# EUROPEAN PATENT SPECIFICATION

(11) **EP 2 294 215 B1**
(45) Date of publication and mention of the grant of the patent: **16.01.2013**
(21) Application number: 09747388.8
(22) Date of filing: 12.05.2009
(51) Int. Cl.: C12Q 1/68, G01N 33/53, G01N 33/574

(54) **TESTS TO PREDICT RESPONSIVENESS OF CANCER PATIENTS TO CHEMOTHERAPY TREATMENT OPTIONS**
TESTS ZUR VORHERSAGE DER ANSPRECHRATE VON KREBSPATIENTEN AUF CHEMOTHERAPEUTISCHE BEHANDLUNGSOPTIONEN
TESTS POUR PRÉDIRE UNE SENSIBILITÉ DE PATIENTS ATTEINTS DE CANCER À DES OPTIONS DE TRAITEMENT DE CHIMIOTHÉRAPIE

(30) Priority: 12.05.2008 US 52573 P; 29.05.2008 US 57182 P
(43) Date of publication of application: 16.03.2011
(60) Divisional application: 12195318.6
(73) Proprietor: Genomic Health, Inc., Redwood City, CA 94063 (US); Aventis Inc., Greenville, DE 08807 (US)
(72) Inventor: SHAK, Steve, Redwood City, CA 94063 (US); BAKER, Joffre, B., Montara, CA 94037 (US); YOSHIZAWA, Carl, Redwood City, CA 94063 (US); SPARANO, Joseph, Pleasantville, NY 10570 (US); GRAY, Robert, Boston, MA 02115 (US)
(74) Representative: Ellis, Katherine Elizabeth Sleigh
(86) International application number: PCT/US2009/043667
(87) International publication number: WO 2009/140304

(56) References cited:
- WO-A1-2006/052862
- WO-A2-2005/100606
- WO-A2-2007/085497
- US-A1- 2006 275 810
- US-A1- 2007 105 133
- US-A1- 2007 134 669
- US-A1- 2008 085 519
- IVANOV OLGA ET AL: "alpha B-crystallin is a novel predictor of resistance to neoadjuvant chemotherapy in breast cancer", BREAST CANCER RESEARCH AND TREATMENT, SPRINGER, NEW YORK, NY, vol. 111, no. 3, 30 October 2007 (2007-10-30), pages 411-417, XP002516398, ISSN: 0167-6806, DOI: DOI:10.1007/S10549-007-9796-0 [retrieved on 2007-10-30]
- TANG SHOU-CHING: "Predictive markers of tubulin-targeting agents in breast cancer", CLINICAL BREAST CANCER MAR 2008 LNKD- PUBMED:18637403,, vol. 8 Suppl 2, 1 March 2008 (2008-03-01), pages S79-S84, XP002636994,
- ABCC1 GENE., [Online] 06 September 2009, XP008147895 Retrieved from the Internet: <URL:http://www.genecards.org/cgi-bin/cardd isp.pl?gene=ABCC1 &search=ABCC1> [retrieved on 2009-10-19]

## Description

### TECHNICAL FIELD

The present disclosure provides genes and gene sets, the expression levels of which are useful for predicting response of cancer patients to chemotherapy. The disclosure further concerns tests using such molecular markers, arrays and kits for use in such methods, and reports comprising the results and/or conclusions of such tests.

### INTRODUCTION

For many patients with cancer, treatment may include surgical resection of the tumor, hormonal therapy, and chemotherapy. A range of chemotherapy choices are available. Ideally, the choice for an individual patient takes into account both the risk of cancer recurrence and the likelihood that the patient will respond to the chemotherapy chosen.

One critical issue in treatment of breast cancer is the identification of which patients are likely to respond to a standard chemotherapy (e.g. an anthracycline and a cyclophosphamide) and which patients are less likely to respond to standard chemotherapy and should therefore be considered for more aggressive chemotherapy (e.g., a chemotherapy regimen that includes a taxane). Currently, no satisfactory tests are available for identifying patients more likely to respond to standard chemotherapy as opposed to treatment with a taxane-containing treatment regimen.

### SUMMARY

The present disclosure provides methods and compositions to facilitate prediction of the likelihood of responsiveness of cancer patients to treatment including a taxane and/or a cyclophosphamide.

The present disclosure provides methods of predicting whether a hormone receptor (HR) positive cancer patient will exhibit a beneficial response to chemotherapy, where the method involves measuring an expression level of a gene, or its expression product, in a tumor sample obtained from the patient, wherein the gene is selected from the group consisting of ABCC1, ABCC5, ABCD1, ACTB, ACTR2, AKT1, AKT2, APC, APOC1, APOE, APRT, BAK1, BAX, BBC3, BCL2L11, BCL2L13, BID, BUB1, BUB3, CAPZA1, CCT3, CD14, CDC25B, CDCAB, CHEK2, CHFR, CSNK1D, CST7, CXCR4, DDR1, DICER1, DUSP1, ECGF1, EIF4E2, ERBB4, ESR1, FAS, GADD45B, GATA3, GCLC, GDF15, GNS, HDAC6, HSPA1A, HSPA1B, HSPA9B, IL7, ILK, LAPTM4B, LILRB1, LIMK2, MAD2L1BP, MAP2K3, MAPK3, MAPRE1, MCL1, MRE11A, NEK2, NFKB1, NME6, NTSR2, PLAU, PLD3, PPP2CA, PRDX1, PRKCH, RAD1, RASSF1, RCC1, REG1A, RELA, RHOA, RHOB, RPN2, RXRA, SHC1, SIRT1, SLC1A3, SLC35B1, SRC, STK10, STMN1, TBCC, TBCD, TNFRSF10A, TOP3B, TSPAN4, TUBA3, TUBA6, TUBB, TUBB2C, UFM1, VEGF, VEGFB, VHL, ZW10, and ZWILCH; using the expression level to determine a likelihood of a beneficial response to a treatment including a taxane, wherein expression of DDR1, EIF4E2, TBCC, STK10, ZW10, BBC3, BAX, BAK1, TSPAN4, SLC1A3, SHC1, CHFR, RHOB, TUBA6, BCL2L13, MAPRE1, GADD45B, HSPA1B, FAS, TUBB, HSPA1A, MCL1, CCT3, VEGF, TUBB2C, AKT1, MAD2L1BP, RPN2, RHOA, MAP2K3, BID, APOE, ESR1, ILK, NTSR2, TOP3B, PLD3, DICER1, VHL, GCLC, RAD1, GATA3, CXCR4, NME6, UFM1, BUB3, CD14, MRE11A, CST7, APOC1, GNS, ABCC5, AKT2, APRT, PLAU, RCC1, CAPZA1, RELA, NFKB1, RASSF1, BCL2L11, CSNK1D, SRC, LIMK2, SIRT1, RXRA, ABCD1, MAPK3, DUSP1, ABCC1, PRKCH, PRDX1, TUBA3, VEGFB, LILRB1, LAPTM4B, HSPA9B, ECGF1, GDF15, ACTR2, IL7, HDAC6, CHEK2, REG1A, APC, SLC35B1, ACTB, PPP2CA, TNFRSF10A, TBCD, ERBB4, CDC25B, or STMN1 is positively correlated with increased likelihood of a beneficial response to a treatment including a taxane, and wherein expression of CDCAB, ZWILCH, NEK2, or BUB1 is negatively correlated with an increased likelihood of a beneficial response to a treatment including a taxane; and generating a report including information based on the likelihood of a beneficial response to chemotherapy including a taxane.

The methods can further involves using a gene expression level to determine a likelihood of a beneficial response to a treatment including a cyclophosphamide, wherein expression of ZW10, BAX, GADD45B, FAS, ESR1, NME6, MRE11A, AKT2, RELA, RASSF1, PRKCH, VEGFB, LILRB1, ACTR2, REG1A, or PPP2CA is positively correlated with increased likelihood of a beneficial response to a treatment including a cyclophosphamide, and wherein expression of DDR1, EIF4E2, TBCC, STK10, BBC3, BAK1, TSPAN4, SHC1, CHFR, RHOB, TUBA6, BCL2L13, MAPRE1, HSPA1, TUBB, HSPA1A, MCL1, CCT3, VEGF, TUBB2C, AKT1, MAD2L1BP, RPN2, RHOA, MAP2K3, BID, APOE, ILK, NTSR2, TOP3B, PLD3, DICER1, VHL, GCLC, RAD1, GATA3, CXCR4, UFM1, BUB3, CD14, CST7, APOC1, GNS, ABCC5, APRT, PLAU, RCC1, CAPZA1, NFKB1, BCL2L11, CSNK1D, SRC, LIMK2, SIRT1, RXRA, ABCD1, MAPK3, CDCA8, DUSP1, ABCC1, PRDX1, TUBA3, LAPTM4B, HSPA9B, ECGF1, GDF15, IL7, HDAC6, ZWILCH, CHEK2, APC, SLC35B1, NEK2, ACTB, BUB1, TNFRSF10A, TBCD, ERBB4, CDC25B, or STMN1 is negatively correlated with an increased likelihood of a beneficial response to a treatment including a cyclophosphamide, and wherein the report includes information based on the likelihood of a beneficial response to chemotherapy including a cyclophosphamide.

The chemotherapy can include an anthracycline. The anthracycline can be doxorubicin. Where the chemotherapy is a taxane, the taxane can be docetaxel.

The methods can accomplish measuring of the gene expression level by quantitative PCR. The methods can accomplish measuring of the gene expression level by detection of an intron-based sequence of an RNA transcript of the gene, wherein the expression of which correlates with the expression of a corresponding exon sequence.

The tumor sample can be a formalin-fixed and paraffin-embedded (FPE) or a frozen tumor section.

The methods of the present disclosure includes methods of predicting whether a hormone receptor (HR) positive cancer patient will exhibit a beneficial response to chemotherapy, the methods involve measuring an expression level of a gene, or its expression product, in a tumor sample obtained from the patient, wherein the gene is selected from the group consisting of ABCA9, ABCC1, ABCC10, ABCC3, ABCD1, ACTB, ACTR2, ACTR3, AKT1, AKT2, APC, APEX1, APOC1, APOE, APRT, BAD, BAK1, BAX, BBC3, BCL2, BCL2L1, BCL2L11, BCL2L13, BID, BIRC3, BIRC4, BUB3, CAPZA1, CCT3, CD14, CD247, CD63, CD68, CDC25B, CHEK2, CHFR, CHGA, COL1A1, COL6A3, CRABP1, CSNK1D, CST7, CTSD, CXCR4, CYBA, CYP1B1, DDR1, DIABLO, DICER1, DUSP1, ECGF1, EIF4E2, ELP3, ERBB4, ERCC1, ESR1, FAS, FLAD1, FOS, FOXA1, FUS, FYN, GADD45B, GATA3, GBP1, GBP2, GCLC, GGPS1, GNS, GPX1, HDAC6, HRAS, HSPA1A, HSPA1B, HSPA5, HSPA9B, IGFBP2, IL2RA, IL7, ILK, KDR, KNS2, LAPTM4B, LILRB1, LIMK1, LIMK2, MAD1L1, MAD2L1BP, MAD2L2, MAP2K3, MAP4, MAPK14, MAPK3, MAPRE1, MCL1, MGC52057, MGMT, MMP11, MRE11A, MSH3, NFKB1, NME6, NPC2, NTSR2, PDGFRB, PECAM1, PIK3C2A, PLAU, PLD3, PMS1, PPP2CA, PRDX1, PRKCD, PRKCH, PTEN, PTPN21, RAB6C, RAD1, RASSF1, RB1, RBM17, RCC1, REG1A, RELA, RHOA, RHOB, RHOC, RPN2, RXRA, RXRB, SEC61A1, SGK, SHC1, SIRT1, SLC1A3, SLC35B1, SOD1, SRC, STAT1, STAT3, STK10, STK11, STMN1, TBCC, TBCD, TBCE, TFF1, TNFRSF10A, TNFRSF10B, TOP3B, TP53BP1, TSPAN4, TUBA3, TUBA6, TUBB, TUBB2C, TUBD1, UFM1, VEGF, VEGFB, VEGFC, VHL, XIST, ZW10, and ZWILCH; using the expression level to determine a likelihood of a beneficial response to a treatment including a taxane, wherein expression of DDR1, ZW10, RELA, BAX, RHOB, TSPAN4, BBC3, SHC1, CAPZA1, STK10, TBCC, EIF4E2, MCL1, RASSF1, VEGF, SLC1A3, DICER1, ILK, FAS, RAB6C, ESR1, MRE11A, APOE, BAK1, UFM1, AKT2, SIRTI, BCL2L13, ACTR2, LIMK2, HDAC6, RPN2, PLD3, RHOA, MAPK14, ECGF1, MAPRE1, HSPA1B, GATA3, PPP2CA, ABCD1, MAD2L1BP, VHL, GCLC, ACTB, BCL2L11, PRDX1, LILRB1, GNS, CHFR, CD68, LIMK1, GADD45B, VEGFB, APRT, MAP2K3, MGC52057, MAPK3, APC, RAD1, COL6A3, RXRB, CCT3, ABCC3, GPX1, TUBB2C, HSPA1A, AKT1, TUBA6, TOP3B, CSNK1D, SOD1, BUB3, MAP4, NFKB1, SEC61A1, MAD1L1, PRKCH, RXRA, PLAU, CD63, CD14, RHOC, STAT1, NPC2, NME6, PDGFRB, MGMT1, GBP1, ERCC1, RCC1, FUS, TUBA3, CHEK2, APOC1, ABCC10, SRC, TUBB, FLAD1, MAD2L2, LAPTM4B, REG1A, PRKCD, CST7, IGFBP2, FYN, KDR, STMN1, RBM17, TP53BP1, CD247, ABCA9, NTSR2, FOS, TNFRSF10A, MSH3, PTEN, GBP2, STK11, ERBB4, TFF1, ABCC1, IL7, CDC25B, TUBD1, BIRC4, ACTR3, SLC35B1, COL1A1, FOXA1, DUSP1, CXCR4, IL2RA, GGPS1, KNS2, RB1, BCL2L1, XIST, BIRC3, BID, BCL2, STAT3, PECAM1, DIABLO, CYBA, TBCE, CYP1B1, APEX1, TBCD, HRAS, TNFRSF10B, ELP3, PIK3C2A, HSPA5, VEGFC, MMP11, SGK, CTSD, BAD, PTPN21, HSPA9B, or PMS1 is positively correlated with increased likelihood of a beneficial response to a treatment including a taxane, and wherein expression of CHGA, ZWILCH, or CRABP1 is negatively correlated with an increased likelihood of a beneficial response to a treatment including a taxane; and generating a report including information based on the likelihood of a beneficial response to chemotherapy including a taxane.

The methods can further involve using a gene expression level to determine a likelihood of a beneficial response to a treatment including a cyclophosphamide, wherein expression of LILRB1, PRKCH, STAT1, GBP1, CD247, IL7, IL2RA, BIRC3, or CRABP1 is positively correlated with increased likelihood of a beneficial response to a treatment including a cyclophosphamide, and wherein expression of DDR1, ZW10, RELA, BAX, RHOB, TSPAN4, BBC3, SHC1, CAPZA1, STK10, TBCC, EIF4E2, MCL1, RASSF1, VEGF, DICER1, ILK, FAS, RAB6C, ESR1, MRE11A, APOE, BAK1, UFM1, AKT2, SIRT1, BCL2L13, ACTR2, LIMK2, HDAC6, RPN2, PLD3, CHGA, RHOA, MAPK14, ECGF1, MAPRE1, HSPA1B, GATA3, PPP2CA, ABCD1, MAD2L1BP, VHL, GCLC, ACTB, BCL2L11, PRDX1, GNS, CHFR, CD68, LIMK1, GADD45B, VEGFB, APRT, MAP2K3, MGC52057, MAPK3, APC, RAD1, COL6A3, RXRB, CCT3, ABCC3, GPX1, TUBB2C, HSPA1A, AKT1, TUBA6, TOP3B, CSNK1D, SOD1, BUB3, MAP4, NFKB1, SEC61A1, MAD1L1, RXRA, PLAU, CD63, CD14, RHOC, NPC2, NME6, PDGFRB, MGMT1, ERCC1, RCC1, FUS, TUBA3, CHEK2, APOC1, ABCC10, SRC, TUBB, FLAD1, MAD2L2, LAPTM4B, REG1A, PRKCD, CST7, IGFBP2, FYN, KDR, STMN1, ZWILCH, RBM17, TP53BP1, ABCA9, NTSR2, FOS, TNFRSF10A, MSH3, PTEN, GBP2, STK11, ERBB4, TFF1, ABCC1, CDC25B, TUBD1, BIRC4, ACTR3, SLC35B1, COL1A1, FOXA1, DUSP1, CXCR4, GGPS1, KNS2, RB1, BCL2L1, XIST, BID, BCL2, STAT3, PECAM1, DIABLO, CYBA, TBCE, CYP1B1, APEX1, TBCD, HRAS, TNFRSF10B, ELP3, PIK3C2A, HSPA5, VEGFC, MMP11, SGK, CTSD, BAD, PTPN21, HSPA9B, or PMS1 is negatively correlated with an increased likelihood of a beneficial response to a treatment including a cyclophosphamide, and wherein the report includes information based on the likelihood of a beneficial response to chemotherapy including a cyclophosphamide.

The chemotherapy can include an anthracycline. The anthracycline can be doxorubicin. Where the chemotherapy is a taxane, the taxane can be docetaxel.

The methods can accomplish measuring of the gene expression level by quantitative PCR. The methods can accomplish measuring of the gene expression level by detection of an intron-based sequence of an RNA transcript of the gene, wherein the expression of which correlates with the expression of a corresponding exon sequence.

The tumor sample can be a formalin-fixed and paraffin-embedded (FPE) or a frozen tumor section.

The methods of the present disclosure include methods of predicting whether a hormone receptor (HR) negative cancer patient will exhibit a beneficial response to chemotherapy, where the methods involve measuring an expression level of a gene, or its expression product, in a tumor sample obtained from the patient, wherein the gene is selected from the group consisting of CD247, TYMS, IGF1R, ACTG2, CCND1, CAPZA1, CHEK2, STMN 1, and ZWILCH; using the expression level to determine a likelihood of a beneficial response to a treatment including a taxane, wherein expression of CD247, TYMS, IGF1R, ACTG2, CAPZA1, CHEK2, STMN1, or ZWILCH is positively correlated with increased likelihood of a beneficial response to a treatment including a taxane, and wherein expression of CCND1 is negatively correlated with an increased likelihood of a beneficial response to a treatment including a taxane; and generating a report including information based on the likelihood of a beneficial response to chemotherapy including a taxane.

The methods can further include a gene expression level to determine a likelihood of a beneficial response to a treatment including a cyclophosphamide, wherein expression of CD247, CCND1, or CAPZA1 is positively correlated with increased likelihood of a beneficial response to a treatment including a cyclophosphamide, and wherein expression of TYMS, IGF1R, ACTG2, CHEK2, STMN1, or ZWILCH is negatively correlated with an increased likelihood of a beneficial response to a treatment including a cyclophosphamide, and wherein the report includes information based on the likelihood of a beneficial response to chemotherapy including a cyclophosphamide.

The chemotherapy can include an anthracycline. The anthracycline can be doxorubicin. Where the chemotherapy is a taxane, the taxane can be docetaxel.

The methods can accomplish measuring of the gene expression level by quantitative PCR. The methods can accomplish measuring of the gene expression level by detection of an intron-based sequence of an RNA transcript of the gene, wherein the expression of which correlates with the expression of a corresponding exon sequence.

The tumor sample can be a formalin-fixed and paraffin-embedded (FPE) or a frozen tumor section.

The methods of the present disclosure include methods of predicting whether a cancer patient will exhibit a beneficial response to chemotherapy, where the methods involve measuring an expression level of a gene, or its expression product, in a tumor sample obtained from the patient, wherein the gene is selected from the group consisting of ABCC1, ABCC10, ABCC5, ACTB, ACTR2, APEX1, APOC1, APRT, BAK1, BAX, BBC3, BCL2L13, BID, BUB1, BUB3, CAPZA1, CCT3, CD247, CD68, CDCA8, CENPA, CENPF, CHEK2, CHFR, CST7, CXCR4, DDR1, DICE1, EIF4E2, GADD45B, GBP1, HDAC6, HSPA1A, HSPA1B, HSPA1L, IL2RA, IL7, ILK, KALPHA1, KIF22, LILRB1, LIMK2, MAD2L1, MAPRE1, MCL1, MRE11A, NEK2, NTSR2, PHB, PLD3, RAD1, RALBP1, RHOA, RPN2, SHC1, SLC1A3, SRC, STAT1, STK10, STMN1, TBCC, TOP3B, TPX2, TSPAN4, TUBA3, TUBA6, TUBB, TUBB2C, TUBB3, TYMS, VEGF, VHL, WNT5A, ZW10, ZWILCH, and ZWINT; using the expression level to determine a likelihood of a beneficial response to a treatment including a taxane, wherein expression of SLC1A3, TBCC, EIF4E2, TUBB, TSPAN4, VHL, BAX, CD247, CAPZA1, STMN1, ABCC1, ZW10, HSPA1B, MAPRE1, PLD3, APRT, BAK1, CST7, SHC1, ZWILCH, SRC, GADD45B, LIMK2, CHEK2, RAD1, MRE11A, DDR1, STK10, LILRB1, BBC3, BUB3, TOP3B, RPN2, ILK, GBP1, TUBB3, NTSR2, BID, BCL2L13, ABCC5, HDAC6, CD68, DICER1, RHOA, CCT3, ACTR2, WNT5A, HSPA1L, APOC1, APEX1, KALPHA1, ABCC10, PHB, TUBB2C, RALBP1, MCL11, HSPA1A, IL2RA, TUBA3, ACTB, KIF22, CXCR4, STAT1, IL7, or CHFR is positively correlated with increased likelihood of a beneficial response to a treatment including a taxane, and wherein expression of CENPA, CDCAB, TPX2, NEK2, TYMS, ZWINT, VEGF, BUB 1, MAD2L1, or CENPF is negatively correlated with an increased likelihood of a beneficial response to a treatment including a taxane; and generating a report including information based on the likelihood of a beneficial response to chemotherapy including a taxane.

The methods can further include using a gene expression level to determine a likelihood of a beneficial response to a treatment including a cyclophosphamide, wherein expression of SLC1A3, TSPAN4, BAX, CD247, CAPZA1, ZW10, CST7, SHC1, GADD45B, MRE11A, STK10, LILRB1, BBC3, BUB3, ILK, GBP1, BCL2L13, CD68, DICER1, RHOA, ACTR2, WNT5A, HSPA1L, APEX1, MCL1, IL2RA, ACTB, STAT1, IL7, or CHFR is positively correlated with increased likelihood of a beneficial response to a treatment including a cyclophosphamide, and wherein expression of TBCC, EIF4E2, TUBB, VHL, STMN1, ABCC1, HSPA1B, MAPRE1, APRT, BAK1, TUBA6, ZWILCH, SRC, LIMK2, CENPA, CHEK2, RAD1, DDR1, CDCAB, TOP3B, RPN2, TUBB3, NTSR2, BID, TPX2, ABCC5, HDAC6, NEK2, TYMS, CCT3, ZWINT, KALPHA1, ABCC10, PHB, TUBB2C, RALBP1, VEGF, HSPA1A, BUB1, MAD2L1, CENPF, TUBA3, KIF22, or CXCR4 is negatively correlated with an increased likelihood of a beneficial response to a treatment including a cyclophosphamide, and wherein the report includes information based on the likelihood of a beneficial response to chemotherapy including a cyclophosphamide.

The chemotherapy can include an anthracycline. The anthracycline can be doxorubicin. Where the chemotherapy is a taxane, the taxane can be docetaxel.

The methods can accomplish measuring of the gene expression level by quantitative PCR. The methods can accomplish measuring of the gene expression level by detection of an intron-based sequence of an RNA transcript of the gene, wherein the expression of which correlates with the expression of a corresponding exon sequence.

The tumor sample can be a formalin-fixed and paraffin-embedded (FPE) or a frozen tumor section.

The present disclosure also provides kits containing one or more (1) extraction buffer/reagents and protocol; (2) reverse transcription buffer/reagents and protocol; and (3) qPCR buffer/reagents and protocol, suitable for performing the method disclosed herein. Also contemplated are arrays having bound polynucleotides that specifically hybridize to one or more genes used in the methods disclosed herein, as well as arrays having bound one or more antibodies that specifically bind a polypeptides expressed by a gene used in the methods disclosed herein.

Various aspects and embodiments will be apparent from the following discussion, including the Examples. Such additional embodiments, without limitation, include any and all of the ESR1 gene combinations discussed and/or specifically listed in Example 2.

### BRIEF DESCRIPTION OF THE DRAWINGS

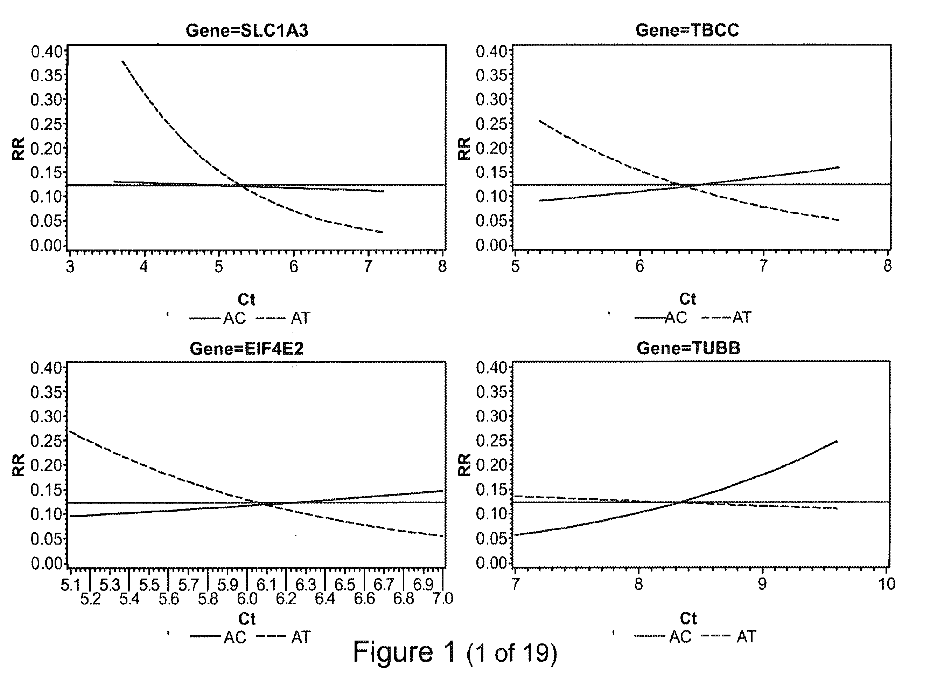

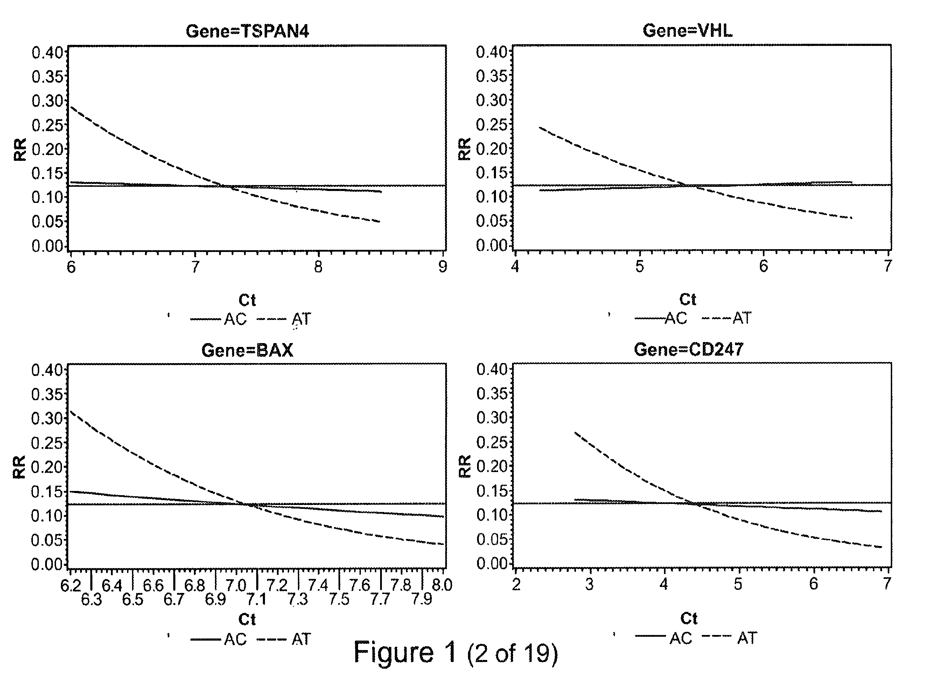

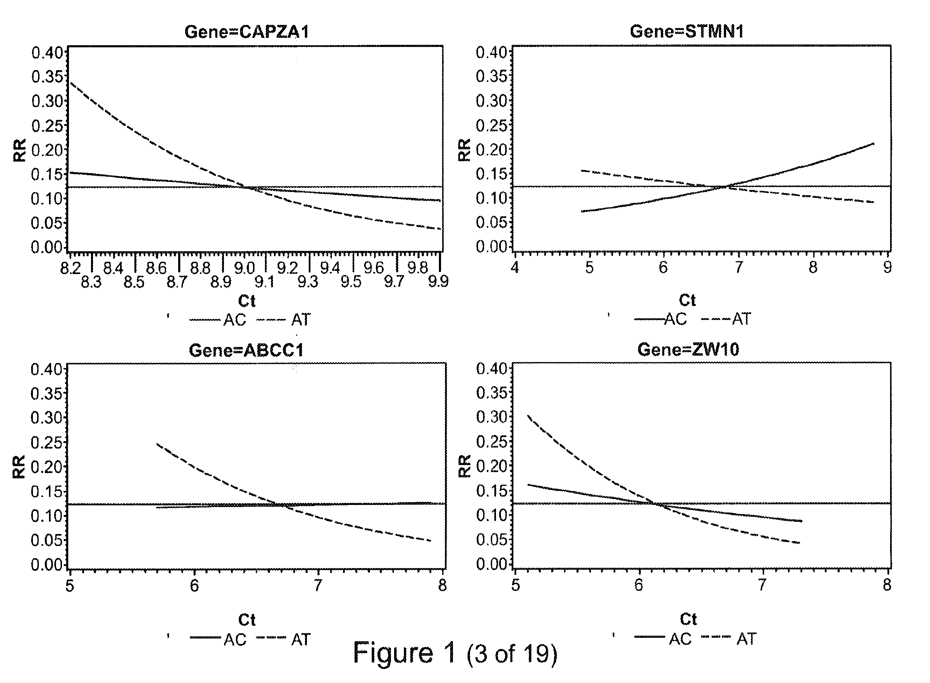

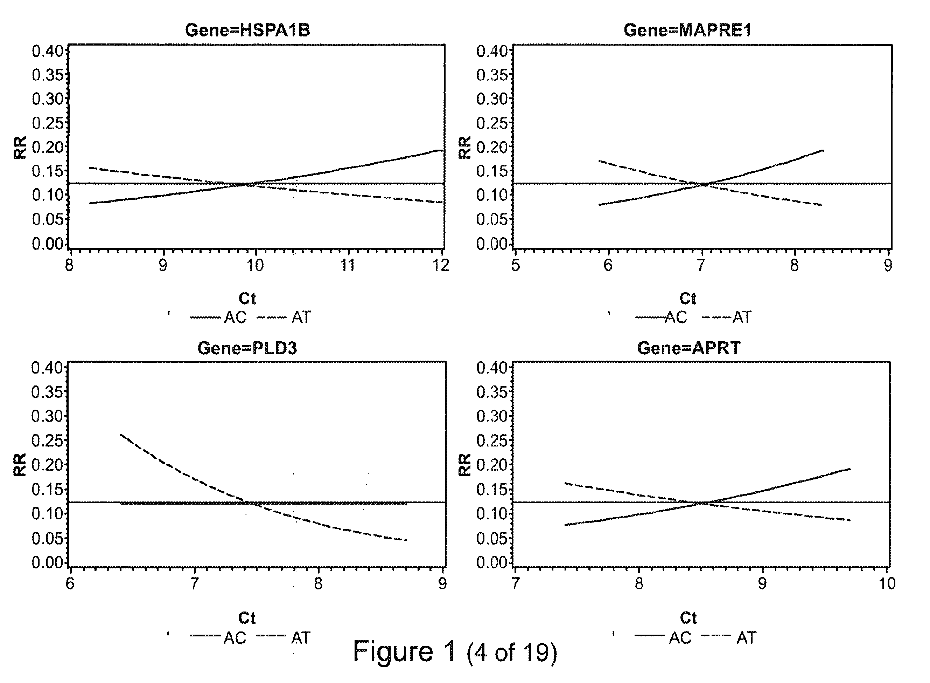

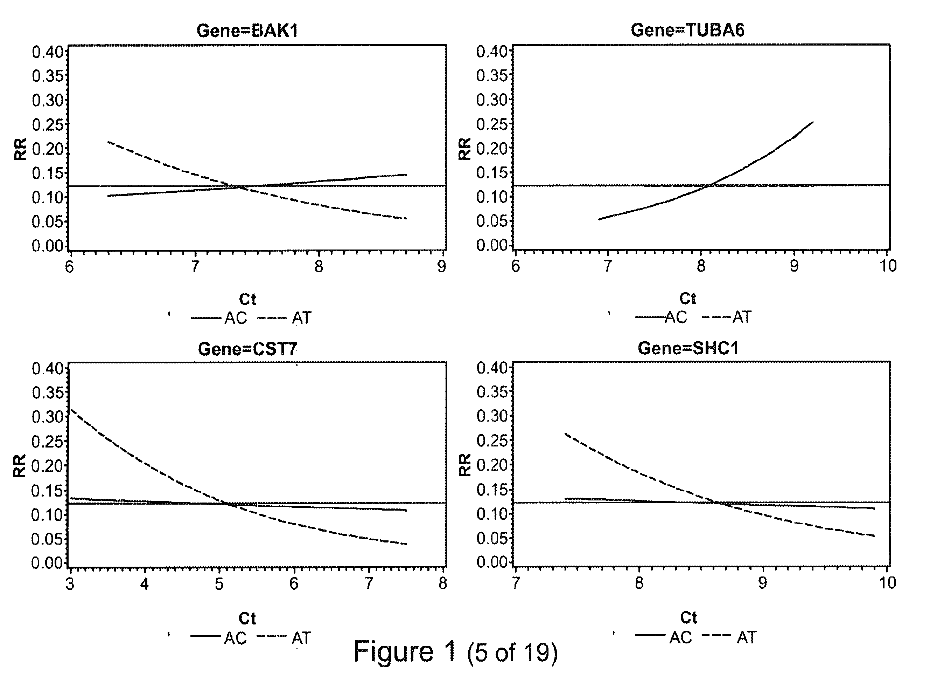

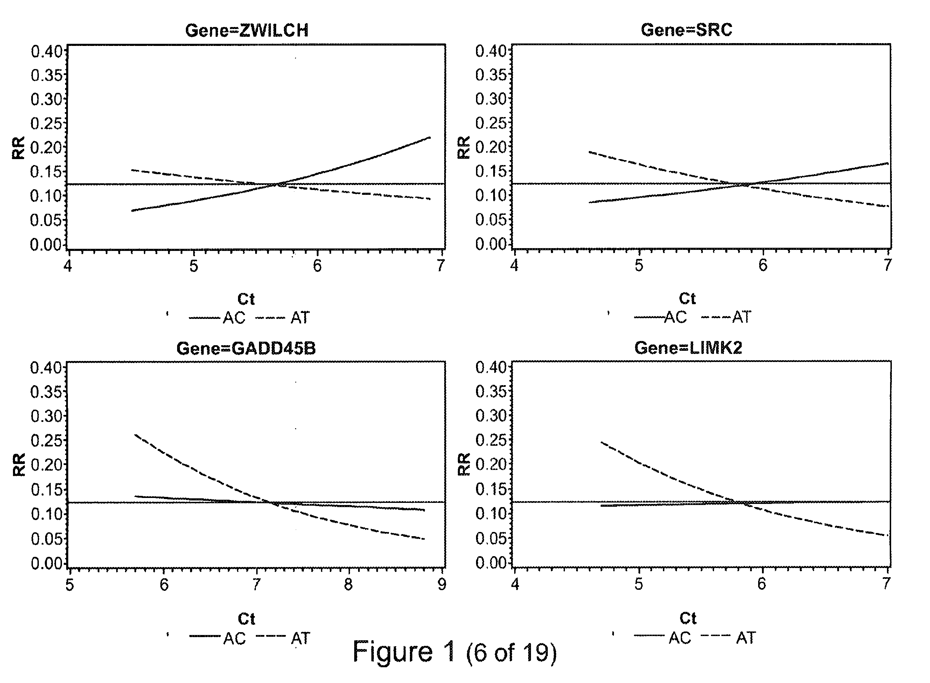

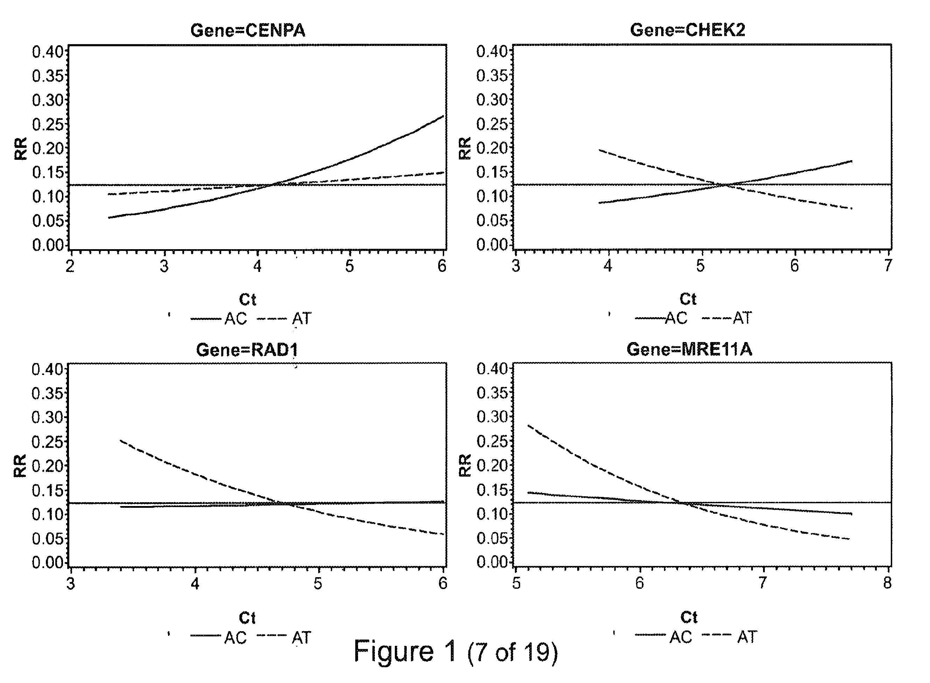

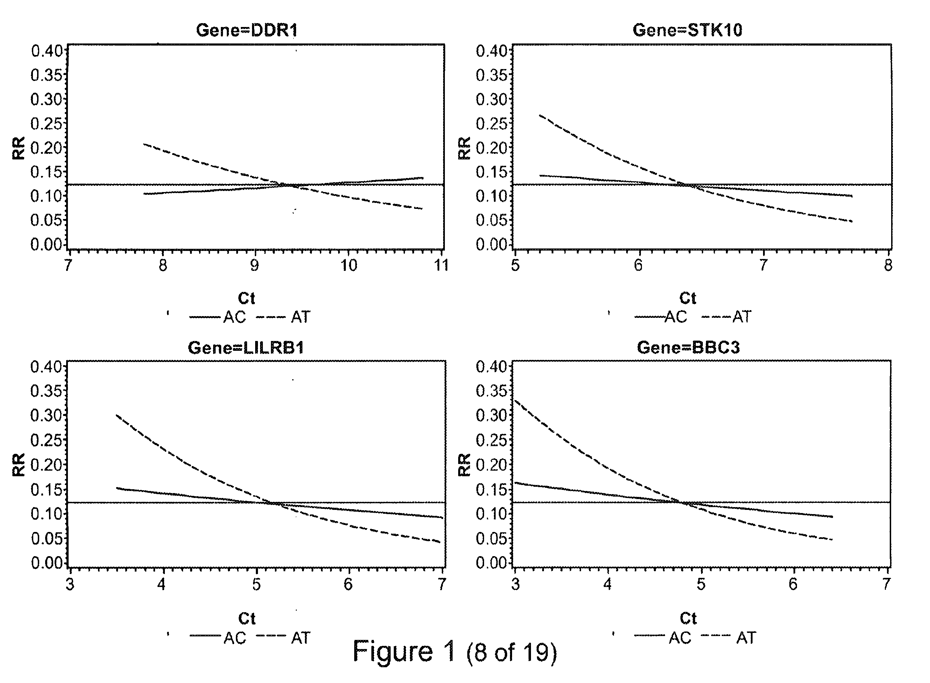

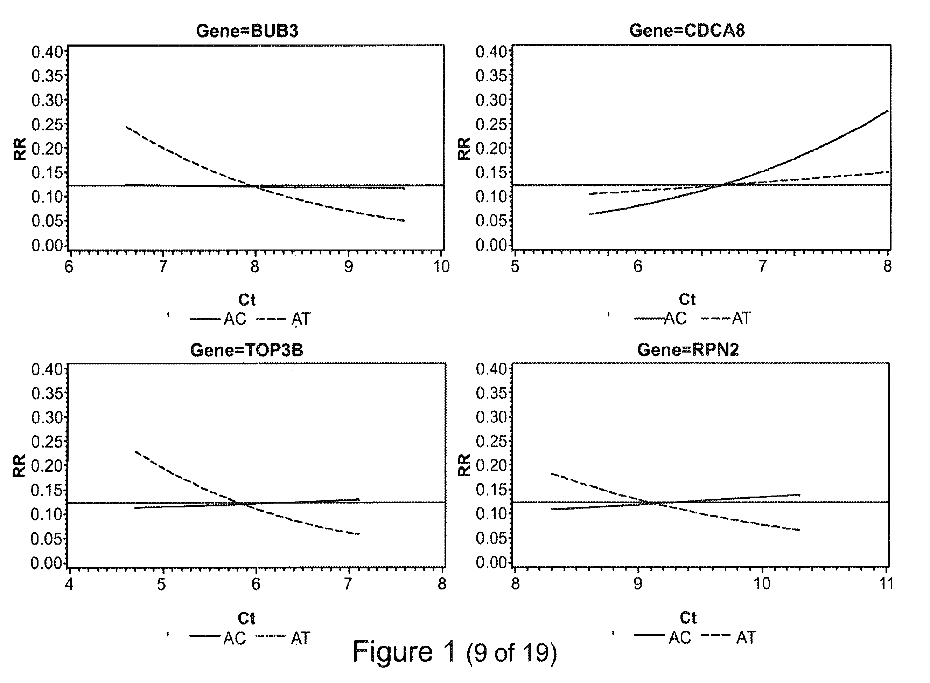

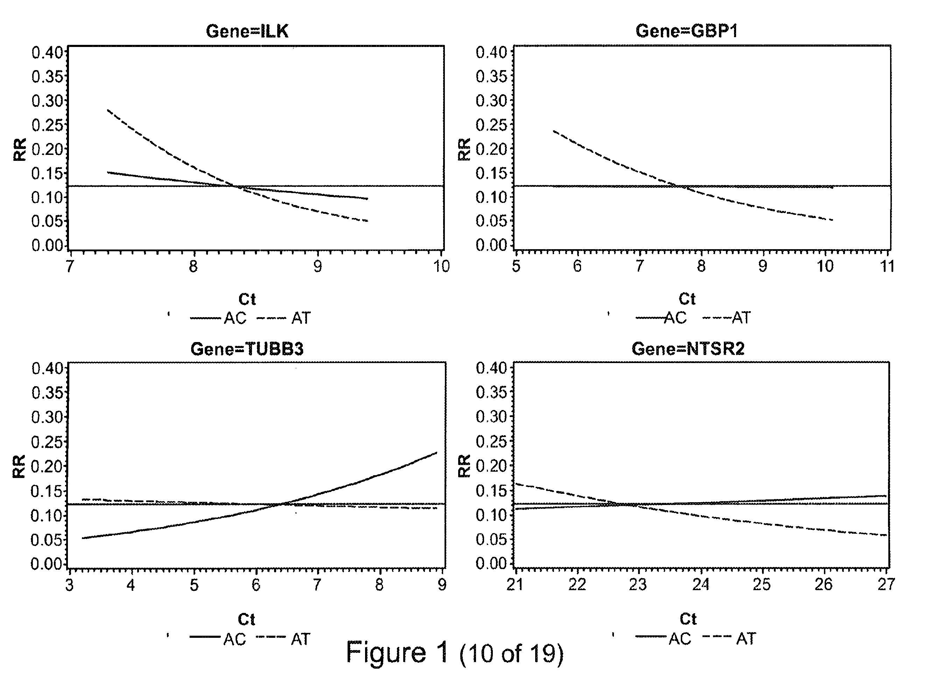

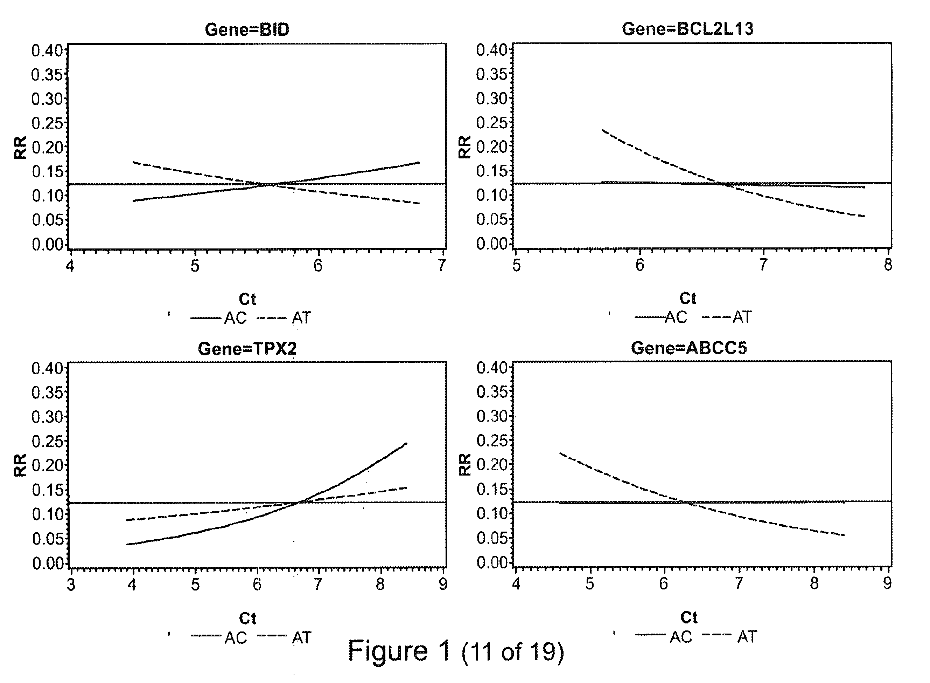

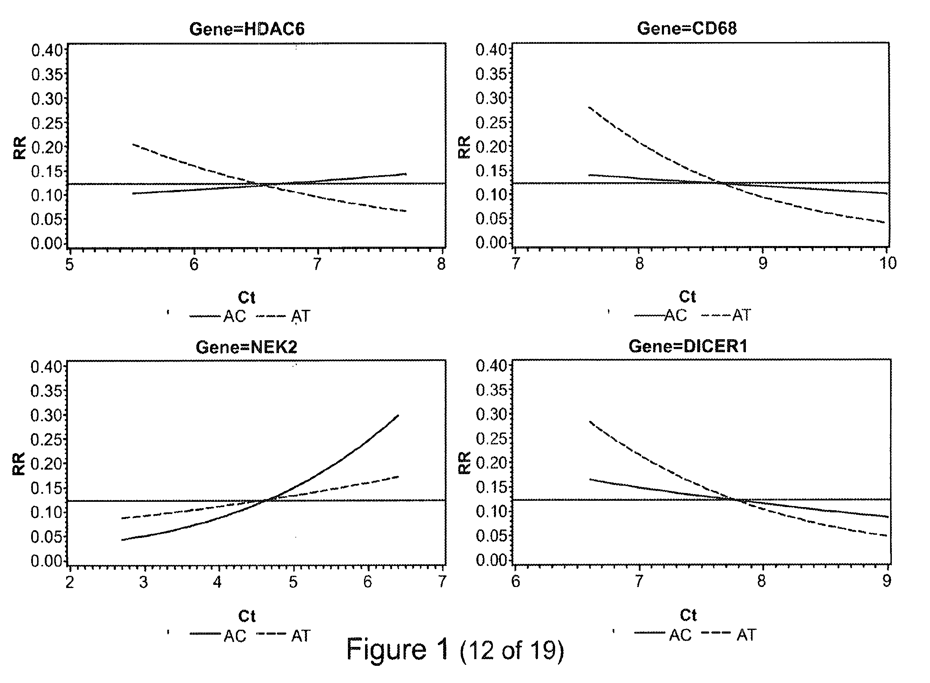

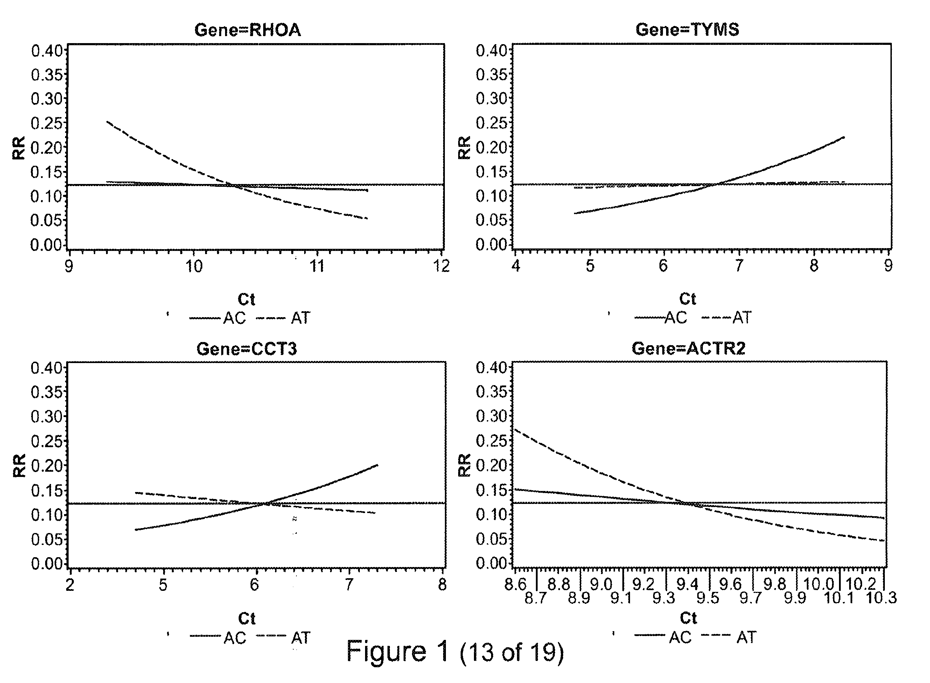

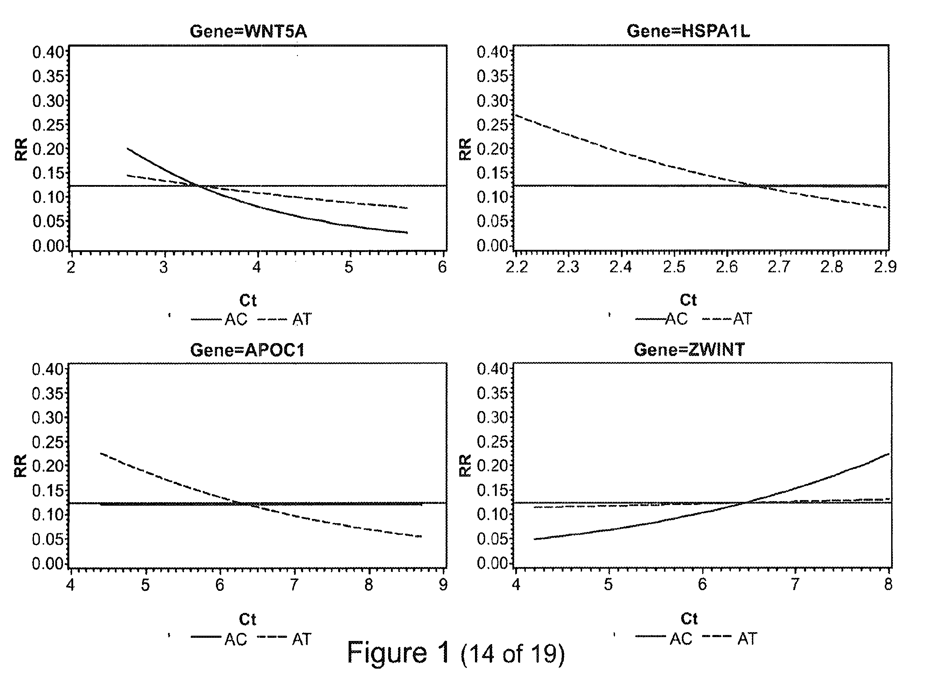

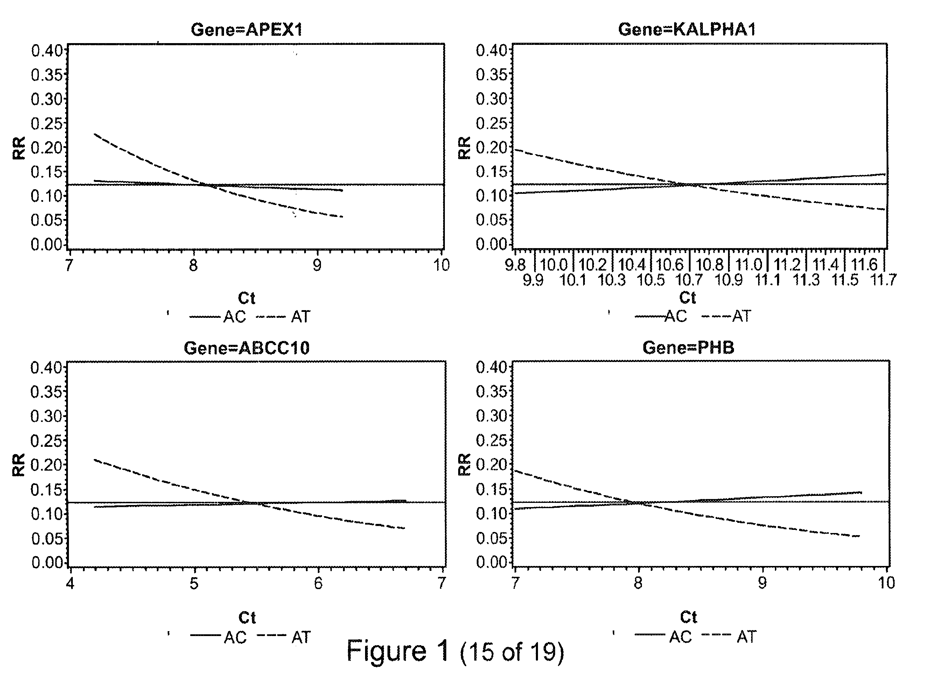

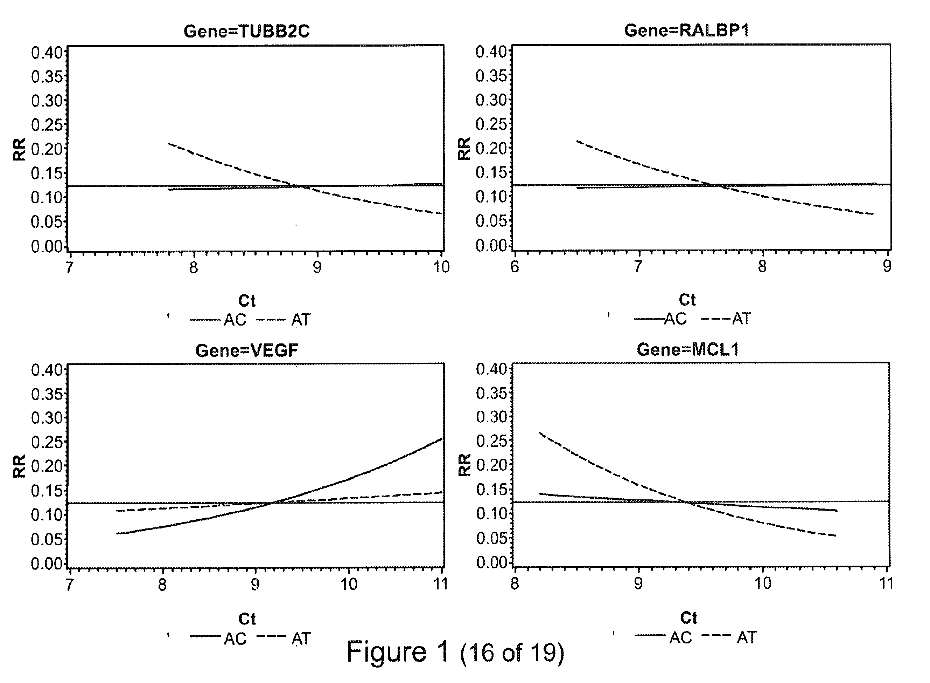

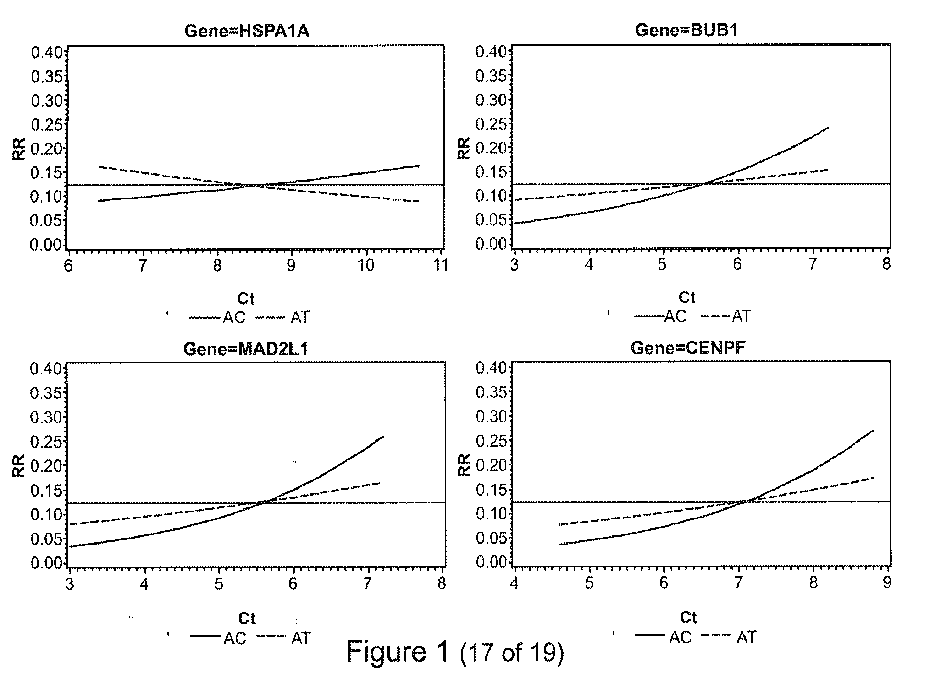

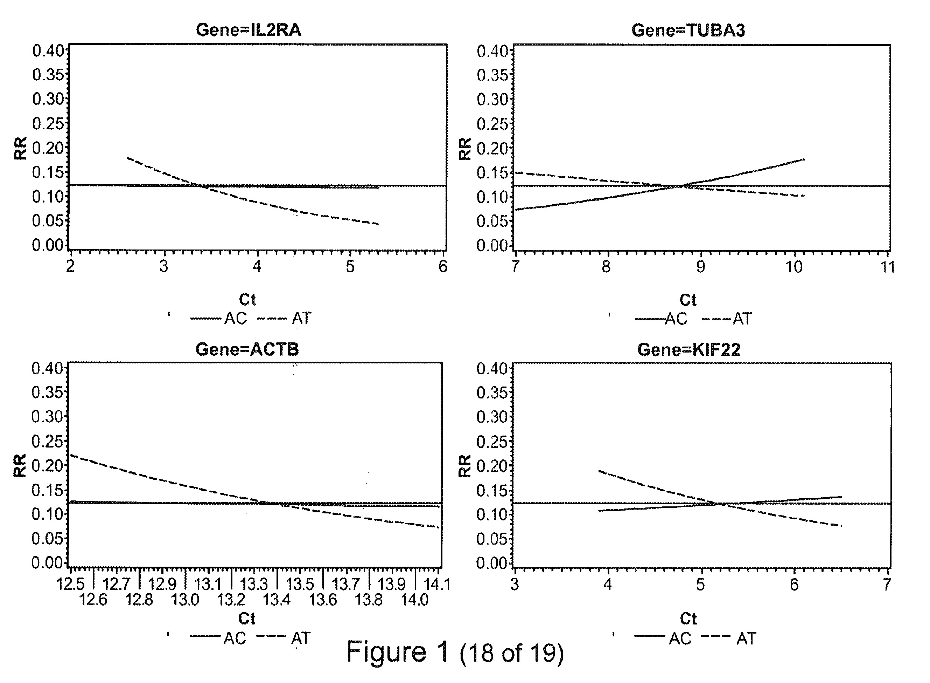

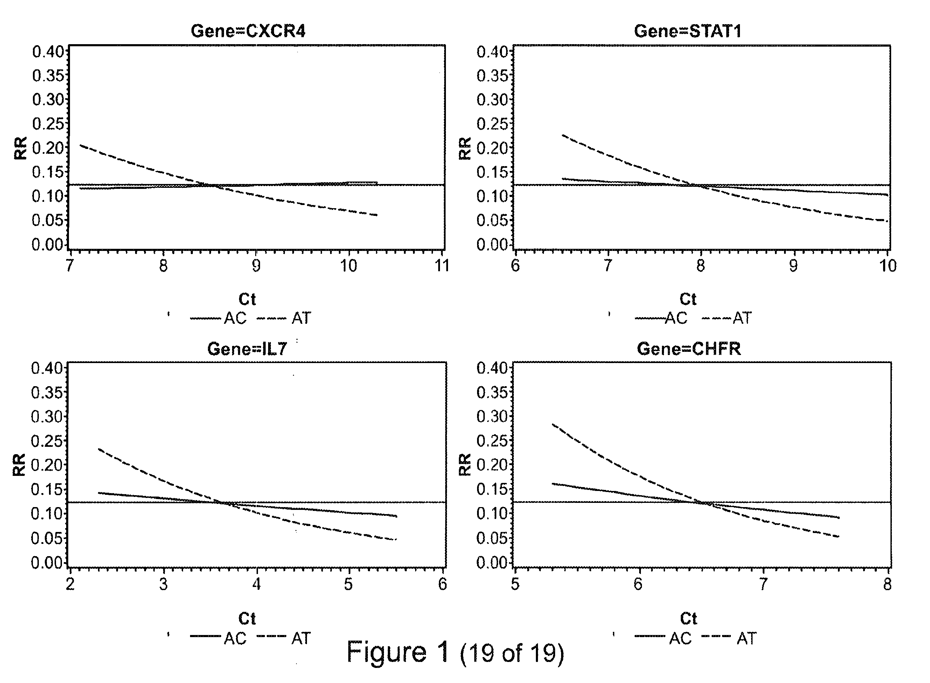

Figure 1 is a set of graphs showing the relationship between normalized expression (represented by "Cₜ") of the indicated gene (gene name provided at top of each graph) and 5-year recurrence rate (RR) of breast cancer in a treatment group receiving anthracycline and a cyclophosphamide (AC prediction curve; smooth line) and the relationship between expression of the indicated gene and RR in a treatment group receiving anthracycline and a taxane (AT prediction curve; hatched line). A horizontal dashed line in each graph represents the overall (i.e., not gene expression-specific) 5-year RR in the study population who were randomized to treatment with either AC or AT. In Figure 1 the patients were included without regard to hormone receptor expression status of the tumor.

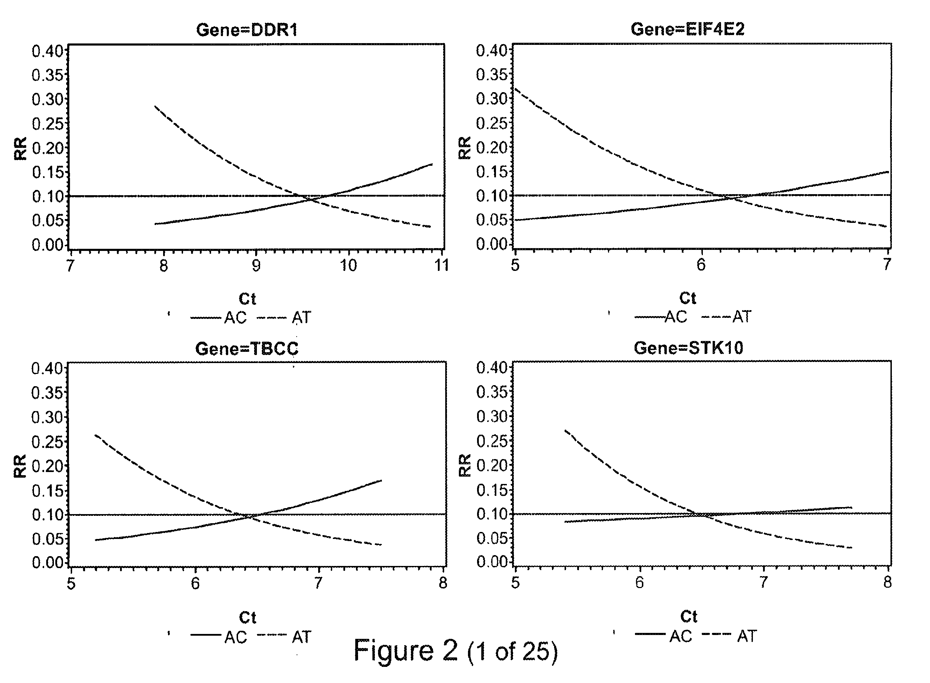

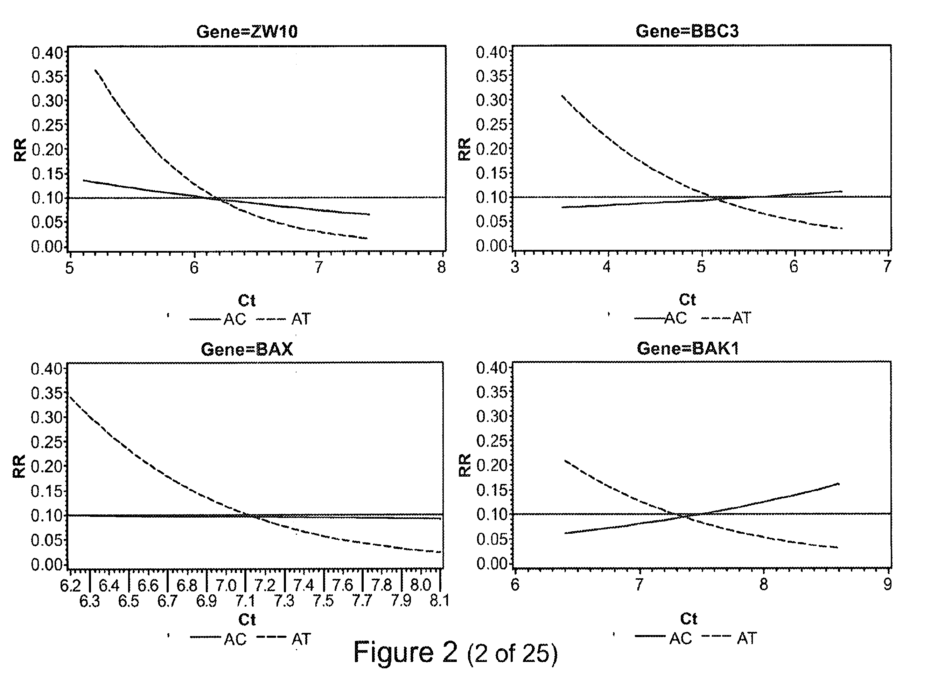

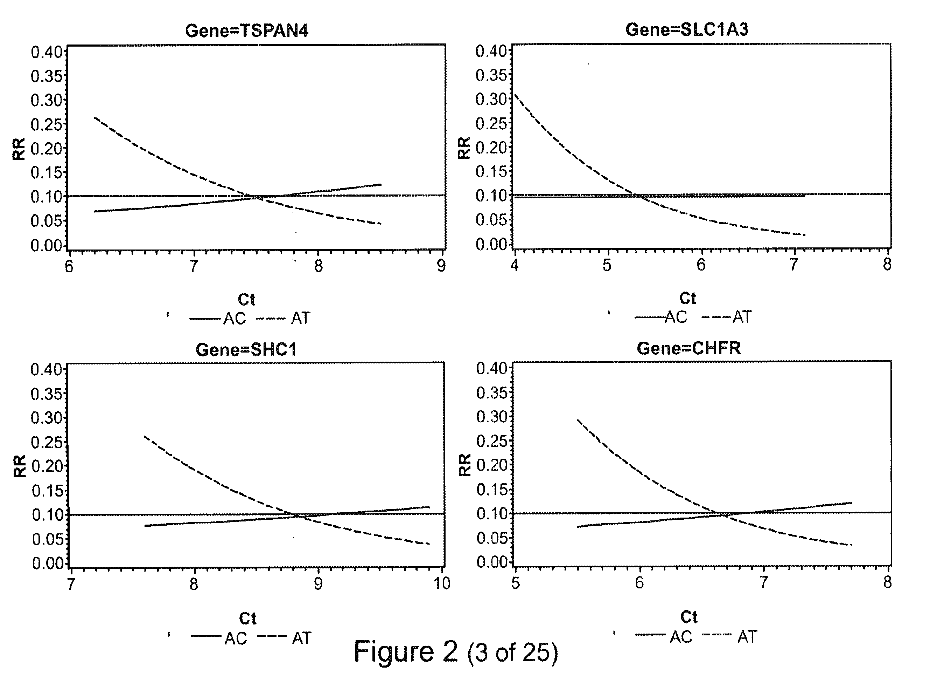

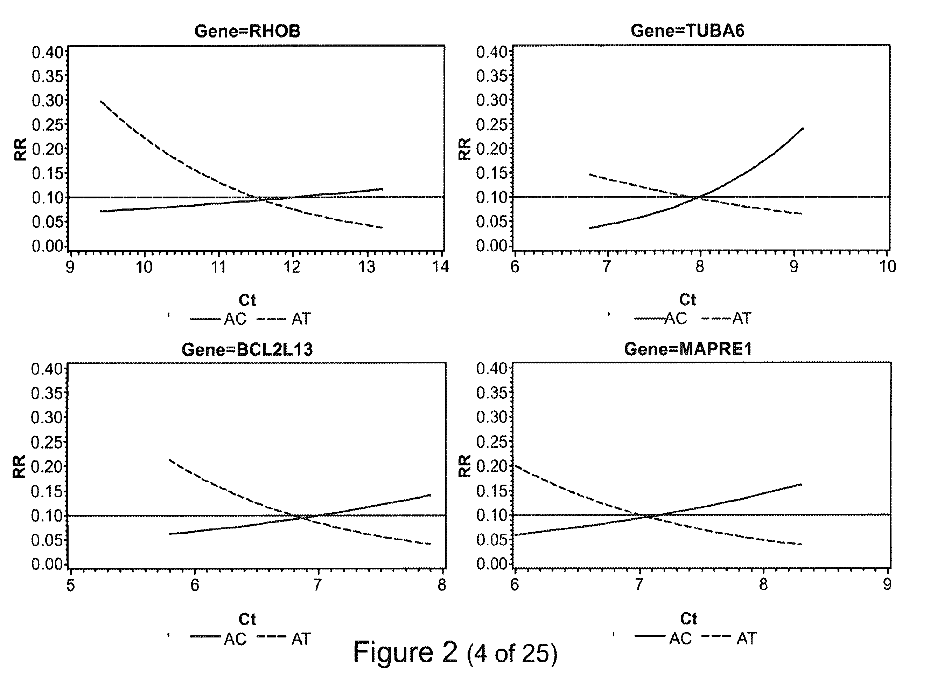

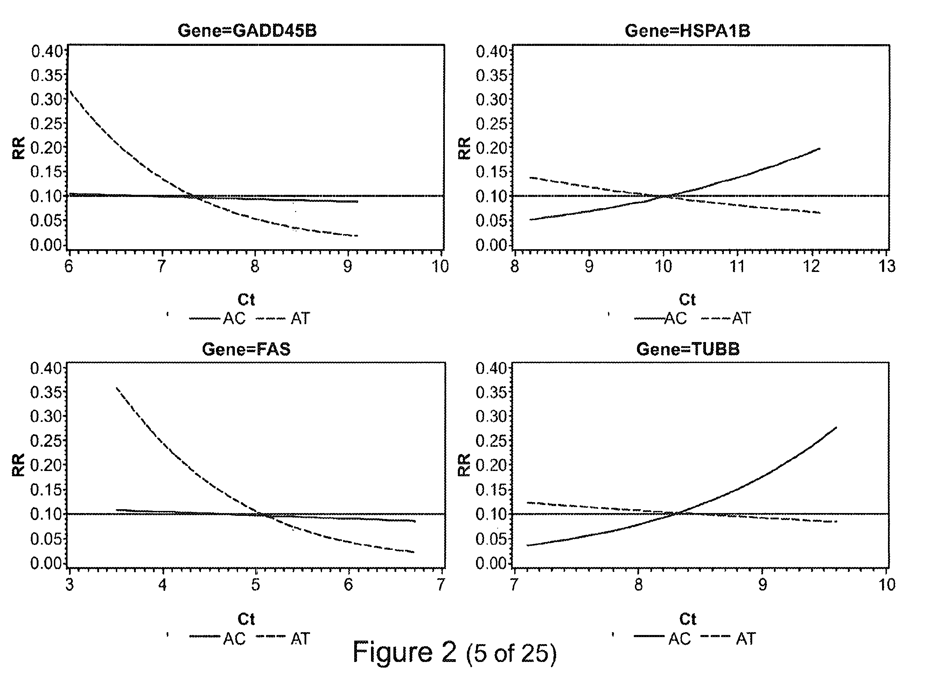

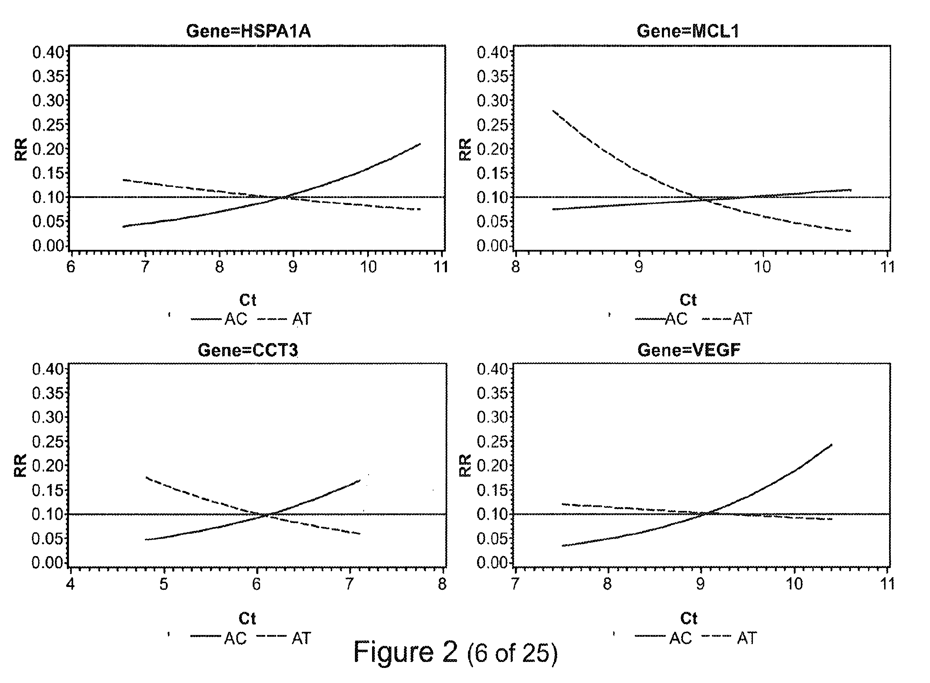

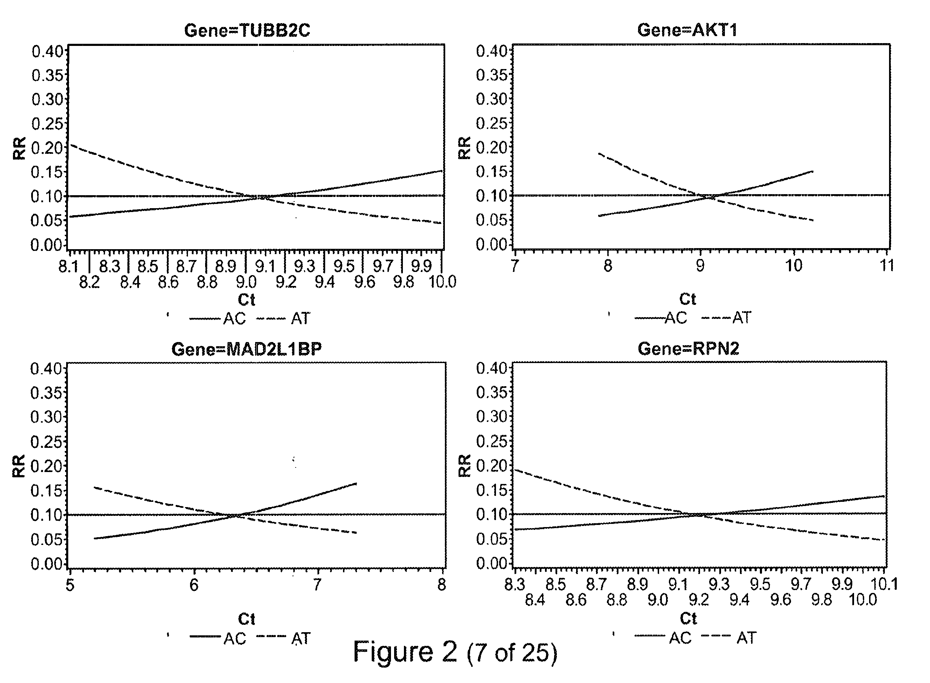

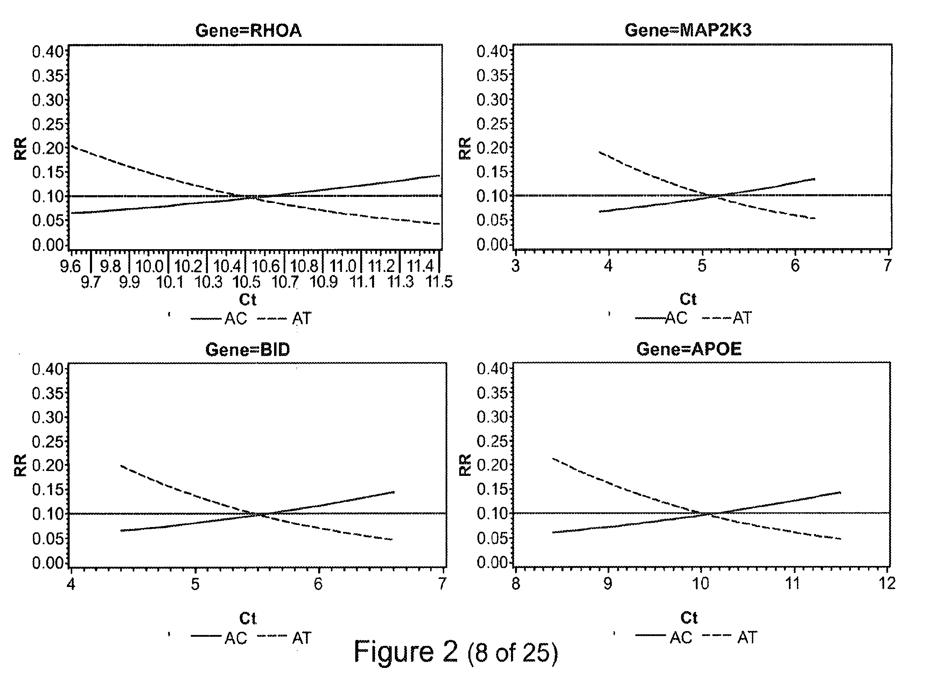

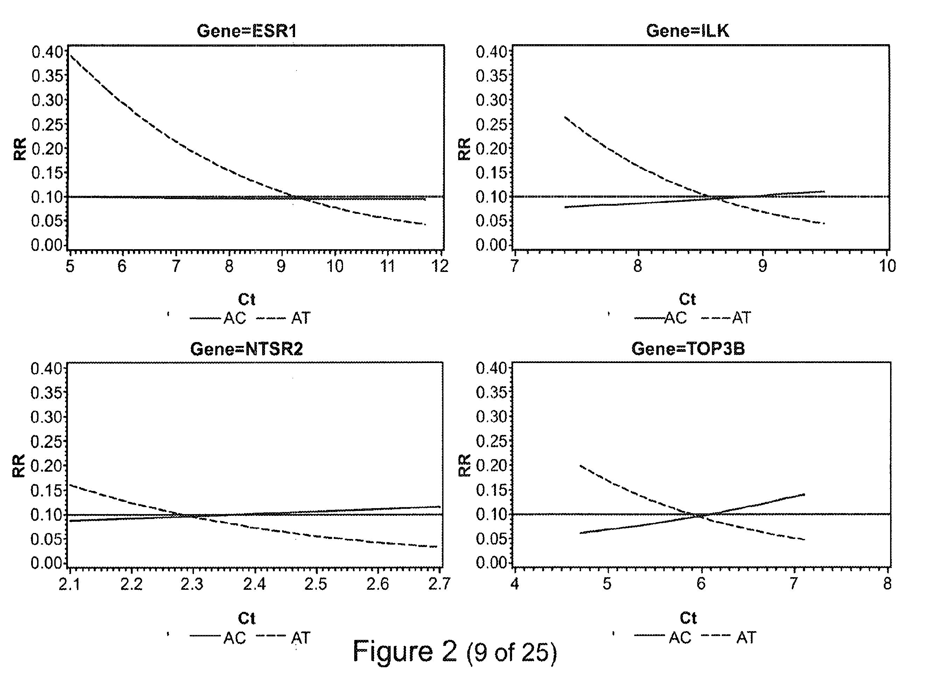

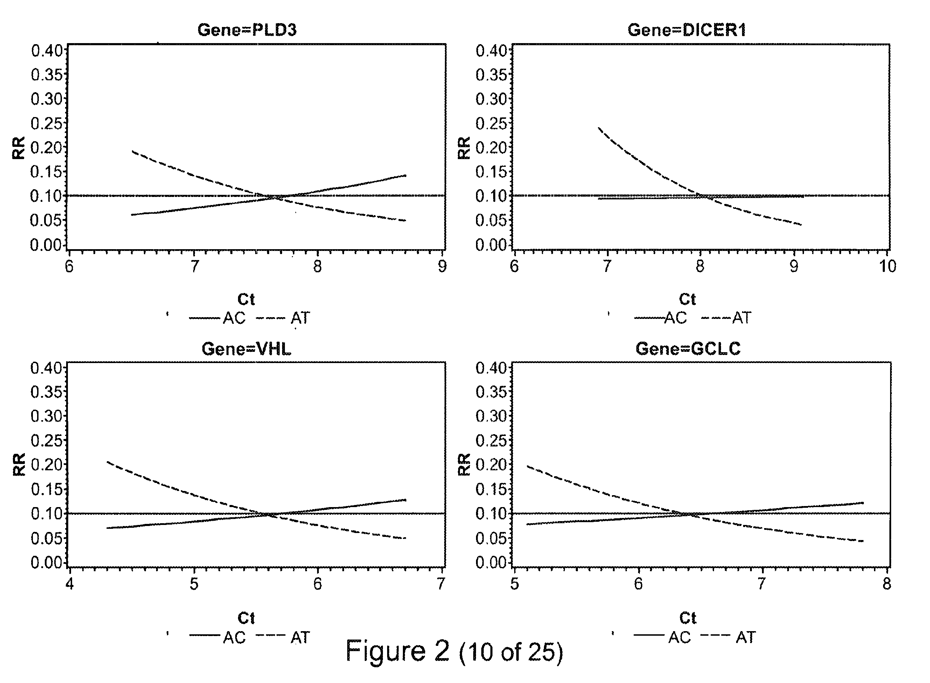

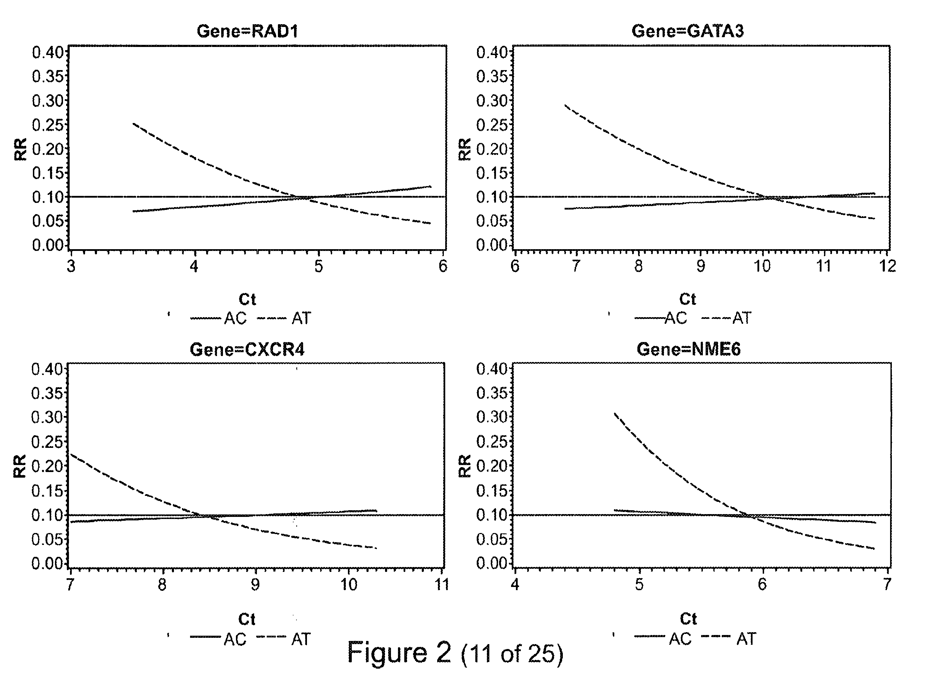

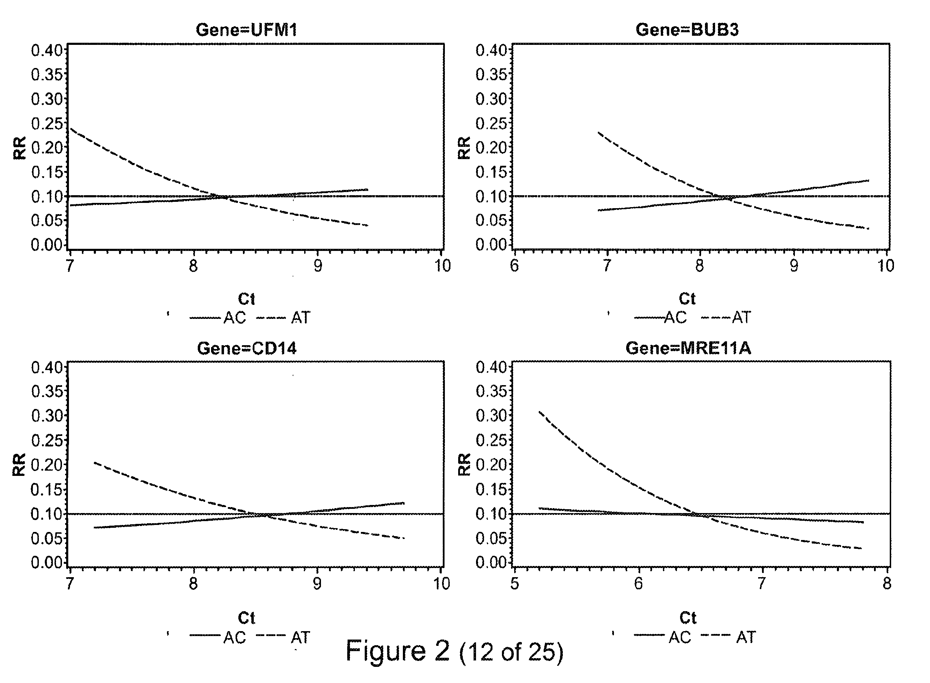

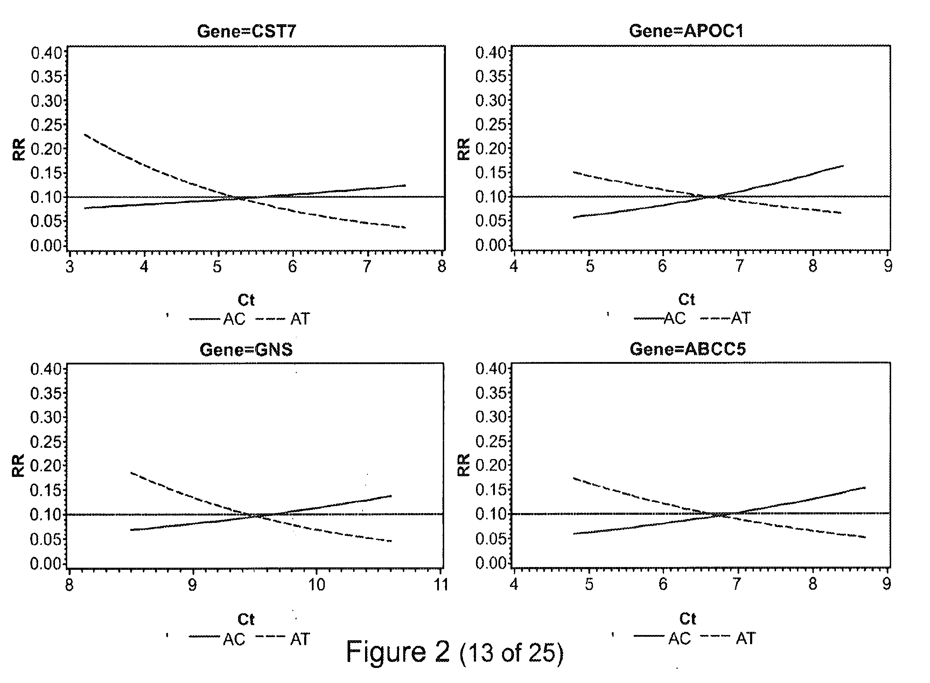

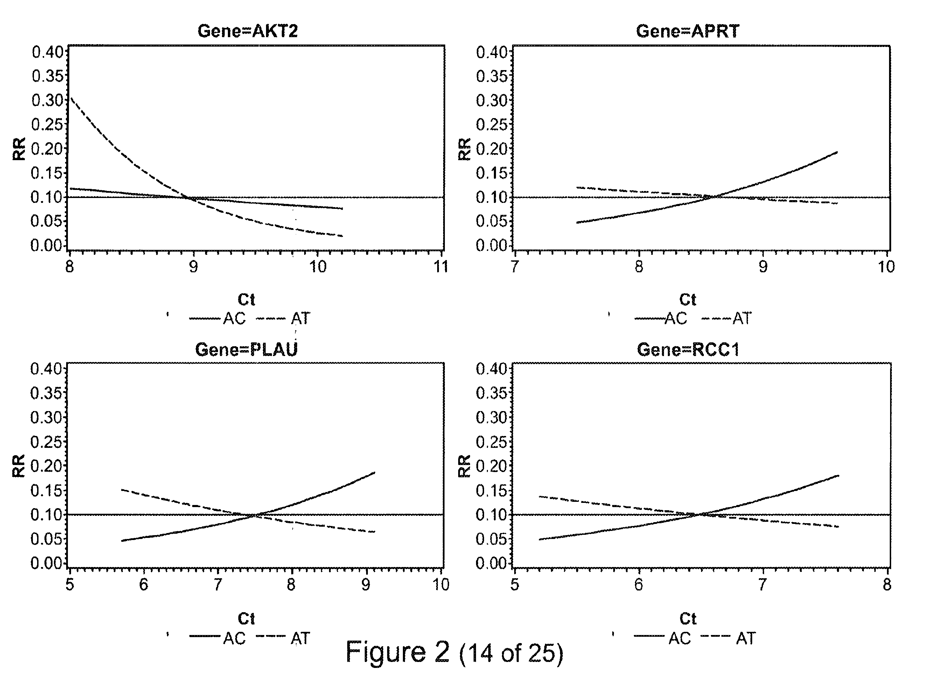

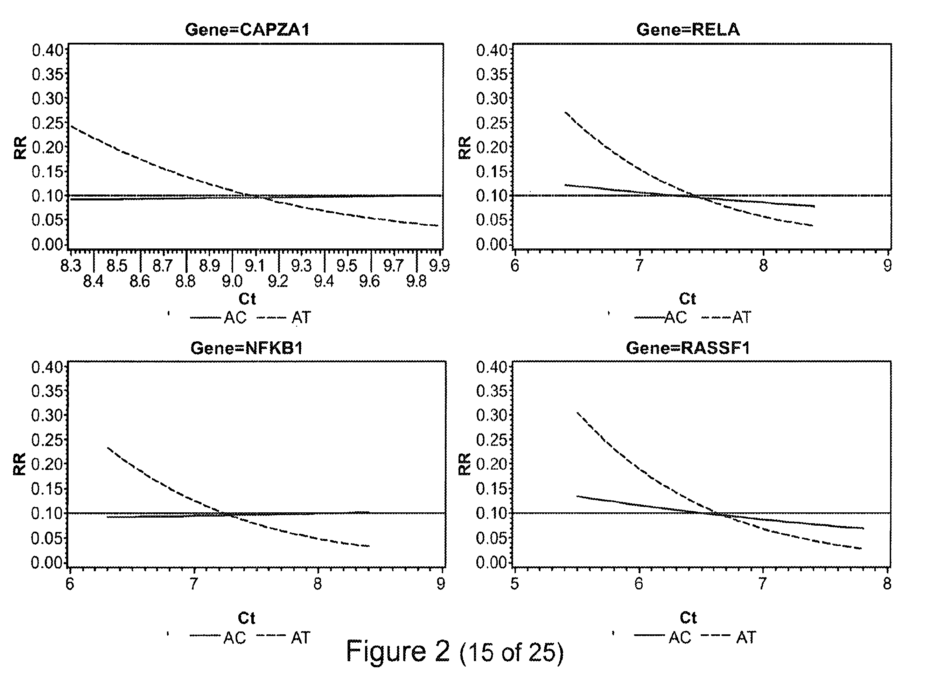

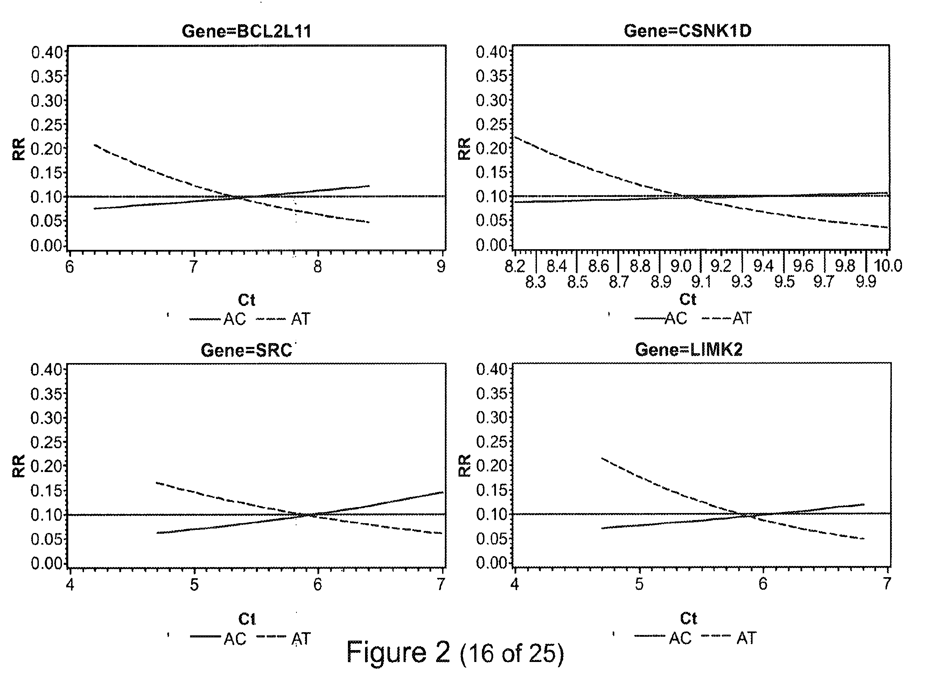

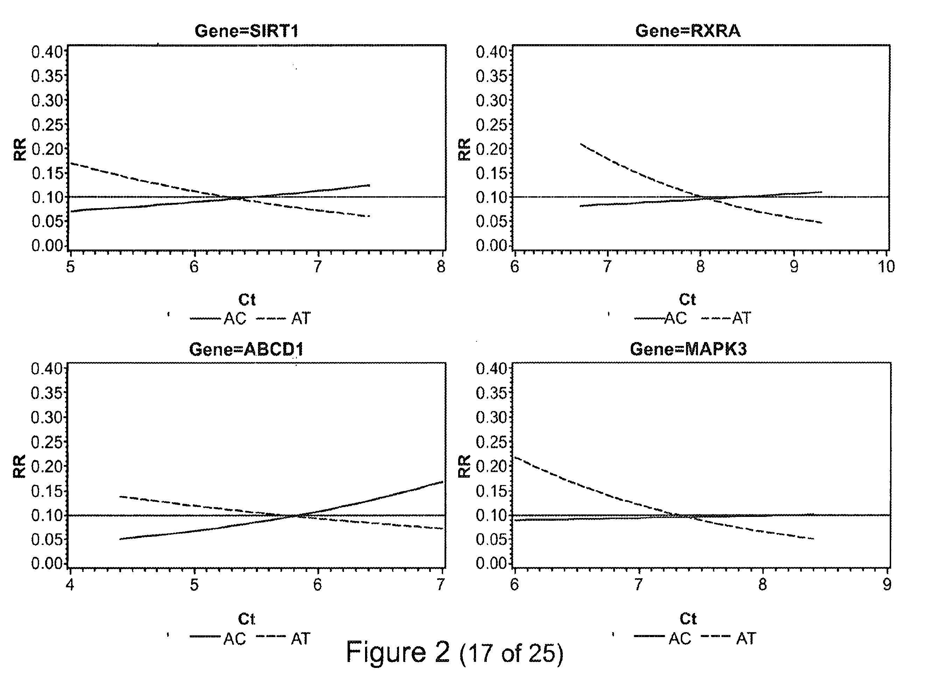

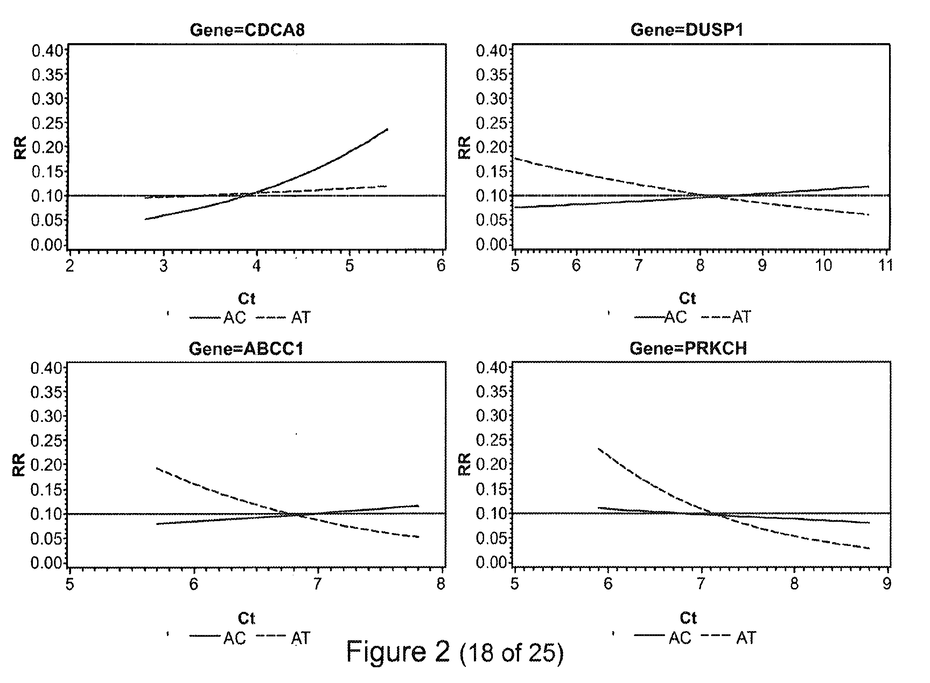

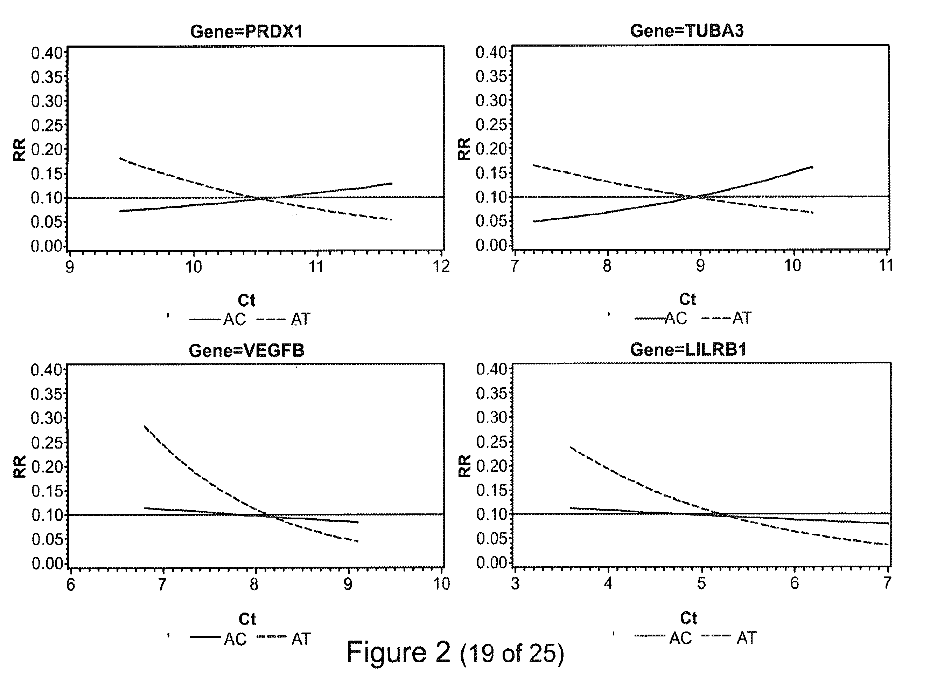

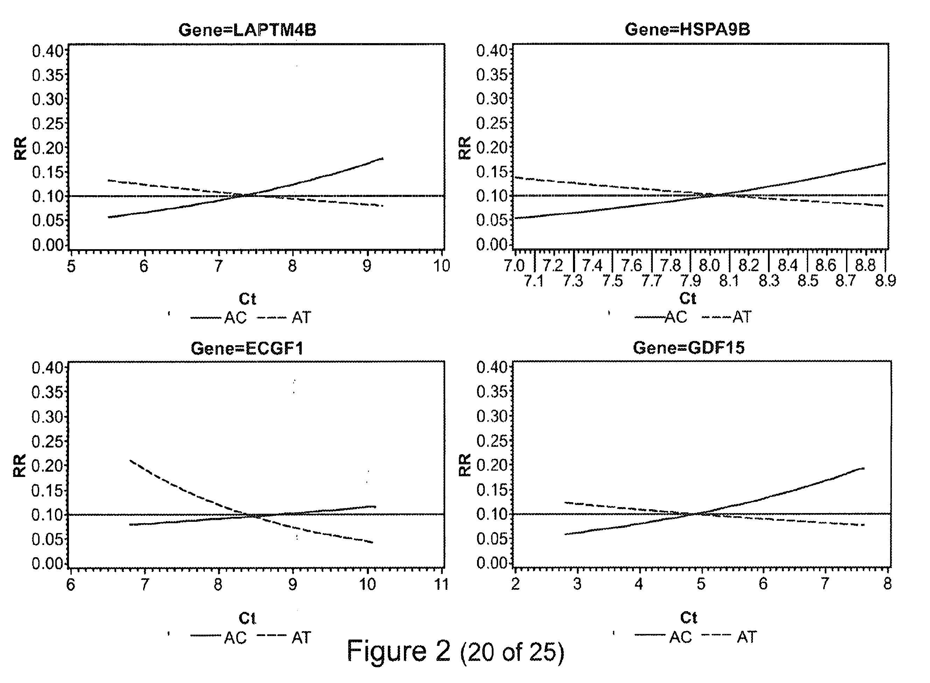

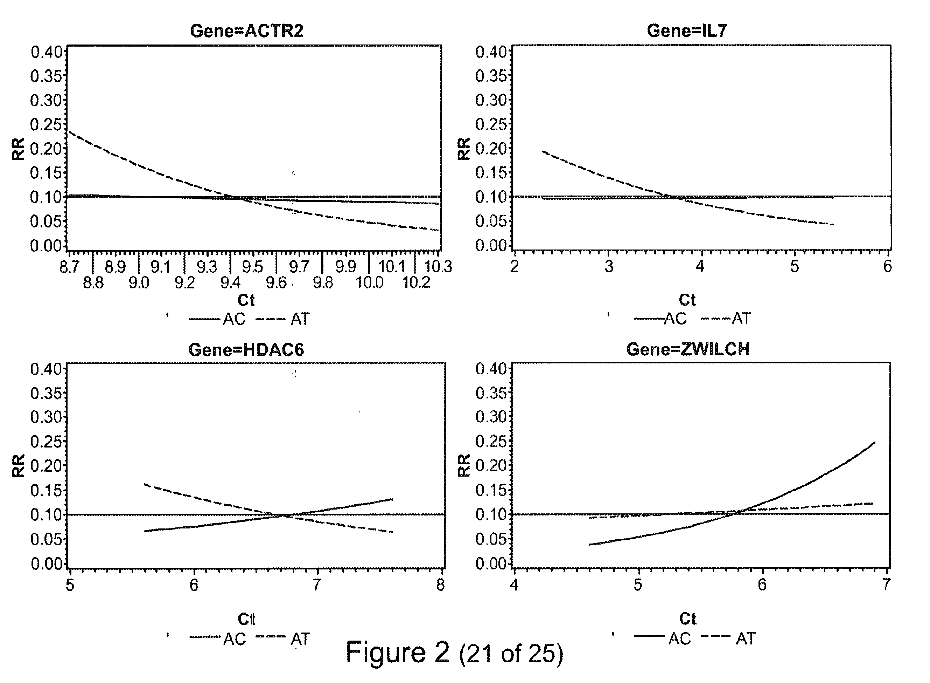

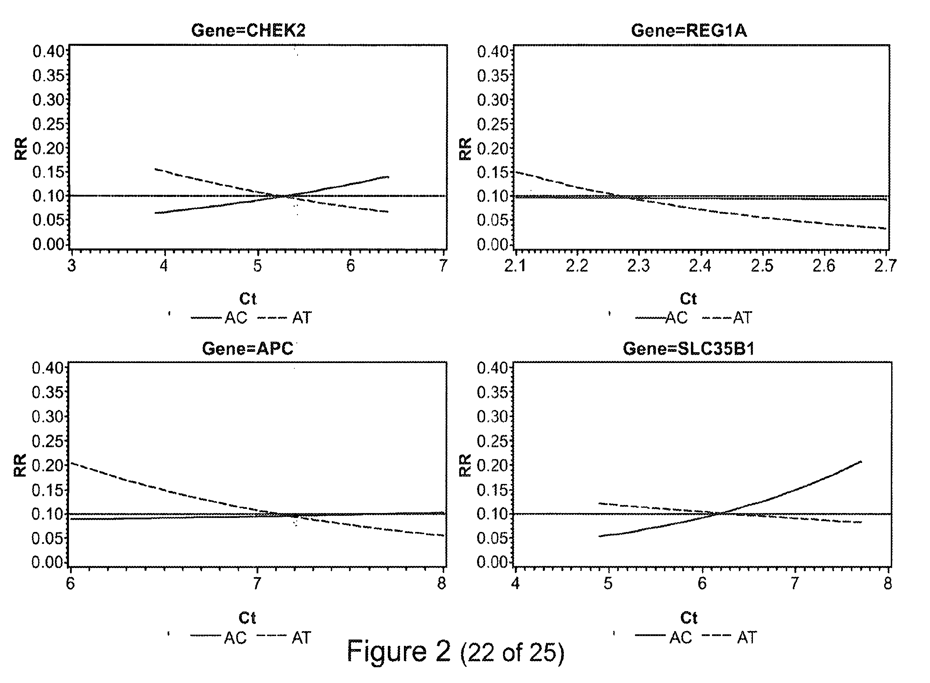

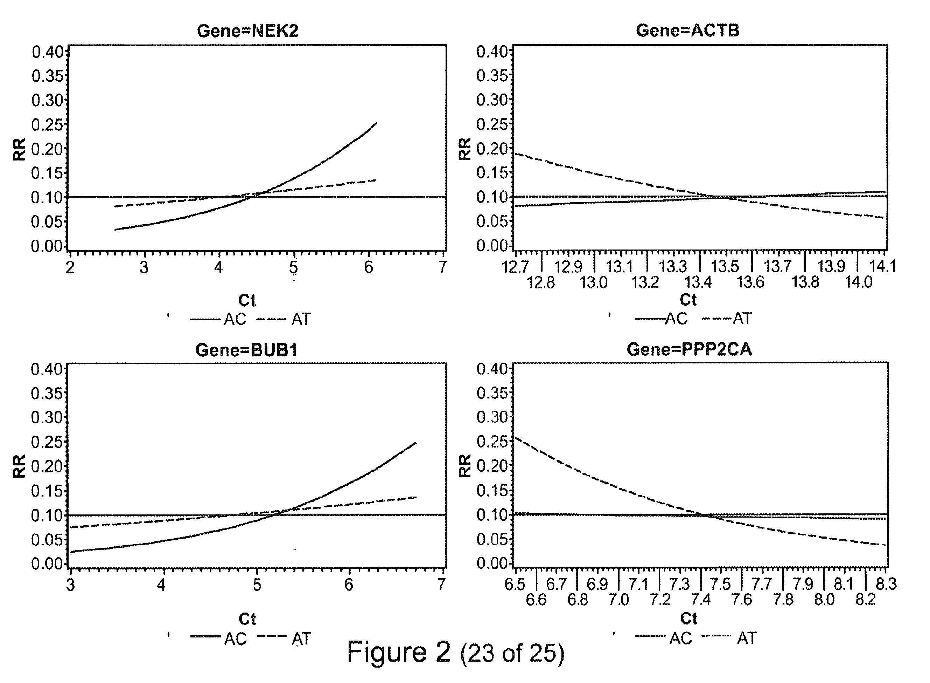

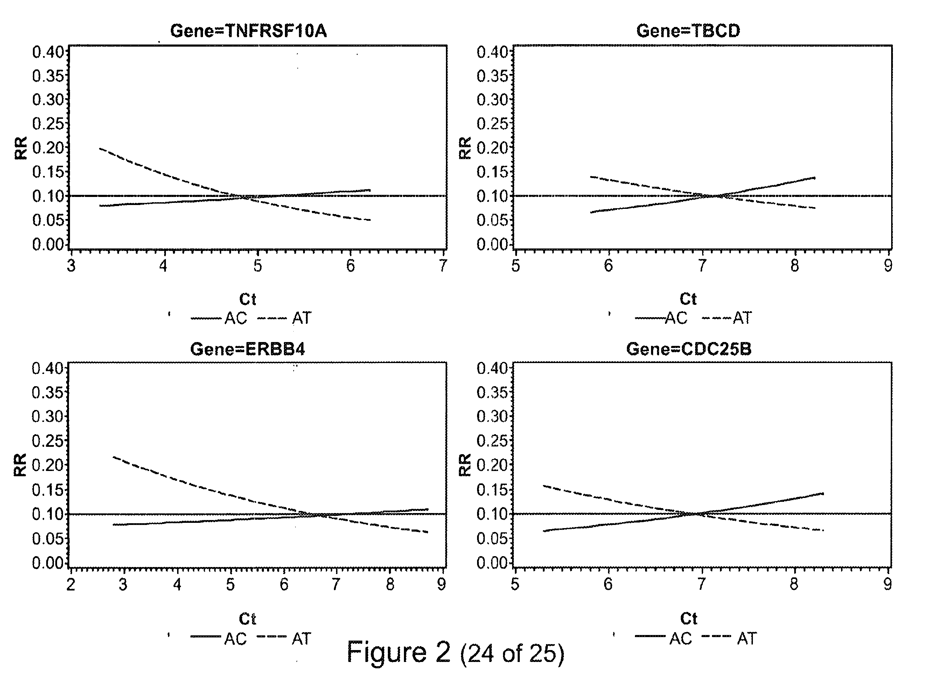

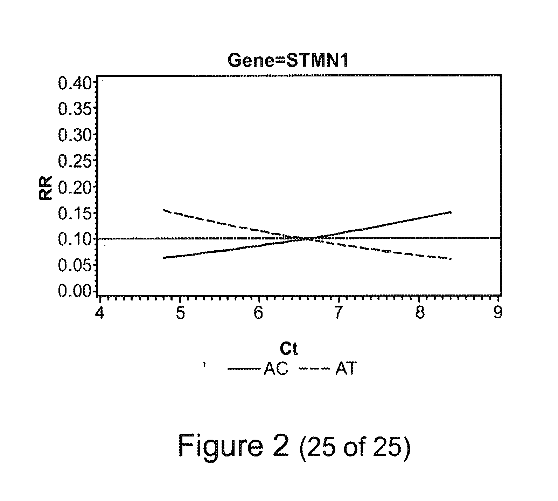

Figure 2 is a set of graphs showing the relationship between normalized expression (represented by "Cₜ") of the indicated gene (gene name provided at top of each graph) and 5-year recurrence rate (RR) of breast cancer in a treatment group receiving anthracycline and a cyclophosphamide (AC prediction curve; smooth line) and the relationship between expression of the indicated gene and RR in a treatment group receiving anthracycline and a taxane (AT prediction curve; hatched line), where the patients in the treatment groups had hormone receptor positive (HR⁺) breast cancer. A horizontal dashed line in each graph represents the overall (i.e., not gene expression-specific) 5-year RR in patients in the study population having HR+ breast cancer who were randomized to treatment with either AC or AT.

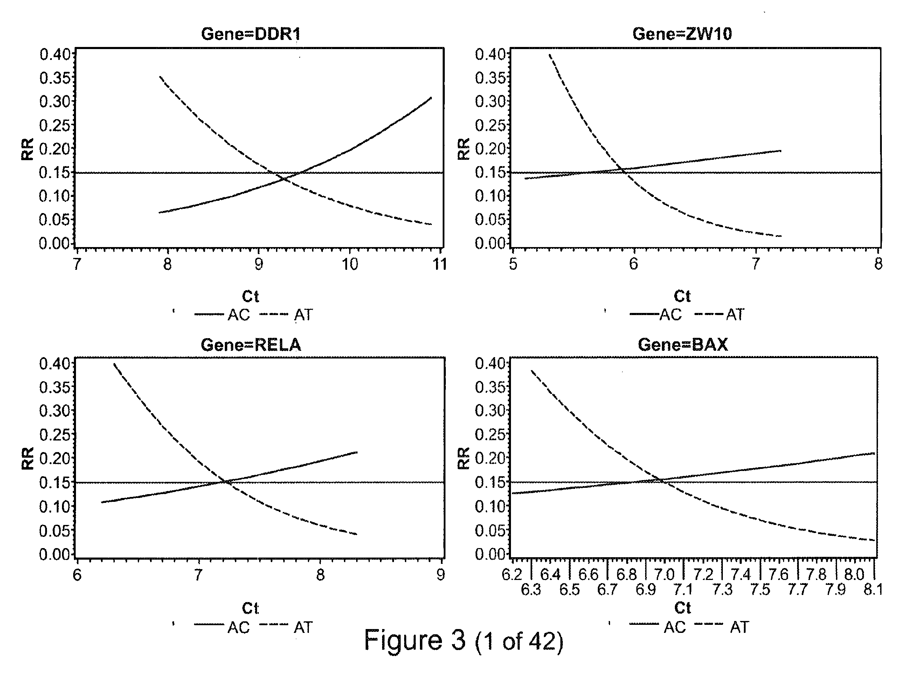

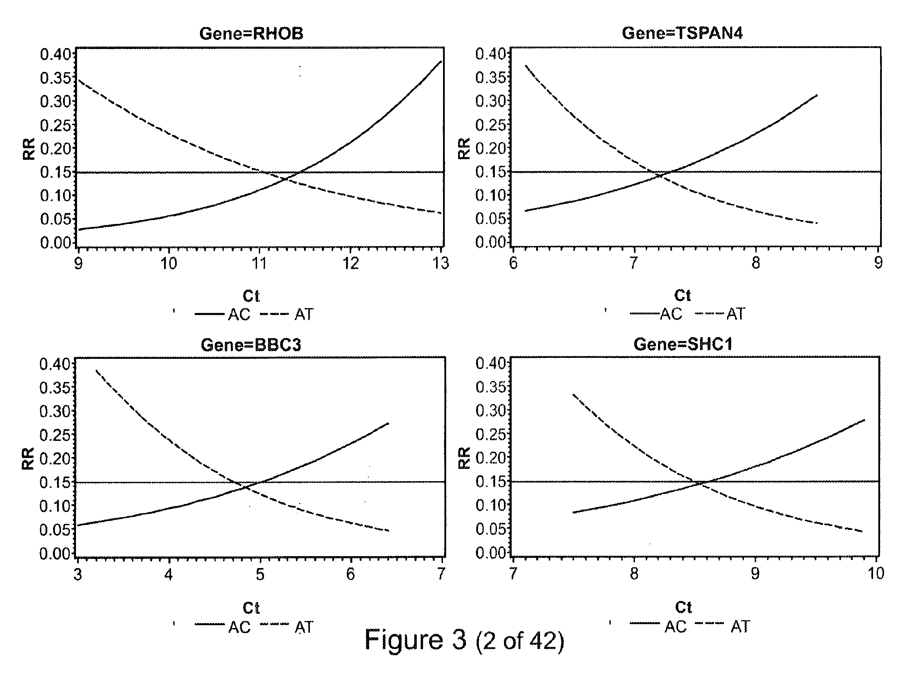

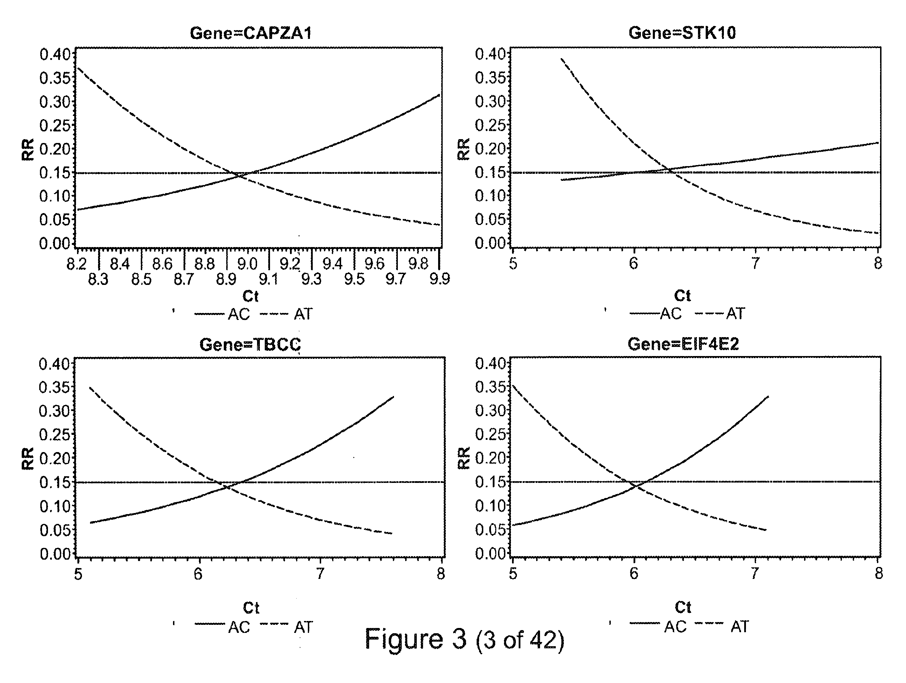

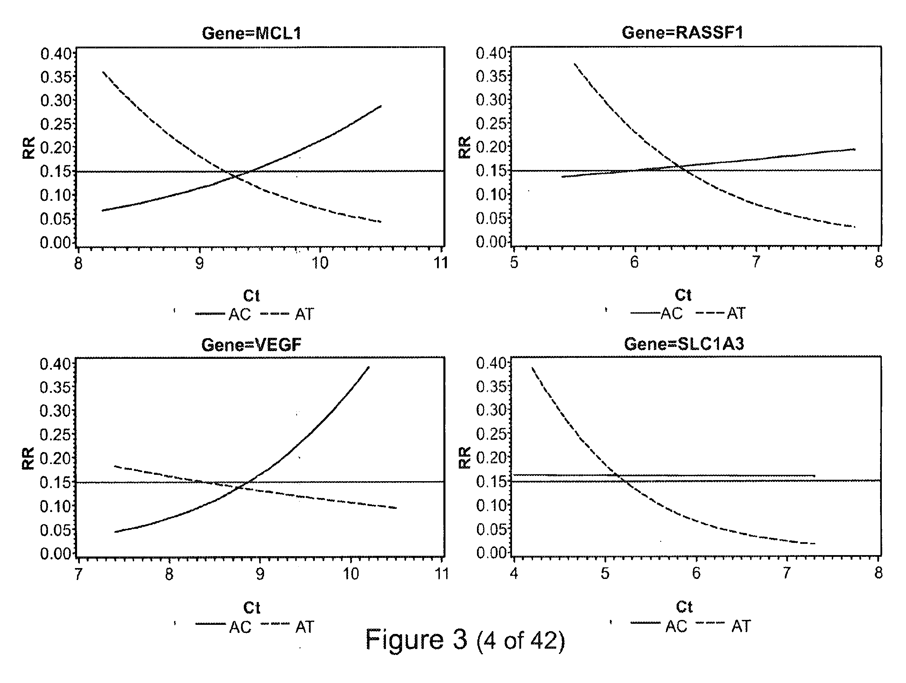

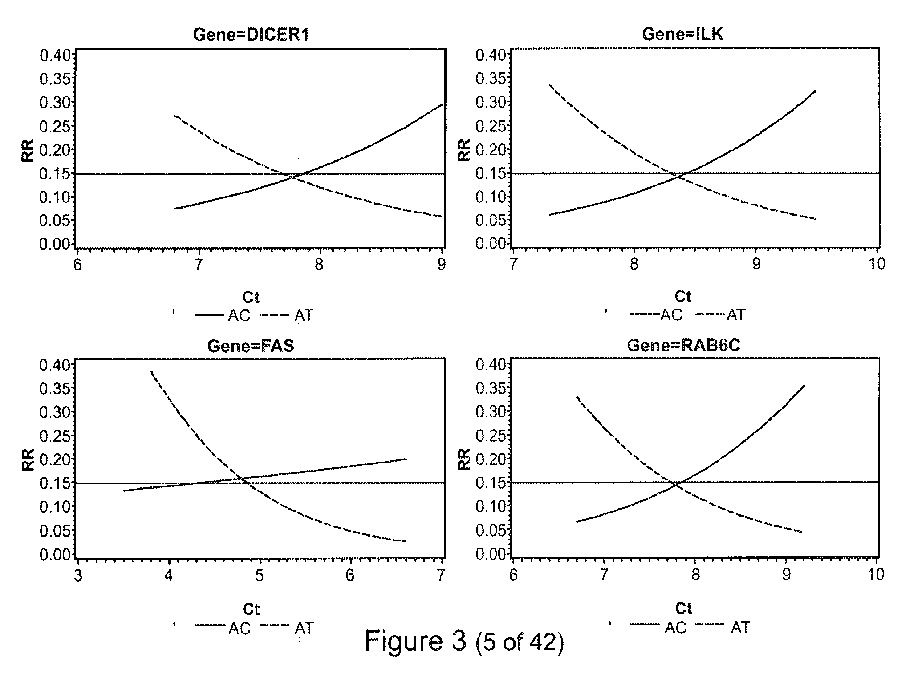

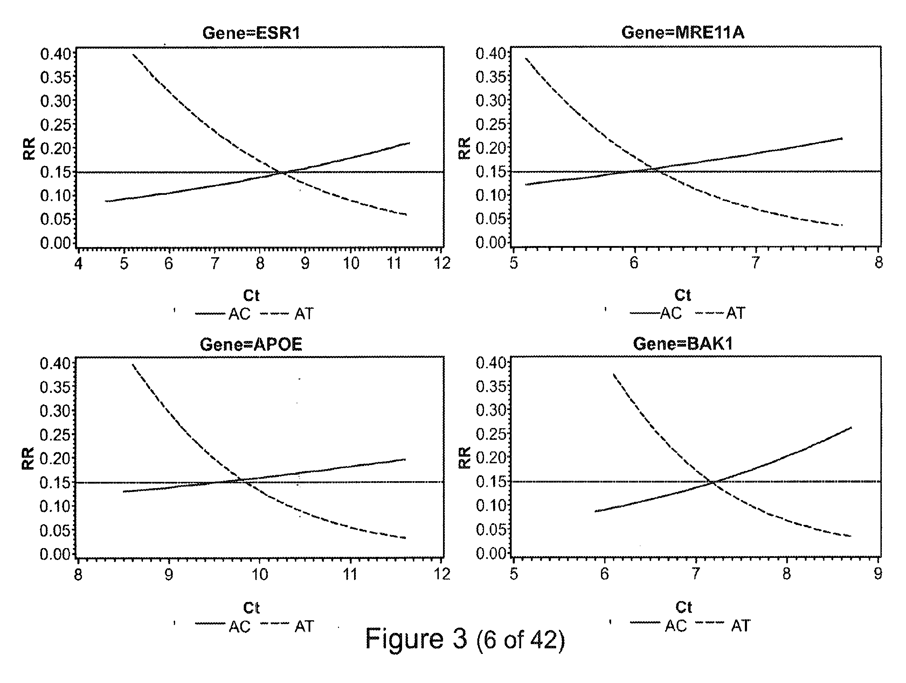

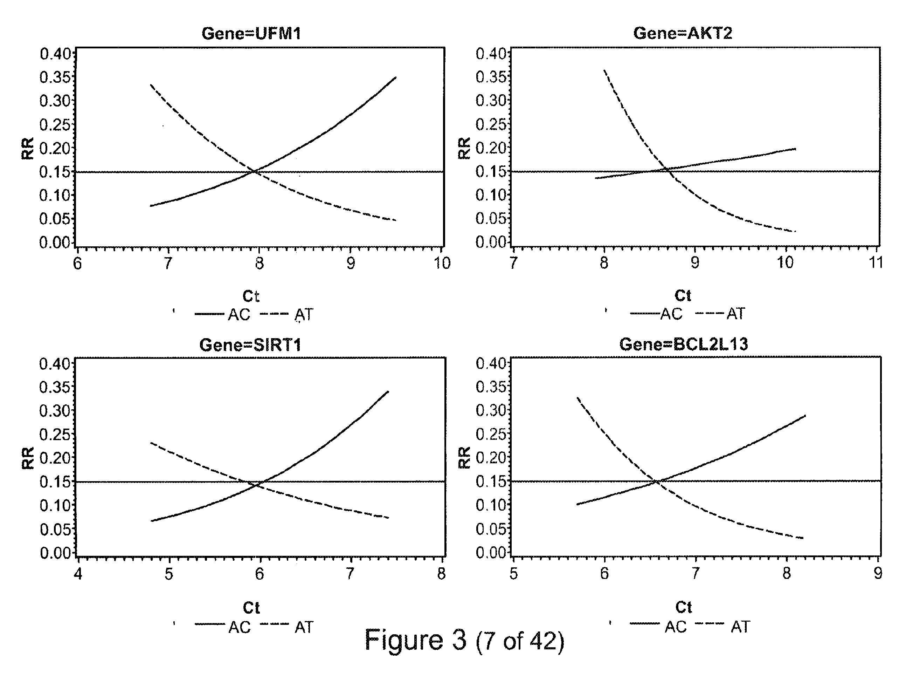

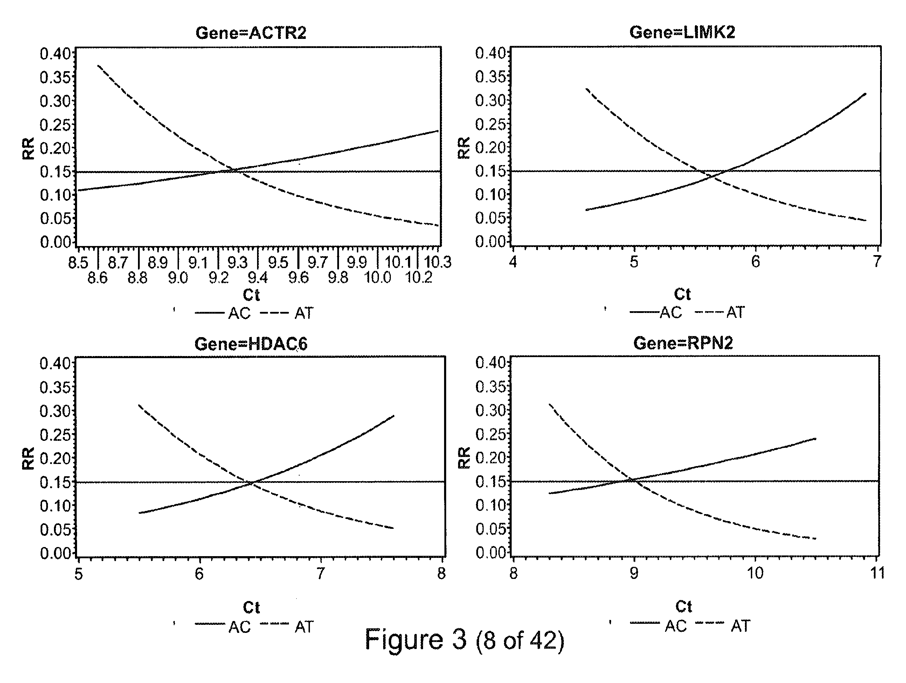

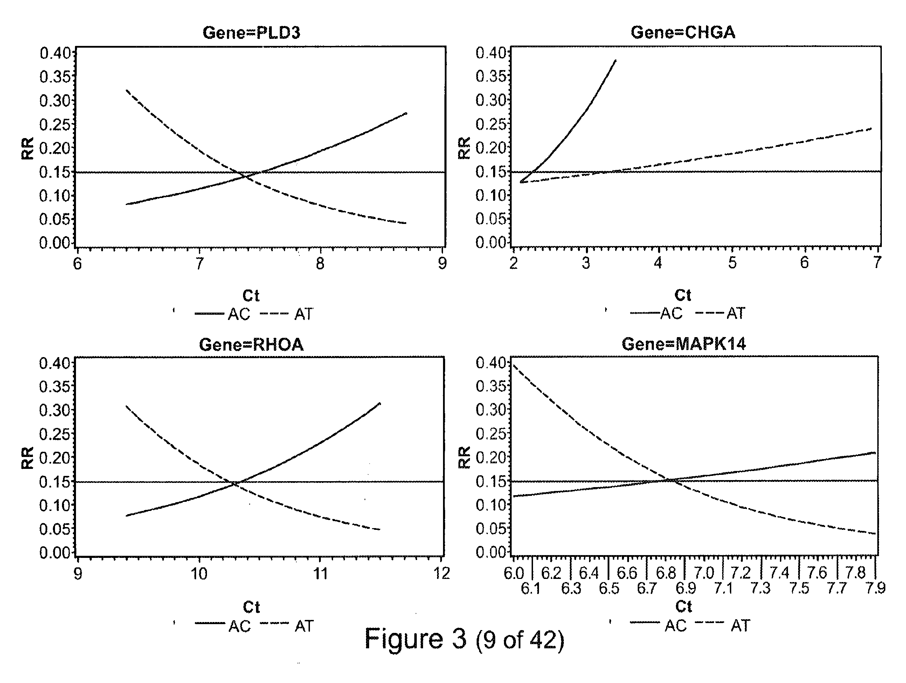

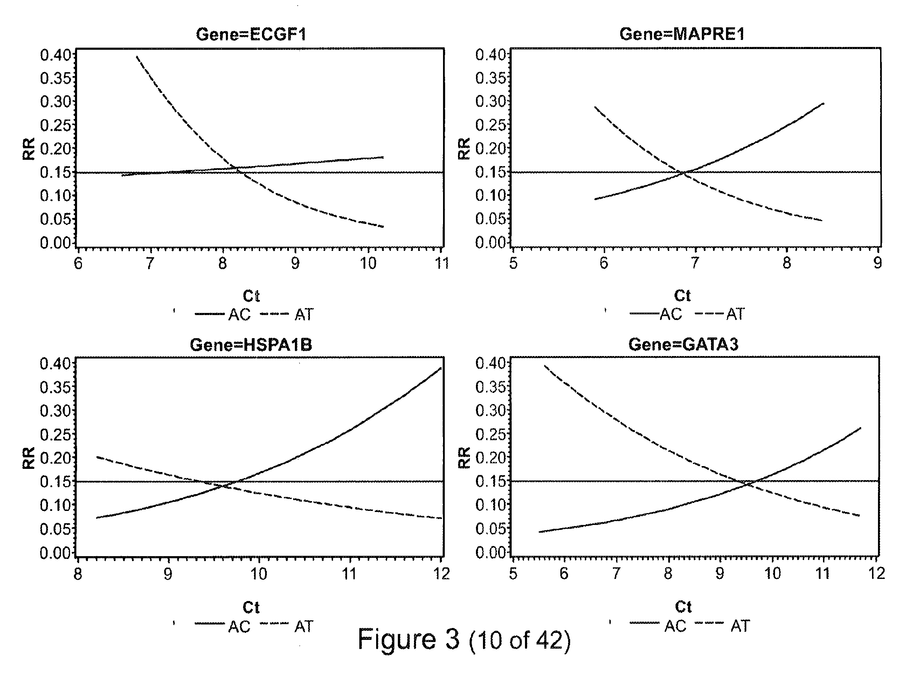

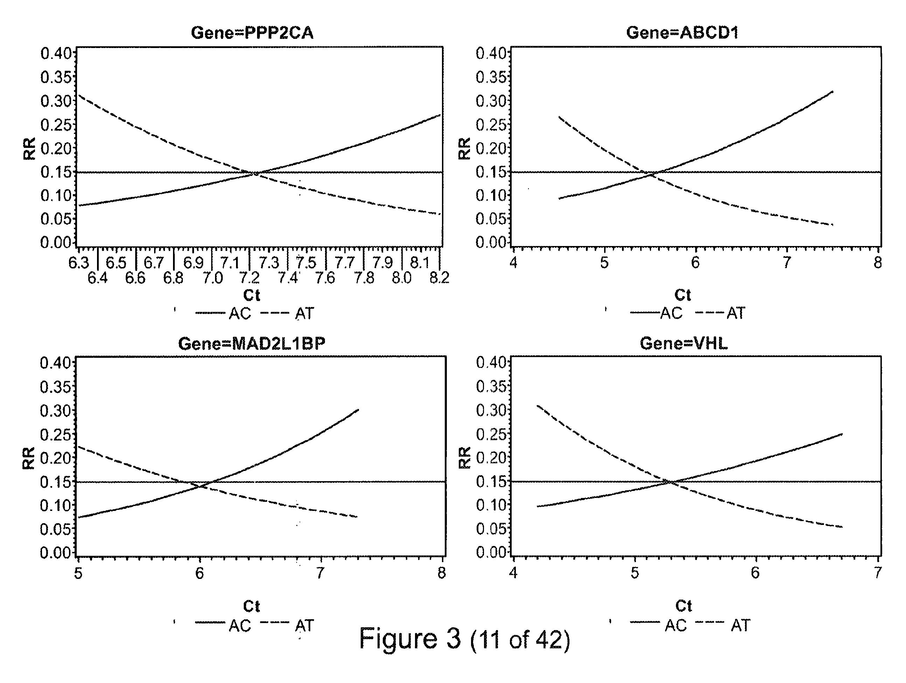

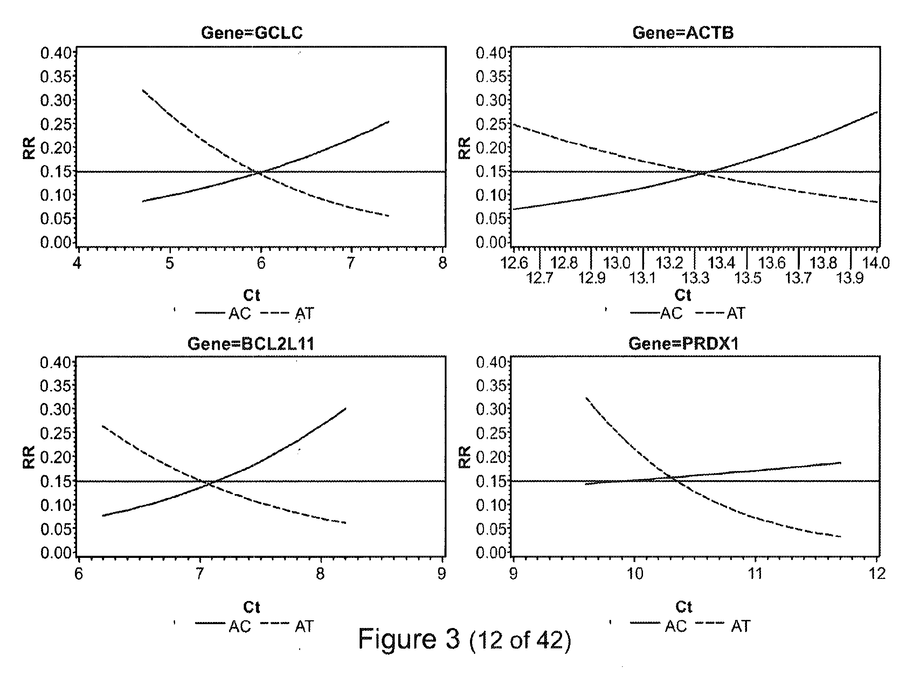

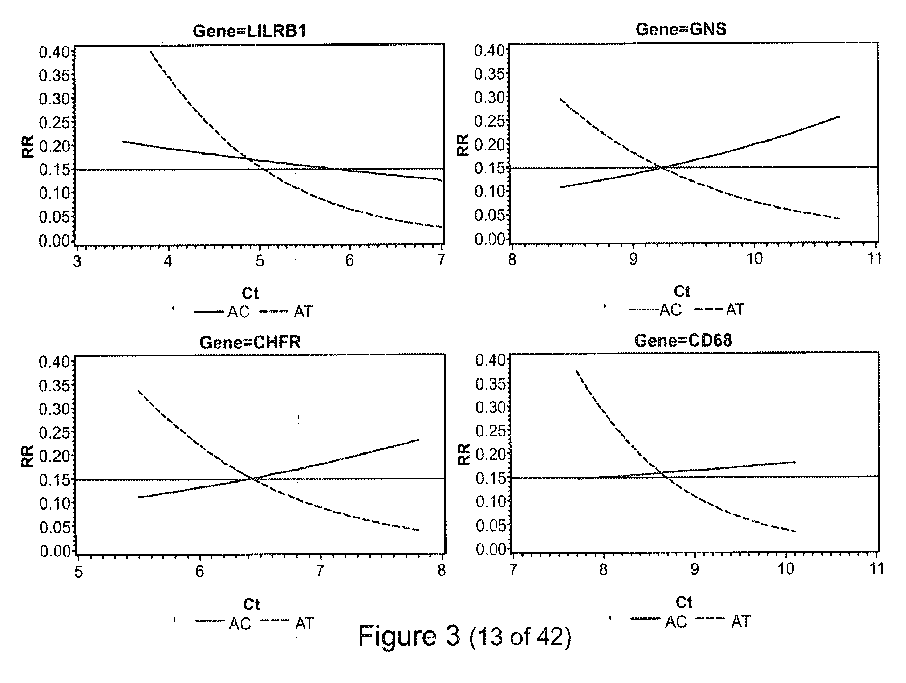

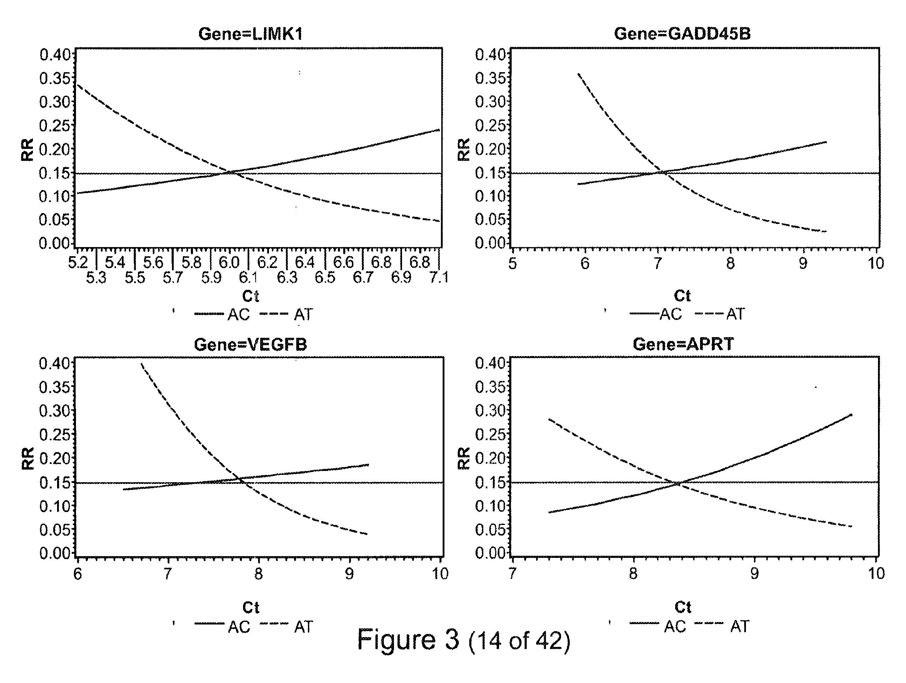

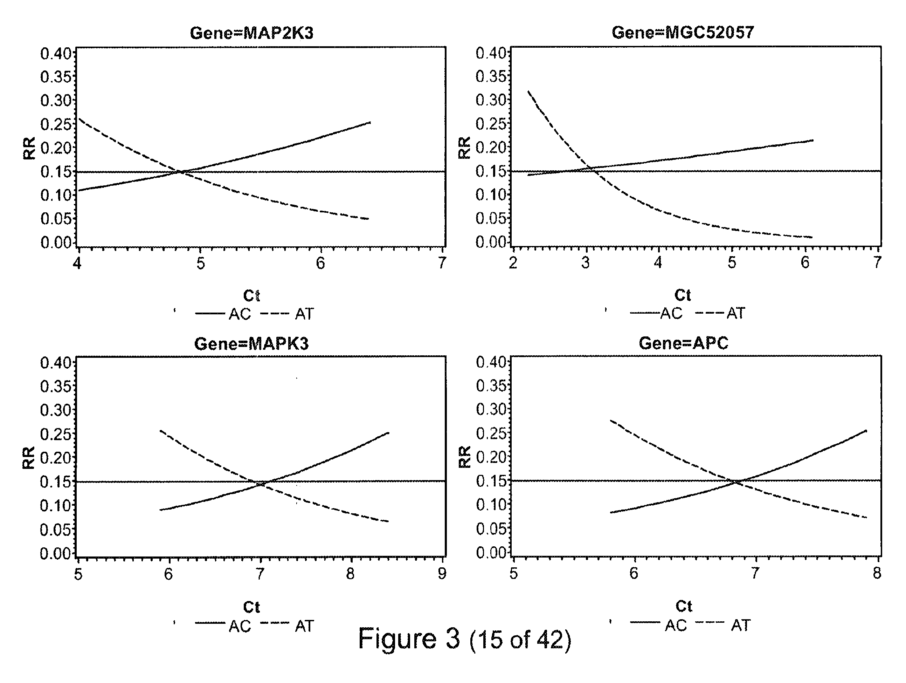

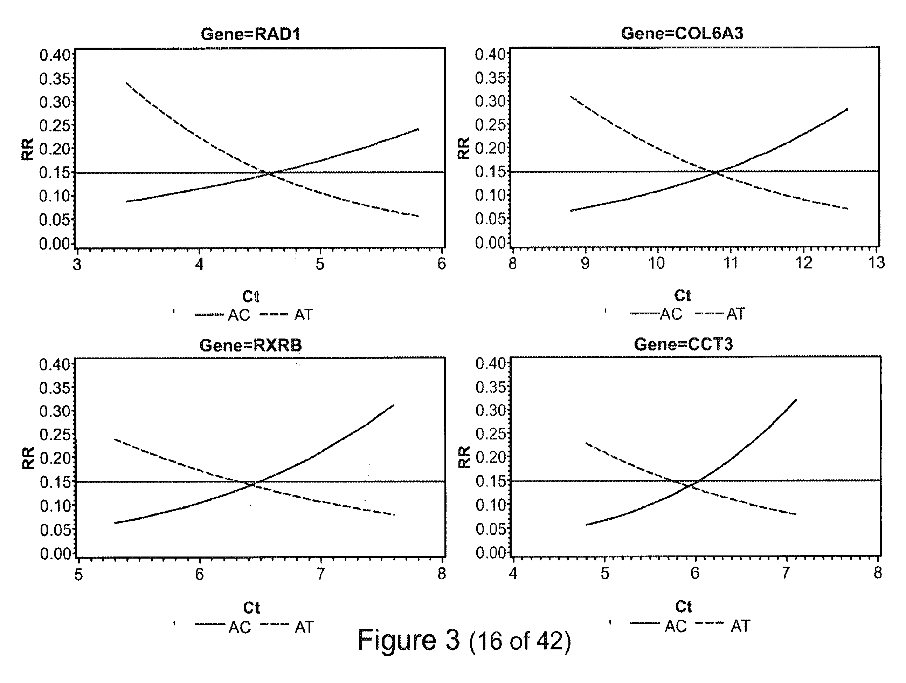

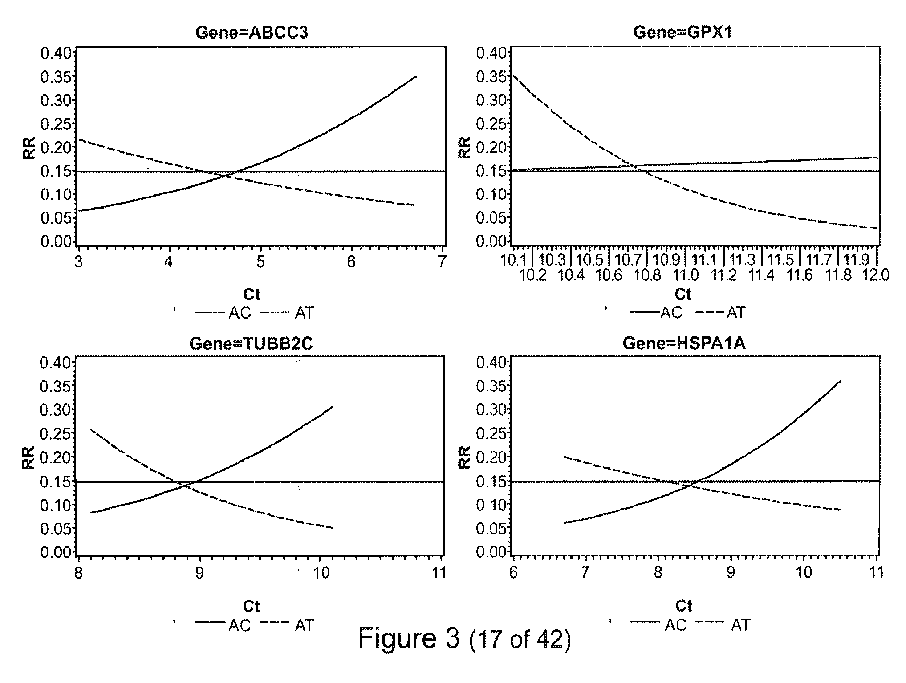

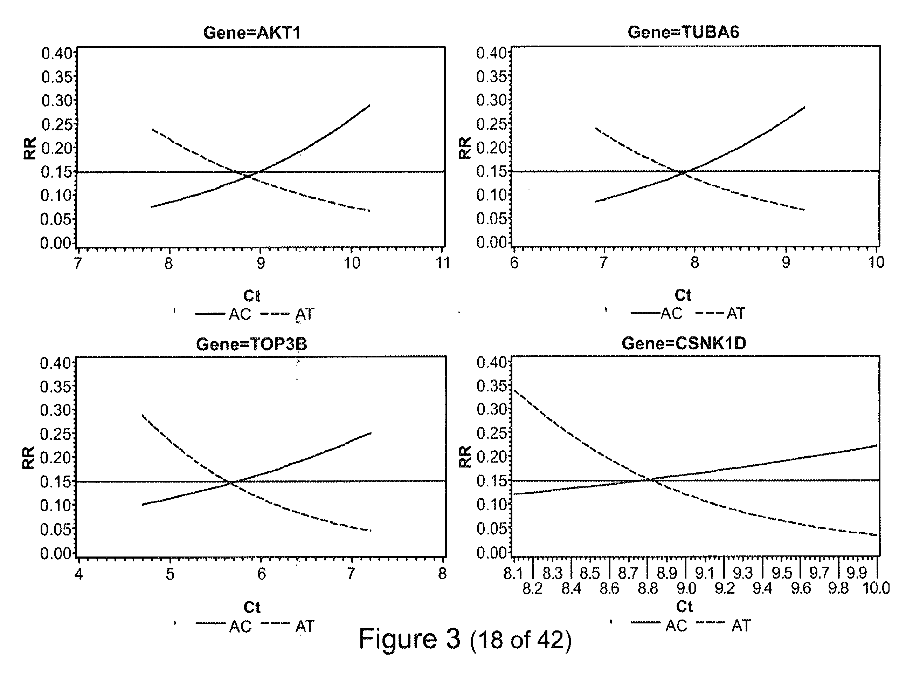

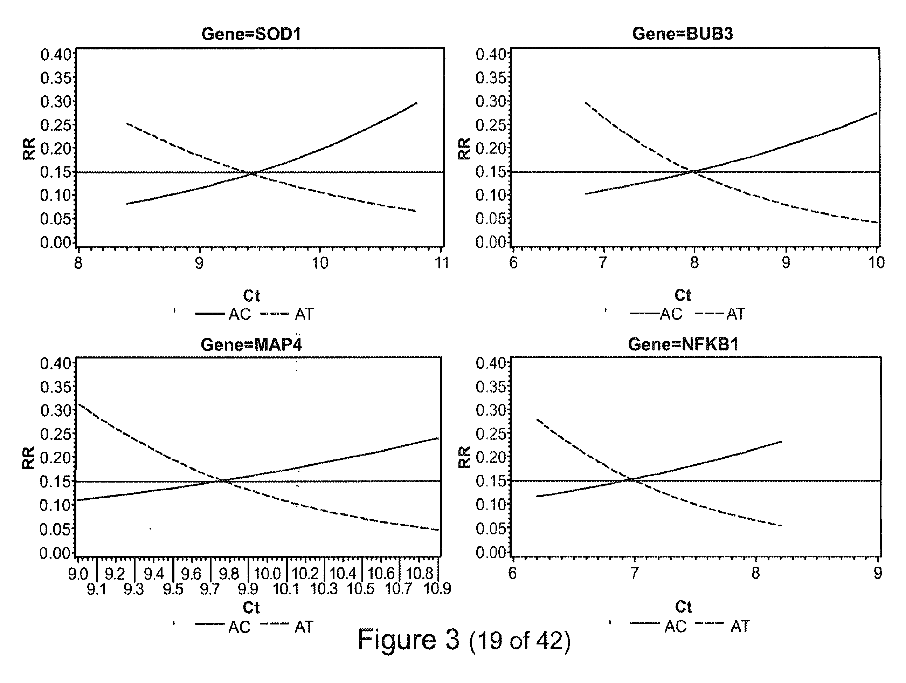

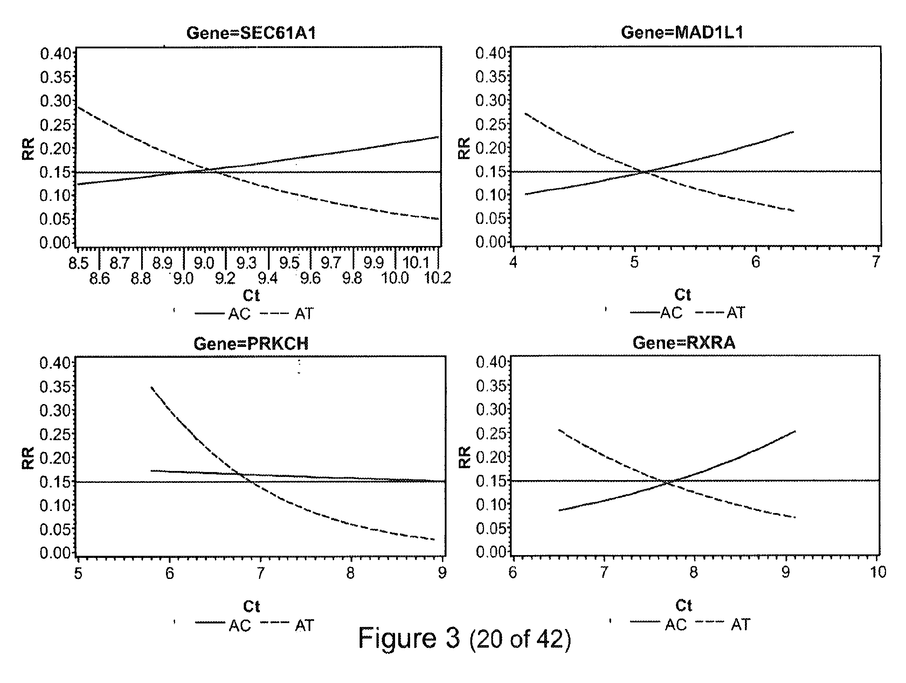

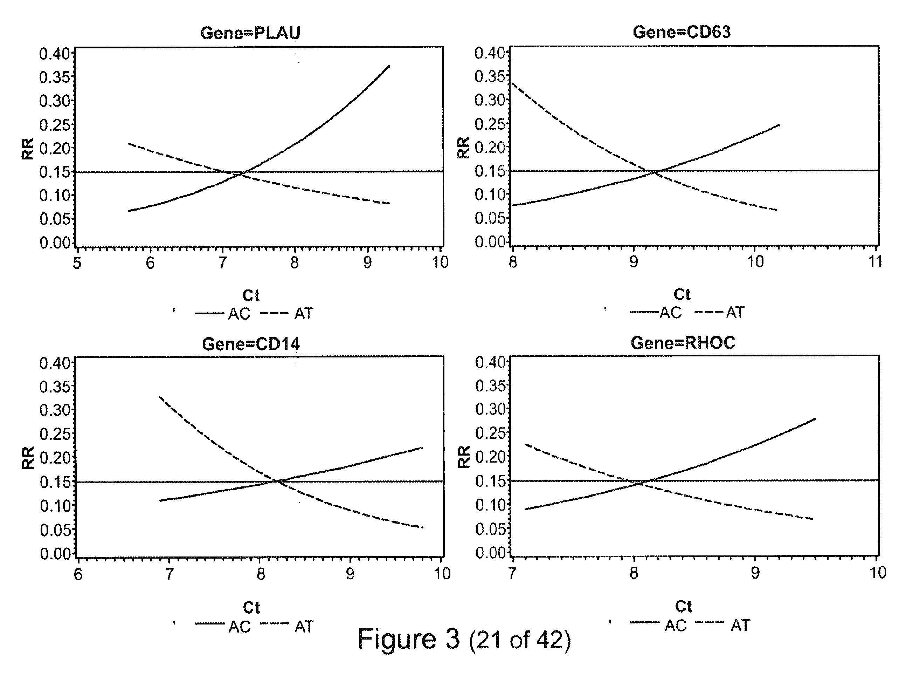

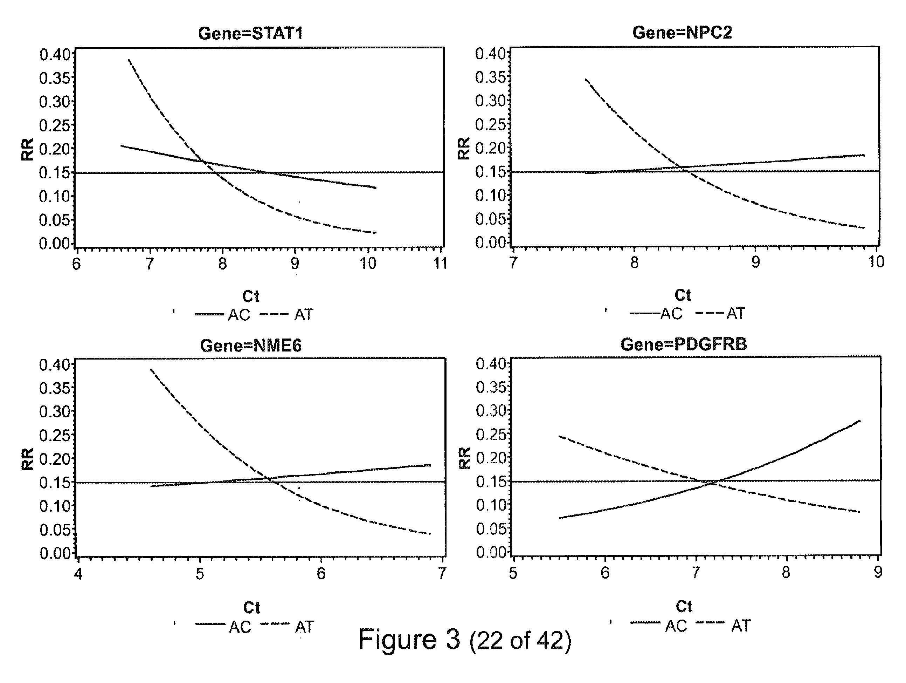

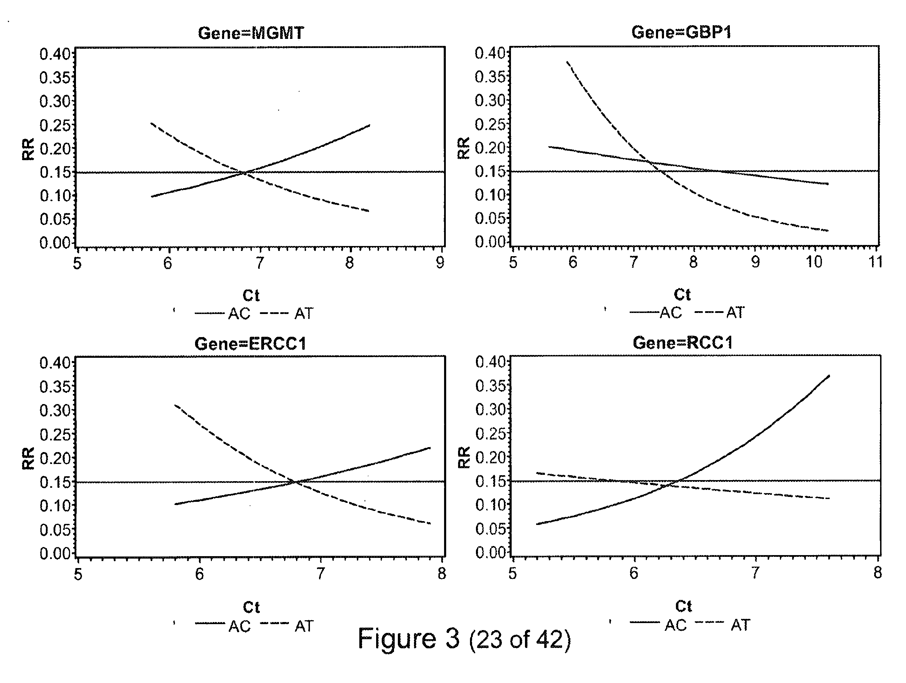

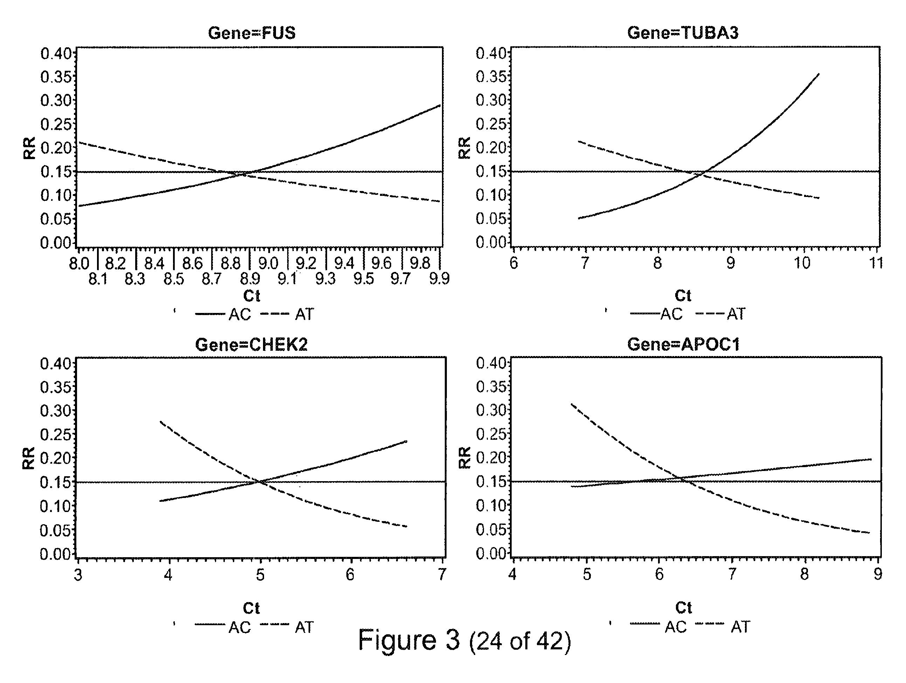

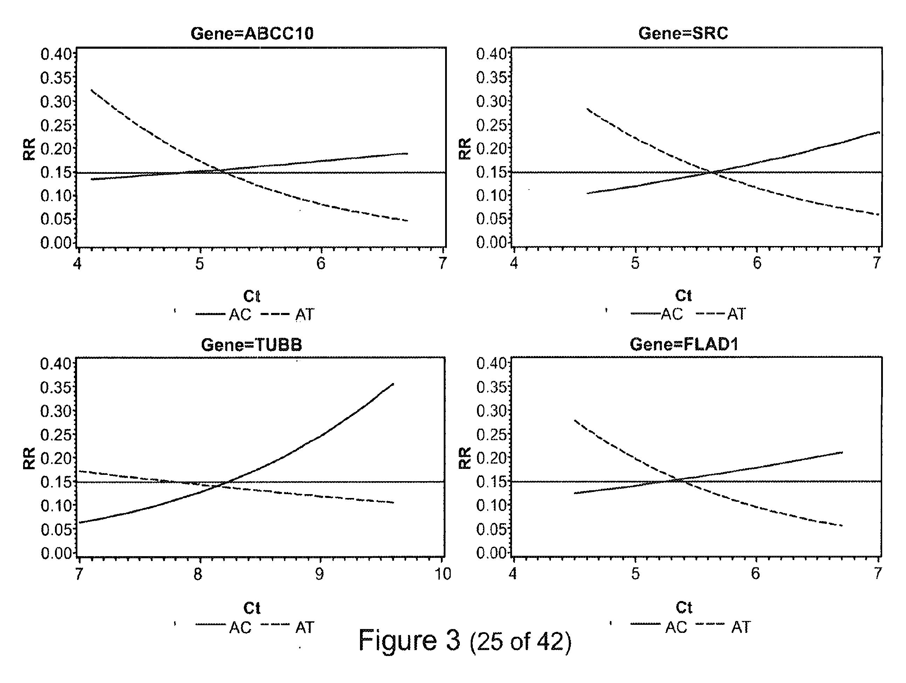

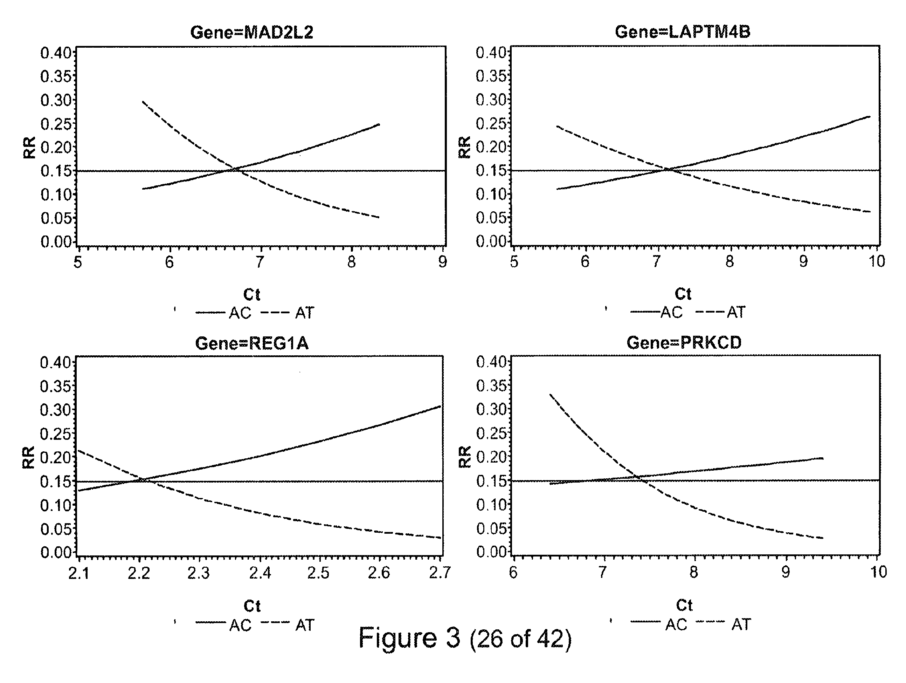

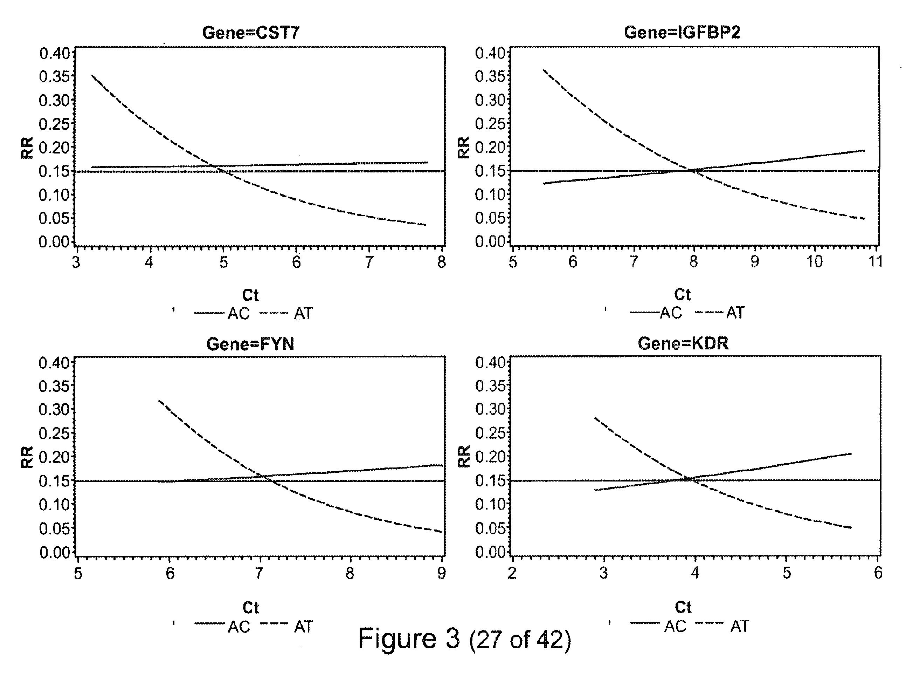

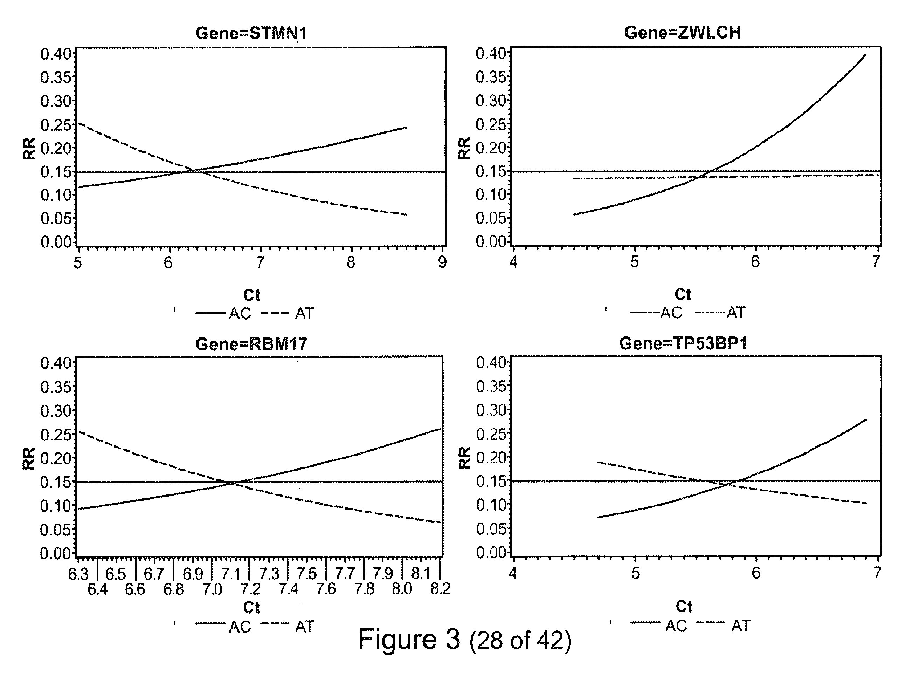

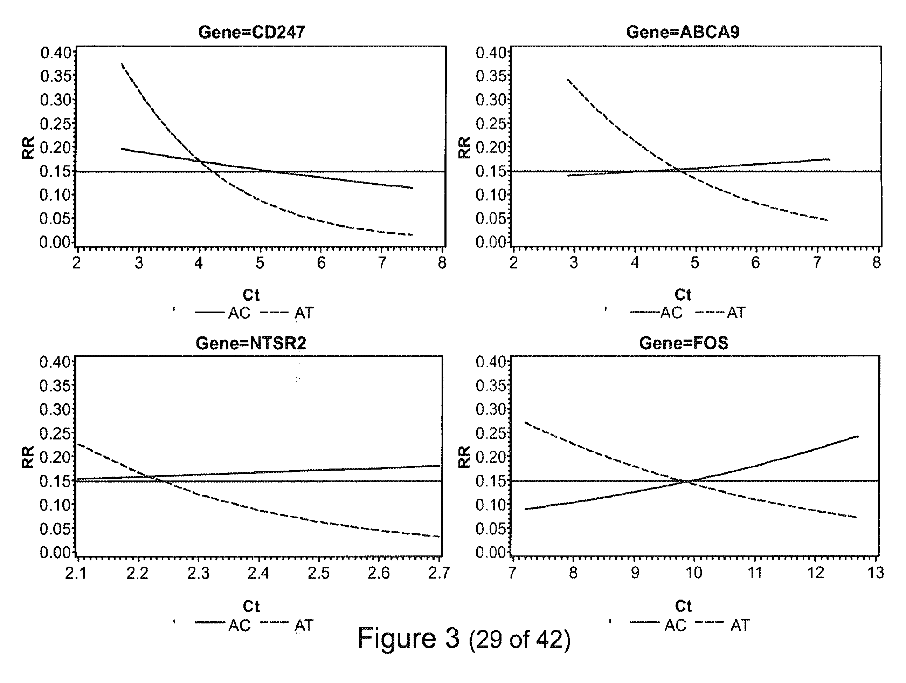

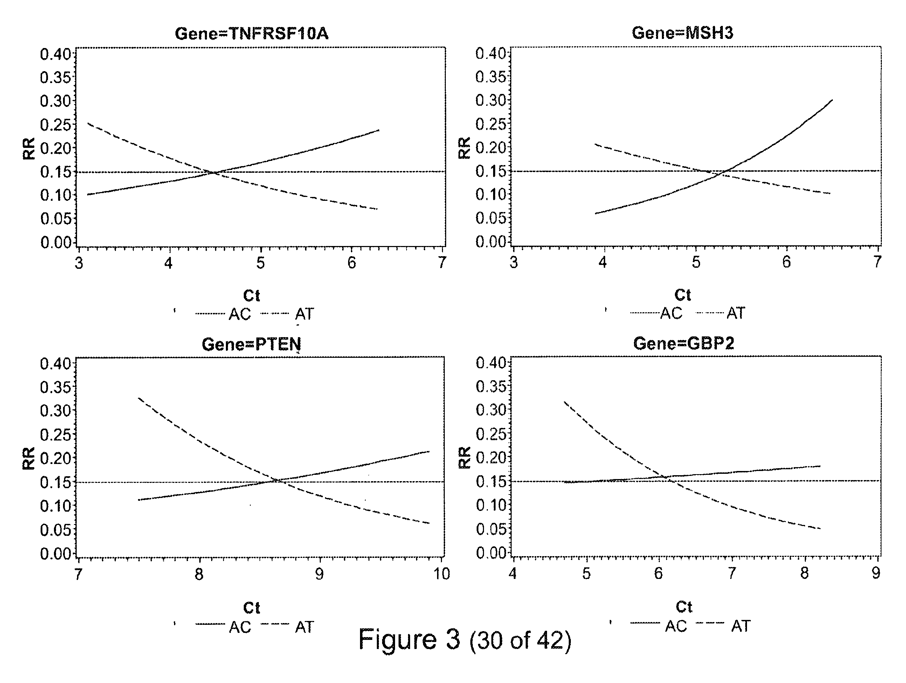

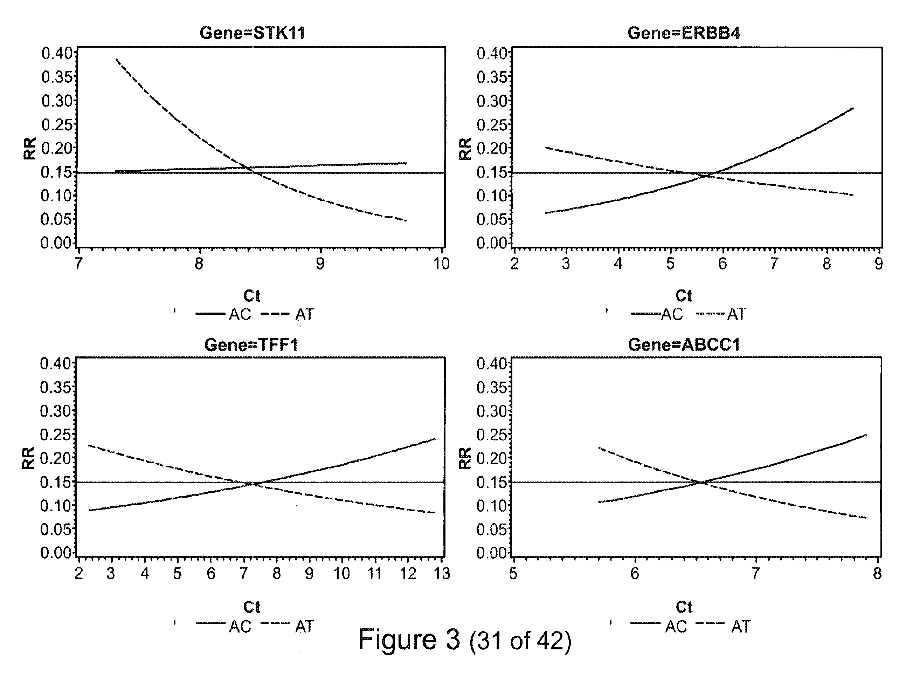

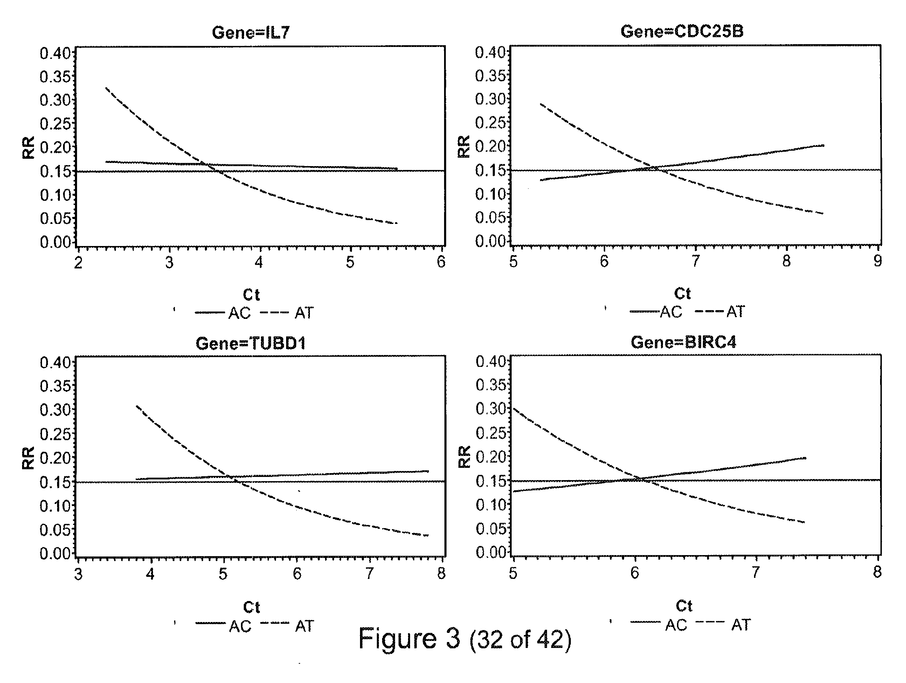

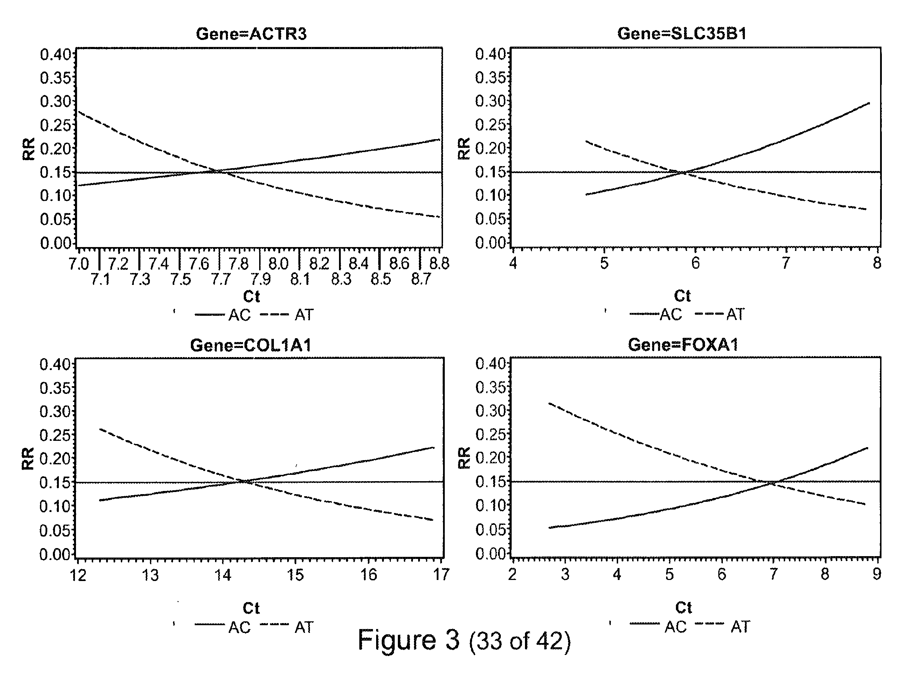

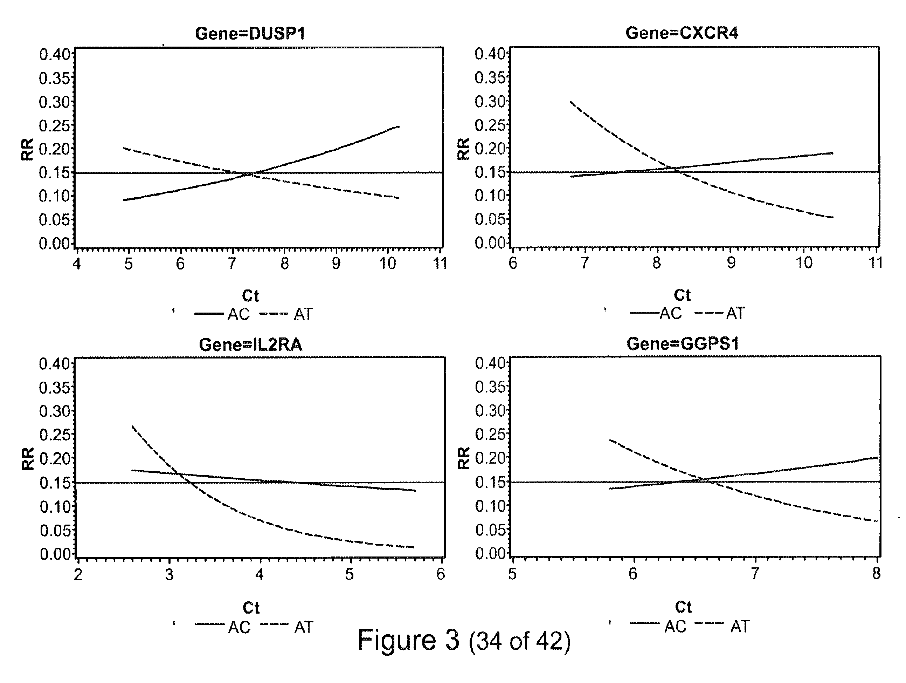

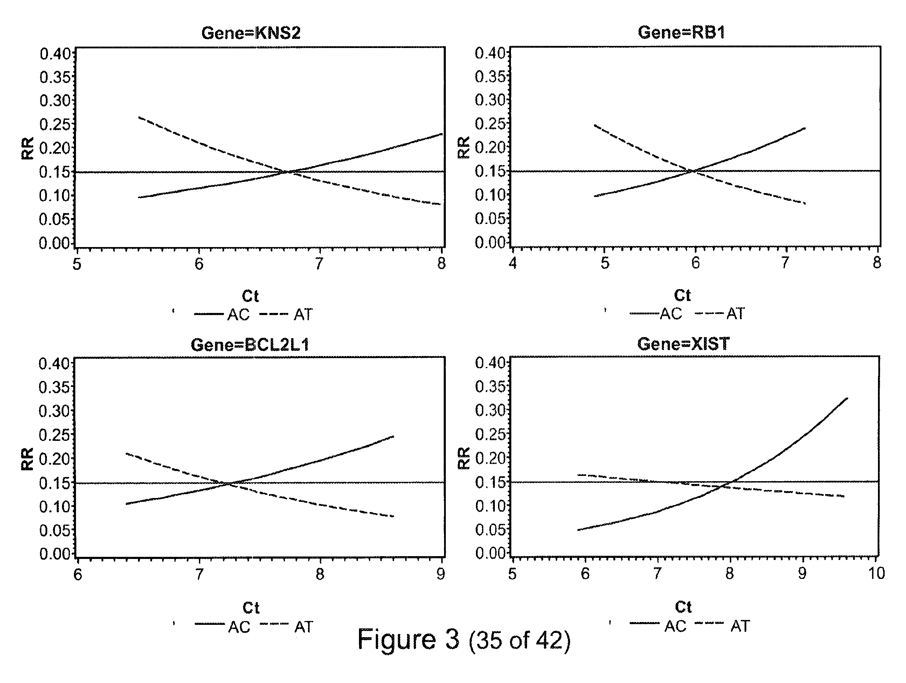

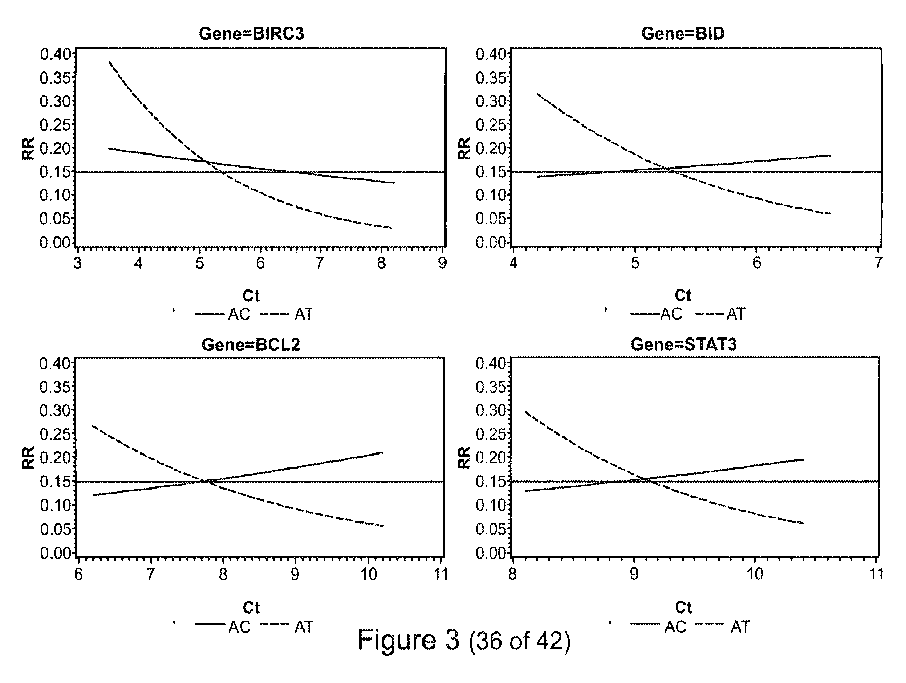

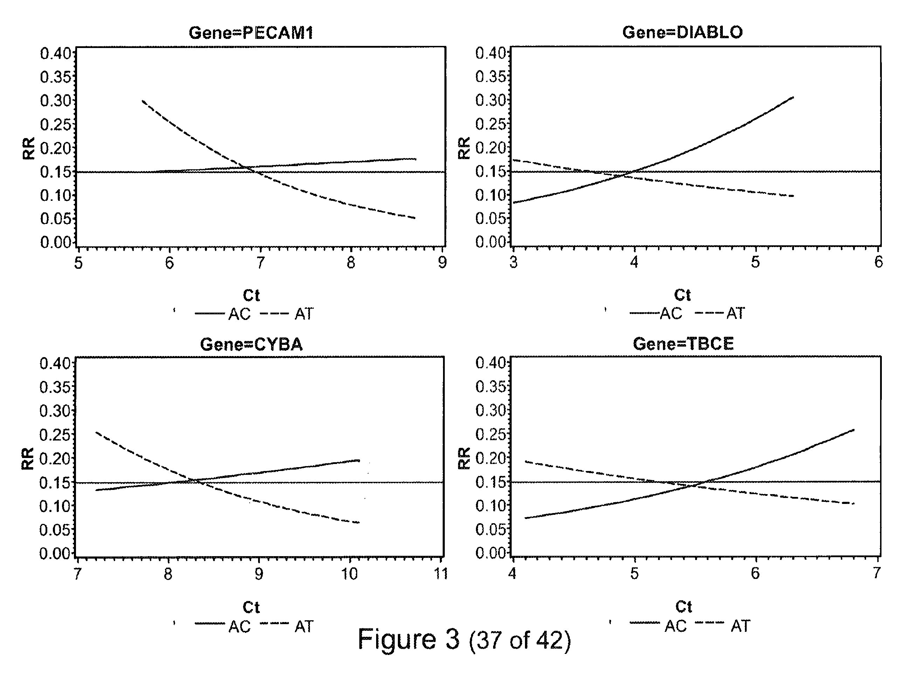

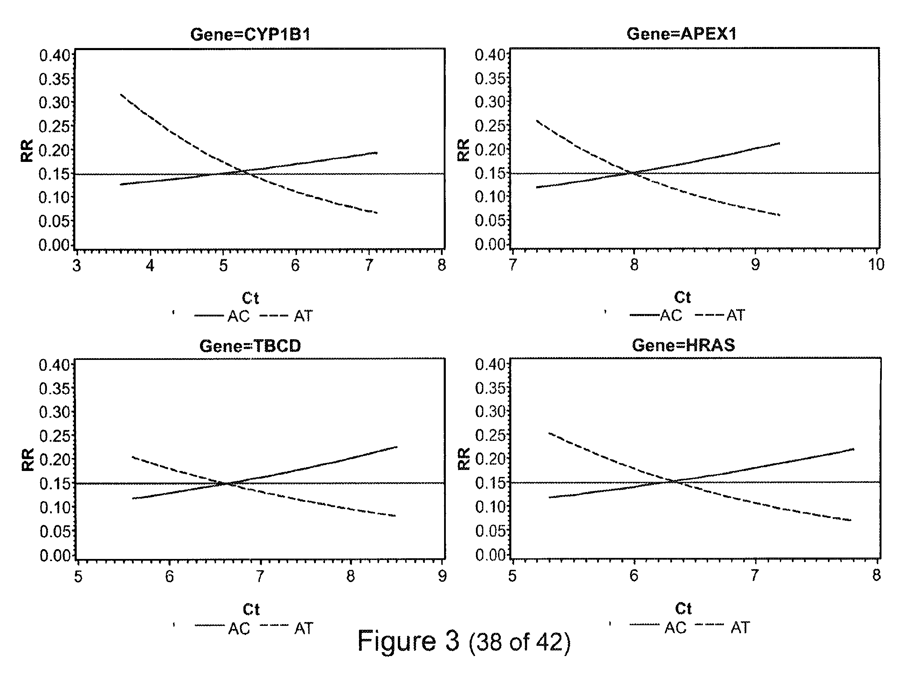

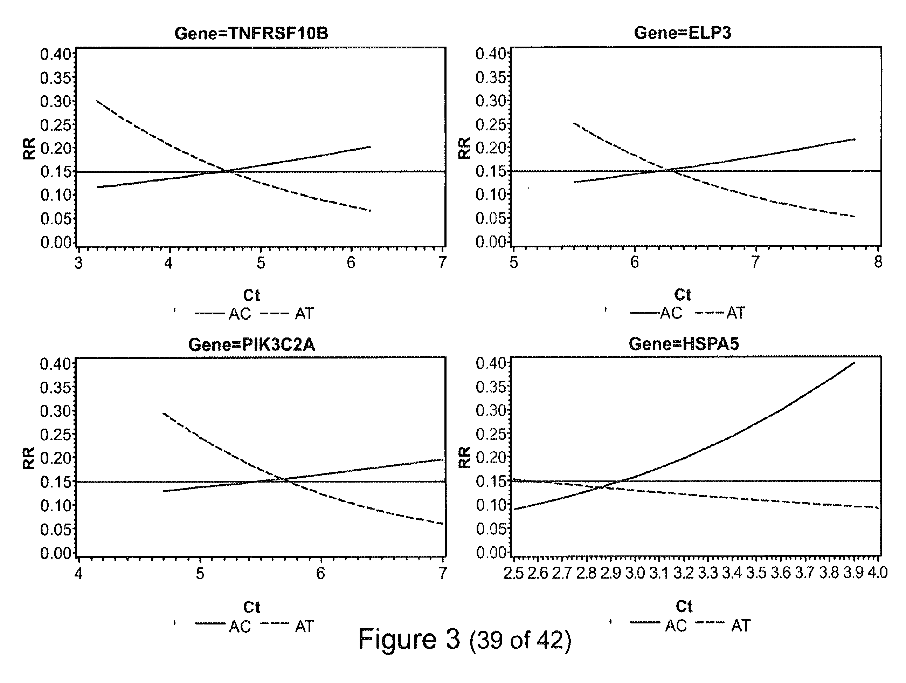

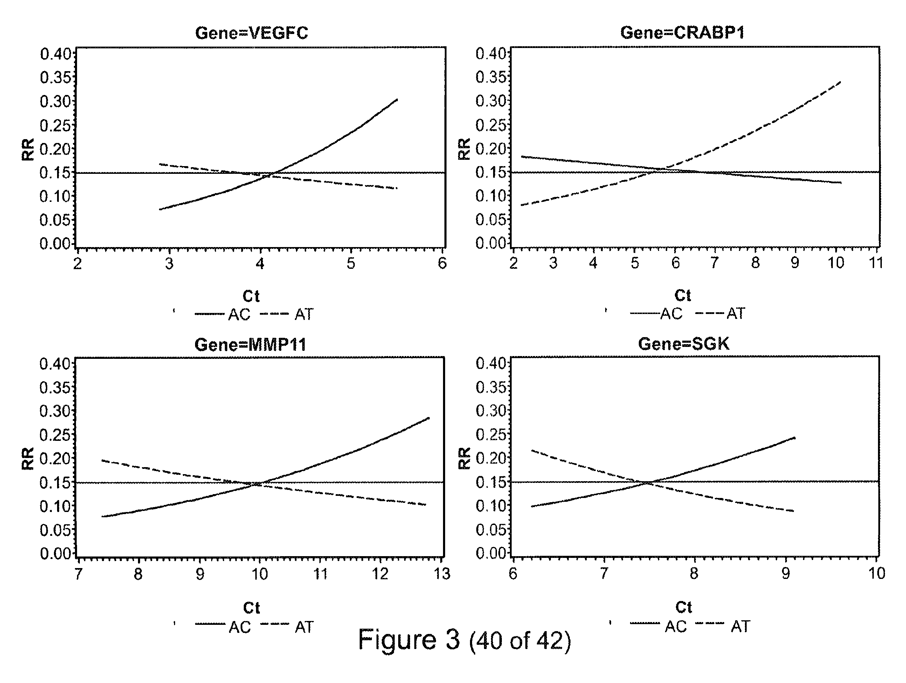

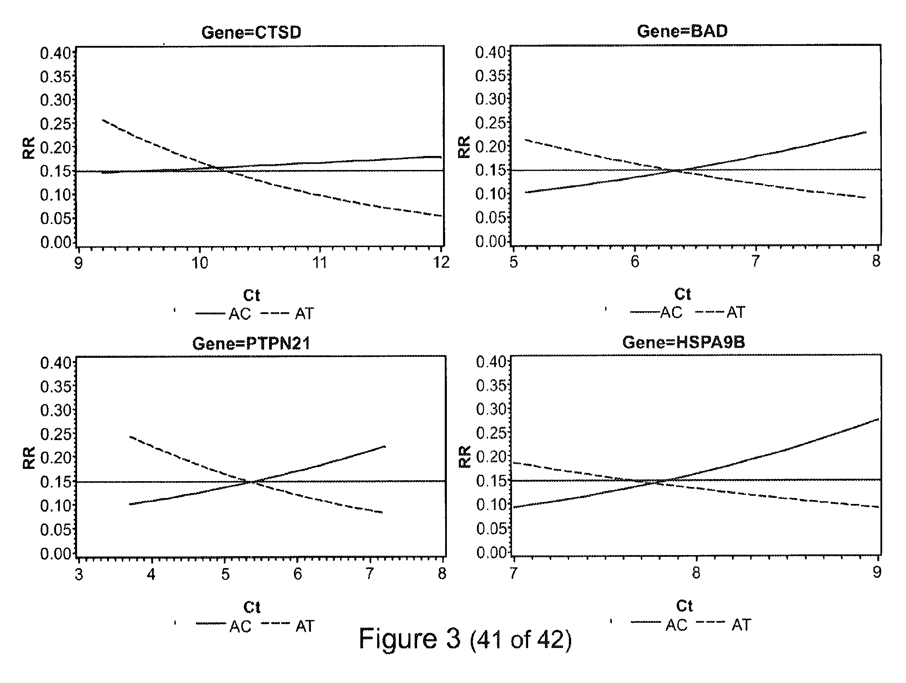

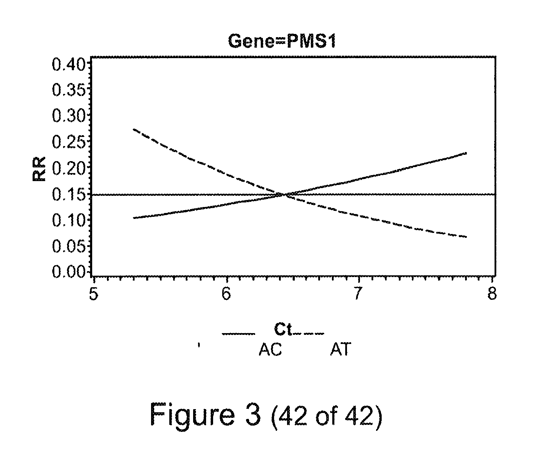

Figure 3 is a set of graphs showing the relationship between normalized expression (represented by "Cₜ") of the indicated gene (gene name provided at top of each graph) and 5-year recurrence rate (RR) of breast cancer in a treatment group receiving anthracycline and a cyclophosphamide (AC prediction curve; smooth line) and the relationship between expression of the indicated gene and RR in a treatment group receiving anthracycline and a taxane (AT prediction curve; hatched line), where the patients in the treatment groups had hormone receptor positive (HR⁺) breast cancer and an Oncotype Dx Recurrence Score of greater than 18. A horizontal dashed line in each graph represents the overall (i.e., not gene expression-specific) 5-year RR in patients in the study having HR+ breast cancer and an Oncotype Dx Recurrence Score greater than 18 who were randomized to treatment with either AC or AT.

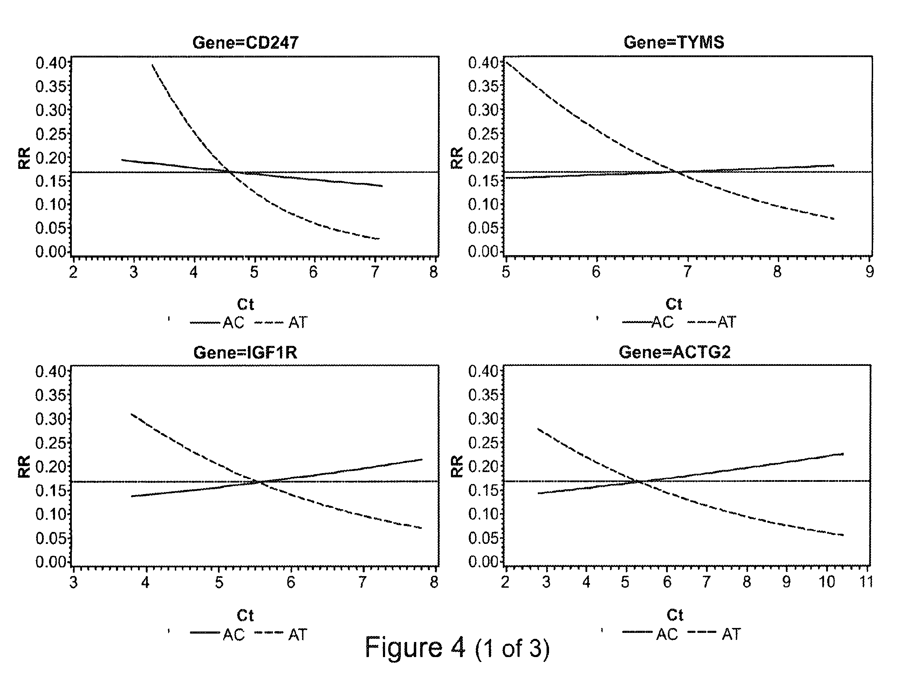

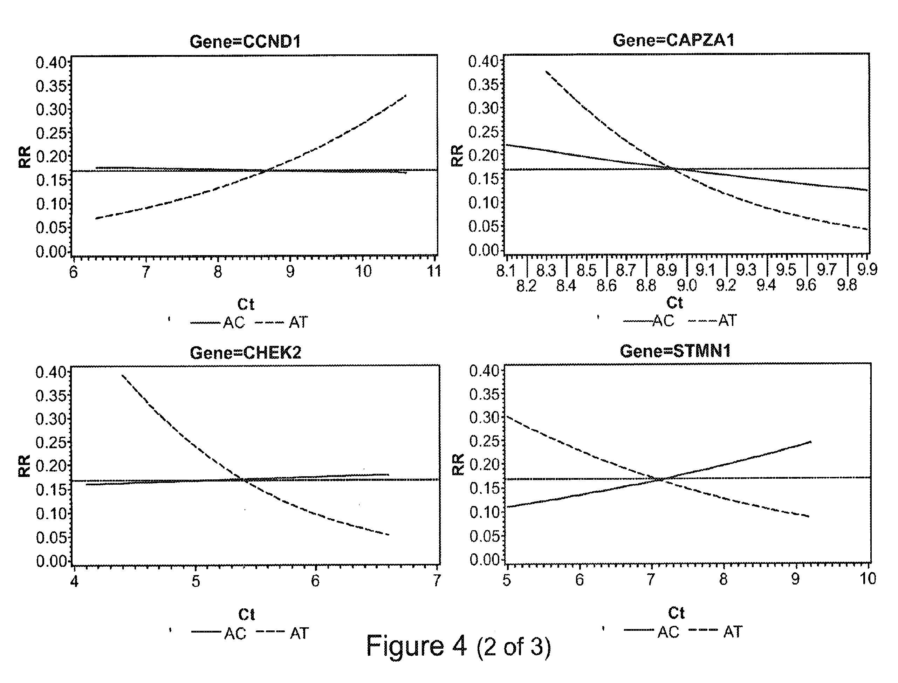

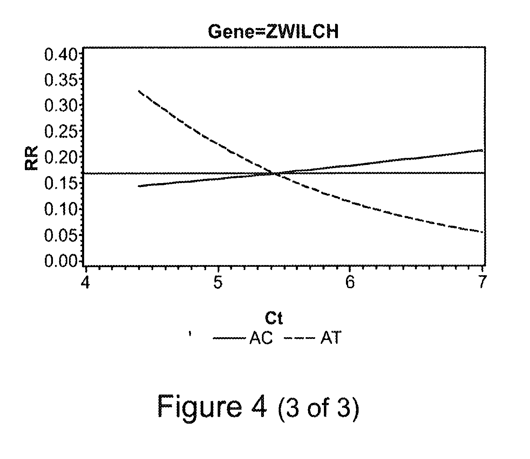

Figure 4 is a set of graphs showing the relationship between normalized expression (represented by "Cₜ") of the indicated gene (gene name provided at top of each graph) and 5-year recurrence rate (RR) of breast cancer in a treatment group receiving an anthracycline and a cyclophosphamide (AC prediction curve; smooth line) and the relationship between expression of the indicated gene and RR in a treatment group receiving anthracycline and a taxane (AT prediction curve; hatched line), where the patients in the treatment groups had hormone receptor negative (HR⁻) breast. A horizontal dashed line in each graph represents the overall (i.e., not gene expression-specific) 5-year RR in patients in the study having HR⁻ breast cancer who were randomized to treatment with either AC or AT

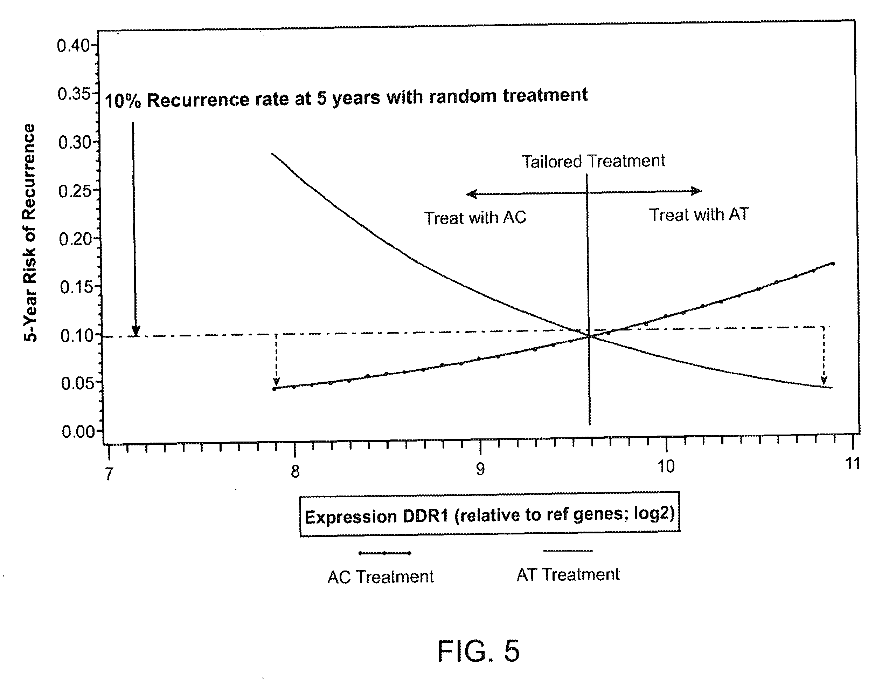

Figure 5 is a graph illustrating the impact of using DDR1 to select HR-positive patients for treatment with AC vs AT. The dotted line depicts the relationship between normalized expression of DDR1 and the 5-year recurrence rate (RR) of breast cancer in the AC treatment group (the AC prediction curve, also referred to as the cyclophosphamide benefit (CB) curve); the solid line depicts the relationship between normalized expression of DDR1 and the 5-year recurrence rate (RR) of breast cancer in the AT treatment group (the AT prediction curve, also referred to as the taxane benefit (TB) curve. Expression is provided on the x-axis as a normalized DDR1 expression level (relative to reference genes; log2). The y-axis provides the risk of cancer recurrence at 5 years.

The following Appendices and Tables are provided in the specification just prior to the claims.

Appendix 1. RT-PCR probe and primer sequences

Appendix 2. RT-PCR amplicon sequences

Table 1.Differential Markers of Response Identified in Breast Cancer Patients, All Patients.

Table 2. Differential Markers of Response Identified in Breast Cancer Patients, HR-Positive Patients

Table 3. Differential Markers of Response Identified in Breast Cancer Patients, HR-Positive Patients, RS > 18

Table 4. Differential Markers of Response Identified in Breast Cancer Patients, HR-Negative Patients.

Table 5. Additional genes involved in NFκB signaling

### DETAILED DESCRIPTION OF EXEMPLARY EMBODIMENTS

### DEFINITIONS

Unless defined otherwise, technical and scientific terms used herein have the same meaning as commonly understood by one of ordinary skill in the art to which this disclosure belongs. See, *e.g.,* Singleton P and Sainsbury D., Dictionary of Microbiology and Molecular Biology 3rd ed., J. Wiley & Sons, Chichester, New York, 2001.

As used herein, the term "anthracycline" refers to a class of antineoplastic antibiotics that are typically derived by *Streptomyces* bacteria (e.g., *Streptomyces peucetius* or *Streptomyces coeruleorubidus*). Although the precise mechanism of action is unknown, anthracyclines are believed to derive their chemotherapeutic activity, at least in part, from their ability to damage DNA by intercalation, metal ion chelation, and the generation of free radicals and can inhibit enzyme activity critical to DNA function. Examples of anthracyclines include daunorubicin, doxorubicin, epirubicin, idarubicin, amrubicin, pirarubicin, valrubicin, zorubicin, caminomycin, detorubicin, esorubicin, marcellomycin, quelamycin, rodorubicin, and aclarubicin, as well as pharmaceutically active salts, acids or derivatives of any of these.

As used herein, the term "taxane" refers to a family of antimitotic/antimicrotubule agents that inhibit cancer cell growth by stopping cell division. Examples of taxanes include paclitaxel, docetaxel, larotaxel, ortataxel, tesetaxel and other related diterpene compounds that have chemotherapeutic activity as well as pharmaceutically active salts, acids or derivatives of any of these. Paclitaxel was originally derived from the Pacific yew tree. Related diterpenes are produced by plants of the genus *Taxus* (yews) and synthetic or semi-synthetic taxanes with chemotherapeutic activity have also been synthesized, e.g., docetaxel, and are encompassed in the term taxane.

As used herein, the term "cyclophosphasmide" refers to a cytotoxic alkylating agent of the nitrogen mustard group, including the chemotherapeutic compound *N,N*-bis(2-chloroethyl)-1,3,2-oxazaphosphinan-2-amine 2-oxide (also known as cytophosphane). It is a highly toxic, immunosuppressive, antineoplastic drug, used in the treatment of Hodgkin's disease, lymphoma, and certain other forms of cancer, such as leukemia and breast cancer.

A "taxane-containing treatment" (also referred to as "taxane-containing regimen" or "taxane-containing treatment regimen") or "cyclophosphamide-containing treatment" (also referred to as "cyclophosphamide-containing regimen" or "cyclophosphamide-containing treatment regimen") is meant to encompass therapies in which a taxane or a cyclophosphamide, respectively, is administered alone or in combination with another therapeutic regimen (e.g., another chemotherapy (e.g., anthracycline), or both). Thus, a taxane-containing treatment can include, for example, administration a taxane in combination with anthracyline, with anthracyline and cyclosphophamide, and the like.

The term "in combination with" such as when used in reference to a therapeutic regimen, refers to administration or two or more therapies over the course of a treatment regimen, where the therapies may be administered together or separately, and, where used in reference to drugs, may be administered in the same or different formulations, by the same or different routes, and in the same or different dosage form type.

The term "prognosis" is used herein to refer to the prediction of the likelihood of cancer-attributable death or progression, including recurrence, of a neoplastic disease, such as breast cancer, in a patient. The concept of prognosis is used in the context of the minimal standard of care. For example, in the context of early stage, ER+ invasive breast care, the minimal standard of care could be surgery plus adjuvant hormonal therapy.

The term "prediction" is used herein to refer to a likelihood that a patient will have a particular clinical outcome following administration of a treatment regimen, e.g., a chemotherapeutic regimen. Clinical benefit may be measured, for example, in terms of clinical outcomes such as disease recurrence, tumor shrinkage, and/or disease progression.

The term "patient" or "subject" as used herein refers to a human patient.

The term "long-term" survival is used herein to refer to survival for at least 3 years, more preferably for at least 8 years, most preferably for at least 10 years following surgery or other treatment.

The term "tumor," as used herein, refers to all neoplastic cell growth and proliferation, whether malignant or benign, and all pre-cancerous and cancerous cells and tissues.

The terms "cancer" and "cancerous" refer to or describe the physiological condition in mammals that is typically characterized by unregulated cell growth.

The term "breast cancer" is used herein to include all forms and stages of breast cancer, including, without limitation, locally advanced breast cancer, invasive breast cancer, and metastatic breast cancer.

A "tumor sample" as used herein is a sample derived from, or containing tumor cells from, a patient's tumor. Examples of tumor samples herein include, but are not limited to, tumor biopsies, circulating tumor cells, circulating plasma proteins, ascitic fluid, primary cell cultures or cell lines derived from tumors or exhibiting tumor-like properties, as well as preserved tumor samples, such as formalin-fixed, paraffin-embedded tumor samples.

The "pathology" of cancer includes all phenomena that compromise the well-being of the patient. This includes, without limitation, abnormal or uncontrollable cell growth, metastasis, interference with the normal functioning of neighboring cells, release of cytokines or other secretory products at abnormal levels, suppression or aggravation of inflammatory or immunological response, neoplasia, premalignancy, malignancy, invasion of surrounding or distant tissues or organs, such as lymph nodes, etc.

As used herein, the term "expression level" as applied to a gene refers to the normalized level of a gene product, e.g. the normalized value determined for the RNA expression level of a gene or for the polypeptide expression level of a gene.

The term "Cₜ" as used herein refers to threshold cycle, the cycle number in quantitative polymerase chain reaction (qPCR) at which the fluorescence generated within a reaction well exceeds the defined threshold, i.e. the point during the reaction at which a sufficient number of amplicons have accumulated to meet the defined threshold.

The terms "threshold" or "thresholding" refer to a procedure used to account for non-linear relationships between gene expression measurements and clinical response as well as to further reduce variation in reported patient scores. When thresholding is applied, all measurements below or above a threshold are set to that threshold value. Non-linear relationship between gene expression and outcome could be examined using smoothers or cubic splines to model gene expression in Cox PH regression on recurrence free interval or logistic regression on recurrence status. Variation in reported patient scores could be examined as a function of variability in gene expression at the limit of quantitation and/or detection for a particular gene.

The term "gene product" or "expression product" are used herein to refer to the RNA transcription products (transcripts) of the gene, including mRNA, and the polypeptide translation products of such RNA transcripts. A gene product can be, for example, an unspliced RNA, an mRNA, a splice variant mRNA, a microRNA, a fragmented RNA, a polypeptide, a post-translationally modified polypeptide, a splice variant polypeptide, etc.

The term "RNA transcript" as used herein refers to the RNA transcription products of a gene, including, for example, mRNA, an unspliced RNA, a splice variant mRNA, a microRNA, and a fragmented RNA.

Unless indicated otherwise, each gene name used herein corresponds to the Official Symbol assigned to the gene and provided by Entrez Gene (URL: http://www.ncbi.nlm.nih.gov/sites/entrez) as of the filing date of this application.

The terms "correlated" and "associated" are used interchangeably herein to refer to a strength of association between two measurements (or measured entities). The disclosure provides genes and gene subsets, the expression levels of which are associated with a particular outcome measure, such as for example between the expression level of a gene and the likelihood of beneficial response to treatment with a drug. For example, the increased expression level of a gene may be positively correlated (positively associated) with an increased likelihood of good clinical outcome for the patient, such as an increased likelihood of long-term survival without recurrence of the cancer and/or beneficial response to a chemotherapy, and the like. Such a positive correlation may be demonstrated statistically in various ways, e.g. by a low hazard ratio. In another example, the increased expression level of a gene may be negatively correlated (negatively associated) with an increased likelihood of good clinical outcome for the patient. In that case, for example, the patient may have a decreased likelihood of long-term survival without recurrence of the cancer and/or beneficial response to a chemotherapy, and the like. Such a negative correlation indicates that the patient likely has a poor prognosis or will respond poorly to a chemotherapy, and this may be demonstrated statistically in various ways, e.g., a high hazard ratio.

A "positive clinical outcome" and "beneficial response" can be assessed using any endpoint indicating a benefit to the patient, including, without limitation, (1) inhibition, to some extent, of tumor growth, including slowing down and complete growth arrest; (2) reduction in the number of tumor cells; (3) reduction in tumor size; (4) inhibition (i.e., reduction, slowing down or complete stopping) of tumor cell infiltration into adjacent peripheral organs and/or tissues; (5) inhibition of metastasis; (6) enhancement of anti-tumor immune response, possibly resulting in regression or rejection of the tumor; (7) relief, to some extent, of one or more symptoms associated with the tumor; (8) increase in the length of survival following treatment; and/or (9) decreased mortality at a given point of time following treatment. Positive clinical response may also be expressed in terms of various measures of clinical outcome. Positive clinical outcome can also be considered in the context of an individual's outcome relative to an outcome of a population of patients having a comparable clinical diagnosis, and can be assessed using various endpoints such as an increase in the duration of Recurrence-Free interval (RFI), an increase in the time of survival as compared to Overall Survival (OS) in a population, an increase in the time of Disease-Free Survival (DFS), an increase in the duration of Distant Recurrence-Free Interval (DRFI), and the like. An increase in the likelihood of positive clinical response corresponds to a decrease in the likelihood of cancer recurrence.

The term "risk classification" means a level of risk (or likelihood) that a subject will experience a particular clinical outcome. A subject may be classified into a risk group or classified at a level of risk based on the methods of the present disclosure, e.g. high, medium, or low risk. A "risk group" is a group of subjects or individuals with a similar level of risk for a particular clinical outcome.

The term "normalized expression" with regard to a gene or an RNA transcript or other expression product (e.g., protein) is used to refer to the level of the transcript (or fragmented RNA) determined by normalization to the level of reference mRNAs, which might be all measured transcripts in the specimen or a particular reference set of mRNAs. A gene exhibits "increased expression" or "increased normalized expression" in a subpopulation of subjects when the normalized expression level of an RNA transcript (or its gene product) is higher in one clinically relevant subpopulation of patients (e.g., patients who are responsive to chemotherapy treatment) than in a related subpopulation (e.g., patients who are not responsive to said chemotherapy). In the context of an analysis of a normalized expression level of a gene in tissue obtained from an individual subject, a gene is exhibits "increased expression" when the normalized expression level of the gene trends toward or more closely approximates the normalized expression level characteristic of such a clinically relevant subpopulation of patients. Thus, for example, when the gene analyzed is a gene that shows increased expression in responsive subjects as compared to non-responsive subjects, then if the expression level of the gene in the patient sample trends toward a level of expression characteristic of a responsive subject, then the gene expression level supports a determination that the individual patient is likely to be a responder. Similarly, where the gene analyzed is a gene that is increased in expression in non-responsive patients as compared to responsive patients, then if the expression level of the gene in the patient sample trends toward a level of expression characteristic of a non-responsive subject, then the gene expression level supports a determination that the individual patient will be nonresponsive. Thus normalized expression of a given gene as disclosed herein can be described as being positively correlated with an increased likelihood of positive clinical response to chemotherapy or as being positively correlated with a decreased likelihood of a positive clinical response to chemotherapy.

The term "recurrence score" or "RS" refers to an algorithm-based indicator useful in determining the likelihood of an event of interest, such as a likelihood of cancer recurrence and/or the likelihood that a patient will respond to a treatment modality as may be assessed by cancer recurrence following therapy with the treatment modality.

The term "hormone receptor positive (HR+) tumor" means a tumor expressing either estrogen receptor (ER+) or progesterone receptor (PR+) above a certain threshold as determined by standard methods, including immunohistochemical staining of nuclei and polymerase chain reaction (PCR) in a biological sample obtained from a patient. The term "hormone receptor negative (HR-) tumor" means a tumor that does not express either estrogen receptor (ER-) or progesterone receptor (PR-) above a certain threshold. The threshold may be measured, for example, using an Allred score or gene expression. See, e.g., J. Harvey, et al., J Clin Oncol 17:1474-1481 (1999); S. Badve, et al., J Clin Oncol 26(15):2473-2481 (2008).

"Overall survival (OS)" refers to the patient remaining alive for a defined period of time, such as 1 year, 5 years, etc, e.g., from the time of diagnosis or treatment.

"Progression-free survival (PFS)" refers to the patient remaining alive, without the cancer getting worse.

"Neoadjuvant therapy" is adjunctive or adjuvant therapy given prior to the primary (main) therapy. Neoadjuvant therapy includes, for example, chemotherapy, radiation therapy, and hormone therapy. Thus, chemotherapy may be administered prior to surgery to shrink the tumor, so that surgery can be more effective, or, in the case of previously unoperable tumors, possible.

The term "polynucleotide," when used in singular or plural, generally refers to any polyribonucleotide or polydeoxribonucleotide, which may be unmodified RNA or DNA or modified RNA or DNA. Thus, for instance, polynucleotides as defined herein include, without limitation, single- and double-stranded DNA, DNA including single- and double-stranded regions, single- and double-stranded RNA, and RNA including single- and double-stranded regions, hybrid molecules comprising DNA and RNA that may be single-stranded or, more typically, double-stranded or include single- and double-stranded regions. In addition, the term "polynucleotide" as used herein refers to triple-stranded regions comprising RNA or DNA or both RNA and DNA. The strands in such regions may be from the same molecule or from different molecules. The regions may include all of one or more of the molecules, but more typically involve only a region of some of the molecules. One of the molecules of a triple-helical region often is an oligonucleotide. The term "polynucleotide" specifically includes cDNAs. The term includes DNAs (including cDNAs) and RNAs that contain one or more modified bases. Thus, DNAs or RNAs with backbones modified for stability or for other reasons are "polynucleotides" as that term is intended herein. Moreover, DNAs or RNAs comprising unusual bases, such as inosine, or modified bases, such as tritiated bases, are included within the term "polynucleotides" as defined herein. In general, the term "polynucleotide" embraces all chemically, enzymatically and/or metabolically modified forms of unmodified polynucleotides, as well as the chemical forms of DNA and RNA characteristic of viruses and cells, including simple and complex cells.

The term "oligonucleotide" refers to a relatively short polynucleotide, including, without limitation, single-stranded deoxyribonucleotides, single- or double-stranded ribonucleotides, RNA:DNA hybrids and double-stranded DNAs. Oligonucleotides, such as single-stranded DNA probe oligonucleotides, are often synthesized by chemical methods, for example using automated oligonucleotide synthesizers that are commercially available. However, oligonucleotides can be made by a variety of other methods, including in vitro recombinant DNA-mediated techniques and by expression of DNAs in cells and organisms.

"Stringency" of hybridization reactions is readily determinable by one of ordinary skill in the art, and generally is an empirical calculation dependent upon probe length, washing temperature, and salt concentration. In general, longer probes require higher temperatures for proper annealing, while shorter probes need lower temperatures. Hybridization generally depends on the ability of denatured DNA to reanneal when complementary strands are present in an environment below their melting temperature. The higher the degree of desired homology between the probe and hybridizable sequence, the higher the relative temperature which can be used. As a result, it follows that higher relative temperatures would tend to make the reaction conditions more stringent, while lower temperatures less so. For additional details and explanation of stringency of hybridization reactions, see Ausubel et al., Current Protocols in Molecular Biology, Wiley Interscience Publishers, (1995).

"Stringent conditions" or "high stringency conditions", as defined herein, typically: (1) employ low ionic strength and high temperature for washing, for example 0.015 M sodium chloride/0.0015 M sodium citrate/0.1% sodium dodecyl sulfate at 50 °C; (2) employ during hybridization a denaturing agent, such as formamide, for example, 50% (v/v) formamide with 0.1 % bovine serum albumin/0.1% Ficoll/0.1% polyvinylpyrrolidone/50 mM sodium phosphate buffer at pH 6.5 with 750 mM sodium chloride, 75 mM sodium citrate at 42 °C; or (3) employ 50% formamide, 5 x SSC (0.75 M NaCl, 0.075 M sodium citrate), 50 mM sodium phosphate (pH 6.8), 0.1% sodium pyrophosphate, 5 x Denhardt's solution, sonicated salmon sperm DNA (50 µg/ml), 0.1% SDS, and 10% dextran sulfate at 42 °C, with washes at 42 °C in 0.2 x SSC (sodium chloride/sodium citrate) and 50% formamide at 55 °C, followed by a high-stringency wash consisting of 0.1 x SSC containing EDTA at 55 °C.

"Moderately stringent conditions" may be identified as described by Sambrook et al., Molecular Cloning: A Laboratory Manual, New York: Cold Spring Harbor Press, 1989, and include the use of washing solution and hybridization conditions (e.g., temperature, ionic strength and % SDS) less stringent that those described above. An example of moderately stringent conditions is overnight incubation at 37 °C. in a solution comprising: 20% formamide, 5 x SSC (150 mM NaCl, 15 mM trisodium citrate), 50 mM sodium phosphate (pH 7.6), 5 x Denhardt's solution, 10% dextran sulfate, and 20 mg/ml denatured sheared salmon sperm DNA, followed by washing the filters in 1 x SSC at about 37-50 °C. The skilled artisan will recognize how to adjust the temperature, ionic strength, etc. as necessary to accommodate factors such as probe length and the like.

In the context of the present disclosure reference to "at least one," "at least two," "at least five," etc. of the genes listed in any particular gene set means any one or any and all combinations of the genes listed.

Herein, numerical ranges or amounts prefaced by the term "about" expressly include the exact range or exact numerical amount.

### GENERAL DESCRIPTION

The disclosed methods are useful to facilitate treatment decisions by providing an assessment of the likelihood of clinical benefit to a treatment that includes a taxane, a treatment that includes a cyclophosphamide, or both. Because taxanes and cyclophosphamide have different mechanisms of action, it is possible that tumors of certain patients exhibit molecular pathology that makes them more likely to respond to one drug type than the other. For example, the methods disclosed herein can be used to facilitate treatment decisions by providing an assessment of the likelihood of clinical benefit to an anthracycline-based treatment that includes a taxane, an anthracycline-based treatment that includes a cyclophosphamide, or an anthracycline-based treatment that includes both a cyclophosphamide and a taxane. Accordingly, such predictive methods are useful to facilitate chemotherapy treatment decisions that are tailored to individual patients. For example, the methods disclosed herein can be used to assess whether there is clinical benefit to addition of a taxane to a chemotherapeutic regimen.

Genes for which expression is correlated either positively or negatively with increased likelihood of response to a treatment that includes a taxane, a treatment that includes a cyclophosphamide, or both are provided in Figures 1-4 and Tables 1-4.

The relationships between expression level of a marker gene of the present disclosure and a positive or negative correlation with likelihood of recurrence of cancer (e.g., breast cancer) following treatment with a taxane-containing regimen or a cyclophosphamide-containing regimen are exemplified in Figures 1-4. The hatched line in each graph represents the relationship between expression of the gene in patients treated with a taxane-containing regimen (e.g., anthracycline plus a taxane) and the 5-year recurrence rate (RR) of cancer (the taxane benefit (TB) prediction curve). The TB prediction line thus represents the correlation of expression of the gene and the likelihood of clinical benefit of a taxane in a treatment regimen. The smooth line in each graph represents the relationship between expression of the gene in patients treated with a cyclophosphamide-containing regimen (e.g., anthracycline plus cyclophosphamide) and the 5-year recurrence rate (RR) of cancer (the cyclophosphamide benefit (CB) prediction curve). The CB prediction curve thus represents the correlation of expression of the gene and the likelihood of clinical benefit of a cyclophosphamide in a treatment regimen. Because the patients in the study also received an anthracycline, the TB prediction curve and CB prediction curve can also be considered an anthracycline plus a taxane (AT) benefit prediction curve and an anthracycline plus a cyclophosphamide (AC) benefit prediction curve, respectively.

Each of the graphs in Figures 1-4 include a horizontal dashed line that represents the overall (i.e., not gene expression-specific) recurrence rate at 5-years in the relevant population who were randomized to treatment with AC or AT. The difference between the TB and CB prediction curves and this horizontal line depicts the extent to which clinical benefit may be improved by a gene expression-guided treatment decision.

Other characteristics of the tumor can be taken into account when assessing likelihood of taxane and/or cyclophosphamide benefit by analysis of expression level of a marker gene disclosed herein. For example, hormone receptor expression status (e.g., ER⁺, ER⁻, PR⁺, PR⁻) can be assessed for the tumor sample, and taken into consideration when evaluating expression levels of the marker gene, e.g., the expression level is compared to expression level correlations to TB and/or CB in a population sharing the same characteristics. For example, Figure 1 provides TB (AT) and CB (AC) prediction curves in all patients in the study discussed in the Examples below without regard to hormone expression status or likelihood of cancer recurrence as predicted by the Oncotype DX RS. Figure 2 provides TB (AT) and CB (AC) prediction curves in hormone receptor positive patients. Figure 3 provides TB (AT) and CB (AC) prediction curves in hormone receptor positive patients having an Oncotype DX RS score of about 18 or greater, which indicates a significant risk of cancer recurrence within 10 years following surgery and tamoxifen therapy. Figure 4 provides TB (AT) and CB (AC) prediction curves in hormone receptor negative patients.

The prediction curves can be used to assess information provided by an expression level of a marker gene disclosed herein and in turn facilitate a treatment decision with respect to selection of a taxane-containing and/or a cyclophosphamide-containing regimen. For example, where a gene exhibits an expression level having a TB (AT) prediction curve having a negative slope as exemplified in Figures 1-4, then increasing normalized expression levels of the gene are positively correlated with a likelihood of clinical benefit of including a taxane in the treatment regimen (since patients who exhibited this expression pattern of the particular gene had lower recurrence rates following a taxane-containing regimen). Conversely, where a gene exhibits an expression level having a TB (AT) prediction curve having a positive slope as exemplified in Figures 1-4, then increasing normalized expression levels of the gene are negatively correlated with a likelihood of clinical benefit of including a taxane in the treatment regimen. Similarly, where a gene exhibits an expression level having a CB (AC) prediction curve having a negative slope as exemplified in Figures 1-4, then increasing normalized expression levels of the gene are positively correlated with a likelihood of clinical benefit of including a cyclophosphamide in the treatment regimen (since patients who exhibited this expression pattern of the particular gene had lower recurrence rates following cyclophosphamide-containing regimen). Conversely, where a gene exhibits an expression level having a CB (AC) prediction curve having a positive slope as exemplified in Figures 1-4, then increasing normalized expression levels of the gene are negatively correlated with a likelihood of clinical benefit of including a cyclophosphamide in the treatment regimen.

Accordingly, the expression levels of the marker genes can be used to facilitate a decision as to whether a taxane should be included or excluded in a treatment regimen, and to facilitate a decision as to whether a cyclophosphamide should be included or excluded in a treatment regimen. The marker genes can be used to facilitate selection of a treatment regimen that includes, a taxane and/or a cyclophosphamide, or neither a taxane nor a cyclophosphamide.

In some instances the marker gene expression level may suggest clinical benefit for both a taxane and a cyclophosphamide, e.g., where increasing expression levels are associated with a recurrence risk below a selected recurrence risk. For example, as illustrated in Fig. 2 for the gene ZW10, increased expression of ZW10 in HR-positive cancer patients is associated with increased likelihood of clinical benefit for both a taxane and for a cyclophosphamide. In addition, because the magnitudes of the slopes are significantly different, patients with increased expression of ZW 10 are predicted to have lower risks of recurrence if treated with AT instead of AC, and patients with decreased expression of ZW10 are predicted to have lower risks of recurrence if treated with AC instead of AT. Thus, the marker genes that are associated with TB (AT) and CT (AC) prediction curves that differ in slope can facilitate a decision in selecting between a taxane-containing regimen and a cyclophosphamide-containing regimen, even where there may be clinical benefit with either or both treatment regimen.

The methods of the present disclosure also can facilitate selection between a taxane-containing regimen and a cyclophosphamide-containing regimen (e.g., between and AT and AC therapy). For example, where the curves in Figures 1-4 have significantly different slopes in the Cox regression model and the TB (AT) and CB (AC) prediction curves cross, expression levels of the marker gene can be used to assess the likelihood the patient will respond to a taxane-containing regimen (e.g., AT) or to a cyclophosphamide-containing regimen (e.g., AC).

For example, Fig. 5 illustrates a plot of the 5-year risk of relapse versus gene expression, presented for an exemplary gene, DDR1. As illustrated in Fig. 5, the expression level of DDR1 can be used to facilitate selection of therapy where treatment with a cyclophosphamide is favored over treatment with a taxane at lower expression levels of DDR1, with a "switch" of the relative clinical benefit of these therapies occurring at a point where the recurrence risk associated with taxane treatment is lower than that associated with cyclophosphamide treatment, thus favoring a treatment regimen including a taxane over a cyclophosphamide.

There are many types of systemic treatment regimens available for patients diagnosed with cancer. For example, the table below lists various chemotherapeutic and hormonal therapies for breast cancer.

**Single Agents Useful in Breast Cancer**

| GENERIC NAME | COMMON TRADE NAME | CLASS |
|---|---|---|
| Cyclophosphamide (C) | Cytoxan® | Nitrogen mustards |
| Doxorubicin | Adriamycin® | Anthracyclines |
| Epirubicin | Pharmorubicin® | Anthracyclines |
| Fluorouracil | | Pyrimidine analogs |
| Methotrexate | Rheumatrex® | Folic acid analogs |
| Paclitaxel | Taxol® | Taxanes (T) |
| Docetaxel | Taxotere® | Taxanes (T) |
| Capecitabine | Xeloda® | Pyrimidine analogs |
| Trastuzumab | Herceptin® | Monoclonal Antibodies |
| Bevacizumab | Avastin® | Monoclonal Antibodies |

**Combinations Useful in Breast Cancer**

| | | |
|---|---|---|
| CAF | Cyclophosphamide, Adriamycin, Fluorouracil | US |
| CMF | Cyclophosphamide, Methotrexate, Fluorouracil | US |
| AC | Adriamycin, Cyclophosphamide | US |
| AT | Adriamycin, Taxane | US |
| ACT | Adriamycin, Cyclophosphamide, Taxane | US |
| TAC | Taxane, Adriamycin, Cyclophosphamide | US |
| TC | Taxane, Cyclophosphamide | US |
| | Fluorouracil, Epirubicin, Cyclophosphamide | Europe |

### GENE EXPRESSION PROFILING

The practice of the methods and compositions of the present disclosure will employ, unless otherwise indicated, conventional techniques of molecular biology (including recombinant techniques), microbiology, cell biology, and biochemistry, which are within the skill of the art. Such techniques are explained fully in the literature, such as, "Molecular Cloning: A Laboratory Manual", 2nd edition (Sambrook et al., 1989); "Oligonucleotide Synthesis" (M.J. Gait, ed., 1984); "Animal Cell Culture" (R.I. Freshney, ed., 1987); "Methods in Enzymology" (Academic Press, Inc.); "Handbook of Experimental Immunology", 4th edition (D.M. Weir & C.C. Blackwell, eds., Blackwell Science Inc., 1987); "Gene Transfer Vectors for Mammalian Cells" (J.M. Miller & M.P. Calos, eds., 1987); "Current Protocols in Molecular Biology" (F.M. Ausubel et al., eds., 1987); and "PCR: The Polymerase Chain Reaction", (Mullis et al., eds., 1994).

Methods of gene expression profiling include methods based on hybridization analysis of polynucleotides, methods based on sequencing of polynucleotides, and proteomics-based methods. Exemplary methods known in the art for the quantification of mRNA expression in a sample include northern blotting and in situ hybridization (Parker & Barnes, Methods in Molecular Biology 106:247-283 (1999)); RNAse protection assays (Hod, Biotechniques 13:852-854 (1992)); and PCR-based methods, such as reverse transcription PCT (RT-PCR) (Weis et al., Trends in Genetics 8:263-264 (1992)). Antibodies may be employed that can recognize sequence-specific duplexes, including DNA duplexes, RNA duplexes, and DNA-RNA hybrid duplexes or DNA-protein duplexes. Representative methods for nucleic acid sequencing analysis include Serial Analysis of Gene Expression (SAGE), and Digital Gene Expression (DGE).

Representative methods of gene expression profiling are disclosed, for example, in U.S. Patent Nos. 7,056,674 and 7,081,340, and in U.S. Patent Publication Nos. 20020095585; 20050095634; 20050260646; and 20060008809. Representative scientific publications including methods of gene expression profiling, including data analysis, include Gianni et al., J Clin Oncol. 2005 Oct 10;23(29):7265-77; Paik et al., N Engl J Med. 2004 Dec 30;351(27):2817-26; and Cronin et al., Am J Pathol. 2004 Jan;164(1):35-42.

### Reverse Transcriptase PCR (RT-PCR)

Typically, mRNA is isolated from a test sample. The starting material is typically total RNA isolated from a human tumor, usually from a primary tumor. Optionally, normal tissues from the same patient can be used as an internal control. mRNA can be extracted from a tissue sample, e.g., from a sample that is fresh, frozen (e.g. fresh frozen), or paraffin-embedded and fixed (e.g. formalin-fixed).

General methods for mRNA extraction are well known in the art and are disclosed in standard textbooks of molecular biology, including Ausubel et al., Current Protocols of Molecular Biology, John Wiley and Sons (1997). Methods for RNA extraction from paraffin embedded tissues are disclosed, for example, in Rupp and Locker, Lab Invest. 56:A67 (1987), and De Andrés et al., BioTechniques 18:42044 (1995). In particular, RNA isolation can be performed using a purification kit, buffer set and protease from commercial manufacturers, such as Qiagen, according to the manufacturer's instructions. For example, total RNA from cells in culture can be isolated using Qiagen RNeasy mini-columns. Other commercially available RNA isolation kits include MasterPure™ Complete DNA and RNA Purification Kit (EPICENTRE®, Madison, WI), and Paraffin Block RNA Isolation Kit (Ambion, Inc.). Total RNA from tissue samples can be isolated using RNA Stat-60 (Tel-Test). RNA prepared from tumor can be isolated, for example, by cesium chloride density gradient centrifugation.

The sample containing the RNA is then subjected to reverse transcription to produce cDNA from the RNA template, followed by exponential amplification in a PCR reaction. The two most commonly used reverse transcriptase enzymes are avilo myeloblastosis virus reverse transcriptase (AMV-RT) and Moloney murine leukemia virus reverse transcriptase (MMLV-RT). The reverse transcription step is typically primed using specific primers, random hexamers, or oligo-dT primers, depending on the circumstances and the goal of expression profiling. For example, extracted RNA can be reverse-transcribed using a GeneAmp RNA PCR kit (Perkin Elmer, CA, USA), following the manufacturer's instructions. The derived cDNA can then be used as a template in the subsequent PCR reaction.

PCR-based methods use a thermostable DNA-dependent DNA polymerase, such as a Taq DNA polymerase. For example, TaqMan® PCR typically utilizes the 5'-nuclease activity of Taq or Tth polymerase to hydrolyze a hybridization probe bound to its target amplicon, but any enzyme with equivalent 5' nuclease activity can be used. Two oligonucleotide primers are used to generate an amplicon typical of a PCR reaction product. A third oligonucleotide, or probe, can be designed to facilitate detection of a nucleotide sequence of the amplicon located between the hybridization sites the two PCR primers. The probe can be detectably labeled, e.g., with a reporter dye, and can further be provided with both a fluorescent dye, and a quencher fluorescent dye, as in a Taqman® probe configuration. Where a Taqman® probe is used, during the amplification reaction, the Taq DNA polymerase enzyme cleaves the probe in a template-dependent manner. The resultant probe fragments disassociate in solution, and signal from the released reporter dye is free from the quenching effect of the second fluorophore. One molecule of reporter dye is liberated for each new molecule synthesized, and detection of the unquenched reporter dye provides the basis for quantitative interpretation of the data.

TaqMan® RT-PCR can be performed using commercially available equipment, such as, for example, ABI PRISM 7700™ Sequence Detection System™ (Perkin-Elmer-Applied Biosystems, Foster City, CA, USA), or Lightcycler (Roche Molecular Biochemicals, Mannheim, Germany). In a preferred embodiment, the 5' nuclease procedure is run on a real-time quantitative PCR device such as the ABI PRISM 7700™ Sequence Detection System™. The system consists of a thermocycler, laser, charge-coupled device (CCD), camera and computer. The system amplifies samples in a 96-well format on a thermocycler. During amplification, laser-induced fluorescent signal is collected in real-time through fiber optics cables for all 96 wells, and detected at the CCD. The system includes software for running the instrument and for analyzing the data.

5'-Nuclease assay data are initially expressed as a threshold cycle ("Cₜ"). Fluorescence values are recorded during every cycle and represent the amount of product amplified to that point in the amplification reaction. The threshold cycle (Cₜ) is generally described as the point when the fluorescent signal is first recorded as statistically significant.

It is desirable to correct for (normalize away) both differences in the amount of RNA assayed and variability in the quality of the RNA used. Therefore, the assay typically measures, and expression analysis of a marker gene incorporates analysis of, the expression of certain reference genes (or "normalizing genes"), including well known housekeeping genes, such as GAPDH. Alternatively, normalization can be based on the mean or median signal (Ct) of all of the assayed genes or a large subset thereof (often referred to as a "global normalization" approach). On a gene-by-gene basis, measured normalized amount of a patient tumor mRNA may be compared to the amount found in a colon cancer tissue reference set. See M. Cronin, et al., Am. Soc. Investigative Pathology 164:35-42 (2004).

Gene expression measurements can be normalized relative to the mean of one or more (e.g., 2, 3, 4, 5, or more) reference genes. Reference-normalized expression measurements can range from 0 to 15, where a one unit increase generally reflects a 2-fold increase in RNA quantity.

RT- PCR is compatible both with quantitative competitive PCR, where internal competitor for each target sequence is used for normalization, and with quantitative comparative PCR using a normalization gene contained within the sample, or a housekeeping gene for RT-PCR. For further details see, e.g. Held et al., Genome Research 6:986-994 (1996).

The steps of a representative protocol for use in the methods of the present disclosure use fixed, paraffin-embedded tissues as the RNA source mRNA isolation, purification, primer extension and amplification can be preformed according to methods available in the art. (see, e.g., Godfrey et al. J. Molec. Diagnostics 2: 84-91 (2000); Specht et al., Am. J. Pathol. 158: 419-29 (2001)). Briefly, a representative process starts with cutting about 10 µm thick sections of paraffin-embedded tumor tissue samples. The RNA is then extracted, and protein and DNA depleted from the RNA-containing sample. After analysis of the RNA concentration, RNA is reverse transcribed using gene specific primers followed by RT-PCR to provide for cDNA amplification products.

### Design of Intron-Based PCR Primers and Probes

PCR primers and probes can be designed based upon exon or intron sequences present in the mRNA transcript of the gene of interest. Primer/probe design can be performed using publicly available software, such as the DNA BLAT software developed by Kent, W.J., Genome Res. 12(4):656-64 (2002), or by the BLAST software including its variations.

Where necessary or desired, repetitive sequences of the target sequence can be masked to mitigate non-specific signals. Exemplary tools to accomplish this include the Repeat Masker program available on-line through the Baylor College of Medicine, which screens DNA sequences against a library of repetitive elements and returns a query sequence in which the repetitive elements are masked. The masked intron sequences can then be used to design primer and probe sequences using any commercially or otherwise publicly available primer/probe design packages, such as Primer Express (Applied Biosystems); MGB assay-by-design (Applied Biosystems); Primer3 (Steve Rozen and Helen J. Skaletsky (2000) Primer3 on the WWW for general users and for biologist programmers. In: Rrawetz et al. (eds.) Bioinformatics Methods and Protocols: Methods in Molecular Biology. Humana Press, Totowa, NJ, pp 365-386).

Other factors that can influence PCR primer design include primer length, melting temperature (Tm), and G/C content, specificity, complementary primer sequences, and 3'-end sequence. In general, optimal PCR primers are generally 17-30 bases in length, and contain about 20-80%, such as, for example, about 50-60% G+C bases, and exhibit Tm's between 50 and 80 °C, e.g. about 50 to 70 °C.

For further guidelines for PCR primer and probe design see, e.g. Dieffenbach, CW. et al, "General Concepts for PCR Primer Design" in: PCR Primer, A Laboratory Manual, Cold Spring Harbor Laboratory Press, New York, 1995, pp. 133-155; Innis and Gelfand, "Optimization of PCRs" in: PCR Protocols, A Guide to Methods and Applications, CRC Press, London, 1994, pp. 5-11; and Plasterer, T.N. Primerselect: Primer and probe design. Methods Mol. Biol. 70:520-527 (1997).

### Quantitative PCR for Gene Expression Analysis

Per VanGuilder et al., BioTechniques 44: 619 (2008), quantitative PCR (qPCR) now represents the method of choice for analyzing gene expression of numerous genes in anywhere from a small number to thousands of samples. For investigators studying gene expression, there is a multitiered technological approach depending on the number of genes and samples being examined. Gene expression microarrays are still the preferred method for large-scale (e.g., whole-genome) discovery experiments. Due to the logistics, sensitivity, and costs of whole-genome micorarrays, there is also a niche for focused microarrays that allow for analysis of a smaller number of genes in a larger number of samples. Nonetheless, for validation of microarray discovery, reverse-transcription quantitative PCR (RT-qPCR) remains the gold standard. The current maturation of real-time qPCR with fluorescent probes allows for rapid and easy confirmation of microarray results in a large number of samples. Often, a whole-genome discovery experiment is not required, as the gene or pathway of interest is already known. In that case, the data collection can begin with qPCR. Finally, qPCR has also shown great utility in biomarker monitoring. In this scenario, previously developed identified targets can be assayed in very large numbers of samples (1000s).

Data Analysis. Analysis of real-time qPCR data has also reached a mature stage of development. Analyses can be either of absolute levels (i.e., numbers of copies of a specific RNA per sample) or relative levels (i.e., sample 1 has twice as much mRNA of a specific gene as sample 2). By far, the majority of analyses use relative quantitation as this is easier to measure and is of primary interest to researchers examining disease states. For absolute quantitation, an RNA standard curve of the gene of interest is required in order to calculate the number of copies. In this case, a serial dilution of a known amount (number of copies) of pure RNA is diluted and subjected to amplification. Like a protein assay, the unknown signal is compared with the curve so as to extrapolate the starting concentration.

The most common method for relative quantitation is the 2^{-ΔΔCT} method. This method relies on two assumptions. The first is that the reaction is occurring with 100% efficiency; in other words, with each cycle of PCR, the amount of product doubles. This can be ascertained through simple experiments as described in the scientific literature. This assumption is also one of the reasons for using a low cycle number when the reaction is still in the exponential phase. In the initial exponential phase of PCR, substrates are not limiting and there is no degradation of products. In practice, this requires setting the crossing threshold or cycle threshold (Cₜ) at the earliest cycle possible. The Cₜ is the number of cycles that it takes each reaction to reach an arbitrary amount of fluorescence. The second assumption of the 2^{-ΔΔCT} method is that there is a gene (or genes) that is expressed at a constant level between the samples. This endogenous control will be used to correct for any difference in sample loading.

Once the Cₜ value is collected for each reaction, it can be used to generate a relative expression level. One 2^{-ΔΔCT} method is now described. In this example, there are two samples (Control and Treated) and we have measured the levels of (i) a gene of interest (Target Gene (TG)) and (ii) an endogenous control gene (Control Gene (CG)). For each sample, the difference in Cₜ values for the gene of interest and the endogenous control is calculated (the ΔCₜ). Next, subtraction of the control-condition ΔCₜ from the treated-condition ΔCₜ yields the ΔΔCₜ. The negative value of this subtraction, the -ΔΔCₜ, is used as the exponent of 2 in the equation and represents the difference in "corrected" number of cycles to threshold. The exponent conversion comes from the fact that the reaction doubles the amount of product per cycle. For example, if the control sample ΔCₜ is 2 and the treated sample ΔCₜ is 4, computing the 2^{-ΔΔCT} (which becomes 2 ⁻⁽⁴⁻²⁾) yields 0.25. This value is often referred to as the RQ, or relative quantity value. This means that the level of the gene of interest in the treated sample is only 25% of the level of that gene in the control sample. This becomes evident because the treated sample took two more cycles of PCR to reach the same amount of product as the control sample and therefore there was less of that cDNA to begin with in the treated sample. The 2^{-ΔΔCT} method is the most common quantitation strategy, but it should be noted that there are other valid methods for analyzing qPCR Cₜ values. Several investigators have proposed alternative analysis methods.

### MassARRAY® System

In MassARRAY-based methods, such as the exemplary method developed by Sequenom, Inc. (San Diego, CA) following the isolation of RNA and reverse transcription, the obtained cDNA is spiked with a synthetic DNA molecule (competitor), which matches the targeted cDNA region in all positions, except a single base, and serves as an internal standard. The cDNA/competitor mixture is PCR amplified and is subjected to a post-PCR shrimp alkaline phosphatase (SAP) enzyme treatment, which results in the dephosphorylation of the remaining nucleotides. After inactivation of the alkaline phosphatase, the PCR products from the competitor and cDNA are subjected to primer extension, which generates distinct mass signals for the competitor- and cDNA-derives PCR products. After purification, these products are dispensed on a chip array, which is pre-loaded with components needed for analysis with matrix-assisted laser desorption ionization time-of-flight mass spectrometry (MALDI-TOF MS) analysis. The cDNA present in the reaction is then quantified by analyzing the ratios of the peak areas in the mass spectrum generated. For further details see, e.g. Ding and Cantor, Proc. Natl. Acad. Sci. USA 100:3059-3064 (2003).

### Other PCR-based Methods

Further PCR-based techniques that can find use in the methods disclosed herein include, for example, BeadArray® technology (Illumina, San Diego, CA; Oliphant et al., Discovery of Markers for Disease (Supplement to Biotechniques), June 2002; Ferguson et al., Analytical Chemistry 72:5618 (2000)); BeadsArray for Detection of Gene Expression® (BADGE), using the commercially available Luminex 100 LabMAP® system and multiple color-coded microspheres (Luminex Corp., Austin, TX) in a rapid assay for gene expression (Yang et al., Genome Res. 11:1888-1898 (2001)); and high coverage expression profiling (HiCEP) analysis (Fukumura et al., Nucl. Acids. Res. 31(16) e94 (2003).

### Microarrays

Expression levels of a gene of interest can also be assessed using the microarray technique. In this method, polynucleotide sequences of interest (including cDNAs and oligonucleotides) are arrayed on a substrate. The arrayed sequences are then contacted under conditions suitable for specific hybridization with detectably labeled cDNA generated from mRNA of a test sample. As in the RT-PCR method, the source of mRNA typically is total RNA isolated from a tumor sample, and optionally from normal tissue of the same patient as an internal control or cell lines. mRNA can be extracted, for example, from frozen or archived paraffin-embedded and fixed (e.g. formalin-fixed) tissue samples.

For example, PCR amplified inserts of cDNA clones of a gene to be assayed are applied to a substrate in a dense array. Usually at least 10,000 nucleotide sequences are applied to the substrate. For example, the microarrayed genes, immobilized on the microchip at 10,000 elements each, are suitable for hybridization under stringent conditions. Fluorescently labeled cDNA probes may be generated through incorporation of fluorescent nucleotides by reverse transcription of RNA extracted from tissues of interest. Labeled cDNA probes applied to the chip hybridize with specificity to each spot of DNA on the array. After washing under stringent conditions to remove non-specifically bound probes, the chip is scanned by confocal laser microscopy or by another detection method, such as a CCD camera. Quantitation of hybridization of each arrayed element allows for assessment of corresponding mRNA abundance.

With dual color fluorescence, separately labeled cDNA probes generated from two sources of RNA are hybridized pair wise to the array. The relative abundance of the transcripts from the two sources corresponding to each specified gene is thus determined simultaneously. The miniaturized scale of the hybridization affords a convenient and rapid evaluation of the expression pattern for large numbers of genes. Such methods have been shown to have the sensitivity required to detect rare transcripts, which are expressed at a few copies per cell, and to reproducibly detect at least approximately two-fold differences in the expression levels (Schena et at, Proc. Natl. Acad. Sci. USA 93(2):106-149 (1996)). Microarray analysis can be performed by commercially available equipment, following manufacturer's protocols, such as by using the Affymetrix GenChip® technology.

### Serial Analysis of Gene Expression (SAGE)

Serial analysis of gene expression (SAGE) is a method that allows the simultaneous and quantitative analysis of a large number of gene transcripts, without the need of providing an individual hybridization probe for each transcript. First, a short sequence tag (about 10-14 bp) is generated that contains sufficient information to uniquely identify a transcript, provided that the tag is obtained from a unique position within each transcript. Then, many transcripts are linked together to form long serial molecules, that can be sequenced, revealing the identity of the multiple tags simultaneously. The expression pattern of any population of transcripts can be quantitatively evaluated by determining the abundance of individual tags, and identifying the gene corresponding to each tag. For more details see, e.g. Velculescu et al., Science 270:484-487 (1995); and Velculescu et al., Cell 88:243-51 (1997).

### Gene Expression Analysis by Nucleic Acid Sequencing

Nucleic acid sequencing technologies are suitable methods for analysis of gene expression. The principle underlying these methods is that the number of times a cDNA sequence is detected in a sample is directly related to the relative expression of the mRNA corresponding to that sequence. These methods are sometimes referred to by the term Digital Gene Expression (DGE) to reflect the discrete numeric property of the resulting data. Early methods applying this principle were Serial Analysis of Gene Expression (SAGE) and Massively Parallel Signature Sequencing (MPSS). See, e.g., S. Brenner, et al., Nature Biotechnology 18(6):630-634 (2000). More recently, the advent of "next-generation" sequencing technologies has made DGE simpler, higher throughput, and more affordable. As a result, more laboratories are able to utilize DGE to screen the expression of more genes in more individual patient samples than previously possible. See, e.g., J. Marioni, Genome Research 18(9):1509-1517 (2008); R. Morin, Genome Research 18(4):610-621 (2008); A. Mortazavi, Nature Methods 5(7):621-628 (2008); N. Cloonan, Nature Methods 5(7):613-619 (2008).

### Isolating RNA from Body Fluids

Methods of isolating RNA for expression analysis from tissue (e.g., breast tissue), blood, plasma and serum (See for example, Tsui NB et al. (2002) 48,1647-53 and references cited therein) and from urine (See for example, Boom R et al. (1990) J Clin Microbiol. 28, 495-503 and reference cited therein) have been described.

### Immunonological methods

Immunological methods (e.g., immunohistochemistry methods)are also suitable for detecting the expression levels of genes and applied to the method disclosed herein. Antibodies (e.g., monoclonal antibodies) that specifically bind a gene product of a gene of interest can be used in such methods. The antibodies can be detected by direct labeling of the antibodies themselves, for example, with radioactive labels, fluorescent labels, haptene labels such as, biotin, or an enzyme such as horse radish peroxidase or alkaline phosphatase. Alternatively, unlabeled primary antibody can be used in conjunction with a labeled secondary antibody specific for the primary antibody. Immunological methods protocols and kits are well known in the art and are commercially available.

### Proteomics

The term "proteome" is defined as the totality of the proteins present in a sample (e.g. tissue, organism, or cell culture) at a certain point of time. Proteomics includes, among other things, study of the global changes of protein expression in a sample (also referred to as "expression proteomics"). Proteomics typically includes the following steps: (1) separation of individual proteins in a sample by 2-D gel electrophoresis (2-D PAGE); (2) identification of the individual proteins recovered from the gel, e.g. my mass spectrometry or N- terminal sequencing, and (3) analysis of the data using bioinformatics.

### GENERAL DESCRIPTION OF EXEMPLARY PROTOCOL

The steps of a representative protocol for profiling gene expression using fixed, paraffin-embedded tissues as the RNA source, including mRNA isolation, purification, primer extension and amplification are provided in various published journal articles. (See, e.g., T.E. Godfrey et al,. J. Molec. Diagnostics 2: 84-91 (2000); K. Specht et al., Am. J. Pathol. 158: 419-29 (2001), M. Cronin, et al., Am J Pathol 164:35-42 (2004)). Briefly, a representative process starts with cutting a tissue sample section (e.g. about 10 µm thick sections of a paraffin-embedded tumor tissue sample). The RNA is then extracted, and protein and DNA are removed. After analysis of the RNA concentration, RNA repair is performed if desired. The sample can then be subjected to analysis, e.g., by reverse transcribed using gene specific promoters followed by RT-PCR.

### KITS

The materials for use in the methods of the present disclosure are suited for preparation of kits produced in accordance with well known procedures. The present disclosure thus provides kits comprising agents, which may include gene-specific or gene-selective probes and/or primers, for quantitating the expression of the disclosed genes for predicting clinical outcome or response to treatment. Such kits may optionally contain reagents for the extraction of RNA from tumor samples, in particular fixed paraffin-embedded tissue samples and/or reagents for RNA amplification. In addition, the kits may optionally comprise the reagent(s) with an identifying description or label or instructions relating to their use in the methods of the present disclosure. The kits may comprise containers (including microtiter plates suitable for use in an automated implementation of the method), each with one or more of the various reagents (typically in concentrated form) utilized in the methods, including, for example, pre-fabricated microarrays, buffers, the appropriate nucleotide triphosphates (e.g., dATP, dCTP, dGTP and dTTP; or rATP, rCTP, rGTP and UTP), reverse transcriptase, DNA polymerase, RNA polymerase, and one or more probes and primers of the present disclosure (e.g., appropriate length poly(T) or random primers linked to a promoter reactive with the RNA polymerase). Mathematical algorithms used to estimate or quantify prognostic and/or predictive information are also properly potential components of kits.

The methods provided by the present disclosure may also be automated in whole or in part.

### REPORTS

The methods of the present disclosure are suited for the preparation of reports summarizing the predictions resulting from the methods of the present disclosure. A "report," as described herein, is an electronic or tangible document which includes report elements that provide information of interest relating to a likelihood assessment and its results. A subject report includes at least a likelihood assessment, e.g., an indication as to the likelihood that a cancer patient will exhibit a beneficial clinical response to a treatment regimen of interest. A subject report can be completely or partially electronically generated, e.g., presented on an electronic display (e.g., computer monitor). A report can further include one or more of: 1) information regarding the testing facility; 2) service provider information; 3) patient data; 4) sample data; 5) an interpretive report, which can include various information including: a) indication; b) test data, where test data can include a normalized level of one or more genes of interest, and 6) other features.

The present disclosure thus provides for methods of creating reports and the reports resulting therefrom. The report may include a summary of the expression levels of the RNA transcripts, or the expression products of such RNA transcripts, for certain genes in the cells obtained from the patients tumor tissue. The report may include a prediction that said subject has an increased likelihood of response to treatment with a particular chemotherapy or the report may include a prediction that the subject has a decreased likelihood of response to the chemotherapy. The report may include a recommendation for treatment modality such as surgery alone or surgery in combination with chemotherapy. The report may be presented in electronic format or on paper.

Thus, in some embodiments, the methods of the present disclosure further includes generating a report that includes information regarding the patient's likelihood of response to chemotherapy, particularly a therapy including cyclophophamide and/or a taxane. For example, the methods disclosed herein can further include a step of generating or outputting a report providing the results of a subject response likelihood assessment, which report can be provided in the form of an electronic medium (e.g., an electronic display on a computer monitor), or in the form of a tangible medium (e.g., a report printed on paper or other tangible medium).

A report that includes information regarding the likelihood that a patient will respond to treatment with chemotherapy, particularly a including cyclophophamide and/or a taxane, is provided to a user. An assessment as to the likelihood that a cancer patient will respond to treatment with chemotherapy, or predicted comparative response to two therapy options, is referred to below as a "response likelihood assessment" or, simply, "likelihood assessment." A person or entity who prepares a report ("report generator") will also perform the likelihood assessment. The report generator may also perform one or more of sample gathering, sample processing, and data generation, e.g., the report generator may also perform one or more of: a) sample gathering; b) sample processing; c) measuring a level of an indicator response gene product(s); d) measuring a level of a reference gene product(s); and e) determining a normalized level of a response indicator gene product(s). Alternatively, an entity other than the report generator can perform one or more sample gathering, sample processing, and data generation.

For clarity, it should be noted that the term "user," which is used interchangeably with "client," is meant to refer to a person or entity to whom a report is transmitted, and may be the same person or entity who does one or more of the following: a) collects a sample; b) processes a sample; c) provides a sample or a processed sample; and d) generates data (e.g., level of a response indicator gene product(s); level of a reference gene product(s); normalized level of a response indicator gene product(s)) for use in the likelihood assessment. In some cases, the person(s) or entity(ies) who provides sample collection and/or sample processing and/or data generation, and the person who receives the results and/or report may be different persons, but are both referred to as "users" or "clients" herein to avoid confusion. In certain embodiments, e.g., where the methods are completely executed on a single computer, the user or client provides for data input and review of data output. A "user" can be a health professional (e.g., a clinician, a laboratory technician, a physician (e.g., an oncologist, surgeon, pathologist), etc.).

In embodiments where the user only executes a portion of the method, the individual who, after computerized data processing according to the methods disclosed herein, reviews data output (e.g., results prior to release to provide a complete report, a complete, or reviews an "incomplete" report and provides for manual intervention and completion of an interpretive report) is referred to herein as a "reviewer." The reviewer may be located at a location remote to the user (e.g., at a service provided separate from a healthcare facility where a user may be located).

Where government regulations or other restrictions apply (e.g., requirements by health, malpractice, or liability insurance), all results, whether generated wholly or partially electronically, are subjected to a quality control routine prior to release to the user.

### COMPUTER-BASED SYSTEMS AND METHODS

The methods and systems described herein can be implemented in numerous ways. In one embodiment of particular interest, the methods involve use of a communications infrastructure, for example the internet. Several embodiments of the invention are discussed below. It is also to be understood that the present disclosure may be implemented in various forms of hardware, software, fiimware, processors, or a combination thereof. The methods and systems described herein can be implemented as a combination of hardware and software. The software can be implemented as an application program tangibly embodied on a program storage device, or different portions of the software implemented in the user's computing environment (e.g., as an applet) and on the reviewer's computing environment, where the reviewer may be located at a remote site associated (e.g., at a service provider's facility).

For example, during or after data input by the user, portions of the data processing can be performed in the user-side computing environment. For example, the user-side computing environment can be programmed to provide for defined test codes to denote a likelihood "score," where the score is transmitted as processed or partially processed responses to the reviewer's computing environment in the form of test code for subsequent execution of one or more algorithms to provide a results and/or generate a report in the reviewer's computing environment. The score can be a numerical score (representative of a numerical value) or a non-numerical score representative of a numerical value or range of numerical values (e.g., "A' representative of a 90-95% likelihood of an outcome; "high" representative of a greater than 50% chance of response (or some other selected threshold of likelihood); "low" representative of a less than 50% chance of response (or some other selected threshold of likelihood); and the like.

The application program for executing the algorithms described herein may be uploaded to, and executed by, a machine comprising any suitable architecture. In general, the machine involves a computer platform having hardware such as one or more central processing units (CPU), a random access memory (RAM), and input/output (I/O) interface(s). The computer platform also includes an operating system and microinstruction code. The various processes and functions described herein may either be part of the microinstruction code or part of the application program (or a combination thereof) which is executed via the operating system. In addition, various other peripheral devices may be connected to the computer platform such as an additional data storage device and a printing device.

As a computer system, the system generally includes a processor unit. The processor unit operates to receive information, which can include test data (e.g., level of a response indicator gene product(s); level of a reference gene product(s); normalized level of a response indicator gene product(s)); and may also include other data such as patient data. This information received can be stored at least temporarily in a database, and data analyzed to generate a report as described above.

Part or all of the input and output data can also be sent electronically; certain output data (e.g., reports) can be sent electronically or telephonically (e.g., by facsimile, e.g., using devices such as fax back). Exemplary output receiving devices can include a display element, a printer, a facsimile device and the like. Electronic forms of transmission and/or display can include email, interactive television, and the like. In an embodiment of particular interest, all or a portion of the input data and/or all or a portion of the output data (e.g., usually at least the final report) are maintained on a web server for access, preferably confidential access, with typical browsers. The data may be accessed or sent to health professionals as desired. The input and output data, including all or a portion of the final report, can be used to populate a patient's medical record which may exist in a confidential database at the healthcare facility.

A system for use in the methods described herein generally includes at least one computer processor (e.g., where the method is carried out in its entirety at a single site) or at least two networked computer processors (e.g., where data is to be input by a user (also preferred to herein as a "client") and transmitted to a remote site to a second computer processor for analysis, where the first and second computer processors are connected by a network, e.g., via an intranet or internet). The system can also include a user component(s) for input; and a reviewer component(s) for review of data, generated reports, and manual intervention. Additional components of the system can include a server component(s); and a database(s) for storing data (e.g., as in a database of report elements, e.g., interpretive report elements, or a relational database (RDB) which can include data input by the user and data output. The computer processors can be processors that are typically found in personal desktop computers (e.g., IBM, Dell, Macintosh), portable computers, mainframes, minicomputers, or other computing devices.

The networked client/server architecture can be selected as desired, and can be, for example, a classic two or three tier client server model. A relational database management system (RDMS), either as part of an application server component or as a separate component (RDB machine) provides the interface to the database.

In one example, the architecture is provided as a database-centric client/server architecture, in which the client application generally requests services from the application server which makes requests to the database (or the database server) to populate the report with the various report elements as required, particularly the interpretive report elements, especially the interpretation text and alerts. The server(s) (e.g., either as part of the application server machine or a separate RDB/relational database machine) responds to the client's requests.

The input client components can be complete, stand-alone personal computers offering a full range of power and features to run applications. The client component usually operates under any desired operating system and includes a communication element (e.g., a modem or other hardware for connecting to a network), one or more input devices (e.g., a keyboard, mouse, keypad, or other device used to transfer information or commands), a storage element (e.g., a hard drive or other computer-readable, computer-writable storage medium), and a display element (e.g., a monitor, television, LCD, LED, or other display device that conveys information to the user). The user enters input commands into the computer processor through an input device. Generally, the user interface is a graphical user interface (GUI) written for web browser applications.

The server component(s) can be a personal computer, a minicomputer, or a mainframe and offers data management, information sharing between clients, network administration and security. The application and any databases used can be on the same or different server.

Other computing arrangements for the client and server(s), including processing on a single machine such as a mainframe, a collection of machines, or other suitable configuration are contemplated. In general, the client and server machines work together to accomplish the processing of the present disclosure.

Where used, the database(s) is usually connected to the database server component and can be any device which will hold data. For example, the database can be a any magnetic or optical storing device for a computer (e.g., CDROM, internal hard drive, tape drive). The database can be located remote to the server component (with access via a network, modem, etc.) or locally to the server component.

Where used in the system and methods, the database can be a relational database that is organized and accessed according to relationships between data items. The relational database is generally composed of a plurality of tables (entities). The rows of a table represent records (collections of information about separate items) and the columns represent fields (particular attributes of a record). In its simplest conception, the relational database is a collection of data entries that "relate" to each other through at least one common field.

Additional workstations equipped with computers and printers may be used at point of service to enter data and, in some embodiments, generate appropriate reports, if desired. The computer(s) can have a shortcut (e.g., on the desktop) to launch the application to facilitate initiation of data entry, transmission, analysis, report receipt, etc. as desired.

### Computer-readable storage media

The present disclosure also contemplates a computer-readable storage medium (e.g. CD-ROM, memory key, flash memory card, diskette, etc.) having stored thereon a program which, when executed in a computing environment, provides for implementation of algorithms to carry out all or a portion of the results of a response likelihood assessment as described herein. Where the computer-readable medium contains a complete program for carrying out the methods described herein, the program includes program instructions for collecting, analyzing and generating output, and generally includes computer readable code devices for interacting with a user as described herein, processing that data in conjunction with analytical information, and generating unique printed or electronic media for that user.

Where the storage medium provides a program which provides for implementation of a portion of the methods described herein (e.g., the user-side aspect of the methods (e.g., data input, report receipt capabilities, etc.)), the program provides for transmission of data input by the user (e.g., via the internet, via an intranet, etc.) to a computing environment at a remote site. Processing or completion of processing of the data is carried out at the remote site to generate a report. After review of the report, and completion of any needed manual intervention, to provide a complete report, the complete report is then transmitted back to the user as an electronic document or printed document (e.g_{.}, fax or mailed paper report). The storage medium containing a program as disclosed herein can be packaged with instructions (e.g., for program installation, use, etc.) recorded on a suitable substrate or a web address where such instructions may be obtained. The computer-readable storage medium can also be provided in combination with one or more reagents for carrying out response likelihood assessment (e.g., primers, probes, arrays, or other such kit components).

All aspects of the present disclosure may also be practiced such that a limited number of additional genes that are co-expressed with the disclosed genes, for example as evidenced by high Pearson correlation coefficients, are included in a prognostic and/or predictive test in addition to and/or in place of disclosed genes.

Having described exemplary embodiments, the same will be more readily understood through reference to the following Examples, which are provided by way of illustration, and are not intended to limit the invention in any way.

### EXAMPLES

The following examples are offered by way of illustration and not by way of limitation.

### EXAMPLE 1: IDENTIFICATION OF DIFFERENTIAL MARKERS OF RESPONSE IN BREAST CANCER PATIENTS

The data from intergroup trial E2197 (Goldstein L, O'Neill A, Sparano J, et al. E2197: phase III AT (doxorubicin/docetaxel) vs. AC (doxorubicin/cyclophosphamide) in the adjuvant treatment of node positive and high risk node negative breast cancer. Proc Am Soc Clin Oncol. 2005; 23:7s. [Abstract 512]) was used to evaluate the relative efficacy of adjuvant treatment of breast cancer patients with an anthracycline (doxorubicin) + a taxane (AT) compared to an anthracycline (doxorubicin) + cyclophosphamide(AC). The trial compared 4 cycles of a standard doxorubicin-cyclophosphamide (AC) combination given every 3 weeks with 4 cycles of doxorubicin plus docetaxel (AT) in patients with 0-3 positive lymph nodes. The trial was powered to detect a 25% reduction in the disease-free survival (DFS) hazard rate (from an anticipated 5-year DFS of 78% for the AC arm to 83% for the AT arm). Tamoxifen (20 mg daily for 5 years) was recommended for hormone receptor-positive patients following completion of chemotherapy, although approximately 40% of patients eventually took an aromatase inhibitor at some point before or after 5 years. The treatment arms were well balanced with regard to median age (51 years), proportion of lymph node-negative disease (65%), and estrogen receptor (ER)-positive disease (64%).

When single genes by treatment (taxane (T) vs cyclophosphamide (C); or AT vs AC) interactions were evaluated, large numbers of genes with significant interaction effects were observed, in all subjects analyzed; in hormone receptor (HR) positive subjects; in HR positive, Oncotype DX Recurrence Score® value > about 18 subjects; and in HR negative subjects. Most of these interactions are in the same "direction", *i.e.,* higher expression is associated with greater T benefit and/or less C benefit. Where Oncotype DX Recurrence Score® (RS) was used, the RS was calculated according to the algorithm described in Paik et al., N Engl J Med. 2004 Dec 30;351(27):2817-26 and in U.S. application publication No. 20050048542, published March 3, 2005, the entire disclosures of which are expressly incorporated by reference herein.

The predictive utility of PR protein expression was evaluated by immunohistochemistry in a central lab and quantitative RNA expression by RT-PCR for 371 genes (including the 21-gene Recurrence Score [RS]) in a representative sample of 734 patients who received at least 3-4 treatment cycles.

### Methods

*Patient Selection:* All recurrences with available tissue and randomly selected patients without recurrence were identified by an ECOG statistician (ratio 3.5 without recurrence to 1 with recurrence).

*Central Immunohistochemistry (IHC) for ER and PR:* IHC was performed on two 1.0-mm tissue microarrays (TMAs), using 4 µm sections, DakoCytomation EnVision+ System® (Dako Corporation, Carpinteria, CA), and standard methodology using anti-ER antibody (clone 1D5, dilution 1:100) and anti-PR antibody 636 (1:200).

TMAs were reviewed centrally and scored by two pathologists who were blinded to outcomes and local laboratory ER/PR status.

Scoring was performed using the Allred method ( see, e. g. Harvey JM, Clark GM, Osborne CK et al. J Clin Oncol 1999;17:1474-1481) scoring the proportion of positive cells (scored on a 0-5 scale) and staining intensity (scored on a 0-3 scale); proportion and intensity scores were added to yield Allred Score of 0 or 2 through 8 with Allred scores > 2 considered positive.

*Genes and RT-PCR analysis:* Candidate genes were selected to represent multiple biological processes. Quantitative RT-PCR analysis was performed by methods known in the art. For each gene, the appropriate mRNA reference sequence (REFSEQ) accession number was identified and the consensus sequence was accessed through the NCBI Entrez nucleotide database. Appendix 1. Besides the REFSEQ, RT-PCR probe and primer sequences are provided in Appendix 1. Sequences for the amplicons that result from the use of these primer sets are listed in Appendix 2.

*Statistical methods:* Single Gene by Treatment Interaction Analyses. The objective of this evaluation was to identify genes whose expression, treated as a continuous variable, is differentially associated with the risk of relapse between patients treated with AC versus those treated with AT. A gene expression by treatment interaction model was employed for this purpose and statistical analyses were performed by using Cox Regression models (SAS version 9.1.3). The Cox regression model that was employed for these analyses includes terms for the main effect of treatment, the main effect of gene expression, and the interaction of treatment and gene expression. This model enables prediction of the association between gene expression and the risk of recurrence for patients treated with AC, and of the association between gene expression and the risk of recurrence for patients treated with AT. The point at which these two curves cross is the level of gene expression at which the predicted risk of recurrence is identical if the patient is treated with AC or with AT. This crossover point is easily calculated from the parameter estimates from this model as the negative of the estimated treatment effect, divided by the estimate of the interaction effect.

All hypothesis tests were reported using two-sided p-values, and p-values of < 0.05 was considered statistically significant. Relapse-Free Interval was defined as the time from study entry to the first evidence of breast cancer relapse, defined as invasive breast cancer in local, regional or distant sites, including the ipsilateral breast, but excluding new primary breast cancers in the opposite breast. Follow-up for relapse was censored at the time of death without relapse, new primary cancer in the opposite breast, or at the time of the patient was last evaluated for relapse.

The variance of the partial likelihood estimators was estimated with a weighted estimate. See R. Gray, Lifetime Data Anal. 15(1):24-40 (2009); K. Chen K, S-H Lo, Biometrika 86:755-764 (1999).

Individual genes by treatment interactions were tested in Cox models for relapse-free interval (RFI) for the HR+ and HR- patients combined and separately. Since there is little chemotherapy benefit for RS<18, the HR+, RS>18 subset was also analyzed.

The interaction between gene expression and treatment for genes could be depicted graphically. As example we present treatment group-specific plots of the 5-year risk of relapse versus DDR1 gene expression.

Supervised principal components (SPC) was used to combine genes into a multigene predictor of differential treatment benefit, and was evaluated via cross-validation (CV). Pre-validation (PV) inference (Tibshirani and Efron, Stat Appl Genet and Mol Biol 2002;1:Article1. Epub 2002 Aug 22), based on 20 replicates of 5 fold cross-validation, was used to estimate and test (via permutations) the utility of the SPC predictors.

### Results

Tables 1-4 include an Estimated Coefficient for each response indicator gene listed in the tables in all subjects analyzed (Table 1); in HR+ subjects (Table 2); in HR⁺ subjects having an Oncotype DX Recurrence Score® value greater than about 18 (Table 3); and in HR negative subjects (Table 4). Figures 1-4 represent graphically the results for each gene summarized in Tables 1-4, respectively. Each graph of Figures 1-4 shows a smooth line representing the model-predicted relationship between expression of the gene and 5-year recurrence rate (RR) in an AC treatment group (the AC prediction curve) and a hatched line representing the model-predicted relationship between gene expression and RR in an AT treatment group (the AT prediction curve). Each of the graphs in Figures 1-4 are presented with 5-year risk of recurrence on the y-axis and normalized expression (Cₜ) on the x-axis, where increasing normalized Cₜ values indicate increasing expression levels.

The Estimated Coefficient referred to in Tables 1-4 is a reflection of the difference between the slopes in the Cox regression model of the AC prediction curve and the AT prediction curve. The magnitude of the Estimated Coefficient is related to the difference between the slopes of the AC prediction curve and the AT prediction curve; the sign of the Estimated Coefficient is an indication of which treatment (AT or AC) becomes the favored treatment as expression of the gene increases. For example, in Table 1, the Estimated Coefficient for SLC1A3 is -0.7577. The magnitude (absolute value = 0.7577) is related to the difference between the slopes of the AC prediction curve and the AT prediction curve (shown in the first panel of Figure 1) for SLC1A3 in this population (all patients, i.e. not stratified by hormone receptor status or by RS). The negative sign indicates that higher expression levels of SLC1A3 favor treatment with AT while lower expression levels of SLC1A3 favor treatment with AC.

The p-value given in Table 1 is a measure of the statistical significance of the difference between the slope of the AC prediction curve and the slope of the AT prediction curve in the Cox regression model, i.e. the probability that the observed difference in slopes is due to chance. Smaller p-values indicate greater statistical significance.

### Analysis of gene expression in all patients in study population (irrespective of HR status and Oncotype Dx® RS score)

Table 1 shows a list of 76 genes whose normalized expression level is differentially associated with response to AT vs. AC treatment in all patients. When the estimated coefficient is <0, high expression of that gene is indicative that AT treatment is more effective than AC treatment; low gene expression of that gene is indicative that AC treatment is more effective than AT treatment. When the estimated coefficient is >0, high expression of that gene is indicative that AC treatment is more effective than AT treatment; low expression of that gene is indicative that AT treatment is more effective than AC treatment.

As noted above, Figure 1 shows a graph for each gene in Table 1. Each graph shows a smooth line representing the model-predicted relationship between expression of the gene and 5-year recurrence rate (RR) in an AC treatment group (the AC prediction curve) and a hatched line representing the model-predicted relationship between gene expression and RR in an AT treatment group (the AT prediction curve). For each gene, the AC prediction curve and the AT prediction curve have statistically significant different slopes in the Cox regression model, indicating that AC or AT can be chosen as a favored treatment based, at least in part, on the expression of the gene. The graph for each gene also shows, as a horizontal dashed line, represents the 12.3% recurrence rate at 5-year RR in all patients analyzed (i.e., without regard to HR status or Oncotype Dx RS).

The first panel of Figure 1, for example, shows the AC-prediction curve and the AT prediction curve for SLC1A3. The curves have significantly different slopes in the Cox regression model and the lines cross, resulting in the ability to discriminate, based on the expression level of SLC1A3, patients who are more likely to respond to AT (or to AC). For SLC1A3, patients with higher expression levels are more likely to respond to AT than AC, while patients with lower expression levels are more likely to respond to AC than AT.

### Analysis of gene expression in HR+ patients in study population

Table 2 shows a list of 97 genes having a normalized expression level that is differently correlated with response to AT vs. AC in hormone receptor (HR)-positive patients (without regard to Oncotype Dx RS value). When the estimated coefficient is <0, high expression of that gene is indicative that AT treatment is more effective than AC treatment; low expression of that gene is indicative that AC treatment is more effective than AT treatment. When the estimated coefficient is >0, high expression of that gene is indicative than AC treatment is more effective than AT treatment; low expression of that gene is indicative that AT treatment is more effective than AC treatment.

The data summarized in Table 2 are provided in graph form for each gene in Figure 2. For each gene, the AC prediction curve and the AT prediction curve have statistically significant different slopes in the Cox regression model, indicating that AC or AT can be chosen as a favored treatment based, at least in part, on the expression of the gene. The graph for each gene also shows, as a horizontal dashed line represents the 10.0% recurrence rate at 5-year RR in HR-positive patients.

### Analysis of gene expression in HR+ patients in the study population having an Oncotype Dx RS of about 18 or greater

Table 3 shows a list of 165 genes whose normalized expression level is differentially associated with response to AT vs. AC in HR-positive patients having a Recurrence Score (RS) > 18. These patients have an increased likelihood of cancer recurrence. When the estimated coefficient is <0, high expression of that gene is indicative that AT treatment is more effective than AC treatment; low expression of that gene is indicative that AC treatment is more effective than AT treatment. When the estimated coefficient is > 0, high expression of that gene is indicative that AC treatment is more effective than AT treatment; low expression of that gene is indicative that AT treatment is more effective than AC treatment.

The data summarized in Table 3 are provided in graph form for each gene in Figure 3. For each gene, the AC prediction curve and the AT prediction curve have statistically significant different slopes in the Cox regression model, indicating that AC or AT can be chosen as a favored treatment based, at least in part, on the expression of the gene. The graph for each gene also shows, as a horizontal dashed line represents the 14.9% recurrence rate at 5-year RR in the HR-positive patient group having an Oncotype Dx RS of about 18 or greater.

### Analysis of gene expression in HR- patients in study population

Table 4 shows a list of 9 genes whose normalized expression level is differentially associated with response to AT vs. AC treatment in HR-negative patients.

The data summarized in Table 4 is provided in graph form for each gene in Figure 4. For each gene, the AC prediction curve and the AT prediction curve have statistically significant different slopes in the Cox regression model, indicating that AC or AT can be chosen as a favored treatment based, at least in part, on the expression of the gene. The graph for each gene also shows, as a horizontal dashed line represents the 16.9% recurrence rate at 5-year RR in the HR-negative patient group.

### Discussion

PR Analysis. There was a weak benefit for AT in PR-negative (AT vs AC hazard ratio [RR]=0.75; p=0.06) and AC in PR-positive disease (RR=1.37; p=0.05) by central immunhistochemistry (Allred score > 2 positive) but not when genomic PR was evaluated by RT-PCR (>5.5 units positive).

RS and Genes Analyzed. Table 1 illustrates genes that can be used as markers of benefit of taxane therapy irrespective of hormone receptor expression status, and facilitate selection of AC vs AT therapy. (Table 1). Several genes strongly predicted taxane benefit when assessed in the context of AT vs AC therapy in the HR-positive subset (Table 2), and especially in the HR-positive, Oncotype Dx RS > 18 subset (Table 3).

Nine genes were identified for which gene expression can be used as markers of benefit of taxane therapy in hormone receptor (HR)-negative breast cancer, and could be used to assess AT vs. AC benefit in the hormone receptor (HR)-negative patients (Table 4).

Of the genes listed in Table 1, SLC1A3 (glial high affinity glutamase transporter 3) is a member of a large family of solute transport proteins, located within the multiple sclerosis locus on 5p.

Of the genes identified in the HR-positive subset (Table 2), DDR1 (discoidin domain receptor 1) is a transmembrane receptor TK the aberrant expression and signaling of which has been linked to accelerated matrix degradation and remodeling, including tumor invasion. Collagen-induced DDR1 activation is believed to be involved in normal mammary cell adhesion, and may distinguish between invasive ductal carcinoma (IDC) and invasive lobular carcinoma (ILC), and further may induce cyclooxygenase-2 and promoter chemoresistance through the NF-κB pathway. EIF4E2 (human transcription initiation factor 4) is an mRNA cap-binding protein.

When differential response markers in HR-positive, RS > 18 patients (Table 3) are ranked in ascending order by p-value, DDR1, RELA, ZW 10, and RhoB are four of the top five genes. RELA is an NF-κB subunit, which plays a role in inflammation, innate immunity, cancer and anti-apoptosis. This gene has also been associated with chemoresistance, and may be necessary for IL-6 induction, which is involved in immune cell homeostasis. ZW10 is a kinetochore protein involved in mitotic spindle formation. It is part of the ROD-ZW10-Zwilch complex, and binds tubulin. RhoB is a low molecular weight GPTase belonging to the RAS superfamily. The Rho protein is pivotal in regulation of actin cytoskeleton. RhoB acts as tumor suppressor gene and inhibits tumor growth and metastases in vitro and in vivo, and activates NF-κB. KO mice for RhoB show increased sensitivity to chemical carcinogenesis and resistance to radiation and cytotoxic induced apoptosis.

DDR1, RELA and RhoB are key elements in the NFκB signaling pathway. Based on these findings, it is expected that other genes in the NFκB pathway are likely to be differentially associated with response to AT vs. AC treatment in HR-positive patients at high risk for cancer recurrence, and such can be used as differential response markers for AT vs. AC treatment. Some additional genes that are known to be involved in NFκB signaling are shown in Table 5.

In the HR-negative subset, CD247 exhibited a correlation of expression with AT vs. AC therapy (p-value < 0.01) and exhibited a strong correlation indicating that expression was positively correlated with increased likelihood of benefit of treatment including a taxane (Fig. 4). The estimated coefficient <0 indicates that high gene expression favors AT treatment, while low gene expression favors AC treatment (see also Fig. 4). CD247, also known as T cell receptor zeta (TCRzeta) functions as an amplification module of the TCR signaling cascade. This gene is downregulated in many chronic infectious and inflammatory processes, such as systemic lupus erythematosus (SLE).

Fig. 5 illustrates an exemplary treatment group-specific plot of the 5-year risk of relapse versus gene expression presented for.an exemplary gene, DDR1.

### EXAMPLE 2:ESR1 GENE COMBINATIONS

Using the differential response markers identified in Table 2, supervised principle component analysis was carried out in HR+ RS>18 patients treated with AT vs AC according the methods of Bair E, Hastie T, Paul D, Tibshirani R. Prediction by supervised principal components. J. Amer. Stat. Assoc. 101:119-137, 2006.

Principal Components can be used in regression problems for dimensionality reduction in a data set by keeping the most important principal components and ignoring the other ones. Supervised principal components (Bair et *al. supra*) is similar to conventional principal components analysis except that it uses a subset of the predictors (i.e. individual genes) that are selected based on their association with relapse-free interval (assessed using Cox regression). In the present example, only the first component was utilized to obtain a score from a weighted combination of genes.

In this patient group, the most heavily weighted gene by supervised principle components analysis was ESR1, indicating that ESR1 is particularly useful when used in combinations with any of the other genes listed in Table 3 in predicting differential response to taxane vs. cyclophosphamide in HR+ high recurrence risk patients. Exemplary combinations of genes include, without limitation:
DDR1 + ESR1, ZW10 + ESR1, RELA + ESR1, BAX + ESR1, RHOB + ESR1, TSPAN4 + ESR1, BBC3 + ESR1, SHC1 + ESR1, CAPZA1 + ESR1, STK10 + ESR1, TBCC + ESR1, EIF4E2 + ESR1, MCL1 + ESR1, RASSF1 + ESR1, VEGF + ESR1, SLC1A3 + ESR1, DICER1 + ESR1, ILK + ESR1, FAS + ESR1, RAB6C + ESR1, ESR1 + ESR1, MRE11A + ESR1, APOE + ESR1, BAK1 + ESR1, UFM1 + ESR1, AKT2 + ESR1, SIRT1 + ESR1, BCL2L13 + ESR1, ACTR2 + ESR1, LIMK2 + ESR1, HDAC6 + ESR1, RPN2 + ESR1, PLD3 + ESR1, CHGA + ESR1, RHOA + ESR1, MAPK14 + ESR1, ECGF1 + ESR1, MAPRE1 + ESR1, HSPA1B + ESR1, GATA3 + ESR1, PPP2CA + ESR1, ABCD1 + ESR1, MAD2L1BP + ESR1, VHL + ESR1, GCLC + ESR1, ACTB + ESR1, BCL2L11 + ESR1, PRDX1 + ESR1, LILRB1 + ESR1, GNS + ESR1, CHFR + ESR1, CD68 + ESR1, LIMK1 + ESR1, GADD45B + ESR1, VEGFB + ESR1, APRT + ESR1, MAP2K3 + ESR1, MGC52057 + ESR1, MAPK3 + ESR1, APC + ESR1, RAD1 + ESR1, COL6A3 + ESR1, RXRB + ESR1, CCT3 + ESR1, ABCC3 + ESR1, GPX1 + ESR1, TUBB2C + ESR1, HSPA1A + ESR1, AKT1 + ESR1, TUBA6 + ESR1, TOP3B + ESR1, CSNK1D + ESR1, SOD1 + ESR1, BUB3 + ESR1, MAP4 + ESR1, NFKB1 + ESR1, SEC61A1 + ESR1, MAD1L1 + ESR1, PRKCH + ESR1, RXRA + ESR1, PLAU + ESR1, CD63 + ESR1, CD14 + ESR1, RHOC + ESR1, STAT1 + ESR1, NPC2 + ESR1, NME6 + ESR1, PDGFRB + ESR1, MGMT + ESR1, GBP1 + ESR1, ERCC1 + ESR1, RCC1 + ESR1, FUS + ESR1, TUBA3 + ESR1, CHEK2 + ESR1, APOC1 + ESR1, ABCC10 + ESR1, SRC + ESR1, TUBB + ESR1, FLAD1 + ESR1, MAD2L2 + ESR1, LAPTM4B + ESR1, REG1A + ESR1, PRKCD + ESR1, CST7 + ESR1, IGFBP2 + ESR1, FYN + ESR1, KDR + ESR1, STMN1 + ESR1, ZWILCH + ESR1, RBM17 + ESR1, TP53BP1 + ESR1, CD247 + ESR1, ABCA9 + ESR1, NTSR2 + ESR1, FOS + ESR1, TNFRSF10A + ESR1, MSH3 + ESR1, PTEN + ESR1, GBP2 + ESR1, STK11 + ESR1, ERBB4 + ESR1, TFF1 + ESR1, ABCC1 + ESR1, IL7 + ESR1, CDC25B + ESR1, TUBD1 + ESR1, BIRC4 + ESR1, ACTR3 + ESR1, SLC35B1 + ESR1, COL1A1 + ESR1, FOXA1 + ESR1, DUSP1 + ESR1, CXCR4 + ESR1, IL2RA + ESR1, GGPS1 + ESR1, KNS2 + ESR1, RB1 + ESR1, BCL2L1 + ESR1, XIST + ESR1, BIRC3 + ESR1, BID + ESR1, BCL2 + ESR1, STAT3 + ESR1, PECAM1 + ESR1, DIABLO + ESR1, CYBA + ESR1, TBCE + ESR1, CYP1B1 + ESR1, APEX1 + ESR1, TBCD + ESR1, HRAS + ESR1, TNFRSF10B + ESR1, ELP3 + ESR1, PIK3C2A + ESR1, HSPA5 + ESR1, VEGFC + ESR1, CRABP1 + ESR1, MMP11 + ESR1, SGK + ESR1, CTSD + ESR1, BAD + ESR1, PTPN21 + ESR1, HSPA9B + ESR1, and PMS1 + ESR1

Any combination of two or more genes from Table 3, said combination not comprising ESR1 is also expected to be useful in predicting differential response to taxane vs. cyclophosphamide in HR+ high recurrence risk patients.

Similarly it is expected that ESR1 is particularly useful when used in combinations with any of the other genes listed in Table 2 in predicting differential response to taxane vs. cyclophosphamide in HR+ patients. Exemplary combinations of genes include:
DDR1 + ESR1, EIF4E2 + ESR1, TBCC + ESR1, STK10 + ESR1, ZW10 + ESR1, BBC3 + ESR1, BAX + ESR1, BAK1 + ESR1, TSPAN4 + ESR1, SLC1A3 + ESR1, SHC1 + ESR1, CHFR + ESR1, RHOB + ESR1, TUBA6 + ESR1, BCL2L13 + ESR1, MAPRE1 + ESR1, GADD45B + ESR1, HSPA1B + ESR1, FAS + ESR1, TUBB + ESR1, HSPA1A + ESR1, MCL1 + ESR1, CCT3 + ESR1, VEGF + ESR1, TUBB2C + ESR1, AKT1 + ESR1, MAD2L1BP + ESR1, RPN2 + ESR1, RHOA + ESR1, MAP2K3 + ESR1, BID + ESR1, APOE + ESR1, ESR1 + ESR1, ILK + ESR1, NTSR2 + ESR1, TOP3B + ESR1, PLD3 + ESR1, DICER1 + ESR1, VHL + ESR1, GCLC + ESR1, RAD1 + ESR1, GATA3 + ESR1, CXCR4 + ESR1, NME6 + ESR1, UFM1 + ESR1, BUB3 + ESR1, CD14 + ESR1, MRE11A + ESR1, CST7 + ESR1, APOC1 + ESR1, GNS + ESR1, ABCC5 + ESR1, AKT2 + ESR1, APRT + ESR1, PLAU + ESR1, RCC1 + ESR1, CAPZA1 + ESR1, RELA + ESR1, NFKB1 + ESR1, RASSF1 + ESR1, BCL2L11 + ESR1, CSNK1D + ESR1, SRC + ESR1, LIMK2 + ESR1, SIRT1 + ESR1, RXRA + ESR1, ABCD1 + ESR1, MAPK3 + ESR1, CDCA8 + ESR1, DUSP1 + ESR1, ABCC1 + ESR1, PRKCH + ESR1, PRDX1 + ESR1, TUBA3 + ESR1, VEGFB + ESR1, LILRB1 + ESR1, LAPTM4B + ESR1, HSPA9B + ESR1, ECGF1 + ESR1, GDF15 + ESR1, ACTR2 + ESR1, IL7 + ESR1, HDAC6 + ESR1, ZWILCH + ESR1, CHEK2 + ESR1, REG1A + ESR1, APC + ESR1, SLC35B1 + ESR1, NEK2 + ESR1, ACTB + ESR1, BUB1 + ESR1, PPP2CA + ESR1, TNFRSF10A + ESR1, TBCD + ESR1, ERBB4 + ESR1, CDC25B + ESR1, and STMN1 + ESR1.

A combination of two or more genes from Table 2, said combination not comprising ESR1 is also expected to be useful in predicting differential response to taxane vs. cyclophosphamide in HR+ patients at high recurrence risk for cancer.

### EXAMPLE 3:GENES OF THE NFκB PATHWAY

When the differential response markers in HR-positive, RS > 18 patients are ranked in ascending order of p-value, three of the top five revealed genes are DDR1, RELA and RHOB. The RELA gene encodes one of the principle subunits of the NFκB transcription factor. Therefore, it is notable that both the DDR1 gene and the RHOB gene stimulate the NFκB signaling pathway. These results indicate that additional genes that stimulate the activity of the NFκB pathway, given in Table 5, also predict increased likelihood of response to AT vs. AC chemotherapy.

### EXAMPLE 4: GENE EXPRESSION PROFILING PROTOCOL

Breast tumor formalin-fixed and paraffin-embedded (FPE) blocks or frozen tumor sections are provided. Fixed tissues are incubated for 5 to 10 hours in 10% neutral-buffered formalin before being alcohol-dehydrated and embedded in paraffin.

RNA is extracted from three 10-µm FPE sections per each patient case. Paraffin is removed by xylene extraction followed by ethanol wash. RNA is isolated from sectioned tissue blocks using the MasterPure Purification kit (Epicenter, Madison, WI); a DNase I treatment step is included. RNA is extracted from frozen samples using Trizol reagent according to the supplier's instructions (Invitrogen Life Technologies, Carlsbad, CA). Residual genomic DNA contamination is assayed by a TaqMan® (Applied Biosystems, Foster City, CA) quantitative PCR assay (no RT control) for ß-actin DNA. Samples with measurable residual genomic DNA are resubjected to DNase I treatment, and assayed again for DNA contamination. TaqMan is a registered trademark of Roche Molecular Systems.

RNA is quantitated using the RiboGreen® fluorescence method (Molecular Probes, Eugene, OR), and RNA size is analyzed by microcapillary electrophoresis using an Agilent 2100 Bioanalyzer (Agilent Technologies, Palo Alto, CA).

Reverse transcription (RT) is performed using a SuperScript® First-Strand Synthesis kit for RT-PCR (Invitrogen Corp., Carlsbad, CA). Total FPE RNA and pooled gene-specific primers are present at 10 to 50 ng/µl and 100 nmol/L (each), respectively.

TaqMan reactions are performed in 384-well plates according to instructions of the manufacturer, using Applied Biosystems Prism 7900HT TaqMan instruments. Expression of each gene is measured either in duplicate 5-µl reactions using cDNA synthesized from 1 ng of total RNA per reaction well, or in single reactions using cDNA synthesized from 2 ng of total RNA. Final primer and probe concentrations are 0.9 µmol/L (each primer) and 0.2 µmol/L, respectively. PCR cycling is performed as follows: 95°C for 10 minutes for one cycle, 95°C for 20 seconds, and 60°C for 45 seconds for 40 cycles. To verify that the RT-PCR signals derives from RNA rather than genomic DNA, for each gene tested a control identical to the test assay but omitting the RT reaction (no RT control) is included. The threshold cycle for a given amplification curve during RT-PCR occurs at the point the fluorescent signal from probe cleavage grows beyond a specified fluorescence threshold setting. Test samples with greater initial template exceed the threshold value at earlier amplification cycle numbers than those with lower initial template quantities.

For normalization of extraneous effects, cycle threshold (C_{T}) measurements obtained by RT-PCR were normalized relative to the mean expression of a set of five reference genes: ATP5E, PGK1, UBB, VDAC2, and GPX1. A one unit increase in reference normalized expression measurements generally reflects a 2-fold increase in RNA quantity.

| Appendix 1 | | | | |
|---|---|---|---|---|
| Gene Name | Accession # | Oligo Name | Oligo Sequence | SEQ ID NO |
| ABCA9 | NM_172386 | T2132/ABCA9.f1 | TTACCCGTGGGAACTGTCTC | 1 |
| ABCA9 | NM 172386 | T2133/ABCA9.r1 | GACCAGTAAATGGGTCAGAGGA | 2 |
| ABCA9 | NM_172386 | T2134/ABCA9.p1 | TCCTCTCACCAGGACAACAACCAC A | 3 |
| ABCB1 | NM 000927 | S8730/ABCB1.f5 | AAACACCACTGGAGCATTGA | 4 |
| ABCB1 | NM_000927 | S8731/ABCB1.r5 | CAAGCCTGGAACCTATAGCC | 5 |
| ABCB1 | NM_000927 | S8732/ABCB1.p5 | CTCGCCAATGATGCTGCTCAAGTT | 6 |
| ABCB5 | NM_178559 | T2072/ABCB5.f1 | AGACAGTCGCCTTGGTCG | 7 |
| ABCB5 | NM_178559 | T2073/ABCB5.r1 | AACCTCTGCAGAAGCTGGAC | 8 |
| ABCB5 | NM_178559 | T2074/ABCB5.p1 | CCGTACTCTTCCCACTGCCATTGA | 9 |
| ABCC10 | NM_033450 | S9064/ABCC10.f1 . | ACCAGTGCCACAATGCAG | 10 |
| ABCC10 | NM_033450 | S9065/ABCC10.r1 . | ATAGCGCTGACCACTGCC | 11 |
| ABCC10 | NM_033450 | S9066/ABCC10.p1 | CCATGAGCTGTAGCCGAATGTCCA | 12 |
| ABCC11 | NM 032583 | T2066/ABCC11.f1 | AAGCCACAGCCTCCATTG | 13 |
| ABCC11 | NM 032583 | T2067/ABCC11.r1 | GGAAGGCTTCACGGATTGT | 14 |
| ABCC11 | NM_032583 | T2068/ABCC11.p1 | TGGAGACAGACACCCTGATCCAGC | 15 |
| ABCC5 | NM_005688 | S5605/ABCC5.f1 | TGCAGACTGTACCATGCTGA | 16 |
| ABCC5 | NM_005688 | S5606/ABCC5.r1 | GGCCAGCACCATAATCCTAT | 17 |
| ABCC5 | NM_005688 | S5607/ABCC5.p1 | CTGCACACGGTTCTAGGCTCCG | 18 |
| ABCD1 | NM_000033 | T1991/ABCD1.f1 | TCTGTGGCCCACCTCTACTC | 19 |
| ABCD1 | NM_000033 | T1992/ABCD1.r1 | GGGTGTAGGAAGTCACAGCC | 20 |
| ABCD1 | NM_000033 | T1993/ABCD1.p1 | AACCTGACCAAGCCACTCCTGGAC | 21 |
| ACTG2 | NM_001615 | S4543/ACTG2.f3 | ATGTACGTCGCCATTCAAGCT | 22 |
| ACTG2 | NM 001615 | S4544/ACTG2.r3 | ACGCCATCACCTGAATCCA | 23 |
| ACTG2 | NM_001615 | S4545/ACTG2.p3 | CTGGCCGCACGACAGGCATC | 24 |
| ACTR2 | NM_005722 | T2380/ACTR2.f1 | ATCCGCATTGAAGACCCA | 25 |
| ACTR2 | NM_005722 | T2381/ACTR2.r1 | ATCCGCTAGAACTGCACCAC | 26 |
| ACTR2 | NM_005722 | T2382/ACTR2.p1 | CCCGCAGAAAGCACATGGTATTCC | 27 |
| ACTR3 | NM_005721 | T2383/ACTR3.f1 | CAACTGCTGAGAGACCGAGA | 28 |
| ACTR3 | NM_005721 | T2384/ACTR3.r1 | CGCTCCTTTACTGCCTTAGC | 29 |
| ACTR3 | NM_005721 | T2385/ACTR3.p1 | AGGAATCCCTCCAGAACAATCCTT GG | 30 |
| AK055699 | NM_194317 | S2097/AK0556.f1 | CTGCATGTGATTGAATAAGAAACAA GA | 31 |
| AK055699 | NM_194317 | S2098/AK0556.r1 | TGTGGACCTGATCCCTGTACAC | 32 |
| AK055699 | NM_194317 | S5057/AK0556.p1 | TGACCACACCAAAGCCTCCCTGG | 33 |
| AKT1 | NM_005163 | S0010/AKT1.f3 | CGCTTCTATGGCGCTGAGAT | 34 |
| AKT1 | NM 005163 | S0012/AKT1.r3 | TCCCGGTACACCACGTTCTT | 35 |
| AKT1 | NM 005163 | S4776/AKT1.p3 | CAGCCCTGGACTACCTGCACTCGG | 36 |
| AKT2 | NM_001626 | S0828/AKT2.f3 | TCCTGCCACCCTTCAAACC | 37 |
| AKT2 | NM_001626 | S0829/AKT2.r3 | GGCGGTAAATTCATCATCGAA | 38 |
| AKT2 | NM_001626 | S4727/AKT2.p3 | CAGGTCACGTCCGAGGTCGACACA | 39 |
| AKT3 | NM_005465 | S0013/AKT3.f2 | TTGTCTCTGCCTTGGACTATCTACA | 40 |
| AKT3 | NM 005465 | S0015/AKT3.r2 | CCAGCATTAGATTCTCCAACTTGA | 41 |
| AKT3 | NM_005465 | S4884/AKT3.p2 | TCACGGTACACAATCTTTCCGGA | 42 |
| ANXA4 | NM_001153 | T1017/ANXA4.f1 | TGGGAGGGATGAAGGAAAT | 43 |
| ANXA4 | MM_001153 | T1018/ANXA4.r1 | CTCATACAGGTCCTGGGCA | 44 |
| ANXA4 | NM 001153 | T1019/ANXA4.p1 | TGTCTCACGAGAGCATCGTCCAGA | 45 |
| APC | NM_000038 | S0022/APC.f4 | GGACAGCAGGAATGTGTTTC | 46 |
| APC | NM_000038 | S0024/APC.r4 | ACCCACTCGATTTGTTTCTG | 47 |
| APC | NM_000038 | S4888/APC.p4 | CATTGGCTCCCCGTGACCTGTA | 48 |
| APEX-1 | NM 001641 | S9947/APEX-1.f1 | GATGAAGCCTTTCGCAAGTT | 49 |
| APEX-1 | NM 001641 | S9948/APEX-1.r1 | AGGTCTCCACACAGCACAAG | 50 |
| APEX-1 | NM_001641 | S9949/APEX-1.p1 | CTTTCGGGAAGCCAGGCCCTT | 51 |
| APOC1 | NM_001645 | S9667/APOC1.f2 | GGAAACACACTGGAGGACAAG | 52 |
| APOC1 | NM_001645 | S9668/APOC1.r2 | CGCATCTTGGCAGAAAGTT | 53 |
| APOC1 | NM 001645 | S9669/APOC1.p2 | TCATCAGCCGCATCAAACAGAGTG | 54 |
| APOD | NM 001647 | T0536/APOD.f1 | GTTTATGCCATCGGCACC | 55 |
| APOD | NM_001647 | T0537/APOD.r1 | GGAATACACGAGGGCATAGTTC | 56 |
| APOD | NM_001647 | T0538/APOD.p1 | ACTGGATCCTGGCCACCGACTATG | 57 |
| APOE | NM_000041 | T1994/APOE.f1 | GCCTCAAGAGCTGGTTCG | 58 |
| APOE | NM 000041 | T1995/APOE.r1 | CCTGCACCTTCTCCACCA | 59 |
| APOE | NM_000041 | T1996/APOE.p1 | ACTGGCGCTGCATGTCTTCCAC | 60 |
| APRT | NM_000485 | T1023/APRT.f1 | GAGGTCCTGGAGTGCGTG | 61 |
| APRT | NM_000485 | T1024/APRT.r1 | AGGTGCCAGCTTCTCCCT | 62 |
| APRT | NM_000485 | T1025/APRT.p1 | CCTTAAGCGAGGTCAGCTCCACCA | 63 |
| ARHA | NM 001664 | S8372/ARHA.f1 | GGTCCTCCGTCGGTTCTC | 64 |
| ARHA | NM_001664 | S8373/ARHA.r1 | GTCGCAAACTCGGAGACG | 65 |
| ARHA | NM_001664 | S8374/ARHA.p1 | CCACGGTCTGGTCTTCAGCTACCC | 66 |
| AURKB | NM_004217 | S7250/AURKB.f1 | AGCTGCAGAAGAGCTGCACAT | 67 |
| AURKB | NM_004217 | S7251/AURKB.r1 | GCATCTGCCAACTCCTCCAT | 68 |
| AURKB | NM 004217 | S7252/AURKB.p1 | TGACGAGCAGCGAACAGCCACG | 69 |
| B-actin | NM_001101 | S0034/B-acti.f2 | CAGCAGATGTGGATCAGCAAG | 70 |
| B-actin | NM_001101 | S0036/B-acti.r2 | GCATTTGCGGTGGACGAT | 71 |
| B-actin | NM_001101 | S4730/B-acti.p2 | AGGAGTATGACGAGTCCGGCCCC | 72 |
| B-Catenin | NM 001904 | S2150/B-Cate.f3 | GGCTCTTGTGCGTACTGTCCTT | 73 |
| B-Catenin | NM 001904 | S2151/B-Cate.r3 | TCAGATGACGAAGAGCACAGATG | 74 |
| B-Catenin | NM_001904 | S5046/B-Cate.p3 | AGGCTCAGTGATGTCTTCCCTGTC ACCAG | 75 |
| BAD | NM_032989 | S2011/BAD.f1 | GGGTCAGGTGCCTCGAGAT | 76 |
| BAD | NM_032989 | S2012/BAD.r1 | CTGCTCACTCGGCTCAAACTC | 77 |
| BAD | NM_032989 | S5058/BAD.p1 | TGGGCCCAGAGCATGTTCCAGATC | 78 |
| BAG1 | NM_004323 | S1386/BAG1.f2 | CGTTGTCAGCACTTGGAATACAA | 79 |
| BAG1 | NM 004323 | S1387/BAG1.r2 | GTTCAACCTCTTCCTGTGGACTGT | 80 |
| BAG1 | NM_004323 | S4731/BAG1.p2 | CCCAATTAACATGACCCGGCAACC AT | 81 |
| Bak | NM_001188 | S0037/Bak.f2 | CCATTCCCACCATTCTACCT | 82 |
| Bak | NM_001188 | S0039/Bak.r2 | GGGAACATAGACCCACCAAT | 83 |
| Bak | NM_001188 | S4724/Bak.p2 | ACACCCCAGACGTCCTGGCCT | 84 |
| Bax | NM_004324 | S0040/Bax.f1 | CCGCCGTGGACACAGACT | 85 |
| Bax | NM 004324 | S0042/Bax.r1 | TTGCCGTCAGAAAACATGTCA | 86 |
| Bax | NM 004324 | S4897/Bax.p1 | TGCCACTCGGAAAAAGACCTCTCG | 87 |
| | | | G | |
| BBC3 | NM 014417 | S1584/BBC3.f2 | CCTGGAGGGTCCTGTACAAT | 88 |
| BBC3 | NM_014417 | S1585/BBC3.r2 | CTAATTGGGCTCCATCTCG | 89 |
| BBC3 | M_014417 | S4890/BBC3.p2 | CATCATGGGACTCCTGCCCTTACC | 90 |
| Bcl2 | NM_000633 | S0043/Bcl2.f2 | CAGATGGACCTAGTACCCACTGAG A | 91 |
| Bcl2 | NM_000633 | S0045/Bcl2.r2 | CCTATGATTTAAGGGCATTTTTCC | 92 |
| Bcl2 | NM_000633 | S4732/Bcl2.p2 | TTCCACGCCGAAGGACAGCGAT | 93 |
| BCL2L11 | NM 138621 | S7139/BCL2L1.f1 | AATTACCAAGCAGCCGAAGA | 94 |
| BCL2L11 | NM_138621 | S7140/BCL2L1.r1 | CAGGCGGACAATGTAACGTA | 95 |
| BCL2L11 | NM 138621 | S7141/BCL2L1.p1 | CCACCCACGAATGGTTATCTTACG ACTG | 96 |
| BCL2L13 | MM_015367 | S9025/BCL2L1.f1 | CAGCGACAACTCTGGACAAG | 97 |
| BCL2L13 | NM_015367 | S9026/BCL2L1.r1 | GCTCTCAGACTGCCAGGAA | 98 |
| BCL2L13 | NM_015367 | S9027/BCL2L1.p1 | CCCCAGAGTCTCCAACTGTGACCA | 99 |
| Bclx | NM 001191 | S0046/Bclx.f2 | CTTTTGTGGAACTCTATGGGAACA | 100 |
| Bclx | NM_001191 | S0048/Bclx.r2 | CAGCGGTTGAAGCGTTCCT | 101 |
| Bclx | NM_001191 | S4898/Bclx.p2 | TTCGGCTCTCGGCTGCTGCA | 102 |
| BCRP | NM_004827 | S0840/BCRP.f1 | TGTACTGGCGAAGAATATTTGGTAA[?] A | 103 |
| BCRP | NM_004827 | S0841/BCRP.r1 | GCCACGTGATTCTTCCACAA | 104 |
| BCRP | NM_004827 | S4836/BCRP.p1 | CAGGGCATCGATCTCTCACCCTGG | 105 |
| BID | NM 001196 | S6273/BID.f3 | GGACTGTGAGGTCAACAACG | 106 |
| BID | NM 001196 | S6274/BID.r3 | GGAAGCCAAACACCAGTAGG | 107 |
| BID | NM_001196 | S6275/BID.p3 | TGTGATGCACTCATCCCTGAGGCT | 108 |
| BIN1 | NM_004305 | S2651/BIN1.f3 | CCTGCAAAAGGGAACAAGAG | 109 |
| BIN1 | NM_004305 | S2652/BIN1.r3 | CGTGGTTGACTCTGATCTCG | 110 |
| BIN1 | NM_004305 | S4954/BIN1.p3 | CTTCGCCTCCAGATGGCTCCC | 111 |
| BRCA1 | NM_007295 | S0049/BRCA1.f2 | TCAGGGGGCTAGAAATCTGT | 112 |
| BRCA1 | NM_007295 | S0051/BRCA1.r2 | CCATTCCAGTTGATCTGTGG | 113 |
| BRCA1 | NM_007295 | S4905/BRCA1.p2 | CTATGGGCCCTTCACCAACATGC | 114 |
| BRCA2 | NM_000059 | S0052/BRCA2.f2 | AGTTCGTGCTTTGCAAGATG | 115 |
| BRCA2 | NM 000059 | S0054/BRCA2.r2 | AAGGTAAGCTGGGTCTGCTG | 116 |
| BRCA2 | NM_000059 | S4985/BRCA2.p2 | CATTCTTCACTGCTTCATAAAGCTC TGCA | 117 |
| BUB1 | NM 004336 | S4294/BUB1.f1 | CCGAGGTTAATCCAGCACGTA | 118 |
| BUB1 | NM_004336 | S4295/BUB1.r1 | AAGACATGGCGCTCTCAGTTC | 119 |
| BUB1 | NM_004336 | S4296/BUB1.p1 | TGCTGGGAGCCTACACTTGGCCC | 120 |
| BUB1B | NM_001211 | S8060/BUB1 B.f1 | TCAACAGAAGGCTGAACCACTAGA | 121 |
| BUB1B | NM 001211 | S8061/BUB1B.r1 | CAACAGAGTTTGCCGAGACACT | 122 |
| BUB1B | NM 001211 | S8062/BUB1B.p1 | TACAGTCCCAGCACCGACAATTCC | 123 |
| BUB3 | NM_004725 | S8475/BUB3.f1 | CTGAAGCAGATGGTTCATCATT | 124 |
| BUB3 | NM_004725 | S8476/BUB3.r1 | GCTGATTCCCAAGAGTCTAACC | 125 |
| BUB3 | NM_004725 | S8477/BUB3.p1 | CCTCGCTTTGTTTAACAGCCCAGG | 126 |
| c-Src | NM_005417 | S7320/c-Src.f1 | TGAGGAGTGGTATTTTGGCAAGA | 127 |
| c-Src | NM 005417 | S7321/c-Src.r1 | CTCTCGGGTTCTCTGCATTGA | 128 |
| c-Src | NM_005417 | S7322/c-Src.p1 | AACCGCTCTGACTCCCGTCTGGTG | 129 |
| C14orf10 | NM 017917 | T2054/C14orf.f1 | GTCAGCGTGGTAGCGGTATT | 130 |
| C14orf10 | NM_017917 | T2055/C14orf.r1 | GGAAGTCTTGGCTAAAGAGGC | 131 |
| C14orf10 | NM_017917 | T2056/C14orf.p1 | AACAATTACTGTCACTGCCGCGGA | 132 |
| C20 orf1 | NM_012112 | S3560/C20 or.f1 | TCAGCTGTGAGCTGCGGATA | 133 |
| C20 orf1 | NM 012112 | S3561/C20 or.r1 | ACGGTCCTAGGTTTGAGGTTAAGA | 134 |
| C20 orf1 | NM 012112 | S3562/C20 or.p1 | CAGGTCCCATTGCCGGGCG | 135 |
| CA9 | NM_001216 | S1398/CA9.f3 | ATCCTAGCCCTGGTTTTTGG | 136 |
| CA9 | NM_001216 | S1399/CA9.r3 | CTGCCTTCTCATCTGCACAA | 137 |
| CA9 | MM_001216 | S4938/CA9.p3 | TTTGCTGTCACCAGCGTCGC | 138 |
| CALD1 | NM_004342 | S4683/CALD1.f2 | CACTAAGGTTTGAGACAGTTCCAG AA | 139 |
| CALD1 | NM_004342 | S4684/CALD1.r2 | GCGAATTAGCCCTCTACAACTGA | 140 |
| CALD1 | NM_004342 | S4685/CALD1.p2 | AACCCAAGCTCAAGACGCAGGACG AG | 141 |
| CAPZA1 | NM 006135 | T2228/CAPZA1.f1 | TCGTTGGAGATCAGAGTGGA | 142 |
| CAPZA1 | NM_006135 | T2229/CAPZA1.r1 | TTAAGCACGCCAACCACC | 143 |
| CAPZA1 | NM_006135 | T2230/CAPZA1.p1 | TCACCATCACACCACCTACAGCCC | 144 |
| CAV1 | NM_001753 | S7151/CAV1.f1 | GTGGCTCAACATTGTGTTCC | 145 |
| CAV1 | NM_001753 | S7152/CAV1.r1 | CAATGGCCTCCATTTTACAG | 146 |
| CAV1 | NM 001753 | S7153/CAV1.p1 | ATTTCAGCTGATCAGTGGGCCTCC | 147 |
| CCNB1 | NM_031966 | S1720/CCNB1.f2 | TTCAGGTTGTTGCAGGAGAC | 148 |
| CCNB1 | NM_031966 | 51721/CCNB1.r2 | CATCTTCTTGGGCACACAAT | 149 |
| CCNB1 | NM_031966 | S4733/CCNB1.p2 | TGTCTCCATTATTGATCGGTTCATG CA | 150 |
| CCND1 | NM_053056 | S0058/CCND1.f3 | GCATGTTCGTGGCCTCTAAGA | 151 |
| CCND1 | NM_053056 | S0060/CCND1.r3 | CGGTGTAGATGCACAGCTTCTC | 152 |
| CCND1 | NM_053056 | S4986/CCND1.p3 | AAGGAGACCATCCCCCTGACGGC | 153 |
| CCNE2 | NM_057749 | S1458/CCNE2.f2 | ATGCTGTGGCTCCTTCCTAACT | 154 |
| CCNE2 | NM_057749 | S1459/CCNE2.r2 | ACCCAAATTGTGATATACAAAAAGG TT | 155 |
| CCNE2 | NM_057749 | S4945/CCNE2.p2 | TACCAAGCAACCTACATGTCAAGA AAGCCC | 156 |
| CCT3 | NM_001008800 | T1053/CCT3.f1 | ATCCAAGGCCATGACTGG | 157 |
| CCT3 | NM_001008800 | T1054/CCT3.r1 | GGAATGACCTCTAGGGCCTG | 158 |
| CCT3 | NM_001008800 | T1055/CCT3.p1 | ACAGCCCTGTATGGCCATTGTTCC | 159 |
| CD14 | NM_000591 | T1997/CD14.f1 | GTGTGCTAGCGTACTCCCG | 160 |
| CD14 | NM 000591 | T1998/CD14.r1 | GCATGGTGCCGGTTATCT | 161 |
| CD14 | NM_000591 | T1999/CD14.p1 | CAAGGAACTGACGCTCGAGGACCT | 162 |
| CD31 | MM_000442 | S1407/CD31.f3 | TGTATTTCAAGACCTCTGTGCACTT | 163 |
| CD31 | NM_000442 | 51408/CD31.r3 | TTAGCCTGAGGAATTGCTGTGTT | 164 |
| CD31 | NM_000442 | S4939/CD31.p3 | TTTATGAACCTGCCCTGCTCCCAC A | 165 |
| CD3z | NM_000734 | S0064/CD3z.f1 | AGATGAAGTGGAAGGCGCTT | 166 |
| CD3z | NM 000734 | S0066/CD3z.r1 | TGCCTCTGTAATCGGCAACTG | 167 |
| CD3z | NM 000734 | S4988/CD3z.p1 | CACCGCGGCCATCCTGCA | 168 |
| CD63 | NM_001780 | T1988/CD63.f1 | AGTGGGACTGATTGCCGT | 169 |
| CD63 | NM_001780 | T1989/CD63.r1 | GGGTAGCCCCCTGGATTAT | 170 |
| CD63 | NM 001780 | T1990/CD63.p1 | TCTGACTCAGGACAAGCTGTGCCC | 171 |
| CD68 | NM_001251 | S0067/CD68.f2 | TGGTTCCCAGCCCTGTGT | 172 |
| CD68 | NM_001251 | S0069/CD68.r2 | CTCCTCCACCCTGGGTTGT | 173 |
| CD68 | NM_001251 | S4734/CD68.p2 | CTCCAAGCCCAGATTCAGATTCGA GTCA | 174 |
| CDC2 | NM_001786 | S7238/CDC2.f1 | GAGAGCGACGCGGTTGTT | 175 |
| CDC2 | NM_001786 | S7239/CDC2.r1 | GTATGGTAGATCCCGGCTTATTATT C | 176 |
| CDC2 | NM_001786 | S7240/CDC2.p1 | TAGCTGCCGCTGCGGCCG | 177 |
| CDC20 | NM 001255 | S4447/CDC20.f1 | TGGATTGGAGTTCTGGGAATG | 178 |
| CDC20 | NM 001255 | S4448/CDC20.r1 | GCTTGCACTCCACAGGTACACA | 179 |
| CDC20 | NM_001255 | S4449/CDC20.p1 | ACTGGCCGTGGCACTGGACAACA | 180 |
| CDC25B | NM_021873 | S1160/CDC25B.f1 | AAACGAGCAGTTTGCCATCAG | 181 |
| CDC25B | NM_021873 | S1161/CDC25B.r1 | GTTGGTGATGTTCCGAAGCA | 182 |
| CDC25B | NM 021873 | S4842/CDC25B.p1 | CCTCACCGGCATAGACTGGAAGCG | 183 |
| CDCA8 | NM_018101 | T2060/CDCA8.f1 | GAGGCACAGTATTGCCCAG | 184 |
| CDCA8 | NM_018101 | T2061/CDCA8.r1 | GAGACGGTTGGAGAGCTTCTT | 185 |
| CDCA8 | NM_018101 | T2062/CDCA8.p1 | ATGTTTCCCAAGGCCTCTGGATCC | 186 |
| CDH1 | NM_004360 | S0073/CDH1.f3 | TGAGTGTCCCCCGGTATCTTC | 187 |
| CDH1 | NM 004360 | S0075/CDH1.r3 | CAGCCGCTTTCAGATTTTCAT | 188 |
| CDH1 | NM_004360 | S4990/CDH1.p3 | TGCCAATCCCGATGAAATTGGAAA TTT | 189 |
| CDK5 | NM 004935 | T2000/CDK5.f1 | AAGCCCTATCCGATGTACCC | 190 |
| CDK5 | NM_004935 | T2001/CDK5.r1 | CTGTGGCATTGAGTTTGGG | 191 |
| CDK5 | NM_004935 | T2002/CDK5.p1 | CACAACATCCCTGGTGAACGTCGT | 192 |
| CDKN1C | NM_000076 | T2003/CDKN1C.f1 | CGGCGATCAAGAAGCTGT | 193 |
| CDKN1C | NM 000076 | T2004/CDKN1C.r1 | CAGGCGCTGATCTCTTGC | 194 |
| CDKN1C | NM 000076 | T2005/CDKN1C.p1 | CGGGCCTCTGATCTCCGATTTCTT | 195 |
| CEGP1 | NM_020974 | S1494/CEGP1.f2 | TGACAATCAGCACACCTGCAT | 196 |
| CEGP1 | NM_020974 | S1495/CEGP1.r2 | TGTGACTACAGCCGTGATCCTTA | 197 |
| CEGP1 | NM_020974 | S4735/CEGP1.p2 | CAGGCCCTCTTCCGAGCGGT | 198 |
| CENPA | NM 001809 | 57082/CENPA.f1 | TAAATTCACTCGTGGTGTGGA | 199 |
| CENPA | NM_001809 | S7083/CENPA.r1 | GCCTCTTGTAGGGCCAATAG | 200 |
| CENPA | NM_001809 | S7084/CENPA.p1 | CTTCAATTGGCAAGCCCAGGC | 201 |
| CENPE | NM_001813 | S5496/CENPE.f3 | GGATGCTGGTGACCTCTTCT | 202 |
| CENPE | NM_001813 | S5497/CENPE.r3 | GCCAAGGCACCAAGTAACTC | 203 |
| CENPE | NM_001813 | S5498/CENPE.p3. | TCCCTCACGTTGCAACAGGAATTA A | 204 |
| CENPF | NM 016343 | S9200/CENPF.f1 | CTCCCGTCAACAGCGTTC | 205 |
| CENPF | NM 016343 | S9201/CENPF.r1 | GGGTGAGTCTGGCCTTCA | 206 |
| CENPF | NM_016343 | S9202/CENPF.p1 | ACACTGGACCAGGAGTGCATCCAG | 207 |
| CGA(CHGA official) | NM_001275 | S3221/CGA(C.f3 | CTGAAGGAGCTCCAAGACCT | 208 |
| CGA(CHGA Official) | NM_001275 | S3222/CGA (C.r3 | CAAAACCGCTGTGTTTCTTC | 209 |
| CGA (CHGA | NM_001275 | S3254/CGA (C.p3 | TGCTGATGTGCCCTCTCCTTGG | 210 |
| official) | | | | |
| CHFR | NM 018223 | S7085/CHFR.f1 | AAGGAAGTGGTCCCTCTGTG | 211 |
| CHFR | NM_018223 | S7086/CHFR.r1 | GACGCAGTCTTTCTGTCTGG | 212 |
| CHFR | NM_018223 | S7087/CHFR.p1 | TGAAGTCTCCAGCTTTGCCTCAGC | 213 |
| Chk1 | NM_001274 | S1422/Chk1.f2 | GATAAATTGGTACAAGGGATCAGC TT | 214 |
| Chk1 | NM_001274 | S1423/Chk1.r2 | GGGTGCCAAGTAACTGACTATTCA | 215 |
| Chk1 | NM_001274 | S4941/Chk1.p2 | CCAGCCCACATGTCCTGATCATAT GC | 216 |
| Chk2 | NM_007194 | S1434/Chk2.f3 | ATGTGGAACCCCCACCTACTT | 217 |
| Chk2 | NM 007194 | S1435/Chk2.r3 | CAGTCCACAGCACGGTTATACC | 218 |
| Chk2 | NM_007194 | S4942/Chk2.p3 | AGTCCCAACAGAAACAAGAACTTC AGGCG | 219 |
| cIAP2 | NM 001165 | S0076/cIAP2.f2 | GGATATTTCCGTGGCTCTTATTCA | 220 |
| cIAP2 | MM_001165 | S0078/cIAP2.r2 | CTTCTCATCAAGGCAGAAAAATCTT | 221 |
| cIAP2 | NM 001165 | S4991/cIAP2.p2 | TCTCCATCAAATCCTGTAAACTCCA GAGCA | 222 |
| CKAP1 | NM_001281 | T2293/CKAP1.f1 | TCATTGACCACAGTGGCG | 223 |
| CKAP1 | NM_001281 | T2294/CKAP1.r1 | TCGTGTACTTCTCCACCCG | 224 |
| CKAP1 | NM_001281 | T2295/CKAP1.p1 | CACGTCCTCATACTCACCAAGGCG | 225 |
| CLU | NM 001831 | S5666/CLU.f3 | CCCCAGGATACCTACCACTACCT | 226 |
| CLU | NM_001831 | S5667/CLU.r3 | TGCGGGACTTGGGAAAGA | 227 |
| CLU | NM_001831 | S5668/CLU.p3 | CCCTTCAGCCTGCCCCACCG | 228 |
| cMet | NM_000245 | S0082/cMet.f2 | GACATTTCCAGTCCTGCAGTCA | 229 |
| cMet | NM_000245 | S0084/cMet.r2 | CTCCGATCGCACACATTTGT | 230 |
| cMet | NM_000245 | S4993/cMet.p2 | TGCCTCTCTGCCCCACCCTTTGT | 231 |
| cMYC | NM_002467 | S0085/cMYC.f3 | TCCCTCCACTCGGAAGGACTA | 232 |
| cMYC | NM_002467 | S0087/cMYC.r3 | CGGTTGTTGCTGATCTGTCTCA | 233 |
| cMYC | NM_002467 | S4994/cMYC.p3 | TCTGACACTGTCCAACTTGACCCT CTT | 234 |
| CNN | NM_001299 | S4564/CNN.f1 | TCCACCCTCCTGGCTTTG | 235 |
| CNN | NM_001299 | S4565/CNN.r1 | TCACTCCCACGTTCACCTTGT | 236 |
| CNN | NM 001299 | 54566/CNN.p1 | TCCTTTCGTCTTCGCCATGCTGG | 237 |
| COL1A1 | NM_000088 | S4531/COL1A1.f1 | GTGGCCATCCAGCTGACC | 238 |
| COL1A1 | NM_000088 | S4532/COL1A1.r1 | CAGTGGTAGGTGATGTTCTGGGA | 239 |
| COL1A1 | NM_000088 | S4533/COL1A1.p1 | TCCTGCGCCTGATGTCCACCG | 240 |
| COL1A2 | NM_000089 | -S4534/COL1A2.f1 | CAGCCAAGAACTGGTATAGGAGCT | 241 |
| COL1A2 | NM 000089 | S4535/COL1A2.r1 | AAACTGGCTGCCAGCATTG | 242 |
| COL1A2 | NM_000089 | S4536/COL1A2.p1 | TCTCCTAGCCAGACGTGTTTCTTGT[?] CCTTG | 243 |
| COL6A3 | NM 004369 | T1062/COL6A3.f1 | GAGAGCAAGCGAGACATTCTG | 244 |
| COL6A3 | NM_004369 | T1063/COL6A3.r1 | AACAGGGAACTGGCCCAC | 245 |
| COL6A3 | NM_004369 | T1064/COL6A3.p1 | CCTCTTTGACGGCTCAGCCAATCT | 246 |
| Contig 51037 | NM_198477 | S2070/Contiq.f1 | CGACAGTTGCGATGAAAGTTCTAA | 247 |
| Contig 51037 | NM_198477 | S2071/Contig.r1 | GGCTGCTAGAGACCATGGACAT | 248 |
| Contig 51037 | NM_198477 | S5059/Contig.p1 | CCTCCTCCTGTTGCTGCCACTAAT GCT | 249 |
| COX2 | NM_000963 | S0088/COX2.f1 | TCTGCAGAGTTGGAAGCACTCTA | 250 |
| COX2 | NM_000963 | S0090/COX2.r1 | GCCGAGGCTTTTCTACCAGAA | 251 |
| COX2 | NM_000963 | S4995/COX2.p1 | CAGGATACAGCTCCACAGCATCGA TGTC | 252 |
| COX7C | NM_001867 | T0219/COX7C.f1 | ACCTCTGTGGTCCGTAGGAG | 253 |
| COX7C | NM_001867 | T0220/COX7C.r1 | CGACCACTTGTTTTCCACTG | 254 |
| COX7C | NM_001867 | T0221/COX7C.p1 | TCTTCCCAGGGCCCTCCTCATAGT | 255 |
| CRABP1 | NM_004378 | S5441/CRABP1.f3 | AACTTCAAGGTCGGAGAAGG | 256 |
| CRABP1 | NM_004378 | S5442/CRABP1.r3 | TGGCTAAACTCCTGCACTTG | 257 |
| CRABP1 | NM_004378 | S5443/CRABP1.p3 | CCGTCCACGGTCTCCTCCTCA | 258 |
| CRIP2 | NM_001312 | S5676/CRIP2.f3 | GTGCTACGCCACCCTGTT | 259 |
| CRIP2 | NM_001312 | S5677/CRIP2.r3 | CAGGGGCTTCTCGTAGATGT | 260 |
| CRIP2 | NM_001312 | S5678/CRIP2.p3 | CCGATGTTCACGCCTTTGGGTC | 261 |
| CRYAB | NM 001885 | S8302/CRYAB.f1 | GATGTGATTGAGGTGCATGG | 262 |
| CRYAB | NM_001885 | S8303/CRYAB.r1 | GAACTCCCTGGAGATGAAACC | 263 |
| CRYAB | NM_001885 | S8304/CRYAB.p1 | TGTTCATCCTGGCGCTCTTCATGT | 264 |
| CSF1 | NM_000757 | S1482/CSF1.f1 | TGCAGCGGCTGATTGACA | 265 |
| CSF1 | NM_000757 | S1483/CSF1.r1 | CAACTGTTCCTGGTCTACAAACTCA | 266 |
| CSF1 | NM_000757 | S4948/CSF1.p1 | TCAGATGGAGACCTCGTGCCAAAT TACA | 267 |
| CSNK1D | NM 001893 | S2332/CSNK1D.f3 | AGCTTTTCCGGAATCTGTTC | 268 |
| CSNK1D | NM_001893 | S2333/CSNK1D.r3 | ATTTGAGCATGTTCCAGTCG | 269 |
| CSNK1D | NM_001893 | S4850/CSNK1D.p3 | CATCGCCAGGGCTTCTCCTATGAC | 270 |
| CST7 | NM_003650 | T2108/CST7.f1 | TGGCAGAACTACCTGCAAGA | 271 |
| CST7 | NM_003650 | T2109/CST7.r1 | TGCTTCAAGGTGTGGTTGG | 272 |
| CST7 | NM_ 003650 | T2110/CST7.p1 | CACCTGCGTCTGGATGACTGTGAC | 273 |
| CTSD | NM_001909 | S1152/CTSD.f2 | GTACATGATCCCCTGTGAGAAGGT | 274 |
| CTSD | NM_001909 | S1153/CTSD.r2 | GGGACAGCTTGTAGCCTTTGC | 275 |
| CTSD | NM_001909 | S4841/CTSD.p2 | ACCCTGCCCGCGATCACACTGA | 276 |
| CTSL | NM_001912 | S1303/CTSL.f2 | GGGAGGCTTATCTCACTGAGTGA | 277 |
| CTSL | NM 001912 | S1304/CTSL.r2 | CCATTGCAGCCTTCATTGC | 278 |
| CTSL | NM_001912 | S4899/CTSL.p2 | TTGAGGCCCAGAGCAGTCTACCAG ATTCT | 279 |
| CTSL2 | NM_001333 | S4354/CTSL2.f1 | TGTCTCACTGAGCGAGCAGAA | 280 |
| CTSL2 | NM_001333 | S4355/CTSL2.r1 | ACCATTGCAGCCCTGATTG | 281 |
| CTSL2 | NM_001333 | S4356/CTSL2.p1 | CTTGAGGACGCGAACAGTCCACCA | 282 |
| CXCR4 | NM_003467 | S5966/CXCR4.f3 | TGACCGCTTCTACCCCAATG | 283 |
| CXCR4 | NM_003467 | S5967/CXCR4.r3 | AGGATAAGGCCAACCATGATGT | 284 |
| CXCR4 | NM_003467 | S5968/CXCR4.p3 | CTGAAACTGGAACACAACCACCCA CAAG | 285 |
| CYBA | NM 000101 | S5300/CYBA.f1 | GGTGCCTACTCCATTGTGG | 286 |
| CYBA | NM_000101 | S5301/CYBA.r1 | GTGGAGCCCTTCTTCCTCTT | 287 |
| CYBA | NM_000101 | S5302/CYBA.p1 | TACTCCAGCAGGCACACAAACACG | 288 |
| CYP1B1 | NM_000104 | S0094/CYP1B1.f3 | CCAGCTTTGTGCCTGTCACTAT | 289 |
| CYP1B1 | NM 000104 | S0096/CYP1B1.r3 | GGGAATGTGGTAGCCCAAGA | 290 |
| CYP1B1 | NM_000104 | S4996/CYP1B1.p3 | CTCATGCCACCACTGCCAACACCT C | 291 |
| CYP2C8 | NM_000770 | S1470/CYP2C8.f2 | CCGTGTTCAAGAGGAAGCTC | 292 |
| CYP2C8 | NM 000770 | S1471/CYP2C8.r2 | AGTGGGATCACAGGGTGAAG | 293 |
| CYP2C8 | NM_000770 | S4946/CYP2C8.p2 | TTTTCTCAACTCCTCCACAAGGCA | 294 |
| CYP3A4 | NM_017460 | S1620/CYP3A4.f2 | AGAACAAGGACAACATAGATCCTT ACATAT | 295 |
| CYP3A4 | NM_017460 | S1621/CYP3A4.r2 | GCAAACCTCATGCCAATGC | 296 |
| CYP3A4 | MM_017460 | S4906/CYP3A4.p2 | CACACCCTTTGGAAGTGGACCCAG AA | 297 |
| DDR1 | NM_001954 | T2156/DDR1.f1 | CCGTGTGGCTCGCTTTCT | 298 |
| DDR1 | NM_001954 | T2157/DDR1.r1 | GGAGATTTCGCTGAAGAGTAACCA | 299 |
| DDR1 | NM 001954 | T2158/DDR1.p1 | TGCCGCTTCCTCTTTGCGGG | 300 |
| DIABLO | NM 019887 | S0808/DIABLO.f1 | CACAATGGCGGCTCTGAAG | 301 |
| DIABLO | NM_019887 | S0809/DIABLO.r1 | ACACAAACACTGTCTGTACCTGAA GA | 302 |
| DIABLO | NM_019887 | S4813/DIABLO.p1 | AAGTTACGCTGCGCGACAGCCAA | 303 |
| DIAPH1 | NM_005219 | S7608/DIAPH1.f1 | CAAGCAGTCAAGGAGAACCA | 304 |
| DIAPH1 | NM_005219 | S7609/DIAPH1.r1 | AGTTTTGCTCGCCTCATCTT | 305 |
| DIAPH1 | NM_005219 | S7610/DIAPH1.p1 | TTCTTCTGTCTCCCGCCGCTTC | 306 |
| DICER1 | NM 177438 | S5294/DICER1.f2 | TCCAATTCCAGCATCACTGT | 307 |
| DICER1 | NM_177438 | S5295/DICER1.r2 | GGCAGTGAAGGCGATAAAGT | 308 |
| DICER1 | NM_177438 | S5296/DICER1.p2 | AGAAAAGCTGTTTGTCTCCCCAGC A | 309 |
| DKFZp564D0462; | NM_198569 | S4405/DKFZp5.f2 | CAGTGCTTCCATGGACAAGT | 310 |
| DKFZp564D0462: | NM_198569 | S4406/DKFZp5.r2 | TGGACAGGGATGATTGATGT | 311 |
| DKFZp564D0462: | NM_198569 | S4407/DKFZp5.p2 | ATCTCCATCAGCATGGGCCAGTTT | 312 |
| DR4 | NM 003844 | S2532/DR4.f2 | TGCACAGAGGGTGTGGGTTAC | 313 |
| DR4 | NM_003844 | S2533/DR4.r2 | TCTTCATCTGATTTACAAGCTGTAC ATG | 314 |
| DR4 | NM_003844 | S4981/DR4.p2 | CAATGCTTCCAACAATTTGTTTGCT TGCC | 315 |
| DR5 | NM 003842 | S2551/DR5.f2 | CTCTGAGACAGTGCTTCGATGACT | 316 |
| DR5 | NM_003842 | S2552/DR5.r2 | CCATGAGGCCCAACTTCCT | 317 |
| DR5 | NM_003842 | S4979/DR5.p2 | CAGACTTGGTGCCCTTTGACTCC | 318 |
| DUSP1 | NM_004417 | S7476/DUSP1.f1 | AGACATCAGCTCCTGGTTCA | 319 |
| DUSP1 | NM 004417 | S7477/DUSP1.r1 | GACAAACACCCTTCCTCCAG | 320 |
| DUSP1 | NM_004417 | S7478/DUSP1.p1 | CGAGGCCATTGACTTCATAGACTC CA | 321 |
| EEF1D | NM 001960 | T2159/EEF1 D.f1 | CAGAGGATGACGAGGATGATGA | 322 |
| EEF1D | NM_001960 | T2160/EEF1D.r1 | CTGTGCCGCCTCCTTGTC | 323 |
| EEF1D | NM 001960 | T2161/EEF1D.p1 | CTCCTCATTGTCACTGCCAAACAG GTCA | 324 |
| EGFR | NM_005228 | S0103/EGFR.f2 | TGTCGATGGACTTCCAGAAC | 325 |
| EGFR | NM_005228 | S0105/EGFR.r2 | ATTGGGACAGCTTGGATCA | 326 |
| EGFR | NM_005228 | S4999/EGFR.p2 | CACCTGGGCAGCTGCCAA | 327 |
| EIF4E | NM 001968 | S0106/EIF4E.f1 | GATCTAAGATGGCGACTGTCGAA | 328 |
| EIF4E | NM 001968 | S0108/EIF4E.r1 | TTAGATTCCGTTTTCTCCTCTTCTG | 329 |
| EIF4E | NM_001968 | S5000/EIF4E.p1 | ACCACCCCTACTCCTAATCCCCCG | 330 |

| Gene Name | Accession # | Oligo Name | Oliqo Sequence | SEQ ID NO |
|---|---|---|---|---|
| | | | ACT | |
| EIF4EL3 | NM 004846 | 54495/EIF4EL.f1 | AAGCCGCGGTTGAATGTG | 331 |
| EIF4EL3 | NM_004846 | S4496/EIF4EL.r1 | TGACGCCAGCTTCAATGATG | 332 |
| EIF4EL3 | NM_004846 | S4497/EIF4EL.p1 | TGACCCTCTCCCTCTCTGGATGGC A | 333 |
| ELP3 | NM_018091 | T2234/ELP3.f1 | CTCGGATCCTAGCCCTCG | 334 |
| ELP3 | NM_018091 | T2235/ELP3.r1 | GGCATTGGAATATCCCTCTGTA | 335 |
| ELP3 | NM_018091 | T2236/ELP3.p1 | CCTCCATGGACTCGAGTGTACCGA | 336 |
| ER2 | NM 001437 | S0109/ER2.f2 | TGGTCCATCGCCAGTTATCA | 337 |
| ER2 | NM_001437 | S0111/ER2.r2 | TGTTCTAGCGATCTTGCTTCACA | 338 |
| ER2 | NM 001437 | S5001/ER2.p2 | ATCTGTATGCGGAACCTCAAAAGA GTCCCT | 339 |
| ErbB3 | NM_001982 | S0112/ErbB3.f1 | CGGTTATGTCATGCCAGATACAC | 340 |
| ErbB3 | NM_001982 | S0114/ErbB3.r1 | GAACTGAGACCCACTGAAGAAAGG | 341 |
| ErbB3 | NM_001982 | S5002/ErbB3.p1 | CCTCAAAGGTACTCCCTCCTCCCG G | 342 |
| ERBB4 | NM_005235 | S1231/ERBB4.f3 | TGGCTCTTAATCAGTTTCGTTACCT | 343 |
| ERBB4 | NM_005235 | S1232/ERBB4.r3 | CAAGGCATATCGATCCTCATAAAGT | 344 |
| ERBB4 | NM_005235 | S4891/ERBB4.p3 | TGTCCCACGAATAATGCGTAAATTC TCCAG | 345 |
| ERCC1 | NM 001983 | S2437/ERCC1.f2 | GTCCAGGTGGATGTGAAAGA | 346 |
| ERCC1 | NM_001983 | S2438/ERCC1.r2 | CGGCCAGGATACACATCTTA | 347 |
| ERCC1 | MM_001983 | S4920/ERCC1.p2 | CAGCAGGCCCTCAAGGAGCTG | 348 |
| ERK1 | MM_002746 | S1560/ERK1.f3 | ACGGATCACAGTGGAGGAAG | 349 |
| ERK1 | MM_002746 | S1561/ERK1.r3 | CTCATCCGTCGGGTCATAGT | 350 |
| ERK1 | NM_002746 | S4882/ERK1.p3 | CGCTGGCTCACCCCTACCTG | 351 |
| ESPL1 | NM_012291 | S5686/ESPL1.f3 | ACCCCCAGACCGGATCAG | 352 |
| ESPL1 | NM_012291 | S5687/ESPL1.r3 | TGTAGGGCAGACTTCCTCAAACA | 353 |
| ESPL1 | NM_012291 | S5688/ESPL1.p3 | CTGGCCCTCATGTCCCCTTCACG | 354 |
| EstR1 | NM_000125 | S0115/EstR1.f1 | CGTGGTGCCCCTCTATGAC | 355 |
| EstR1 | NM 000125 | S0117/EstR1.r1 | GGCTAGTGGGCGCATGTAG | 356 |
| EstR1 | NM_000125 | S4737/EstR1.p1 | CTGGAGATGCTGGACGCCC | 357 |
| fas | NM_000043 | S0118/fas.f1 | GGATTGCTCAACAACCATGCT | 358 |
| fas | NM_000043 | S0120/fas.r1 | GGCATTAACACTTTTGGACGATAA | 359 |
| fas | NM_000043 | S5003/fas.p1 | TCTGGACCCTCCTACCTCTGGTTC TTACGT | 360 |
| fasl | NM_000639 | S0121/fasl.f2 | GCACTTTGGGATTCTTTCCATTAT | 361 |
| fasl | NM 000639 | S0123/fasl.r2 | GCATGTAAGAAGACCCTCACTGAA | 362 |
| fasl | NM_000639 | S5004/fasl.p2 | ACAACATTCTCGGTGCCTGTAACA AAGAA | 363 |
| FASN | NM 004104 | S8287/FASN.f1 | GCCTCTTCCTGTTCGACG | 364 |
| FASN | MM_004104 | S8288/FASN.r1 | GCTTTGCCCGGTAGCTCT | 365 |
| FASN | MM_004104 | S8289/FASN.p1 | TCGCCCACCTACGTACTGGCCTAC | 366 |
| FBXO5 | NM_012177 | S2017/FBXO5.r1 | GGATTGTAGACTGTCACCGAAATT C | 367 |
| FBXO5 | NM_012177 | S2018/FBXO5.f1 | GGCTATTCCTCATTTTCTCTACAAA GTG | 368 |
| FBXO5 | NM_012177 | S5061/FBX05.p1 | CCTCCAGGAGGCTACCTTCTTCAT GTTCAC | 369 |
| FDFT1 | NM_004462 | T2006/FDFT1.f1 | AAGGAAAGGGTGCCTCATC | 370 |
| FDFT1 | NM 004462 | T2007/FDFT1.r1 | GAGCCACAAGCAGCACAGT | 371 |
| FDFT1 | NM_004462 | T2008/FDFTt .p1 | CATCACCCACAAGGACAGGTTGCT | 372 |
| FGFR1 | NM_023109 | S0818/FGFR1.f3 | CACGGGACATTCACCACATC | 373 |
| FGFR1 | MM_023109 | S0819/FGFR1.r3 | GGGTGCCATCCACTTCACA | 374 |
| FGFR1 | NM_023109 | S4816/FGFR1.p3 | ATAAAAAGACAACCAACGGCCGAC TGC | 375 |
| FHIT | NM_002012 | S2443/FHIT.f1 | CCAGTGGAGCGCTTCCAT | 376 |
| FHIT | NM 002012 | S2444/FHIT.r1 | CTCTCTGGGTCGTCTGAAACAA | 377 |
| FHIT | NM 002012 | S4921/FHIT.p1 | TCGGCCACTTCATCAGGACGCAG | 378 |
| FIGF | NM_004469 | S8941/FIGF.f1 | GGTTCCAGCTTTCTGTAGCTGT | 379 |
| FIGF | NM_004469 | S8942/FIGF.r1 | GCCGCAGGTTCTAGTTGCT | 380 |
| FIGF | NM_004469 | S8943/FIGF.p1 | ATTGGTGGCCACACCACCTCCTTA | 381 |
| FLJ20354 (DEPDC1 official) | NM_017779 | S4309/FLJ203.f1 | GCGTATGATTTCCCGAATGAG | 382 |
| FLJ20354 (DEPDC1 official) | NM_017779 | S4310/FLJ203.r1 | CAGTGACCTCGTACCCATTGC | 383 |
| FLJ20354 (DEPDC1 official) | NM_017779 | S4311/FLJ203.p1 | ATGTTGATATGCCCAAACTTCATGA | 384 |
| FOS | NM_005252 | S6726/FOS.f1 | CGAGCCCTTTGATGACTTCCT | 385 |
| FOS | NM 005252 | S6727/FOS.r1 | GGAGCGGGCTGTCTCAGA | 386 |
| FOS | NM 005252 | S6728/FOS.p1 | TCCCAGCATCATCCAGGCCCAG | 387 |
| FOXM1 | NM_021953 | S2006/FOXM1.f1 | CCACCCCGAGCAAATCTGT | 388 |
| FOXM1 | NM_021953 | S2007/FOXM1.r1 | AAATCCAGTCCCCCTACTTTGG | 389 |
| FOXM1 | NM_021953 | S4757/FOXM1.p1 | CCTGAATCCTGGAGGCTCACGCC | 390 |
| FUS | NM_004960 | S2936/FUS.f1 | GGATAATTCAGACAACAACACCAT CT | 391 |
| FUS | NM_004960 | S2937/FUS.r1 | TGAAGTAATCAGCCACAGACTCAA T | 392 |
| FUS | NM_004960 | S4801/FUS.p1 | TCAATTGTAACATTCTCACCCAGGC CTTG | 393 |
| FYN | NM_002037 | S5695/FYN.f3 | GAAGCGCAGATCATGAAGAA | 394 |
| FYN | NM_02037 | S5696/FYN.r3 | CTCCTCAGACACCACTGCAT | 395 |
| FYN | NM_002037 | S5697/FYN.p3 | CTGAAGCACGACAAGCTGGTCCAG | 396 |
| G1P3 | NM_002038 | T1086/G1P3.f1 | CCTCCAACTCCTAGCCTCAA | 397 |
| G1P3 | NM_002038 | T1087/G1P3.r1 | GGCGCATGCTTGTAATCC | 398 |
| G1P3 | NM_002038 | T1088/G1P3.p1 | TGATCCTCCTGTCTCAACCTCCCA | 399 |
| GADD45 | NM 001924 | S5835/GADD45.f3 | GTGCTGGTGACGAATCCA | 400 |
| GADD45 | NM 001924 | S5836/GADD45.r3 | CCCGGCAAAAACAAATAAGT | 401 |
| GADD45 | NM 001924 | S5837/GADD45.p3 | TTCATCTCAATGGAAGGATCCTGC C | 402 |
| GADD45B | NM_015675 | S6929/GADD45.f1 | ACCCTCGACAAGACCACACT | 403 |
| GADD45B | NM_015675 | S6930/GADD45.r1 | TGGGAGTTCATGGGTACAGA | 404 |
| GADD45B | NM_015675 | S6931/GADD45.p1 | AACTTCAGCCCCAGCTCCCAAGTC | 405 |
| GAGE1 | NM_001468 | T2162/GAGE1.f1 | AAGGGCAATCACAGTGTTAAAAGA A | 406 |

| Gene Name | Accession # | Oligo Name | Oligo Sequence | SEQ ID NO |
|---|---|---|---|---|
| GAGE1 | NM_001468 | T2163/GAGE1.r1 | GGAGAACTTCAATGAAGAATTTTCC A | 407 |
| GAGE1 | NM_001468 | T2164/GAGE1.p1 | CATAGGAGCAGCCTGCAACATTTC AGCAT | 408 |
| GAPDH | NM_002046 | S0374/GAPDH.f1 | ATTCCACCCATGGCAAATTC | 409 |
| GAPDH | NM_002046 | S0375/GAPDH.r1 | GATGGGATTTCCATTGATGACA | 410 |
| GAPDH | NM 002046 | S4738/GAPDH.p1 | CCGTTCTCAGCCTTGACGGTGC | 411 |
| GATA3 | NM_002051 | S0127/GATA3.f3 | CAAAGGAGCTCACTGTGGTGTCT | 412 |
| GATA3 | NM_002051 | S0129/GATA3.r3 | GAGTCAGAATGGCTTATTCACAGA TG | 413 |
| GATA3 | NM_002051 | S5005/GATA3.p3 | TGTTCCAACCACTGAATCTGGACC | 414 |
| GBP1 | NM_002053 | S5698/GBP1.f1 | TTGGGAAATATTTGGGCATT | 415 |
| GBP1 | NM_002053 | S5699/GBP1.r1 | AGAAGCTAGGGTGGTTGTCC | 416 |
| GBP1 | NM_002053 | S5700/GBP1.p1 | TTGGGACATTGTAGACTTGGCCAG AC | 417 |
| GBP2 | NM_004120 | S5707/GBP2.f2 | GCATGGGAACCATCAACCA | 418 |
| GBP2 | NM 004120 | S5708/GBP2.r2 | TGAGGAGTTTGCCTTGATTCG | 419 |
| GBP2 | NM_004120 | S5709/GBP2.p2 | CCATGGACCAACTTCACTATGTGA CAGAGC | 420 |
| GCLC | NM_001498 | S0772/GCLC.f3 | CTGTTGCAGGAAGGCATTGA | 421 |
| GCLC | NM_001498 | S0773/GCLC.r3 | GTCAGTGGGTCTCTAATAAAGAGA TGAG | 422 |
| GCLC | NM_001498 | S4803/GCLC.p3 | CATCTCCTGGCCCAGCATGTT | 423 |
| GDF15 | NM_004864 | S7806/GDF15.f1 | CGCTCCAGACCTATGATGACT | 424 |
| GDF15 | MM_004864 | S7807/GDF15.r1 | ACAGTGGAAGGACCAGGACT | 425 |
| GDF15 | NM_004864 | S7808/GDF15.p1 | TGTTAGCCAAAGACTGCCACTGCA | 426 |
| GGPS1 | NM_004837 | S1590/GGPS1.f1 | CTCCGACGTGGCTTTCCA | 427 |
| GGPS1 | NM_004837 | S1591/GGPS1.r1 | CGTAATTGGCAGAATTGATGACA | 428 |
| GGPS1 | NM_004837 | S4896/GGPS1.p1 | TGGCCCACAGCATCTATGGAATCC C | 429 |
| GLRX | NM_002064 | T2165/GLRX.f1 | GGAGCTCTGCAGTAACCACAGAA | 430 |
| GLRX | NM_002064 | T2166/GLRX.r1 | CAATGCCATCCAGCTCTTGA | 431 |
| GLRX | NM 002064 | T2167/GLRX.p1 | AGGCCCCATGCTGACGTCCCTC | 432 |
| GNS | NM_002076 | T2009/GNS.f1 | GGTGAAGGTTGTCTCTTCCG | 433 |
| GNS | NM_002076 | T2010/GNS.r1 | CAGCCCTTCCACTTGTCTG | 434 |
| GNS | NM_002076 | T2011/GNS.p1 | AAGAGCCCTGTCTTCAGAAGGCCC | 435 |
| GPR56 | NM_005682 | T2120/GPR56.f1 | TACCCTTCCATGTGCTGGAT | 436 |
| GPR56 | NM 005682 | T2121/GPR56.r1 | GCTGAAGAGGCCCAGGTT | 437 |
| GPR56 | NM_005682 | T2122/GPR56.p1 | CGGGACTCCCTGGTCAGCTACATC | 438 |
| GPX1 | NM_000581 | S8296/GPX1.f2 | GCTTATGACCGACCCCAA | 439 |
| GPX1 | NM_000581 | S8297/GPX1.r2 | AAAGTTCCAGGCAACATCGT | 440 |
| GPX1 | NM_000581 | S8298/GPX1.p2 | CTCATCACCTGGTCTCCGGTGTGT | 441 |
| GRB7 | NM 005310 | S0130/GRB7.f2 | CCATCTGCATCCATCTTGTT | 442 |
| GRB7 | NM_005310 | S0132/GRB7.r2 | GGCCACCAGGGTATTATCTG | 443 |
| GRB7 | NM_005310 | S4726/GRB7.p2 | CTCCCCACCCTTGAGAAGTGCCT | 444 |
| GSK3B | NM_002093 | T0408/GSK3B.f2 | GACAAGGACGGCAGCAAG | 445 |
| GSK3B | NM 002093 | T0409/GSK3B.r2 | TTGTGGCCTGTCTGGACC | 446 |

| Gene Name | Accession # | Oliqo Name | Oligo Sequence | SEQ ID NO |
|---|---|---|---|---|
| GSK3B | NM_002093 | T0410/GSK3B.p2 | CCAGGAGTTGCCACCACTGTTGTC | 447 |
| GSR | NM 000637 | S8633/GSR.f1 | GTGATCCCAAGCCCACAATA | 448 |
| GSR | NM_000637 | S8634/GSR.r1 | TGTGGCGATCAGGATGTG | 449 |
| GSR | NM_000637 | S8635/GSR.p1 | TCAGTGGGAAAAAGTACACCGCCC | 450 |
| GSTM1 | NM_000561 | S2026/GSTM1.r1 | GGCCCAGCTTGAATTTTTCA | 451 |
| GSTM1 | NM_000561 | S2027/GSTM1.f1 | AAGCTATGAGGAAAAGAAGTACAC GAT | 452 |
| GSTM1 | NM_000561 | S4739/GSTM1.p1 | TCAGCCACTGGCTTCTGTCATAATC AGGAG | 453 |
| GSTp | NM_000852 | S0136/GSTp.f3 | GAGACCCTGCTGTCCCAGAA | 454 |
| GSTp | NM 000852 | S0138/GSTp.r3 | GGTTGTAGTCAGCGAAGGAGATC | 455 |
| GSTp | NM_000852 | S5007/GSTp.p3 | TCCCACAATGAAGGTCTTGCCTCC CT | 456 |
| GUS | NM 000181 | S0139/GUS.f1 | CCCACTCAGTAGCCAAGTCA | 457 |
| GUS | M_000181 | S0141/GUS.r1 | CACGCAGGTGGTATCAGTCT | 458 |
| GUS | NM 000181 | S4740/GUS.p1 | TCAAGTAAACGGGCTGTTTTCCAAA[?] CA | 459 |
| HDAC6 | NM_006044 | S9451/HDAC6.f1 | TCCTGTGCTCTGGAAGCC | 460 |
| HDAC6 | NM-006044 | S9452/HDAC6.r1 | CTCCACGGTCTCAGTTGATCT | 461 |
| HDAC6 | NM_006044 | S9453/HDAC6.p1 | CAAGAACCTCCCAGAAGGGCTCAA | 462 |
| HER2 | NM 004448 | S0142/HER2.f3 | CGGTGTGAGAAGTGCAGCAA | 463 |
| HER2 | NM_004448 | S0144/HER2.r3 | CCTCTCGCAAGTGCTCCAT | 464 |
| HER2 | NM_004448 | S4729/HER2.p3 | CCAGACCATAGCACACTCGGGCAC | 465 |
| HIF1A | NM_001530 | S1207/HIF1A.f3 | TGAACATAAAGTCTGCAACATGGA | 466 |
| HIF1A | NM_001530 | S1208/HIF1A.r3 | TGAGGTTGGTTACTGTTGGTATCAT ATA | 467 |
| HIF1A | NM_001530 | S4753/HIF1A.p3 | TTGCACTGCACAGGCCACATTCAC | 468 |
| HNF3A | NM 004496 | S0148/HNF3A.f1 | TCCAGGATGTTAGGAACTGTGAAG | 469 |
| HNF3A | NM 004496 | S0150/HNF3A.r1 | GCGTGTCTGCGTAGTAGCTGTT | 470 |
| HNF3A | NM_004496 | S5008/HNF3A.p1 | AGTCGCTGGTTTCATGCCCTTCCA | 471 |
| HRAS | NM_005343 | S8427/HRAS.f1 | GGACGAATACGACCCCACT | 472 |
| HRAS | NM_005343 | S8428/HRAS.r1 | GCACGTCTCCCCATCAAT | 473 |
| HRAS | NM 005343 | S8429/HRAS.p1 | ACCACCTGCTTCCGGTAGGAATCC | 474 |
| HSPA1A | NM_005345 | S6708/HSPA1A.f1 | CTGCTGCGACAGTCCACTA | 475 |
| HSPA1A | NM_005345 | S6709/HSPA1A.r1 | CAGGTTCGCTCTGGGAAG | 476 |
| HSPA1A | NM_005345 | S6710/HSPA1A.p1 | AGAGTGACTCCCGTTGTCCCAAGG | 477 |
| HSPA1B | NM_005346 | S6714/HSPA1B.f1 | GGTCCGCTTCGTCTTTCGA | 478 |
| HSPA1B | NM 005346 | S6715/HSPA1B.r1 | GCACAGGTTCGCTCTGGAA | 479 |
| HSPA1B | NM_005346 | S6716/HSPA1B.p1 | TGACTCCCGCGGTCCCAAGG | 480 |
| HSPA1L | NM_005527 | T2015/HSPA1L.f1 | GCAGGTGTGATTGCTGGAC | 481 |
| HSPA1L | NM_005527 | T2016/HSPA1L.r1 | ACCATAGGCAATGGCAGC | 482 |
| HSPA1L | NM_005527 | T2017/HSPA1L.p1 | AAGAATCATCAATGAGCCCACGGC | 483 |
| HSPA5 | NM 005347 | S7166/HSPA5.f1 | GGCTAGTAGAACTGGATCCCAACA | 484 |
| HSPA5 | NM_005347 | S7167/HSPA5.r1 | GGTCTGCCCAAATGCTTTTC | 485 |
| HSPA5 | NM_005347 | S7168/HSPA5.p1 | TAATTAGACCTAGGCCTCAGCTGC ACTGCC | 86 |
| HSPA9B | NM_004134 | T2018/HSPA9B.f1 | GGCCACTAAAGATGCTGGC | 487 |
| HSPA9B | NM_004134 | T2019/HSPA9B.r1 | AGCAGCTGTGGGCTCATT | 488 |
| HSPA9B | NM 004134 | T2020/HSPA9B.p1 | ATCACCCGAAGCACATTCAGTCCA | 489 |
| HSPB1 | MM_001540 | S6720/HSPB1.f1 | CCGACTGGAGGAGCATAAA | 490 |
| HSPB1 | NM_001540 | S6721/HSPB1.r1 | ATGCTGGCTGACTCTGCTC | 491 |
| HSPB1 | MM_001540 | S6722/HSPB1.p1 | CGCACTTTTCTGAGCAGACGTCCA | 492 |
| HSPCA | NM 005348 | S7097/HSPCA.f1 | CAAAAGGCAGAGGCTGATAA | 493 |
| HSPCA | NM 005348 | S7098/HSPCA.r1 | AGCGCAGTTTCATAAAGCAA | 494 |
| HSPCA | NM_005348 | S7099/HSPCA.p1 | TGACCAGATCCTTCACAGACTTGT CGT | 495 |
| ID1 | NM_002165 | S0820/ID1.f1 | AGAACCGCAAGGTGAGCAA | 496 |
| ID1 | NM_002165 | S0821/ID1.r1 | TCCAACTGAAGGTCCCTGATG | 497 |
| ID1 | NM_002165 | S4832/ID1.p1 | TGGAGATTCTCCAGCACGTCATCG AC | 498 |
| IFITM1 | NM_003641 | S7768/IFTM1.f1 | CACGCAGAAAACCACACTTC | 499 |
| IFITM1 | NM_003641 | S7769/IFITM1.r1 | CATGTTCCTCCTTGTGCATC | 500 |
| IFITM1 | NM 003641 | S7770/IFITM1.p1 | CAACACTTCCTTCCCCAAAGCCAG | 501 |
| IGF1R | NM_000875 | S1249/IGF1R.f3 | GCATGGTAGCCGAAGATTTCA | 502 |
| IGF1R | NM 000875 | S1250/IGF1R.r3 | TTTCCGGTAATAGTCTGTCTCATAG ATATC | 503 |
| IGF1R | NM_000875 | S4895/IGF1R.p3 | CGCGTCATACCAAAATCTCCGATTT TGA | 504 |
| IGFBP2 | NM_000597 | S1128/IGFBP2.f1 | GTGGACAGCACCATGAACA | 505 |
| IGFBP2 | NM_000597 | S1129/IGFBP2.r1 | CCTTCATACCCGACTTGAGG | 506 |
| IGFBP2 | NM_000597 | S4837/IGFBP2.p1 | CTTCCGGCCAGCACTGCCTC | 507 |
| IGFBP3 | NM_000598 | S0157/IGFBP3.f3 | ACGCACCGGGTGTCTGA | 508 |
| IGFBP3 | NM_000598 | S0159/IGFBP3.r3 | TGCCCTTTCTTGATGATGATTATC | 509 |
| IGFBP3 | NM_000598 | S5011/IGFBP3.p3 | CCCAAGTTCCACCCCCTCCATTCA | 510 |
| IGFBP5 | NM_000599 | S1644/IGFBP5.f1 | TGGACAAGTACGGGATGAAGCT | 511 |
| IGFBP5 | NM_000599 | S1645/IGFBP5.r1 | CGAAGGTGTGGCACTGAAAGT | 512 |
| IGFBP5 | NM_000599 | S4908/IGFBP5.p1 | CCCGTCAACGTACTCCATGCCTGG- | 513 |
| IL-7 | NM 000880 | S5781/IL-7.f1 | GCGGTGATTCGGAAATTCG | 514 |
| IL-7 | NM_000880 | S5782/IL-7.r1 | CTCTCCTGGGCACCTGCTT | 515 |
| IL-7 | NM_000880 | S5783/IL-7.p1 | CTCTGGTCCTCATCCAGGTGCGC | 516 |
| IL-8 | NM_000584 | S5790/IL-8.f1 | AAGGAACCATCTCACTGTGTGTAA AC | 517. |
| IL-8 | NM_000584 | S5791/IL-8.r1 | ATCAGGAAGGCTGCCAAGAG | 518 |
| IL-8 | NM_000584 | S5792/IL-8.p1 | TGACTTCCAAGCTGGCCGTGGC | 519 |
| IL2RA | NM 000417 | T2147/IL2RA.f1 | TCTGCGTGGTTCCTTTCTCA | 520 |
| IL2RA | NM 000417 | T2148/IL2RA.r1 | TTGAAGGATGTTTATTAGGCAACGT | 521 |
| IL2RA | NM 000417 | T2149/IL2RA.p1 | CGCTTCTGACTGCTGATTCTCCCG TT | 522 |
| IL6 | NM_000600 | S0760/IL6.f3 | CCTGAACCTTCCAAAGATGG | 523 |
| IL6 | NM_000600 | S0761/IL6.r3 | ACCAGGCAAGTCTCCTCATT | 524 |
| IL6 | NM_000600 | S4800/IL6.p3 | CCAGATTGGAAGCATCCATCTTTTT CA | 525 |
| IL8RB | NM_001557 | T2168/IL8RB.f1 | CCGCTCCGTCACTGATGTCT | 526 |
| IL8RB | NM 001557 | T2169/IL8RB.r1 | GCAAGGTCAGGGCAAAGAGTA | 527 |

| Gene Name | Accession # | Oligo Name | Oligo Sequence | SEQ ID NO |
|---|---|---|---|---|
| IL8RB | NM_001557 | T2170/IL8RB.p1 | CCTGCTGAACCTAGCCTTGGCCGA | 528 |
| ILK | NM 001014794 | T0618/ILK.f1 | CTCAGGATTTTCTCGCATCC | 529 |
| ILK | NM_001014794 | T0619/ILK.r1 | AGGAGCAGGTGGAGACTGG | 530 |
| ILK | NM_001014794 | T0620/ILK.p1 | ATGTGCTCCCAGTGCTAGGTGCCT | 531 |
| ILT-2 | NM_006669 | S1611/ILT-2.f2 | AGCCATCACTCTCAGTGCAG | 532 |
| ILT-2 | NM 006669 | S1612/I T-2.r2 | ACTGCAGAGTCAGGGTCTCC | 533 |
| ILT-2 | NM_006669 | S4904/ILT-2.p2 | CAGGTCCTATCGTGGCCCCTGA | 534 |
| INCENP | NM_020238 | T2024/INCENP.f1 | GCCAGGATACTGGAGTCCATC | 535 |
| INCENP | NM_020238 | T2025/INCENP.r1 | CTTGACCCTTGGGGTCCT | 536 |
| INCENP | NM_020238 | T2026/INCENP.p1 | TGAGCTCCCTGATGGCTACACCC | 537 |
| IRAK2 | NM 001570 | T2027/IRAK2.f1 | GGATGGAGTTCGCCTCCT | 538 |
| IRAK2 | NM 001570 | T2028/IRAK2.r1 | CGCTCCATGGACTTGATCTT | 539 |
| IRAK2 | NM_001570 | T2029/IRAK2.p1 | CGTGATCACAGACCTGACCCAGCT | 540 |
| IRS1 | NM_005544 | S1943/IRS1.f3 | CCACAGCTCACCTTCTGTCA | 541 |
| IRS1 | NM_005544 | S1944/IRS1.r3 | CCTCAGTGCCAGTCTCTTCC | 542 |
| IRS1 | NM 005544 | S5050/IRS1.p3 | TCCATCCCAGCTCCAGCCAG | 543 |
| ITGB1 | NM_002211 | S7497/ITGB1.f1 | TCAGAATTGGATTTGGCTCA | 544 |
| ITGB1 | NM_002211 | S7498/ITGB1.r1 | CCTGAGCTTAGCTGGTGTTG | 545 |
| ITGB1 | NM_002211 | S7499/ITGBI.p1 | TGCTAATGTAAGGCATCACAGTCTT[?] TTCCA | 546 |
| K-Alpha-1 | NM_006082 | S8706/K-Alph.f2 | TGAGGAAGAAGGAGAGGAATACTA AT | 547 |
| K-Alpha-1 | NM_006082 | S8707/K-Alph.r2 | CTGAAATTCTGGGAGCATGAC | 548 |
| K-Alpha-1 | NM_006082 | S8708/K-Alph.p2 | TATCCATTCCTTTTGGCCCTGCAG | 549 |
| KDR | NM_002253 | S1343/KDR.f6 | GAGGACGAAGGCCTCTACAC | 550 |
| KDR | NM_002253 | S1344/KDR.r6 | AAAAATGCCTCCACTTTTGC | 551 |
| KDR | NM_002253 | S4903/KDR.p6 | CAGGCATGCAGTGTTCTTGGCTGT | 552 |
| Ki-67 | NM_002417 | S0436/Ki-67.f2 | CGGACTTTGGGTGCGACTT | 553 |
| Ki-67 | NM_002417 | S0437/Ki-67.r2 | TTACAACTCTTCCACTGGGACGAT | 554 |
| Ki-67 | NM 002417 | S4741/Ki-67.p2 | CCACTTGTCGAACCACCGCTCGT | 555 |
| KIF11 | NM_004523 | T2409/KIF11.f2 | TGGAGGTTGTAAGCCAATGT | 556 |
| KIF11 | NM_004523 | T2410/KIF11.r2 | TGCCTTACGTCCATCTGATT | 557 |
| KIF11 | NM_004523 | T2411/KIF11.p2 | CAGTGATGTCTGAACTTGAAGCCT CACA | 558 |
| KIF22 | NM_007317 | S8505/KIF22.f1 | CTAAGGCACTTGCTGGAAGG | 559 |
| KIF22 | NM_007317 | S8506/KIF22.r1 | TCTTCCCAGCTCCTGTGG | 560 |
| KIF22 | NM_007317 | S8507/KIF22.p1 | TCCATAGGCAAGCACACTGGCATT | 561 |
| KIF2C | NM 006845 | S7262/KIF2C.f1 | AATTCCTGCTCCAAAAGAAAGTCTT | 562 |
| KIF2C | NM_006845 | S7263/KIF2C.r1 | CGTGATGCGAAGCTCTGAGA | 563 |
| KIF2C | NM_006845 | S7264/KIFC.p1 | AAGCCGCTCCACTCGCATGTCC | 564 |
| KIFC1 | NM_002263 | S8517/KIFC1.f1 | CCACAGGGTTGAAGAACCAG | 565 |
| KIFC1 | NM_002263 | S8519/KIFC1.r1 | CACCTGATGTGCCAGACTTC | 566 |
| KIFC1 | NM 002263 | S8520/KIFC1.p1 | AGCCAGTTCCTGCTGTTCCTGTCC | 567 |
| KLK10 | NM_002776 | S2624/KLK10.f3 | GCCCAGAGGCTCCATCGT | 568 |
| KLK10 | NM_002776 | S2625/KLK10.r3 | CAGAGGTTTGAACAGTGCAGACA | 569 |
| KLK10 | NM_002776 | S4978/KLK10.p3 | CCTCTTCCTCCCCAGTCGGCTGA | 570 |
| KNS2 | NM_005552 | T2030/KNS2.f1 | CAAACAGAGGGTGGCAGAAG | 571 |
| KNS2 | NM 005552 | T2031/KNS2.r1 | GAGGCTCTCACGGCTCCT | 572 |
| KNS2 | NM_005552 | T2032/KNS2.p1 | CGCTTCTCCATGTTCTCAGGGTCA | 573 |
| KNTC1 | NM_014708 | T2126/KNTC1.f1 | AGCCGAGGCTTTGTTGAA | 574 |
| KNTC1 | NM_014708 | T2127/KNTC1.r1 | TGGGCTATGAGCACAGCTT | 575 |
| KNTC1 | NM 014708 | T2128/KNTC1.p1 | TTCATATCCAGTACCGGCGATCGG | 576 |
| KNTC2 | NM 006101 | S7296/KNTC2.f1 | ATGTGCCAGTGAGCTTGAGT | 577 |
| KNTC2 | NM_006101 | S7297/KNTC2.r1 | TGAGCCCCTGGTTAACAGTA | 578 |
| KNTC2 | NM_006101 | S7298/KNTC2.p1 | CCTTGGAGAAACACAAGCACCTGC | 579 |
| KRT14 | NM_000526 | S1853/KRT14.f1 | GGCCTGCTGAGATCAAAGAC | 580 |
| KRT14 | NM_000526 | S1854/KRT14.r1 | GTCCACTGTGGCTGTGAGAA | 581 |
| KRT14 | NM_000526 | S5037/KRT14.p1 | TGTTCCTCAGGTCCTCAATGGTCTT G | 582 |
| KRT17 | NM 000422 | S0172/KRT17.f2 | CGAGGATTGGTTCTTCAGCAA | 583 |
| KRT17 | NM_000422 | S0173/KRT17.p2 | CACCTCGCGGTTCAGTTCCTCTGT | 584 |
| KRT17 | NM_000422 | S0174/KRT17.r2 | ACTCTGCACCAGCTCACTGTTG | 585 |
| KRT19 | NM_002276 | S1515/KRT19.f3 | TGAGCGGCAGAATCAGGAGTA | 586 |
| KRT19 | NM 002276 | S1516/KRT19.r3 | TGCGGTAGGTGGCAATCTC | 587 |
| KRT19 | NM 002276 | S4866/KRT19.p3 | CTCATGGACATCAAGTCGCGGCTG | 588 |
| KRT5 | NM_000424 | S0175/KRT5.f3 | TCAGTGGAGAAGGAGTTGGA | 589 |
| KRT5 | NM_000424 | S0177/KRT5.r3 | TGCCATATCCAGAGGAAACA | 590 |
| KRT5 | NM_000424 | S5015/KRT5.p3 | CCAGTCAACATCTCTGTTGTCACAA GCA | 591 |
| L1CAM | NM_000425 | T1341/L1CAM.f1 | CTTGCTGGCCAATGCCTA | 592 |
| L1CAM | NM_000425 | T1342/L1CAM.r1 | TGATTGTCCGCAGTCAGG | 593 |
| L1CAM | NM_000425 | T1343/L1CAM.p1 | ATCTACGTTGTCCAGCTGCCAGCC | 594 |
| LAMC2 | NM_005562 | S2826/LAMC2.f2 | ACTCAAGCGGAAATTGAAGCA | 595 |
| LAMC2 | NM_005562 | S2827/LAMC2.r2 | ACTCCCTGAAGCCGAGACACT | 596 |
| LAMC2 | NM_005562 | S4969/LAMC2.p2 | AGGTCTTATCAGCACAGTCTCCGC CTCC | 597 |
| LAPTM4B | NM_018407 | T2063/LAPTM4.f1 | AGCGATGAAGATGGTCGC | 598 |
| LAPTM4B | NM_018407 | T2064/LAPTM4.r1 | GACATGGCAGCACAAGCA | 599 |
| LAPTM4B | NM_018407 | T2065/LAPTM4.p1 | CTGGACGCGGTTCTACTCCAACAG | 600 |
| LIMK1 | NM_016735 | T0759/LIMK1.f1 | GCTTCAGGTGTTGTGACTGC | 601 |
| LIMK1 | NM_016735 | T0760/LIMK1.r1 | AAGAGCTGCCCATCCTTCTC | 602 |
| LIMK1 | NM_016735 | T0761/LIMK1.p1 | TGCCTCCCTGTCGCACCAGTACTA | 603 |
| LIMK2 | NM_005569 | T2033/LIMK2.f1 | CTTTGGGCCAGGAGGAAT | 604 |
| LIMK2 | NM_005569 | T2034/LIMK2.r1 | CTCCCACAATCCACTGCC | 605 |
| LIMK2 | NM_005569 | T2035/LIMK2.p1 | ACTCGAATCCACCCAGGAACTCCC | 606 |
| MAD1L1 | NM_003550 | S7299/MAD1L1.f1 | AGAAGCTGTCCCTGCAAGAG | 607 |
| MAD1L1 | NM_003550 | S7300/MAD1L1.r1 | AGCCGTACCAGCTCAGACTT | 608 |
| MAD1L1 | NM_003550 | S7301/MAD1L1.p1 | CATGTTCTTCACAATCGCTGCATCC | 609 |
| MAD2L1 | NM_002358 | S7302/MAD2L1.f1 | CCGGGAGCAGGGAATCAC | 610 |
| MAD2L1 | NM_002358 | S7303/MAD2L1.r1 | ATGCTGTTGATGCCGAATGA | 611 |
| MAD2L1 | NM_002358 | S7304/MAD2L1.p1 | CGGCCACGATTTCGGCGCT | 612 |
| MAD2L1BP | NM_014628 | T2123/MAD2L1.f1 | CTGTCATGTGGCAGACCTTC | 613 |
| MAD2L1BP | NM_014628 | T2124/MAD2L1.r1 | TAAATGTCACTGGTGCCTGG | 614 |
| MAD2L1BP | NM 014628 | T2125/MAD2L1.p1 | CGAACCACGGCTTGGGAAGACTAC | 615 |
| MAD2L2 | NM_006341 | T1125/MAD2L2.f1 | GCCCAGTGGAGAAATTCGT | 616 |
| MAD2L2 | NM_006341 | T1126/MAD2L2.r1 | GCGAGTCTGAGCTGATGGA | 617 |
| MAD2L2 | NM_006341 | T1127/MAD2L2.p1 | TTTGAGATCACCCAGCCTCCACTG | 618 |
| MAGE2 | NM_005361 | S5623/MAGE2.f1 | CCTCAGAAATTGCCAGGACT | 619 |
| MAGE2 | NM 005361 | S5625/MAGE2.p1 | TTCCCGTGATCTTCAGCAAAGCCT | 620 |
| MAGE2 | NM_005361 | S5626/MAGE2.r1 | CCAAAGACCAGCTGCAAGTA | 621 |
| MAGE6 | NM_005363 | S5639/MAGE6.f3 | AGGACTCCAGCAACCAAGAA | 622 |
| MAGE6 | NM_005363 | S5640/MAGE6.r3 | GAGTGCTGCTTGGAACTCAG | 623 |
| MAGE6 | NM 005363 | S5641/MAGE6.p3 | CAAGCACCTTCCCTGACCTGGAGT | 624 |
| MAP2 | NM 031846 | S8493/MAP2.f1 | CGGACCACCAGGTCAGAG | 625 |
| MAP2 | NM_031846 | S8494/MAP2.r1 | CAGGGGTAGTGGGTGTTGAG | 626 |
| MAP2 | MM_031846 | S8495/MAP2.p1 | CCACTCTTCCCTGCTCTGCGAATT | 627 |
| MAP2K3 | NM_002756 | T2090/MAP2K3.f1 | GCCCTCCAATGTCCTTATCA | 628 |
| MAP2K3 | NM 002756 | T2091/MAP2K3.r1 | GTAGCCACTGATGCCAAAGTC | 629 |
| MAP2K3 | NM_002756 | T2092/MAP2K3.p1 | CACATCTTCACATGGCCCTCCTTG | 630 |
| MAP4 | NM_002375 | S5724/MAP4.f1 | GCCGGTCAGGCACACAAG | 631 |
| MAP4 | NM_002375 | S5725/MAP4.r1 | GCAGCATACACACAACAAAATGG | 632 |
| MAP4 | NM_002375 | S5726/MAP4.p1 | ACCAACCAGTCCACGCTCCAAGGG | 633 |
| MAP6 | NM 033063 | T2341/MAP6.f2 | CCCTCAACCGGCAAATCC | 634 |
| MAP6 | NM_033063 | T2342/MAP6.r2 | CGTCCATGCCCTGAATTCA | 635 |
| MAP6 | NM_033063 | T2343/MAP6.p2 | TGGCGAGTGCAGTGAGCAGCTCC | 636 |
| MAPK14 | NM_139012 | S5557/MAPK14.f2 | TGAGTGGAAAAGCCTGACCTATG | 637 |
| MAPK14 | NM_139012 | S5558/MAPK14.r2 | GGACTCCATCTCTTCTTGGTCAA | 638 |
| MAPK14 | NM_139012 | S5559/MAPK14.p2 | TGAAGTCATCAGCTTTGTGCCACC ACC | 639 |
| MAPK8 | NM_002750 | T2087/MAPK8.f1 | CAACACCCGTACATCAATGTCT | 640 |
| MAPK8 | NM_002750 | T2088/MAPK8.r1 | TCATCTAACTGCTTGTCAGGGA | 641 |
| MAPK8 | NM_002750 | T2089/MAPK8.p1 | CTGAAGCAGAAGCTCCACCACCAA | 642 |
| MAPRE1 | NM_012325 | T2180/MAPRE1.f1 | GACCTTGGAACCTTTGGAAC | 643 |
| MAPRE1 | NM_012325 | T2181/MAPRE1.r1 | CCTAGGCCTATGAGGGTTCA | 644 |
| MAPRE1 | NM_012325 | T2182/MAPRE1.p1 | CAGCCCTGTAAGACCTGTTGACAG CA | 645 |
| MAPT | NM_016835 | S8502/MAPT.f1 | CACAAGCTGACCTTCCGC | 646 |
| MAPT | NM 016835 | S8503/MAPT.r1 | ACTTGTACACGATCTCCGCC | 647 |
| MAPT | NM_016835 | S8504/MAPT.p1 | AGAACGCCAAAGCCAAGACAGACC | 648 |
| Maspin | NM_002639 | S0836/Maspin.f2 | CAGATGGCCACTTTGAGAACATT | 649 |
| Maspin | NM_002639 | S0837/Maspin.r2 | GGCAGCATTAACCACAAGGATT | 650 |
| Maspin | NM_002639 | S4835/Maspin.p2 | AGCTGACAACAGTGTGAACGACCA GACC | 651 |
| MCL1 | NM_021960 | S5545/MCL1.f1 | CTTCGGAAACTGGACATCAA | 652 |
| MCL1 | NM 021960 | S5546/MCL1.r1 | GTCGCTGAAAACATGGATCA | 653 |
| MCL1 | NM_021960 | S5547/MCL1.p1 | TCACTCGAGACAACGATTTCACATC G | 654 |
| MCM2 | NM_004526 | S1602/MCM2.f2 | GACTTTTGCCCGCTACCTTTC | 655 |
| MCM2 | NM_004526 | S1603/MCM2.r2 | GCCACTAACTGCTTCAGTATGAAG AG | 656 |
| MCM2 | NM_004526 | S4900/MCM2.p2 | ACAGCTCATTGTTGTCACGCCGGA | 657 |
| MCM6 | NM 005915 | S1704/MCM6.f3 | TGATGGTCCTATGTGTCACATTCA | 658 |
| MCM6 | NM_005915 | S1705/MCM6.r3 | TGGGACAGGAAACACACCAA | 659 |
| MCM6 | NM 005915 | S4919/MCM6.p3 | CAGGTTTCATACCAACACAGGCTT CAGCAC | 660 |
| MCP1 | NM_002982 | S1955/MCP1.f1 | CGCTCAGCCAGATGCAATC | 661 |
| MCP1 | NM_002982 | S1956/MCP1.r1 | GCACTGAGATCTTCCTATTGGTGA A | 662 |
| MCP1 | NM_002982 | S5052/MCP1.p1 | TGCCCCAGTCACCTGCTGTTA | 663 |
| MGMT | NM_002412 | S1922/MGMT.f1 | GTGAAATGAAACGCACCACA | 664 |
| MGMT | NM_002412 | S1923/MGMT.r1 | GACCCTGCTCACAACCAGAC | 665 |
| MGMT | NM 002412 | S5045/MGMT.p1 | CAGCCCTTTGGGGAAGCTGG | 666 |
| MMP12 | NM_002426 | S4381/MMP12.f2 | CCAACGCTTGCCAAATCCT | 667 |
| MMP12 | NM_002426 | S4382/MMP12.r2 | ACGGTAGTGACAGCATCAAAACTC | 668 |
| MMP12 | NM_002426 | S4383/MMP12.p2 | AACCAGCTCTCTGTGACCCCAATT | 669 |
| MMP2 | NM_004530 | S1874/MMP2.f2 | CCATGATGGAGAGGCAGACA | 670 |
| MMP2 | NM 004530 | S1875/MMP2.r2 | GGAGTCCGTCCTTACCGTCAA | 671 |
| MMP2 | NM_004530 | S5039/MMP2.p2 | CTGGGAGCATGGCGATGGATACCC | 672 |
| MMP9 | NM_004994 | S0656/MMP9.f1 | GAGAACCAATCTCACCGACA | 673 |
| MMP9 | NM_004994 | S0657/MMP9.r1 | CACCCGAGTGTAACCATAGC | 674 |
| MMP9 | NM 004994 | S4760/MMP9.p1 | ACAGGTATTCCTCTGCCAGCTGCC | 675 |
| MRE11 A | NM 005590 | T2039/MRE11A.f1 | GCCATGCTGGCTCAGTCT | 676 |
| MRE11 A | NM_005590 | T2040/MRE11A.r1 | CACCCAGACCCACCTAACTG | 677 |
| MRE11 A | NM_005590 | T2041/MRE11A.p1 | CACTAGCTGATGTGGCCCACAGCT | 678 |
| MRP1 | NM_004996 | S0181/MRP1.f1 | TCATGGTGCCCGTCAATG | 679 |
| MRP1 | NM_004996 | S0183/MRP1.r1 | CGATTGTCTTTGCTCTTCATGTG | 680 |
| MRP1 | NM_004996 | S5019/MRP1.p1 | ACCTGATACGTCTTGGTCTTCATCG CCAT | 681 |
| MRP2 | NM 000392 | S0184/MRP2.f3 | AGGGGATGACTTGGACACAT | 682 |
| MRP2 | NM_000392 | S0186/MRP2.r3 | AAAACTGCATGGCTTTGTCA | 683 |
| MRP2 | NM_000392 | S5021/MRP2.p3 | CTGCCATTCGACATGACTGCAATTT | 684 |
| MRP3 | NM_003786 | S0187/MRP3.f1 | TCATCCTGGCGATCTACTTCCT | 685 |
| MRP3 | NM_003786 | S0189/MRP3.r1 | CCGTTGAGTGGAATCAGCAA | 686 |
| MRP3 | NM_003786 | S5023/MRP3.p1 | TCTGTCCTGGCTGGAGTCGCTTTC AT | 687 |
| MSH3 | NM 002439 | S5940/MSH3.f2 | TGATTACCATCATGGCTCAGA | 688 |
| MSH3 | NM_002439 | S5941/MSH3.r2 | CTTGTGAAAATGCCATCCAC | 689 |
| MSH3 | NM_002439 | S5942/MSH3.p2 | TCCCAATTGTCGCTTCTTCTGCAG | 690 |
| MUC1 | NM_002456 | S0782/MUC1.f2 | GGCCAGGATCTGTGGTGGTA | 691 |
| MUC1 | NM_002456 | S0783/MUC1.r2 | CTCCACGTCGTGGACATTGA | 692 |
| MUC1 | NM 002456 | S4807/MUC1.p2 | CTCTGGCCTTCCGAGAAGGTACC | 693 |
| MX1 | NM_002462 | S7611/MX1.f1 | GAAGGAATGGGAATCAGTCATGA | 694 |
| MX1 | NM_002462 | S7612/MX1.r1 | GTCTATTAGAGTCAGATCCGGGAC AT | 695 |
| MX1 | NM_002462 | S7613/MX1.p1 | TCACCCTGGAGATCAGCTCCCGA | 696 |
| MYBL2 | NM_002466 | S3270/MYBL2.f1 | GCCGAGATCGCCAAGATG | 697 |
| MYBL2 | NM_002466 | S3271/MYBL2.r1 | CTTTTGATGGTAGAGTTCCAGTGAT TC | 698 |
| MYBL2 | NM 002466 | S4742/MYBL2.p1 | CAGCATTGTCTGTCCTCCCTGGCA | 699 |
| MYH11 | NM_002474 | S4555/MYH11.f1 | CGGTACTTCTCAGGGCTAATATATA CG | 700 |
| MYH11 | NM 002474 | S4556/MYH11.r1 | CCGAGTAGATGGGCAGGTGTT | 701 |
| MYH11 | NM_002474 | S4557/MYH11.p1 | CTCTTCTGCGTGGTGGTCAACCCC TA | 702 |
| NEK2 | NM_002497 | S4327/NEK2.f1 | GTGAGGCAGCGCGACTCT | 703 |
| NEK2 | NM_002497 | S4328/NEK2.r1 | TGCCAATGGTGTACAACACTTCA | 704 |
| NEK2 | NM_002497 | S4329/NEK2.p1 | TGCCTTCCCGGGCTGAGGACT | 705 |
| NFKBp50 | NM_003998 | S9661/NFKBp5.f3 | CAGACCAAGGAGATGGACCT | 706 |
| NFKBp50 | NM 003998 | S9662/NFKBp5.r3 | AGCTGCCAGTGCTATCCG | 707 |
| NFKBp50 | NM_003998 | S9663/NFKBp5.p3 | AAGCTGTAAACATGAGCCGCACCA | 708 |
| NFKBp65 | NM_021975 | S0196/NFKBp6.f3 | CTGCCGGGATGGCTTCTAT | 709 |
| NFKBp65 | NM_021975 | S0198/NFKBp6.r3 | CCAGGTTCTGGAAACTGTGGAT | 710 |
| NFKBp65 | NM_021975 | S5030/NFKBp6.p3 | CTGAGCTCTGCCCGGACCGCT | 711 |
| NME6 | NM 005793 | T2129/NME6.f1 | CACTGACACCCGCAACAC | 712 |
| NME6 | NM_005793 | T2130/NME6.r1 | GGCTGCAATCTCTCTGCTG | 713 |
| NME6 | NM_005793 | T2131/NME6.p1 | AACCACAGAGTCCGAACCATGGGT | 714 |
| NPC2 | NM_006432 | T2141/NPC2.f1 | CTGCTTCTTTCCCGAGCTT | 715 |
| NPC2 | NM 006432 | T2142/NPC2.r1 | AGCAGGAATGTAGCTGCCA | 716 |
| NPC2 | NM 006432 | T2143/NPC2.p1 | ACTTCGTTATCCGCGATGCGTTTC | 717 |
| NPD009 (ABAT Official) | NM_020686 | S4474/NPD009.f3 | GGCTGTGGCTGAGGCTGTAG | 718 |
| NPD009 (ABAT official) | NM_020686 | S4475/NPD009.r3 | GGAGCATTCGAGGTCAAATCA | 719 |
| NPD009 (ABAT official) | NM_020686 | S4476/NPD009.p3 | TTCCCAGAGTGTCTCACCTCCAGC AGAG | 720 |
| NTSR2 | NM 012344 | T2332/NTSR2.f2 | CGGACCTGAATGTAATGCAA | 721 |
| NTSR2 | NM_012344 | T2333/NTSR2.r2 | CTTTGCCAGGTGACTAAGCA | 722 |
| NTSR2 | NM_012344 | T2334/NTSR2.p2 | AATGAACAGAACAAGCAAAATGAC CAGC | 723 |
| NUSAP1 | NM_016359 | S7106/NUSAP1.f1 | CAAAGGAAGAGCAACGGAAG | 724 |
| NUSAP1 | NM_016359 | S7107/NUSAP1.r1 | ATTCCCAAAACCTTTGCTT | 725 |
| NUSAP1 | NM_016359 | S7108/NUSAP1.p1 | TTCTCCTTTCGTTCTTGCTCGCGT | 726 |
| p21 | NM 000389 | S0202/p21.f3 | TGGAGACTCTCAGGGTCGAAA | 727 |
| p21 | NM_000389 | S0204/p21.r3 | GGCGTTTGGAGTGGTAGAAATC | 728 |
| p21 | NM_000389 | S5047/p21.p3 | CGGCGGCAGACCAGCATGAC | 729 |
| p27 | NM_004064 | S0205/p27.f3 | CGGTGGACCACGAAGAGTTAA | 730 |
| p27 | NM_004064 | S0207/p27.r3 | GGCTCGCCTCTTCCATGTC | 731 |
| p27 | NM 004064 | S4750/p27.p3 | CCGGGACTTGGAGAAGCACTGCA | 732 |
| PCTK1 | NM_006201 | T2075/PCTK1.f1 | TCACTACCAGCTGACATCCG | 733 |
| PCTK1 | NM_006201 | T2076/PCTK1.r1 | AGATGGGGCTATTGAGGGTC | 734 |
| PCTK1 | NM_006201 | T2077/PCTK1.p1 | CTTCTCCAGGTAGCCCTCAGGCAG | 735 |
| PDGFRb | NM 002609 | S1346/PDGFRb.f3 | CCAGCTCTCCTTCCAGCTAC | 736 |
| PDGFRb | NM_002609 | S1347/PDGFRb.r3 | GGGTGGCTCTCACTTAGCTC | 737 |
| PDGFRb | NM 002609 | S4931/PDGFRb.p3 | ATCAATGTCCCTGTCCGAGTGCTG | 738 |
| PFDN5 | NM_145897 | T2078/PFDN5.f1 | GAGAAGCACGCCATGAAAC | 739 |
| PFDN5 | NM_145897 | T2079/PFDN5.r1 | GGCTGTGAGCTGCTGAATCT | 740 |
| PFDN5 | NM_145897 | T2080/PFDN5.p1 | TGACTCATCATTTCCATGACGGCC | 741 |
| PGK1 | NM 000291 | S0232/PGK1.f1 | AGAGCCAGTTGCTGTAGAACTCAA | 742 |
| PGK1 | NM 000291 | S0234/PGK1.r1 | CTGGGCCTACACAGTCCTTCA | 743 |
| PGK1 | NM_000291 | S5022/PGK1.p1 | TCTCTGCTGGGCAAGGATGTTCTG TTC | 744 |
| PHB | NM_002634 | T2171/PHB.f1 | GACATTGTGGTAGGGGAAGG | 745 |
| PHB | NM_002634 | T2172/PHB.r1 | CGGCAGTCAAAGATAATTGG | 746 |
| PHB | NM_002634 | T2173/PHB.p1 | TCATTTTCTCATCCCGTGGGTACAG A | 747 |
| PI3KC2A | NM_002645 | S2020/PI3KC2.r1 | CACACTAGCATTTTCTCCGCATA | 748 |
| PI3KC2A | NM_002645 | S2021/PI3KC2.f1 | ATACCAATCACCGCACAAACC | 749 |
| PI3KC2A | NM_002645 | S5062/PI3KC2.p1 | TGCGCTGTGACTGGACTTAACAAA TAGCCT | 750 |
| PIM1 | NM_002648 | S7858/PIM1.f3 | CTGCTCAAGGACACCGTCTA | 751 |
| PIM1 | NM_002648 | S7859/PIM1.r3 | GGATCCACTCTGGAGGGC | 752 |
| PIM1 | NM_002648 | S7860/PIM1.p3 | TACACTCGGGTCCCATCGAAGTCC | 753 |
| PIM2 | NM_006875 | T2144/PIM2.f1 | TGGGGACATTCCCTTTGAG | 754 |
| PIM2 | NM_006875 | T2145/PIM2.r1 | GACATGGGCTGGGAAGTG | 755 |
| PIM2 | NM 006875 | T2146/PIM2.p1 | CAGCTTCCAGAATCTCCTGGTCCC | 756 |
| PLAUR | NM_002659 | S1976/PLAUR.f3 | CCCATGGATGCTCCTCTGAA | 757 |
| PLAUR | NM_002659 | S1977/PLAUR.r3 | CCGGTGGCTACCAGACATTG | 758 |
| PLAUR | NM_002659 | S5054/PLAUR.p3 | CATTGACTGCCGAGGCCCCATG | 759 |
| PLD3 | NM_012268 | S8645/PLD3.f1 | CCAAGTTCTGGGTGGTGG | 760 |
| PLD3 | NM_012268 | S8646/PLD3.r1 | GTGAACGCCAGTCCATGTT | 761 |
| PLD3 | NM 012268 | S8647/PLD3.p1 | CCAGACCCACTTCTACCTGGGCAG | 762 |
| PLK | NM_005030 | S3099/PLK.f3 | AATGAATACAGTATTCCCAAGCACAT T | 763 |
| PLK | NM_005030 | S3100/PLK.r3 | TGTCTGAAGCATCTTCTGGATGA | 764 |
| PLK | NM_005030 | S4825/PLK.p3 | AACCCCGTGGCCGCCTCC | 765 |
| PMS1 | NM_000534 | S5894/PMS1,f2. | CTTACGGTTTTCGTGGAGAAG | 766 |
| PMS1 | NM 000534 | S5895/PMS1.r2 | AGCAGCCGTTCTTGTTGTAA | 767 |
| PMS1 | NM_000534 | S5896/PMS1.p2 | CCTCAGCTATACAACAAATTGACCC CAAG | 768 |
| PMS2 | NM 000535 | S5878/PMS2.f3 | GATGTGGACTGCCATTCAAA | 769 |
| PMS2 | NM_000535 | S5879/PMS2.r3 | TGCGAGATTAGTTGGCTGAG | 770 |
| PMS2 | NM 000535 | S5880/PMS2.p3 | TCGAAATTTACATCCGGTATCTTCC TGG | 771 |
| PP591 | NM_025207 | S8657/PP591.f1 | CCACATACCGTCCAGCCTA | 772 |
| PP591 | NM_025207 | S8658/PP591.r1 | GAGGTCATGTGCGGGAGT | 773 |
| PP591 | NM 025207 | S8659/PP591.p1 | CCGCTCCTCTTCTTCGTTCTCCAG | 774 |
| PPP2CA | NM_002715 | T0732/PPP2CA.f1 | GCAATCATGGAACTTGACGA | 775 |
| PPP2CA | NM_002715 | T0733/PPP2CA.r1 | ATGTGGCTCGCCTCTACG | 776 |
| PPP2CA | NM_002715 | T0734/PPP2CA.p1 | TTTCTTGCAGTTTGACCCAGCACC | 777 |
| PR | NM_000926 | S1336/PR.f6 | GCATCAGGCTGTCATTATGG | 778 |
| PR | NM 000926 | S1337/PR.r6 | AGTAGTTGTGCTGCCCTTCC | 779 |
| PR | NM_000926 | S4743/PR.p6 | TGTCCTTACCTGTGGGAGCTGTAA GGTC | 780 |
| PRDX1 | NM_002574 | T1241/PRDX1.f1 | AGGACTGGGACCCATGAAC | 781 |
| PRDX1 | NM_002574 | T1242/PRDX1.r1 | CCCATAATCCTGAGCAATGG | 782 |
| PRDX1 | NM_002574 | T1243/PRDX1.p1 | TCCTTTGGTATCAGACCCGAAGCG | 783 |
| PRDX2 | NM_005809 | S8761/PRDX2.f1 | GGTGTCCTTCGCCAGATCAC | 784 |
| PRDX2 | NM 005809 | S8762/PRDX2.r1 | CAGCCGCAGAGCCTCATC | 785 |
| PRDX2 | NM_005809 | S8763/PRDX2.p1 | TTAATGATTTGCCTGTGGGACGCT CC | 786 |
| PRKCA | NM_002737 | S7369/PRKCA.f1 | CAAGCAATGCGTCATCAATGT | 787 |
| PRKCA | NM_002737 | S7370/PRKCA.r1 | GTAAATCCGCCCCCTCTTCT | 788 |
| PRKCA | NM_002737 | S7371/PRKCA.p1 | CAGCCTCTGCGGAATGGATCACAC T | 789 |
| PRKCD | NM_006254 | S1738/PRKCD.f2 | CTGACACTTGCCGCAGAGAA | 790 |
| PRKCD | NM_006254 | S1739/PRKCD.r2 | AGGTGGTCCTTGGTCTGGAA | 791 |
| PRKCD | NM_006254 | S4923/PRKCD.p2 | CCCTTTCTCACCCACCTCATCTGCA[?] C | 792 |
| PRKCG | NM_002739 | T2081/PRKCG.f1 | GGGTTCTAGACGCCCCTC | 793 |
| PRKCG | NM 002739 | T2082/PRKCG.r1 | GGACGGCTGTAGAGGCTGTAT | 794 |
| PRKCG | NM_002739 | T2083/PRKCG.p1 | CAAGCGTTCCTGGCCTTCTGAACT | 795 |
| PRKCH | NM_006255 | T2084/PRKCH.f1 | CTCCACCTATGAGCGTCTGTC | 796 |
| PRKCH | NM_006255 | T2085/PRKCH.r1 | CACACTTTCCCTCCTTTTGG | 797 |
| PRKCH | NM_006255 | T2086/PRKCH.p1 | TCCTGTTAACATCCCAAGCCCACA | 798 |
| pS2 | NM_003225 | S0241/pS2.f2 | GCCCTCCCAGTGTGCAAAT | 799 |
| pS2 | NM_003225 | S0243/pS2.r2 | CGTCGATGGTATTAGGATAGAAGC A | 800 |
| pS2 | NM_003225 | S5026/pS2.p2 | TGCTGTTTCGACGACACCGTTCG | 801 |
| PTEN | NM_000314 | S0244/PTEN.f2 | TGGCTAAGTGAAGATGACAATCAT G | 802 |
| PTEN | NM_000314 | S0246/PTEN.r2 | TGCACATATCATTACACCAGTTCGT | 803 |
| PTEN | NM_000314 | S5027/PTEN.p2 | CCTTTCCAGCTTTACAGTGAATTGC TGCA | 804 |
| PTPD1 | NM_007039 | S3069/PTPD1.f2 | CGCTTGCCTAACTCATACTTTCC | 805 |
| PTPD1 | NM_07039 | S3070/PTPD1.r2 | CCATTCAGACTGCGCCACTT | 806 |
| PTPD1 | NM_007039 | S4822/PTPD1.p2 | TCCACGCAGCGTGGCACTG | 807 |
| PTTG1 | NM_004219 | S4525/PTTG1.f2 | GGCTACTCTGATCTATGTTGATAAG GAA | 808 |
| PTTG1 | NM 004219 | S4526/PTTG1.r2 | GCTTCAGCCCATCCTTAGCA | 809 |
| PTTG1 | NM 004219 | S4527/PTTG1.p2 | CACACGGGTGCCTGGTTCTCCA | 810 |
| RAB27B | NM_04163 | S4336/RAB27B.f1 | GGGACACTGCGGGACAAG | 811 |
| RAB27B | NM_004163 | S4337/RAB27B.r1 | GCCCATGGCGTCTCTGAA | 812 |
| RAB27B | MM_004163 | S4338/RAB27B.p1 | CGGTTCCGGAGTCTCACCACTGCA T | 813 |
| RAB31 | NM_006868 | S9306/RAB31.f1 | CTGAAGGACCCTACGCTCG | 814 |
| RAB31 | NM_006868 | S9307/RAB31.r1 | ATGCAAAGCCAGTGTGCTC | 815 |
| RAB31 | NM_006868 | S9308/RAB31.p1 | CTTCTCAAAGTGAGGTGCCAGGCC | 816 |
| RAB6C | NM 032144 | S5535/RAB6C.f1 | GCGACAGCTCCTCTAGTTCCA | 817 |
| RAB6C | NM_032144 | S5537/RAB6C.p1 | TTCCCGAAGTCTCCGCCCG | 818 |
| RAB6C | NM_032144 | S5538/RAB6C.r1 | GGAACACCAGCTTGAATTTCCT | 819 |
| RAD1 | NM_002853 | T2174/RAD1.f1 | GAGGAGTGGTGACAGTCTGC | 820 |
| RAD1 | NM_002853 | T2175/RAD1.r1 | GCTGCAGAAATCAAAGTCCA | 821 |
| RAD1 | NM_002853 | T2176/RAD1.p1 | TCAATACACAGGAACCTGAGGAGA CCC | 822 |
| RAD54L | NM 003579 | S4369/RAD54L.f1 | AGCTAGCCTCAGTGACACACATG | 823 |
| RAD54L | NM_003579 | S4370/RAD54L.r1 | CCGGATCTGACGGCTGTT | 824 |
| RAD54L | NM_003579 | S4371/RAD54L.p1 | ACACAACGTCGGCAGTGCAACCTG | 825 |
| RAF1 | NM_002880 | S5933/RAF1.f3 | CGTCGTATGCGAGAGTCTGT | 826 |
| RAF1 | NM_002880 | S5934/RAF1.r3 | TGAAGGCGTGAGGTGTAGAA | 827 |
| RAF1 | NM_002880 | S5935/RAF1.p3 | TCCAGGATGCCTGTTAGTTCTCAG CA | 828 |
| RALBP1 | NM_006788 | S5853/RALBP1.f1 | GGTGTCAGATATAAATGTGCAAAT GC | 829 |
| RALBP1 | NM_006788 | S5854/RALBP1.r1 | TTCGATATTGCCAGCAGCTATAAA | 830 |
| RALBP1 | NM_006788 | S5855/RALBP1.p1 | TGCTGTCCTGTCGGTCTCAGTACG TTCA | 831 |
| RAP1GDS1 | NM 021159 | S5306/RAP1GD.f2 | TGTGGATGCTGGATTGATTT | 832 |
| RAP1GDS1 | NM_021159 | S5307/RAP1GD.r2 | AAGCAGCACTTCCTGGTCTT | 833 |
| RAP1GDS1 | NM 021159 | S5308/RAP1GD.p2 | CCACTGGTGCAGCTGCTAAATAGC A | 834 |
| RASSF1 | M_007182 | S2393/RASSF1.f3 | AGTGGGAGACACCTGACCTT | 835 |
| RASSF1 | NM_007182 | S2394/RASSF1.r3 | TGATCTGGGCATTGTACTCC | 836 |
| RASSF1 | NM_007182 | S4909/RASSF1.p3 | TTGATCTTCTGCTCAATCTCAGCTT GAGA | 837 |
| RB1 | NM_000321 | S2700/RB1.f1 | CGAAGCCCTTACAAGTTTCC | 838 |
| RB1 | NM_000321 | S2701/RB1.r1 | GGACTCTTCAGGGGTGAAAT | 839 |
| RB1 | NM 000321 | S4765/RB1.p1 | CCCTTACGGATTCCTGGAGGGAAC | 840 |
| RBM17 | NM_032905 | T2186/RBM17.f1 | CCCAGTGTACGAGGAACAAG | 841 |
| RBM17 | NM_032905 | T2187/RBM17.r1 | TTAGCGAGGAAGGAGTTGCT | 842 |
| RBM17 | NM_032905 | T2188/RBM17.p1 | ACAGACCGAGATCTCCAACCGGAC | 843 |
| RCC1 | NM_001269 | S8854/RCC1.f1 | GGGCTGGGTGAGAATGTG | 844 |
| RCC1 | NM 001269 | S8855/RCC1.r1 | CACAACATCCTCCGGAATG | 845 |
| RCC1 | NM_001269 | S8856/RCC1.p1 | ATACCAGGGCCGGCTTCTTCCTCT | 846 |
| REG1A | NM_002909 | T2093/REG1A.f1 | CCTACAAGTCCTGGGGCA | 847 |
| REG1A | NM_002909 | T2094/REG1A.r1 | TGAGGTCAGGCTCACACAGT | 848 |
| REG1A | NM_002909 | T2095/REG1A.p1 | TGGAGCCCCAAGCAGTGTTAATCC | 849 |
| RELB | NM 006509 | T2096/RELB.f1 | GCGAGGAGCTCTACTTGCTC | 850 |
| RELB | NM_006509 | T2097/RELB.r1 | GCCCTGCTGAACACCACT | 851 |
| RELB | NM_006509 | T2098/RELB.p1 | TGTCCTCTTTCTGCACCTTGTCGC | 852 |
| RhoB | NM_004040 | S8284/RhoB.f1 | AAGCATGAACAGGACTTGACC | 853 |
| RhoB | NM_004040 | S8285/RhoB.r1 | CCTCCCCAAGTCAGTTGC | 854 |
| RhoB | NM_004040 | S8286/RhoB.p1 | CTTTCCAACCCCTGGGGAAGACAT | 855 |
| rhoC | NM_175744 | S2162/rhoC.f1 | CCCGTTCGGTCTGAGGAA | 856 |
| rhoc | NM_175744 | S2163/rhoC.r1 | GAGCACTCAAGGTAGCCAAAGG | 857 |
| rhoc | NM_175744 | S5042/rhoC.p1 | TCCGGTTCGCCATGTCCCG | 858 |
| RIZ1 | NM_012231 | S1320/RIZ1.f2 | CCAGACGAGCGATTAGAAGC | 859 |
| RIZ1 | NM_012231 | S1321/RIZ1.r2 | TCCTCCTCTTCCTCCTCCTC | 860 |
| RIZ1 | MM_012231 | S4761/RIZ1.p2 | TGTGAGGTGAATGATTTGGGGGA | 861 |
| ROCK1 | NM 005406 | S8305/ROCK1.f1 | TGTGCACATAGGAATGAGCTTC | 862 |
| ROCK1 | NM 005406 | S8306/ROCK1.r1 | GTTTAGCACGCAATTGCTCA | 863 |
| ROCK1 | NM_005406 | S8307/ROCK1.p1 | TCACTCTCTTTGCTGGCCAACTGC | 864 |
| RPL37A | NM_000998 | T2418/RPL37A.f2 | GATCTGGCACTGTGGTTCC | 865 |
| RPL37A | NM_000998 | T2419/RPL37A.r2 | TGACAGCGGAAGTGGTATTG | 866 |
| RPL37A | NM_000998 | T2420/RPL37A.p2 | CACCGCCAGCCACTGTCTTCAT | 867 |
| RPLPO | NM 001002 | S0256/RPLPO.f2 | CCATTCTATCATCAACGGGTACAA | 868 |
| RPLPO | NM_001002 | S0258/RPLPO.r2 | TCAGCAAGTGGGAAGGTGTAATC | 869 |
| RPLPO | NM_001002 | S4744/RPLPO.p2 | TCTCCACAGACAAGGCCAGGACTC G | 870 |
| RPN2 | NM_002951 | T1158/RPN2.f1 | CTGTCTTCCTGTTGGCCCT | 871 |
| RPN2 | NM_002951 | T1159/RPN2.r1 | GTGAGGTAGTGAGTGGGCGT | 872 |
| RPN2 | NM_002951 | T1160/RPN2.p1 | ACAATCATAGCCAGCACCTGGGCT | 873 |
| RPS6KB1 | NM 003161 | S2615/RPS6KB.f3 | GCTCATTATGAAAAACATCCCAAAC | 874 |
| RPS6KB1 | NM_003161 | S2616/RPS6KB.r3 | AAGAAACAGAAGTTGTCTGGCTTT CT | 875 |
| RPS6KB1 | NM_003161 | S4759/RPS6KB.p3 | CACACCAACCAATAATTTCGCATT | 876 |
| RXRA | NM_002957 | S8463/RXRA.f1 | GCTCTGTTGTGTCCTGTTGC | 877 |
| RXRA | NM_002957 | S8464/RXRA.r1 | GTACGGAGAAGCCACTTCACA | 878 |
| RXRA | NM_002957 | S8465/RXRA.p1 | TCAGTCACAGGAAGGCCAGAGCC | 879 |
| RXRB | NM_021976 | S8490/RXRB.f1 | CGAGGAGATGCCTGTGGA | 880 |
| RXRB | NM_021976 | S8491/RXRB.r1 | CAACGCCCTGGTCACTCT | 881 |
| RXRB | NM_021976 | S8492/RXRB.p1 | CTGTTCCACAGCAAGCTCTGCCTC | 882 |
| S100A10 | NM_002966 | S9950/S100A1.f1 | ACACCAAAATGCCATCTCAA | 883 |
| S100A10 | NM 002966 | S9951/S100A1.r1 | TTTATCCCCAGCGAATTTGT | 884 |
| S100A10 | NM_002966 | S9952/S100A1.p1 | CACGCCATGGAAACCATGATGTTT | 885 |
| SEC61A | NM_013336 | S8648/SEC61 A.f1 | CTTCTGAGCCCGTCTCCC | 886 |
| SEC61A | NM_013336 | S8649/SEC61A.r1 | GAGAGCTCCCCTTCCGAG | 887 |
| SEC61A | NM_013336 | S8650/SEC61A.p1 | CGCTTCTGGAGCAGCTTCCTCAAC | 888 |
| SEMA3F | NM 004186 | S2857/SEMA3F.f3 | CGCGAGCCCCTCATTATACA | 889 |
| SEMA3F | NM 004186 | S2858/SEMA3F.r3 | CACTCGCCGTTGACATCCT | 890 |
| SEMA3F | NM_004186 | S4972/SEMA3F.p3 | CTCCCCACAGCGCATCGAGGAA | 891 |
| SFN | NM_006142 | S9953/SFN.f1 | GAGAGAGCCAGTCTGATCCA | 892 |
| SFN | MM_006142 | S9954/SFN.r1 | AGGCTGCCATGTCCTCATA | 893 |
| SFN | NM 006142 | S9955/SFN.p1 | CTGCTCTGCCAGCTTGGCCTTC | 894 |
| SGCB | NM_000232 | S5752/SGCB.f1 | CAGTGGAGACCAGTTGGGTAGTG | 895 |
| SGCB | NM_000232 | S5753/SGCB.r1 | CCTTGAAGAGCGTCCCATCA | 896 |
| SGCB | NM_000232 | S5754/SGCB.p1 | CACACATGCAGAGCTTGTAGCGTA CCCA | 897 |
| SGK | NM_005627 | S8308/SGK.f1 | TCCGCAAGACACCTCCTG | 898 |
| SGK | NM_005627 | S8309/SGK.r1 | TGAAGTCATCCTTGGCCC | 899 |
| SGK | NM_005627 | S8310/SGK.p1 | TGTCCTGTCCTTCTGCAGGAGGC | 900 |
| SGKL | NM_170709 | T2183/SGKL.f1 | TGCATTCGTTGGTTTCTCTT | 901 |
| SGKL | NM_170709 | T2184/SGKL.r1 | TTTCTGAATGGCAAACTGCT | 902 |
| SGKL | NM_170709 | T2185/SGKL.p1 | TGCACCTCCTTCAGAAGACTTATTT TTGTG | 903 |
| SHC1 | NM_003029 | S6456/SHC1.f1 | CCAACACCTTCTTGGCTTCT | 904 |
| SHC1 | NM_003029 | S6457/SHC1.r1 | CTGTTATCCCAACCCAAACC | 905 |
| SHC1 | NM_003029 | S6458/SHC1.p1 | CCTGTGTTCTTGCTGAGCACCCTC | 906 |
| SIR2 | NM 012238 | S1575/SIR2.f2 | AGCTGGGGTGTCTGTTTCAT | 907 |
| SIR2 | NM_012238 | S1576/SIR2.r2 | ACAGCAAGGCGAGCATAAAT | 908 |
| SIR2 | NM_012238 | S4885/SIR2.p2 | CCTGACTTCAGGTCAAGGGATGG | 909 |
| SLC1A3 | NM 004172 | S8469/SLCIA3.f1 | GTGGGGAGCCCATCATCT | 910 |
| SLC1A3 | NM_004172 | S8470/SLCIA3.r1 | CCAGTCCACACTGAGTGCAT | 911 |
| SLC1A3 | MM_004172 | S8471/SLCIA3.p1 | CCAAGCCATCACAGGCTCTGCATA | 912 |
| SLC25A3 | NM_213611 | T0278/SLC25A.f2 | TCTGCCAGTGCTGAATTCTT | 913 |
| SLC25A3 | MM_213611 | T0279/SLC25A.r2 | TTCGAACCTTAGCAGCTTCC | 914 |
| SLC25A3 | NM_213611 | T0280/SLC25A.p2 | TGCTGACATTGCCCTGGCTCCTAT | 915 |
| SLC35B1 | NM 005827 | S8642/SLC35B.f1 | CCCAACTCAGGTCCTTGGTA | 916 |
| SLC35B1 | NM 005827 | S8643/SLC35B.r1 | CAAGAGGGTCACCCCAAG | 917 |
| SLC35B1 | NM_005827 | S8644/SLC35B.p1 | ATCCTGCAAGCCAATCCCAGTCAT | 918 |
| SLC7A11 | NM_014331 | T2045/SLC7A1.f1 | AGATGCATACTTGGAAGCACAG | 919 |
| SLC7A11 | NM_014331 | T2046/SLC7A1.r1 | AACCTAGGACCAGGTAACCACA | 920 |
| SLC7A11 | NM_014331 | T2047/SLC7A1.p1 | CATATCACACTGGGAGGCAATGCA | 921 |
| SLC7A5 | NM 003486 | S9244/SLC7A5.f2 | GCGCAGAGGCCAGTTAAA | 922 |
| SLC7A5 | NM_003486 | S9245/SLC7A5.r2 | AGCTGAGCTGTGGGTTGC | 923 |
| SLC7A5 | NM_003486 | S9246/SLC7A5.p2 | AGATCACCTCCTCGAACCCACTCC | 924 |
| SNAI2 | NM_003068 | S7824/SNAI2.f1 | GGCTGGCCAAACATAAGCA | 925 |
| SNAI2 | NM_003068 | S7825/SNAI2.r1 | TCCTTGTCACAGTATTTACAGCTGA A | 926 |
| SNAI2 | NM_003068 | S7826/SNA12.p1 | CTGCACTGCGATGCCCAGTCTAGA AAATC | 927 |
| SNCA | NM_007308 | T2320/SNCA.f1 | AGTGACAAATGTTGGAGGAGC | 928 |
| SNCA | NM_007308 | T2321/SNCA.r1 | CCCTCCACTGTCTTCTGGG | 929 |
| SNCA | NM_007308 | T2322/SNCA.p1 | TACTGCTGTCACACCCGTCACCAC | 930 |
| SNCG | NM_003087 | T1704/SNCG.f1 | ACCCACCATGGATGTCTTC | 931 |
| SNCG | NM_003087 | T1705/SNCG.r1 | CCTGCTTGGTCTTTTCCAC | 932 |
| SNCG | NM_003087 | T1706/SNCG.p1 | AAGAAGGGCTTCTCCATCGCCAAG | 933 |
| SOD1 | NM 000454 | S7683/SOD1.f1 | TGAAGAGAGGCATGTTGGAG | 934 |
| SOD1 | NM_000454 | S7684/SOD1.r1 | AATAGACACATCGGCCACAC | 935 |
| SOD1 | NM_000454 | S7685/SOD1.p1 | TTTGTCAGCAGTCACATTGCCCAA | 936 |
| SRI | NM_003130 | T2177/SRI.f1 | ATACAGCACCAATGGAAAGATCAC | 937 |
| SRI | NM_003130 | T2178/SRI.r1 | TGTCTGTAAGAGCCCTCAGTTTGA | 938 |
| SRI | NM_003130 | T2179/SRI.pl | TTCGACGACTACATCGCCTGCTGC | 939 |
| STAT1 | NM_007315 | S1542/STAT1.f3 | GGGCTCAGCTTTCAGAAGTG | 940 |
| STAT1 | NM_007315 | S1543/STAT1.r3 | ACATGTTCAGCTGGTCCACA | 941 |
| STAT1 | NM_007315 | S4878/STAT1.p3 | TGGCAGTTTTCTTCTGTCACCAAAA | 942 |
| STAT3 | NM_003150 | S1545/STAT3.f1 | TCACATGCCACTTTGGTGTT | 943 |
| STAT3 | NM 003150 | S1546/STAT3.r1 | CTTGCAGGAAGCGGCTATAC | 944 |
| STAT3 | NM_003150 | S4881/STAT3.p1 | TCCTGGGAGAGATTGACCAGCA | 945 |
| STK10 | NM_005990 | T2099/STK10.f1 | CAAGAGGGACTCGGACTGC | 946 |
| STK10 | NM_005990 | T2100/STK10.r1 | CAGGTCAGTGGAGAGATTGGT | 947 |
| STK10 | NM 005990 | T2101/STK10.p1 | CCTCTGCACCTCTGAGAGCATGGA | 948 |
| STK11 | NM_000455 | S9454/STK11.f1 | GGACTCGGAGACGCTGTG | 949 |
| STK11 | NM_000455 | S9455/STK11.r1 | GGGATCCTTCGCAACTTCTT | 950 |
| STK11 | NM_000455 | S9456/STK11.p1 | TTCTTGAGGATCTTGACGGCCCTC | 951 |
| STK15 | NM_003600 | S0794/STK15.f2 | CATCTTCCAGGAGGACCACT | 952 |
| STK15 | NM 003600 | S0795/STK15.r2 | TCCGACCTTCAATCATTTCA | 953 |
| STK15 | NM 003600 | S4745/STK15.p2 | CTCTGTGGCACCCTGGACTACCTG | 954 |
| STMN1 | NM_005563 | S5838/STMN1.f1 | AATACCCAACGCACAAATGA | 955 |
| STMN1 | NM_005563 | S5839/STMNI.r1 | GGAGACAATGCAAACCACAC | 956 |
| STMN1 | NM_005563 | S5840/STMN1.p1 | CACGTTCTCTGCCCCGTTTCTTG | 957 |
| STMY3 | NM 005940 | S2067/STMY3.f3 | CCTGGAGGCTGCAACATACC | 958 |
| STMY3 | NM_005940 | S2068/STMY3.r3 | TACAATGGCTTTGGAGGATAGCA | 959 |
| STMY3 | NM 005940 | S4746/STMY3.p3 | ATCCTCCTGAAGCCCTTTTCGCAG C | 960 |
| SURV | NM_001168 | S0259/SURV.f2 | TGTTTTGATTCCCGGGCTTA | 961 |
| SURV | NM_001168 | S0261/SURV.r2 | CAAAGCTGTCAGCTCTAGCAAAAG | 962 |
| SURV | NM_001168 | S4747/SURV.p2 | TGCCTTCTTCCTCCCTCACTTCTCA CCT | 963 |
| TACC3 | NM_006342 | S7124/TACC3.f1 | CACCCTTGGACTGGAAAACT | 964 |
| TACC3 | NM_006342 | S7125/TACC3.r1 | CCTTGATGAGCTGTTGGTTC | 965 |
| TACC3 | NM_006342 | S7126/TACC3.p1 | CACACCCGGTCTGGACACAGAAAG | 966 |
| TBCA | NM_004607 | T2284/TBCA.f1 | GATCCTCGCGTGAGACAGA | 967 |
| TBCA | NM_004607 | T2285/TBCA.r1 | CACTTTTTCTTTGACCAACCG | 968 |
| TBCA | NM_004607 | T2286/TBCA.p1 | TTCACCACGCCGGTCTTGATCTT | 969 |
| TBCC | NM 003192 | T2302/TBCC.f1 | CTGTTTTCCTGGAGGACTGC | 970 |
| TBCC | NM_003192 | T2303/TBCC.r1 | ACTGTGTATGCGGAGCTGTT | 971 |
| TBCC | NM_003192 | T2304/TBCC.p1 | CCACTGCCAGCACGCAGTCAC | 972 |
| TBCD | NM_005993 | T2287/TBCD.f1 | CAGCCAGGTGTACGAGACATT | 973 |
| TBCD | NM_005993 | T2288/TBCD.r1 | ACCTCGTCCAGCACATCC | 974 |
| TBCD | NM 005993 | T2289/TBCD.p1 | CTCACCTACAGTGACGTCGTGGGC | 975 |
| TBCE | NM 003193 | T2290/TBCE.f1 | TCCCGAGAGAGGAAAGCAT | 976 |
| TBCE | NM_003193 | T2291/TBCE.r1 | GTCGGGTGCCTGCATTTA | 977 |
| TBCE | NM_003193 | T2292/TBCE.p1 | ATACACAGTCCCTTCGTGGCTCCC | 978 |
| TBD | NM_016261 | S3347/TBD.f2 | CCTGGTTGAAGCCTGTTAATGC | 979 |
| TBD | NM_016261 | S3348/TBD.r2 | TGCAGACTTCTCATATTTGCTAAAG G | 980 |
| TBD | NM_016261 | S4864/TBD.p2 | CCGCTGGGTTTTCCACACGTTGA | 981 |
| TCP1 | NM 030752 | T2296/TCP1.f1 | CCAGTGTGTGTAACAGGGTCAC | 982 |
| TCP1 | NM 030752 | T2297/TCP1.r1 | TATAGCCTTGGGCCACCC | 983 |
| TCP1 | NM 030752 | T2298/TCP1.p1 | AGAATTCGACAGCCAGATGCTCCA | 984 |
| TFRC | NM_003234 | S1352/TFRC.f3 | GCCAACTGCTTTCATTTGTG | 985 |
| TFRC | NM_003234 | S1353/TFRC.r3 | ACTCAGGCCCATTTCCTTTA | 986 |
| TFRC | NM_003234 | S4748/TFRC.p3 | AGGGATCTGAACCAATACAGAGCA GACA | 987 |
| THBS1 | NM 003246 | S6474/THBS1.f1 | CATCCGCAAAGTGACTGAAGAG | 988 |
| THBS1 | NM_003246 | S6475/THBS1.r1 | GTACTGAACTCCGTTGTGATAGCA TAG | 989 |
| THBS1 | NM_003246 | S6476/THBS1.p1 | CCAATGAGCTGAGGCGGCCTCC | 990 |
| TK1 | NM_003258 | S0866/TK1.f2 | GCCGGGAAGACCGTAATTGT | 991 |
| TK1 | NM_003258 | S0927/TK1.r2 | CAGCGGCACCAGGTTCAG | 992 |
| TK1 | NM_003258 | S4798/TK1.p2 | CAAATGGCTTCCTCTGGAAGGTCC CA | 993 |
| TOP2A | NM_001067 | S0271/TOP2A.f4 | AATCCAAGGGGGAGAGTGAT | 994 |
| TOP2A | NM_001067 | S0273/TOP2A.r4 | GTACAGATTTTGCCCGAGGA | 995 |
| TOP2A | NM_001067 | S4777/TOP2A.p4 | CATATGGACTTTGACTCAGCTGTG GC | 996 |
| TOP3B | NM 003935 | T2114/TOP3B.f1 | GTGATGCCTTCCCTGTGG | 997 |
| TOP3B | NM_003935 | T2115/TOP3B.r1 | TCAGGTAGTCGGGTGGGTT | 998 |
| TOP3B | NM_003935 | T2116/TOP3B.p1 | TGCTTCTCCAGCATCTTCACCTCG | 999 |
| TP | NM_001953 | S0277/TP.f3 | CTATATGCAGCCAGAGATGTGACA | 1000 |
| TP | NM_001953 | S0279/TP.r3 | CCACGAGTTTCTTACTGAGAATGG | 1001 |
| TP | NM_001953 | S4779/TP.p3 | ACAGCCTGCCACTCATCACAGCC | 1002 |
| TP53BP1 | NM_005657 | S1747/TP53BP.f2 | TGCTGTTGCTGAGTCTGTTG | 1003 |
| TP53BP1 | NM_005657 | S1748/TP53BP.r2 | CTTGCCTGGCTTCACAGATA | 1004 |
| TP53BP1 | NM_005657 | S4924/TP53BP.p2 | CCAGTCCCCAGAAGACCATGTCTG | 1005 |
| TPT1 | NM_003295 | S9098/TPT1.f1 | GGTGTCGATATTGTCATGAACC | 1006 |
| TPT1 | NM_003295 | S9099/TPT1.r1 | GTAATCTTTGATGTACTTCTTGTAG GC | 1007 |
| TPT1 | NM_003295 | S9100/TPT1.p1 | TCACCTGCAGGAAACAAGTTTCAC AAA | 1008 |
| TRAG3 | NM_004909 | S5881/TRAG3.f1 | GACGCTGGTCTGGTGAAGATG | 1009 |
| TRAG3 | NM 004909 | S5882/TRAG3.r1 | TGGGTGGTTGTTGGACAATG | 1010 |
| TRAG3 | NM_004909 | S5883/TRAG3.p1 | CCAGGAAACCACGAGCCTCCAGC | 1011 |
| TRAIL | NM_003810 | S2539/TRAIL.f1 | CTTCACAGTGCTCCTGCAGTCT | 1012 |
| TRAIL | NM_003810 | S2540/TRAIL.r1 | CATCTGCTTCAGCTCGTTGGT | 1013 |
| TRAIL | NM_003810 | S4980/TRAIL.p1 | AAGTACACGTAAGTTACAGCCACA CA | 1014 |
| TS | NM_001071 | S0280/TS.f1 | GCCTCGGTGTGCCTTTCA | 1015 |
| TS | NM_001071 | S0282/TS.r1 | CGTGATGTGCGCAATCATG | 1016 |
| TS | NM_001071 | S4780/TS.p1 | CATCGCCAGCTACGCCCTGCTC | 1017 |
| TSPAN4 | NM_003271 | T2102/TSPAN4.f1 | CTGGTCAGCCTTCAGGGAC | 1018 |
| TSPAN4 | NM_003271 | T2103/TSPAN4.r1 | CTTCAGTTCTGGGCTGGC | 1019 |
| TSPAN4 | NM_003271 | T2104/TSPAN4.p1 | CTGAGCACCGCCTGGTCTCTTTC | 1020 |
| TTK | NM 003318 | S7247/TTK.f1 | TGCTTGTCAGTTGTCAACACCTT | 1021 |
| TTK | NM_003318 | S7248/TTK.r1 | TGGAGTGGCAAGTATTTGATGCT | 1022 |
| TTK | NM_003318 | S7249/TTK.p1 | TGGCCAACCTGCCTGTTTCCAGC | 1023 |
| TUBA1 | NM_006000 | S8578/TUBA1.f1 | TGTCACCCCGACTCAACGT | 1024 |
| TUBA1 | NM 006000 | S8579/TUBA1.r1 | ACGTGGACTGAGATGCATTCAC | 1025 |
| TUBA1 | NM_006000 | S8580/TUBA1.p1 | AGACGCACCGCCCGGACTCAC | 1026 |
| TUBA2 | NM_006001 | S8581/TUBA2.f1 | AGCTCAACATGCGTGAGTGT | 1027 |
| TUBA2 | NM_006001 | S8582/TUBA2.r1 | ATTGCCGATCTGGACTCCT | 1028 |
| TUBA2 | NM_006001 | S8583/TUBA2.p1 | ATCTCTATCCACGTGGGGCAGGC | 1029 |
| TUBA3 | NM_006009 | S8584/TUBA3.f1 | CTCTTACATCGACCGCCTAAGAG | 1030 |
| TUBA3 | NM 006009 | S8585/TUBA3.r1 | GCTGATGGCGGAGACGAA | 1031 |
| TUBA3 | NM_006009 | S8586/TUBA3.p1 | CGCGCTGTAAGAAGCAACAACCTC TCC | 1032 |
| TUBA4 | NM_025019 | T2415/TUBA4.f3 | GAGGAGGGTGAGTTCTCCAA | 1033 |
| TUBA4 | NM_025019 | T2416/TUBA4.r3 | ATGCCCACCTCCTTGTAATC | 1034 |
| TUBA4 | NM_025019 | T2417/TUBA4.p3 | CCATGAGGATATGACTGCCCTGGA | 1035 |
| TUBA6 | NM_032704 | S8590/TUBA6.f1 | GTCCCTTCGCCTCCTTCAC | 1036 |
| TUBA6 | NM_032704 | S8591/TUBA6.r1 | CGTGGATGGAGATGCACTCA | 1037 |
| TUBA6 | NM_032704 | S8592/TUBA6.p1 | CCGCAGACCCCTTCAAGTTCTAGT CATG | 1038 |
| TUBA8 | NM 018943 | T2412/TUBA8.f2 | CGCCCTACCTATACCAACCT | 1039 |
| TUBA8 | NM_018943 | T2413/TUBA8.r2 | CGGAGAGAAGCAGTGATTGA | 1040 |
| TUBA8 | NM_018943 | T2414/TUBA8.p2 | CAACCGCCTCATCAGTCAGATTGT G | 1041 |
| TUBB | NM_001069 | S5820/TUBB.f1 | CGAGGACGAGGCTTAAAAAC | 1042 |
| TUBB | NM_001069 | S5821/TUBB.r1 | ACCATGCTTGAGGACAACAG | 1043 |
| TUBB | NM_001069 | S5822/TUBB.p1 | TCTCAGATCAATCGTGCATCCTTAG TGAA | 1044 |
| TUBB classIII | NM_006086 | S8090/TUBB c.f3 | CGCCCTCCTGCAGTATTTATG | 1045 |
| TUBB classIII | NM_006086 | S8091/TUBB c.r3 | ACAGAGACAGGAGCAGCTCACA | 1046 |
| TUBB classIII | NM_006086 | S8092/TUBB c.p3 | CCTCGTCCTCCCCACCTAGGCCA | 1047 |
| TUBB1 | NM_030773 | S8093/TUBB1.f1 | ACACTGACTGGCATCCTGCTT | 1048 |
| TUBB1 | NM_030773 | S8094/TUBB1.r1 | GCTCTGTAGCTCCCCATGTACTAG T | 1049 |
| TUBB1 | NM_030773 | S8095/TUBB1.p1 | AGCCTCCAGAAGAGCCAGGTGCCT | 1050 |
| TUBB2 | NM_006088 | S8096/TUBB2.f1 | GTGGCCTAGAGCCTTCAGTC | 1051 |
| TUBB2 | NM_006088 | S8097/TUBB2.r1 | CAGGCTGGGAGTGAATAAAGA | 1052 |
| TUBB2 | NM 006088 | S8098/TUBB2.p1 | TTCACACTGCTTCCCTGCTTTCCC | 1053 |
| TUBB5 | NM_006087 | S8102/TUBB5.f1 | ACAGGCCCCATGCATCCT | 1054 |
| TUBB5 | NM_006087 | S8103/TUBB5.r1 | TGTTTCTCTCCCAGATAAGCTAAGG | 1055 |
| TUBB5 | NM_006087 | S8104/TUBB5.p1 | TGCCTCACTCCCCTCAGCCCC | 1056 |
| TUBBM | NM_032525 | S8105/TUBBM.f1 | CCCTATGGCCCTGAATGGT | 1057 |
| TUBBM | NM_032525 | S8106/TUBBM.r1 | ACTAATTACATGACTTGGCTGCATT T | 1058 |
| TUBBM | NM 032525 | S8107/TUBBM.p1 | TGAGGGGCCGACACCAACACAAT | 1059 |
| TUBBOK | NM 178014 | S8108/TUBBOK.f1 | AGTGGAATCCTTCCCTTTCC | 1060 |
| TUBBOK | NM_178014 | S8109/TUBBOK.r1 | CCCTTGATCCCTTTCTCTGA | 1061 |
| TUBBOK | NM_178014 | S8110/TUBBOK.p1 | CCTCACTCAGCTCCTTTCCCCTGA | 1062 |
| TUBBP | NM_178012 | S8111/TUBBP.f1 | GGAAGGAAAGAAGCATGGTCTACT | 1063 |
| TUBBP | NM 178012 | S8112/TUBBP.r1 | AAAAAGTGACAGGCAACAGTGAAG | 1064 |
| TUBBP | NM_178012 | S8113/TUBBP.p1 | CACCAGAGACCCAGCGCACACCTA | 1065 |
| TUBG1 | NM_001070 | T2299/TUBG1.f1 | GATGCCGAGGGAAATCATC | 1066 |
| TUBG1 | NM_001070 | T2300/TUBG1.r1 | CCAGAACTCGAACCCAATCT | 1067 |
| TUBG1 | NM_001070 | T2301/TUBG1.p1 | ATTGCCGCACTGGCCCAACTGTAG | 1068 |
| TWIST1 | NM_000474 | S7929/TWIST1.f1 | GCGCTGCGGAAGATCATC | 1069 |
| TWIST1 | NM_000474 | S7930/TWIST1.r1 | GCTTGAGGGTCTGAATCTTGCT | 1070 |
| TWIST1 | NM_000474 | S7931/TWIST1.p1 | CCACGCTGCCCTCGGACAAGC | 1071 |
| TYRO3 | NM 006293 | T2105/TYRO3.f1 | CAGTGTGGAGGGGATGGA | 1072 |
| TYRO3 | NM 006293 | T2106/TYRO3.r1 | CAAGTTCTGGACCACAGCC | 1073 |
| TYRO3 | NM_006293 | T2107/TYRO3.p1 | CTTCACCCACTGGATGTCAGGCTC | 1074 |
| UFM1 | NM_016617 | T1284/UFM1.f2 | AGTTGTCGTGTGTTCTGGATTCA | 1075 |
| UFM1 | NM_016617 | T1285/UFM1.r2 | CGTCAGCGTGATCTTAAAGGAA | 1076 |
| UFM1 | NM 016617 | T1286/UFM1.p2 | TCCGGCACCACCATGTCGAAGG | 1077 |
| upa | NM 002658 | S0283/upa.f3 | GTGGATGTGCCCTGAAGGA | 1078 |
| upa | NM_002658 | S0285/upa.r3 | CTGCGGATCCAGGGTAAGAA | 1079 |
| upa | NM_002658 | S4769/upa.p3 | AAGCCAGGCGTCTACACGAGAGTC TCAC | 1080 |
| V-RAF | NM_001654 | S5763/V-RAF.f1 | GGTTGTGCTCTACGAGCTTATGAC | 1081 |
| V-RAF | NM_001654 | S5764/V-RAF.r1 | CGGCCCACCATAAAGATAATCT | 1082 |
| V-RAF | NM 001654 | S5765/V-RAF.p1 | TGCCTTACAGCCACATTGGCTGCC | 1083 |
| VCAM1 | NM 001078 | S3505/VCAM1.f1 | TGGCTTCAGGAGCTGAATACC | 1084 |
| VCAM1 | NM_001078 | S3506/VCAM1.r1 | TGCTGTCGTGATGAGAAAATAGTG | 1085 |
| VCAM1 | NM_001078 | S3507/VCAM1.p1 | CAGGCACACACAGGTGGACACAA AT | 1086 |
| VEGF | NM_003376 | S0286/VEGF.f1 | CTGCTGTCTTGGGTGCATTG | 1087 |
| VEGF | NM_003376 | S0288/VEGF.r1 | GCAGCCTGGGACCACTTG | 1088 |
| VEGF | NM_003376 | S4782/VEGF.p1 | TTGCCTTGCTGCTCTACCTCCACC A | 1089 |
| VEGFB | NM_003377 | S2724/VEGFB.f1 | TGACGATGGCCTGGAGTGT | 1090 |
| VEGFB | NM_003377 | S2725/VEGFB.r1 | GGTACCGGATCATGAGGATCTG | 1091 |
| VEGFB | NM 003377 | S4960/VEGFB.p1 | CTGGGCAGCACCAAGTCCGGA | 1092 |
| VEGFC | NM_005429 | S2251/VEGFC.f1 | CCTCAGCAAGACGTTATTTGAAATT | 1093 |
| VEGFC | NM_005429 | S2252/VEGFC.r1 | AAGTGTGATTGGCAAAACTGATTG | 1094 |
| VEGFC | NM_005429 | S4758/VEGFC.p1 | CCTCTCTCTCAAGGCCCCAAACCA GT | 1095 |
| VHL | NM_000551 | T1359/VHL.f1 | CGGTTGGTGACTTGTCTGC | 1096 |
| VHL | NM_000551 | T1360/VHL.r1 | AAGACTTGTCCCTGCCTCAC | 1097 |
| VHL | NM_000551 | T1361/VHL.p1 | ATGCCTCAGTCTTCCCAAAGCAGG | 1098 |
| VIM | NM_003380 | S0790/VIM.f3 | TGCCCTTAAAGGAACCAATGA | 1099 |
| VIM | NM_003380 | S0791/VIM.r3 | GCTTCAACGGCAAAGTTCTCTT | 1100 |
| VIM | NM_003380 | S4810/VIM.p3 | ATTTCACGCATCTGGCGTTCCA | 1101 |
| WAVE3 | NM 006646 | T2640/WAVE3.f1 | CTCTCCAGTGTGGGCACC | 1102 |
| WAVE3 | NM 006646 | T2641/WAVE3.r1 | GCGGTGTAGCTCCCAGAGT | 1103 |
| WAVE3 | NM_006646 | T2642/WAVE3.p1 | CCAGAACAGATGCGAGCAGTCCAT | 1104 |
| Wnt-5a | NM_003392 | S6183/Wnt-5a.f1 | GTATCAGGACCACATGCAGTACAT C | 1105 |
| Wnt-5a | NM_003392 | S6184/Wnt-5a.r1 | TGTCGGAATTGATACTGGCATT | 1106 |
| Wnt-5a | NM_003392 | S6185/Wnt-5a.p1 | TTGATGCCTGTCTTCGCGCCTTCT | 1107 |
| XIAP | NM 001167 | S0289/XIAP.f1 | GCAGTTGGAAGACACAGGAAAGT | 1108 |
| XIAP | NM_001167 | S0291/XIAP.r1 | TGCGTGGCACTATTTTCAAGA | 1109 |
| XIAP | NM_001167 | S4752/XIAP.p1 | TCCCCAAATTGCAGATTTATCAACG GC | 1110 |
| XIST | M97168 | S1844/XIST.f1 | CAGGTCAGGCAGAGGAAGTC | 1111 |
| XIST | M97168 | S1845/XIST.r1 | CCTAACAAGCCCCAAATCAA | 1112 |
| XIST | M97168 | S8271/XIST.p1 | TGCATTGCATGAGCTAAACCTATCT GA | 1113 |
| ZW10 | NM_004724 | T2117/ZW10.f1 | TGGTCAGATGCTGCTGAAGT | 1114 |
| ZW10 | NM_004724 | T2118/ZW10.r1 | ATCACAGCATGAAGGGATGG | 1115 |
| ZW10 | NM_004724 | T2119/ZW10.p1 | TATCCTTAGGCCGCTGGCATCTTG | 1116 |
| ZWILCH | NM_017975 | T2057/ZWILCH.f1 | GAGGGAGCAGACAGTGGGT | 1117 |
| ZWILCH | NM_017975 | T2058/ZWILCH.r1 | TCAGAGCCCTTGCTAAGTCAC | 1118 |
| ZWILCH | NM_017975 | T2059/ZWILCH.p1 | CCACGATCTCCGTAACCATTTGCA | 1119 |
| ZWINT | NM_007057 | S8920/ZWINT.f1 | TAGAGGCCATCAAAATTGGC | 1120 |
| ZWINT | NM_007057 | S8921/ZWINT.r1 | TCCGTTTCCTCTGGGCTT | 1121 |
| ZWINT | NM_007057 | S8922/ZWINT.p1 | ACCAAGGCCCTGACTCAGATGGAG | 1122 |

| **Appendix 2** | | | |
|---|---|---|---|
| Gene Name | Accession # | Amplicon Sequence | SEQ ID NO: |
| ABCA9 | NM_080283 | TTACCCGTGGGAACTGTCTCCAAATACATACTTCCTCTCA CCAGGACAACAACCACAGGATCCTCTGACCCATTTACTG GTC | 401 |
| ABCB1 | NM_000927 | AAACACCACTGGAGCATTGACTACCAGGCTCGCCAATGA TGCTGCTCAAGTTAAAGGGGCTATAGGTTCCAGGCTTG | 402 |
| ABCB5 | NM_178559 | AGACAGTCGCCTTGGTCGGTCTCAATGGCAGTGGGAAGA GTACGGTAGTCCAGCTTCTGCAGAGGTT | 403 |
| ABCC10 | NM_03345 0 | ACCAGTGCCACAATGCAGTGGCTGGACATTCGGCTACAG CTCATGGGGGCGGCAGTGGTCAGCGCTAT | 404 |
| ABCC11 | NM 032583 | AAGCCACAGCCTCCATTGACATGGAGACAGACACCCTGA TCCAGCGCACAATCCGTGAAGCCTTCC | 405 |
| ABCC5 | NM_005688 | TGCAGACTGTACCATGCTGACCATTGCCCATCGCCTGCA CACGGTTCTAGGCTCCGATAGGATTATGGTGCTGGCC | 406 |
| ABCD1 | NM_000033 | TCTGTGGCCCACCTCTACTCCAACCTGACCAAGCCACTC CTGGACGTGGCTGTGACTTCCTACACCC | 407 |
| ACTG2 | NM_001615 | ATGTACGTCGCCATTCAAGCTGTGCTCTCCCTCTATGCCT CTGGCCGCACGACAGGCATCGTCCTGGATTCAGGTGATG GCGT | 408 |
| ACTR2 | NM_005722 | ATCCGCATTGAAGACCCACCCCGCAGAAAGCACATGGTA TTCCTGGGTGGTGCAGTTCTAGCGGAT | 409 |
| ACTR3 | NM_005721 | CAACTGCTGAGAGACCGAGAAGTAGGAATCCCTCCAGAA CAATCCTTGGAAACTGCTAAGGCAGTAAAGGAGCG | 410 |
| AK055699 | NM_194317 | CTGCATGTGATTGAATAAGAAACAAGAAAGTGACCACACC AAAGCCTCCCTGGCTGGTGTACAGGGATCAGGTCCACA | 411 |
| AKT1 | NM_005163 | CGCTTCTATGGCGCTGAGATTGTGTCAGCCCTGGACTAC CTGCACTCGGAGAAGAACGTGGTGTACCGGGA | 412 |
| AKT2 | NM_001626 | TCCTGCCACCCTTCAAACCTCAGGTCACGTCCGAGGTCG ACACAAGGTACTTCGATGATGAATTTACCGCC | 413 |
| AKT3 | NM_005465 | TTGTCTCTGCCTTGGACTATCTACATTCCGGAAAGATTGT GTACCGTGATCTCAAGTTGGAGAATCTAATGCTGG | 414 |
| ANXA4 | NM_001153 | TGGGAGGGATGAAGGAAATTATCTGGACGATGCTCTCGT GAGACAGGATGCCCAGGACCTGTATGAG | 415 |
| APC | NM_000038 | GGACAGCAGGAATGTGTTTCTCCATACAGGTCACGGGGA GCCAATGGTTCAGAAACAAATCGAGTGGGT | 416 |
| APEX-1 | NM 001641 | GATGAAGCCTTTCGCAAGTTCCTGAAGGGCCTGGCTTCC CGAAAGCCCCTTGTGCTGTGTGGAGACCT | 417 |
| APOC1 | NM_001645 | GGAAACACACTGGAGGACAAGGCTCGGAACTCATCAG CCGCATCAAACAGAGTGAACTTTCTGCCAAGATGCG | 418 |
| APOD | NM_001647 | GTTTATGCCATCGGCACCGTACTGGATCCTGGCCACCGA CTATGAGAACTATGCCCTCGTGTATTCC | 419 |
| APOE | NM_000041 | GCCTCAAGAGCTGGTTCGAGCCCCTGGTGGAAGACATG CAGCGCCAGTGGGCCGGGCTGGTGGAGAAGGTGCAGG | 420 |
| APRT | NM_000485 | GAGGTCCTGGAGTGCGTGAGCCTGGTGGAGCTGACCTC GCTTAAGGGCAGGGAGAAGCTGGCACCT | 421 |
| ARHA | NM_001664 | GGTCCTCCGTCGGTTCTCTCATTAGTCCACGGTCTGGTC TTCAGCTACCCGCCTTCGTCTCCGAGTTTGCGAC | 422 |
| AURKB | NM_004217 | AGCTGCAGAAGAGCTGCACATTTGACGAGCAGCGAACAG CCACGATCATGGAGGAGTTGGCAGATGC | 423 |
| B-actin | NM_001101 | CAGCAGATGTGGATCAGCAAGCAGGAGTATGACGAGTCC GGCCCCTCCATCGTCCACCGCAAATGC | 424 |
| BAD | NM_032989 | GGGTCAGGTGCCTCGAGATCGGGCTTGGGCCCAGAGCA TGTTCCAGATCCCAGAGTTTGAGCCGAGTGAGCAG | 425 |
| BAG1 | NM_004323 | CGTTGTCAGCACTTGGAATACAAGATGGTTGCCGGGTCA TGTTAATTGGGAAAAAGAACAGTCCACAGGAAGAGGTTG AAC | 426 |
| Bak | NM 001188 | CCATTCCCACCATTCTACCTGAGGCCAGGACGTCTGGGG TGTGGGGATTGGTGGGTCTATGTTCCC | 427 |
| Bax | NM_004324 | CCGCCGTGGACACAGACTCCCCCGAGAGGTCTTTTTCC GAGTGGCAGCTGACATGTTTCTGACGGCAA | 428 |
| BBC3 | NM_014417 | CCTGGAGGGTCCTGTACAATCTCATCATGGGACTCCTGC CCTTACCCAGGGGCCACAGAGCCCCCGAGATGGAGCCC AATTAG | 429 |
| B-Catenin | NM_001904 | GGCTCTTGTGCGTACTGTCCTTCGGGCTGGTGACAGGGA AGACATCACTGAGCCTGCCATCTGTGCTCTTCGTCATCTG A | 430 |
| Bcl2 | NM_000633 | CAGATGGACCTAGTACCCACTGAGATTTCCACGCCGAAG GACAGCGATGGGAAAATGCCCTTAAATCATAGG | 431 |
| BCL2L11 | NM_138621 | AATTACCAAGCAGCCGAAGACCACCCACGAATGGTTATC TTACGACTGTTACGTTACATTGTCCGCCTG | 432 |
| BCL2L13 | NM_015367 | CAGCGACAACTCTGGACAAGTCAGTCCCCCAGAGTCTCC AACTGTGACCACTTCCTGGCAGTCTGAGAGC | 433 |
| Bclx | NM_001191 | CTTTTGTGGAACTCTATGGGAACAATGCAGCAGCCGAGA GCCGAAAGGGCCAGGAACGCTTCAACCGCTG | 434 |
| BCRP | NM_004827 | TGTACTGGCGAAGAATATTTGGTAAAGCAGGGCATCGAT CTCTCACCCTGGGGCTTGTGGAAGAATCACGTGGC | 435 |
| BID | NM_001196 | GGACTGTGAGGTCAACAACGGTTCCAGCCTCAGGGATGA GTGCATCACAAACCTACTGGTGTTTGGCTTCC | 436 |
| BIN1 | NM_004305 | CCTGCAAAAGGGAACAAGAGCCCTTCGCCTCCAGATGGC TCCCCTGCCGCCACCCCCGAGATCAGAGTCAACCACG | 437 |
| BRCA1 | NM_007295 | TCAGGGGGCTAGAAATCTGTTGCTATGGGCCCTTCACCA ACATGCCCACAGATCAACTGGAATGG | 438 |
| BRCA2 | NM_000059 | AGTTCGTGCTTTGCAAGATGGTGCAGAGCTTTATGAAGC AGTGAAGAATGCAGCAGACCCAGCTTACCTT | 439 |
| BUB1 | NM_004336 | CCGAGGTTAATCCAGCACGTATGGGGCCAAGTGTAGGCT CCCAGCAGGAACTGAGAGCGCCATGTCTT | 440 |
| BUB1B | NM 001211 | TCAACAGAAGGCTGAACCACTAGAAAGACTACAGTCCCA GCACCGACAATTCCAAGCTCGAGTGTCTCGGCAAACTCT GTTG | 441 |
| BUB3 | NM_004725 | CTGAAGCAGATGGTTCATCATTTCCTGGGCTGTTAAACAA AGCGAGGTTAAGGTTAGACTCTTGGGAATCAGC | 442 |
| C14orf10 | NM_017917 | GTCAGCGTGGTAGCGGTATTCTCCGCGGCAGTGACAGTA ATTGTTTTTGCCTCTTTAGCCAAGACTTCC | 443 |
| C20_orf1 | NM_012112 | TCAGCTGTGAGCTGCGGATACCGCCCGGCAATGGACC TGCTCTTAACCTCAAACCTAGGACCGT | 444 |
| CA9 | NM_001216 | ATCCTAGCCCTGGTTTTTGGCCTCCTTTTTGCTGTCACCA GCGTCGCGTTCCTTGTGCAGATGAGAAGGCAG | 445 |
| CALD1 | NM_004342 | CACTAAGGTTTGAGACAGTTCCAGAAAGAACCCAAGCTC AAGACGCAGGACGAGCTCAGTTGTAGAGGGCTAATTCGC | 446 |
| CAPZA1 | NM_006135 | TCGTTGGAGATCAGAGTGGAAGTTCACCATCACACCACC TACAGCCCAGGTGGTTGGCGTGCTTAA | 447 |
| CAV1 | NM_001753 | GTGGCTCAACATTGTGTTCCCATTTCAGCTGATCAGTGG GCCTCCAAGGAGGGGCTGTAAAATGGAGGCCATTG | 448 |
| CCNB1 | NM_031966 | TTCAGGTTGTTGCAGGAGACCATGTACATGACTGTCTCCA TTATTGATCGGTTCATGCAGAATAATTGTGTGCCCAAGAA GATG | 449 |
| CCND1 | NM 053056 | GCATGTTCGTGGCCTCTAAGATGAAGGAGACCATCCCCC TGACGGCCGAGAAGCTGTGCATCTACACCG | 450 |
| CCNE2 | NM_057749 | ATGCTGTGGCTCCTTCCTAACTGGGGCTTTCTTGACATGT AGGTTGCTTGGTAATAACCTTTTTGTATATCACAATTTGG GT | 451 |
| CCT3 | NM_0010088 00 | ATCCAAGGCCATGACTGGTGTGGAACAATGGCCATACAG GGCTGTTGCCCAGGCCCTAGAGGTCATTCC | 452 |
| CD14 | NM_000591 | GTGTGCTAGCGTACTCCCGCCTCAAGGAACTGACGCTCG AGGACCTAAAGATAACCGGCACCATGC | 453 |
| CD31 | NM_000442 | TGTATTTCAAGACCTCTGTGCACTTATTTATGAACCTGCC CTGCTCCCACAGAACACAGCAATTCCTCAGGCTAA | 454 |
| CD3z | NM_000734 | AGATGAAGTGGAAGGCGCTTTTCACCGCGGCCATCCTGC AGGCACAGTTGCCGATTACAGAGGCA | 455 |
| CD63 | NM_001780 | AGTGGGACTGATTGCCGTGGGTGTCGGGGCACAGCTTG TCCTGAGTCAGACCATAATCCAGGGGGCTACCC | 456 |
| CD68 | NM_001251 | TGGTTCCCAGCCCTGTGTCCACCTCCAAGCCCAGATTCA GATTCGAGTCATGTACACAACCCAGGGTGGAGGAG | 457 |
| CDC2 | NM 001786 | GAGAGCGACGCGGTTGTTGTAGCTGCCGCTGCGGCCGC CGCGGAATAATAAGCCGGGATCTACCATAC | 458 |
| CDC20 | NM_001255 | TGGATTGGAGTTCTGGGAATGTACTGGCCGTGGCACTGG ACAACAGTGTGTACCTGTGGAGTGCAAGC | 459 |
| CDC25B | NM_021873 | AAACGAGCAGTTTGCCATCAGACGCTTCCAGTCTATGCC GGTGAGGCTGCTGGGCCACAGCCCCGTGCTTCGGAACA TCACCAAC | 460 |
| CDCA8 | NM_018101 | GAGGCACAGTATTGCCCAGCTGGATCCAGAGGCCTTGG GAAACATTAAGAAGCTCTCCAACCGTCTC | 461 |
| CDH1 | NM_004360 | TGAGTGTCCCCCGGTATCTTCCCCGCCCTGCCAATCCCG ATGAAATTGGAAATTTTATTGATGAAAATCTGAAAGCGGC TG | 462 |
| CDK5 | NM 004935 | AAGCCCTATCCGATGTACCCGGCCACAACATCCCTGGTG AACGTCGTGCCCAAACTCAATGCCACAG | 463 |
| CDKN1C | NM_000076 | CGGCGATCAAGAAGCTGTCCGGGCCTCTGATCTCCGATT TCTTCGCCAAGCGCAAGAGATCAGCGCCTG | 464 |
| CEGP1 | NM_020974 | TGACAATCAGCACACCTGCATTCACCGCTCGGAAGAGGG CCTGAGCTGCATGAATAAGGATCACGGCTGTAGTCACA | 465 |
| CENPA | NM_001809 | TAAATTCACTCGTGGTGTGGACTTCAATTGGCAAGCCCA GGCCCTATTGGCCCTACAAGAGGC | 466 |
| CENPE | NM_001813 | GGATGCTGGTGACCTCTTCTTCCCTCACGTTGCAACAGG AATTAAAGGCTAAAAGAAAACGAAGAGTTACTTGGTGCCT TGGC | 467 |
| CENPF | NM_016343 | CTCCCGTCAACAGCGTTCTTTCCAAACACTGGACCAGGA GTGCATCCAGATGAAGGCCAGACTCACCC | 468 |
| CGA (CHGA official) | NM_001275 | CTGAAGGAGCTCCAAGACCTCGCTCTCCAAGGCGCCAAG GAGAGGGCACATCAGCAGAAGAAACACAGCGGTTTTG | 469 |
| CHFR | NM_018223 | AAGGAAGTGGTCCCTCTGTGGCAAGTGATGAAGTCTCCA GCTTTGCCTCAGCTCTCCCAGACAGAAAGACTGCGTC | 470 |
| Chk1 | NM 001274 | GATAAATTGGTACAAGGGATCAGCTTTTCCCAGCCCACAT GTCCTGATCATATGCTTTTGAATAGTCAGTTACTTGGCAC CC | 471 |
| Chk2 | NM_007194 | ATGTGGAACCCCCACCTACTTGGCGCCTGAAGTTCTTGT TTCTGTTGGGACTGCTGGGTATAACCGTGCTGTGGACTG | 472 |
| clAP2 | NM_001165 | GGATATTTCCGTGGCTCTTATTCAAACTCTCCATCAAATC CTGTAAACTCCAGAGCAAATCAAGATTTTTCTGCCTTGAT GAGAAG | 473 |
| CKAP1 | NM_001281 | TCATTGACCACAGTGGCGCCCGCCTTGGTGAGTATGAGG ACGTGTCCCGGGTGGAGAAGTACACGA | 474 |
| CLU | NM_001831 | CCCCAGGATACCTACCACTACCTGCCCTTCAGCCTGCCC CACCGGAGGCCTCACTTCTTCTTTCCCAAGTCCCGCA | 475 |
| cMet | NM_000245 | GACATTTCCAGTCCTGCAGTCAATGCCTCTCTGCCCCAC CCTTTGTTCAGTGTGGCTGGTGCCACGACAAATGTGTGC GATCGGAG | 476 |
| cMYC | NM_002467 | TCCCTCCACTCGGAAGGACTATCCTGCTGCCAAGAGGGT CAAGTTGGACAGTGTCAGAGTCCTGAGACAGATCAGCAA CAACCG | 477 |
| CNN | NM_001299 | TCCACCCTCCTGGCTTTGGCCAGCATGGCGAAGACGAAA GGAAACAAGGTGAACGTGGGAGTGA | 478 |
| COL1A1 | NM 000088 | GTGGCCATCCAGCTGACCTTCCTGCGCCTGATGTCCACC GAGGCCTCCCAGAACATCACCTACCACTG | 479 |
| COL1A2 | NM_000089 | CAGCCAAGAACTGGTATAGGAGCTCCAAGGACAAGAAAC ACGTCTGGCTAGGAGAAACTATCAATGCTGGCAGCCAGT TT | 480 |
| COL6A3 | NM_004369 | GAGAGCAAGCGAGACATTCTGTTCCTCTTTGACGGCTCA GCCAATCTTGTGGGCCAGTTCCCTGTT | 481 |
| Contig 51037 | M_198477 | CGACAGTTGCGATGAAAGTTCTAATCTCTTCCCTCCTCCT GTTGCTGCCACTAATGCTGATGTCCATGGTCTCTAGCAG CC | 482 |
| COX2 | NM_000963 | TCTGCAGAGTTGGAAGCACTCTATGGTGACATCGATGCT GTGGAGCTGTATCCTGCCCTTCTGGTAGAAAAGCCTCGG C | 483 |
| COX7C | NM 001867 | ACCTCTGTGGTCCGTAGGAGCCACTATGAGGAGGGCCCT GGGAAGAATTTGCCATTTTCAGTGGAAAACAAGTGGTCG | 484 |
| CRABP1 | NM_004378 | AACTTCAAGGTCGGAGAAGGCTTTGAGGAGGAGACCGTG GACGGACGCAAGTGCAGGAGTTTAGCCA | 485 |
| CRIP2 | NM_001312 | GTGCTACGCCACCCTGTTCGGACCCAAAGGCGTGAACAT CGGGGGCGCGGGCTCCTACATCTACGAGAAGCCCCTG | 486 |
| CRYAB | NM_001885 | GATGTGATTGAGGTGCATGGAAAACATGAAGAGCGCCAG GATGAACATGGTTTCATCTCCAGGGAGTTC | 487 |
| CSF1 | NM_000757 | TGCAGCGGCTGATTGACAGTCAGATGGAGACCTCGTGCC AAATTACATTTGAGTTTGTAGACCAGGAACAGTTG | 488 |
| CSNK1D | NM_001893 | AGCTTTTCCGGAATCTGTTCCATCGCCAGGGCTTCTCCTA TGACTACGTGTTCGACTGGAACATGCTCAAAT | 489 |
| CST7 | NM_003650 | TGGCAGAACTACCTGCAAGAAAAACCAGCACCTGCGTCT GGATGACTGTGACTTCCAAACCAACCACACCTTGAAGCA | 490 |
| CTSD | NM_001909 | GTACATGATCCCCTGTGAGAAGGTGTCCACCCTGCCCGC GATCACACTGAAGCTGGGAGGCAAAGGCTACAAGCTGTC CC | 491 |
| CTSL | NM_001912 | GGGAGGCTTATCTCACTGAGTGAGCAGAATCTGGTAGAC TGCTCTGGGCCTCAAGGCAATGAAGGCTGCAATGG | 492 |
| CTSL2 | NM_001333 | TGTCTCACTGAGCGAGCAGAATCTGGTGGACTGTTCGCG TCCTCAAGGCAATCAGGGCTGCAATGGT | 493 |
| CXCR4 | NM_003467 | TGACCGCTTCTACCCCAATGACTTGTGGGTGGTTGTGTTC CAGTTTCAGCACATCATGGTTGGCCTTATCCT | 494 |
| CYBA | NM_000101 | GGTGCCTACTCCATTGTGGCGGGCGTGTTTGTGTGCCTG CTGGAGTACCCCCGGGGGAAGAGGAAGAAGGGCTCCAC | 495 |
| CYP1B1 | NM_000104 | CCAGCTTTGTGCCTGTCACTATTCCTCATGCCACCACTGC CAACACCTCTGTCTTGGGCTACCACATTCCC | 496 |
| CYP2C8 | NM_000770 | CCGTGTTCAAGAGGAAGCTCACTGCCTTGTGGAGGAGTT GAGAAAAACCAAGGCTTCACCCTGTGATCCCACT | 497 |
| CYP3A4 | NM_017460 | AGAACAAGGACAACATAGATCCTTACATATACACACCCTT TGGAAGTGGACCCAGAAACTGCATTGGCATGAGGTTTGC | 498 |
| DDR1 | NM_001954 | CCGTGTGGCTCGCTTTCTGCAGTGCCGCTTCCTCTTTGC GGGGCCCTGGTTACTCTTCAGCGAAATCTCC | 499 |
| DIABLO | NM_019887 | CACAATGGCGGCTCTGAAGAGTTGGCTGTCGCGCAGCG TAACTTCATTCTTCAGGTACAGACAGTGTTTGTGT | 500 |
| DIAPH1 | NM_005219 | CAAGCAGTCAAGGAGAACCAGAAGCGGCGGGAGACAGA AGAAAAGATGAGGCGAGCAAAACT | 501 |
| DICER1 | NM_177438 | TCCAATTCCAGCATCACTGTGGAGAAAAGCTGTTTGTCTC CCCAGCATACTTTATCGCCTTCACTGCC | 502 |
| DKFZp564 D0462; | NM_198569 | CAGTGCTTCCATGGACAAGTCCTTGTCAAAACTGGCCCA TGCTGATGGAGATCAAACATCAATCATCCCTGTCCA | 503 |
| DR4 | NM_003844 | TGCACAGAGGGTGTGGGTTACACCAATGCTTCCAACAAT TTGTTTGCTTGCCTCCCATGTACAGCTTGTAAATCAGATG | 504 |
| | | AAGA | |
| DR5 | NM_003842 | CTCTGAGACAGTGCTTCGATGACTTTGCAGACTTGGTGC CCTTTGACTCCTGGGAGCCGCTCATGAGGAAGTTGGGCC TCATGG | 505 |
| DUSP1 | NM_004417 | AGACATCAGCTCCTGGTTCAACGAGGCCATTGACTTCATA GACTCCATCAAGAATGCTGGAGGAAGGGTGTTTGTC | 506 |
| EEF1D | NM_001960 | CAGAGGATGACGAGGATGATGACATTGACCTGTTTGGCA GTGACAATGAGGAGGAGGACAAGGAGGCGGCACAG | 507 |
| EGFR | NM_005228 | TGTCGATGGACTTCCAGAACCACCTGGGCAGCTGCCAAA AGTGTGATCCAAGCTGTCCCAAT | 508 |
| EIF4E | NM_001968 | GATCTAAGATGGCGACTGTCGAACCGGAAACCACCCCTA CTCCTAATCCCCCGACTACAGAAGAGGAGAAAACGGAAT CTAA | 509 |
| EIF4EL3 | NM 004846 | AAGCCGCGGTTGAATGTGCCATGACCCTCTCCCTCTCTG GATGGCACCATCATTGAAGCTGGCGTCA | 510 |
| ELP3 | NM_018091 | CTCGGATCCTAGCCCTCGTGCCTCCATGGACTCGAGTGT ACCGAGTACAGAGGGATATTCCAATGCC | 511 |
| ER2 | NM_001437 | TGGTCCATCGCCAGTTATCACATCTGTATGCGGAACCTCA AAAGAGTCCCTGGTGTGAAGCAAGATCGCTAGAACA | 512 |
| ErbB3 | NM_001982 | CGGTTATGTCATGCCAGATACACACCTCAAAGGTACTCC CTCCTCCCGGGAAGGCACCCTTTCTTCAGTGGGTCTCAG TTC | 513 |
| ERBB4 | NM_005235 | TGGCTCTTAATCAGTTTCGTTACCTGCCTCTGGAGAATTT ACGCATTATTCGTGGGACAAAACTTTATGAGGATCGATAT GCCTTG | 514 |
| ERCC1 | NM_001983 | GTCCAGGTGGATGTGAAAGATCCCCAGCAGGCCCTCAAG GAGCTGGCTAAGATGTGTATCCTGGCCG | 515 |
| ERK1 | NM_002746 | ACGGATCACAGTGGAGGAAGCGCTGGCTCACCCCTACCT GGAGCAGTACTATGACCCGACGGATGAG | 516 |
| ESPL1 | MM_012291 | ACCCCCAGACCGGATCAGGCAAGCTGGCCCTCATGTCC CCTTCACGGTGTTTGAGGAAGTCTGCCCTACA | 517 |
| EstR1 | NM_000125 | CGTGGTGCCCCTCTATGACCTGCTGCTGGAGATGCTGGA CGCCCACCGCCTACATGCGCCCACTAGCC | 518 |
| fas | NM 000043 | GGATTGCTCAACAACCATGCTGGGCATCTGGACCCTCCT ACCTCTGGTTCTTACGTCTGTTGCTAGATTATCGTCCAAA AGTGTTAATGCC | 519 |
| fasl | NM_000639 | GCACTTTGGGATTCTTTCCATTATGATTCTTTGTTACAGGC ACCGAGAATGTTGTATTCAGTGAGGGTCTTCTTACATGC | 520 |
| FASN | NM_004104 | GCCTCTTCCTGTTCGACGGCTCGCCCACCTACGTACTGG CCTACACCCAGAGCTACCGGGCAAAGC | 521 |
| FBXO5 | NM_012177 | GGCTATTCCTCATTTTCTCTACAAAGTGGCCTCAGTGAAC ATGAAGAAGGTAGCCTCCTGGAGGAGAATTTCGGTGACA GTCTACAATCC | 522 |
| FDFT1 | NM_004462 | AAGGAAAGGGTGCCTCATCCCAGCAACCTGTCCTTGTGG GTGATGATCACTGTGCTGCTTGTGGCTC | 523 |
| FGFR1 | NM_023109 | CACGGGACATTCACCACATCGACTACTATAAAAGACAAC CAACGGCCGACTGCCTGTGAAGTGGATGGCACCC | 524 |
| FHIT | NM 002012 | CCAGTGGAGCGCTTCCATGACCTGCGTCCTGATGAAGTG GCCGATTTGTTTCAGACGACCCAGAGAG | 525 |
| FIGF | NM_004469 | GGTTCCAGCTTTCTGTAGCTGTAAGCATTGGTGGCCACA CCACCTCCTTACAAAGCAACTAGAACCTGCGGC | 526 |
| FLJ20354 (DEPDC1 official) | NM_017779 | GCGTATGATTTCCCGAATGAGTCAAAATGTTGATATGCCC AAACTTCATGATGCAATGGGTACGAGGTCACTG | 527 |
| FOS | NM_005252 | CGAGCCCTTTGATGACTTCCTGTTCCCAGCATCATCCAG GCCCAGTGGCTCTGAGACAGCCCGCTCC | 528 |
| FOXM1 | NM_021953 | CCACCCCGAGCAAATCTGTCCTCCCCAGAACCCCTGAAT CCTGGAGGCTCACGCCCCCAGCCAAAGTAGGGGGACTG GATTT | 529 |
| FUS | NM 004960 | GGATAATTCAGACAACAACACCATCTTTGTGCAAGGCCTG GGTGAGAATGTTACAATTGAGTCTGTGGCTGATTACTTCA | 530 |
| FYN | NM_002037 | GAAGCGCAGATCATGAAGAAGCTGAAGCACGACAAGCTG GTCCAGCTCTATGCAGTGGTGTCTGAGGAG | 531 |
| G1P3 | NM_002038 | CCTCCAACTCCTAGCCTCAAGTGATCCTCCTGTCTCAACC TCCCAAGTAGGATTACAAGCATGCGCC | 532 |
| GADD45 | NM_001924 | GTGCTGGTGACGAATCCACATTCATCTCAATGGAAGGAT CCTGCCTTAAGTCAACTTATTTGTTTTTGCCGGG | 533 |
| GADD45B | NM_015675 | ACCCTCGACAAGACCACACTTTGGGACTTGGGAGCTGGG GCTGAAGTTGCTCTGTACCCATGAACTCCCA | 534 |
| GAGE1 | NM_001468 | AAGGGCAATCACAGTGTTAAAAGAAGACATGCTGAAATGT TGCAGGCTGCTCCTATGTTGGAAAATTCTTCATTGAAGTT CTCC | 535 |
| GAPDH | NM_002046 | ATTCCACCCATGGCAAATTCCATGGCACCGTCAAGGCTG AGAACGGGAAGCTTGTCATCAATGGAAATCCCATC | 536 |
| GATA3 | NM_002051 | CAAAGGAGCTCACTGTGGTGTCTGTGTTCCAACCACTGA ATCTGGACCCCATCTGTGAATAAGCCATTCTGACTC | 537 |
| GBP1 | NM_002053 | TTGGGAAATATTTGGGCATTGGTCTGGCCAAGTCTACAAT GTCCCAATATCAAGGACAACCACCCTAGCTTCT | 538 |
| GBP2 | NM 004120 | GCATGGGAACCATCAACCAGCAGGCCATGGACCAACTTC ACTATGTGACAGAGCTGACAGATCGAATCAAGGCAAACT CCTCA | 539 |
| GCLC | NM_001498 | CTGTTGCAGGAAGGCATTGATCATCTCCTGGCCCAGCAT GTTGCTCATCTCTTTATTAGAGACCCACTGAC | 540 |
| GDF15 | NM_004864 | CGCTCCAGACCTATGATGACTTGTTAGCCAAAGACTGCC ACTGCATATGAGCAGTCCTGGTCCTTCCACTGT | 541 |
| GGPS1 | NM_004837 | CTCCGACGTGGCTTTCCAGTGGCCCACAGCATCTATGGA ATCCCATCTGTCATCAATTCTGCCAATTACG | 542 |
| GLRX | NM_002064 | GGAGCTCTGCAGTAACCACAGAACAGGCCCCATGCTGAC GTCCCTCCTCAAGAGCTGGATGGCATTG | 543 |
| GNS | NM_002076 | GGTGAAGGTTGTCTCTTCCGAGGGCCTTCTGAAGACAGG GCTCTTGAACAGACAAGTGGAAGGGCTG | 544 |
| GPR56 | NM_005682 | TACCCTTCCATGTGCTGGATCCGGGACTCCCTGGTCAGC TACATCACCAACCTGGGCCTCTTCAGC | 545 |
| GPX1 | NM_000581 | GCTTATGACCGACCCCAAGCTCATCACCTGGTCTCCGGT GTGTCGCAACGATGTTGCCTGGAACTTT | 546 |
| GRB7 | NM_005310 | CCATCTGCATCCATCTTGTTTGGGCTCCCCACCCTTGAGA AGTGCCTCAGATAATACCCTGGTGGCC | 547 |
| GSK3B | NM_002093 | GACAAGGACGGCAGCAAGGTGACAACAGTGGTGGCAAC TCCTGGGCAGGGTCCAGACAGGCCACAA | 548 |
| GSR | NM_000637 | GTGATCCCAAGCCCACAATAGAGGTCAGTGGGAAAAAGT ACACCGCCCCACACATCCTGATCGCCACA | 549 |
| GSTM1 | NM_000561 | AAGCTATGAGGAAAAGAAGTACACGATGGGGGACGCTCC | 550 |
| | | TGATTATGACAGAAGCCAGTGGCTGAATGAAAAATTCAAG CTGGGCC | |
| GSTp | NM_000852 | GAGACCCTGCTGTCCCAGAACCAGGGAGGCAAGACCTT CATTGTGGGAGACCAGATCTCCTTCGCTGACTACAACC | 551 |
| GUS | NM_000181 | CCCACTCAGTAGCCAAGTCACAATGTTTGGAAAACAGCC CGTTTACTTGAGCAAGACTGATACCACCTGCGTG | 552 |
| HDAC6 | NM_006044 | TCCTGTGCTCTGGAAGCCCTTGAGCCCTTCTGGGAGGTT CTTGTGAGATCAACTGAGACCGTGGAG | 553 |
| HER2 | NM_004448 | CGGTGTGAGAAGTGCAGCAAGCCCTGTGCCCGAGTGTG CTATGGTCTGGGCATGGAGCACTTGCGAGAGG | 554 |
| HIF1A | NM 001530 | TGAACATAAAGTCTGCAACATGGAAGGTATTGCACTGCAC AGGCCACATTCACGTATATGATACCAACAGTAACCAACCT CA | 555 |
| HNF3A | NM_004496 | TCCAGGATGTTAGGAACTGTGAAGATGGAAGGGCATGAA ACCAGCGACTGGAACAGCTACTACGCAGACACGC | 556 |
| HRAS | NM_005343 | GGACGAATACGACCCCACTATAGAGGATTCCTACCGGAA GCAGGTGGTCATTGATGGGGAGACGTGC | 557 |
| HSPA1A | NM_005345 | CTGCTGCGACAGTCCACTACCTTTTCGAGAGTGACTCC CGTTGTCCCAAGGCTTCCCAGAGCGAACCTG | 558 |
| HSPA1B | NM_005346 | GGTCCGCTTCGTCTTTCGAGAGTGACTCCCGCGGTCCCA AGGCTTTCCAGAGCGAACCTGTGC | 559 |
| HSPA1L | NM_005527 | GCAGGTGTGATTGCTGGACTTAATGTGCTAAGAATCATCA ATGAGCCCACGGCTGCTGCCATTGCCTATGGT | 560 |
| HSPA5 | NM_005347 | GGCTAGTAGAACTGGATCCCAACACCAAACTCTTAATTAG ACCTAGGCCTCAGCTGCACTGCCCGAAAAGCATTTGGGC AGACC | 561 |
| HSPA9B | NM_004134 | GGCCACTAAAGATGCTGGCCAGATATCTGGACTGAATGT GCTTCGGGTGATTAATGAGCCCACAGCTGCT | 562 |
| HSPB1 | NM 001540 | CCGACTGGAGGAGCATAAAAGCGCAGCCGAGCCCAGCG CCCCGCACTTTTCTGAGCAGACGTCCAGAGCAGAGTCAG CCAGCAT | 563 |
| HSPCA | NM_005348 | CAAAAGGCAGAGGCTGATAAGAACGACAAGTCTGTGAAG GATCTGGTCATCTTGCTTTATGAAACTGCGCT | 564 |
| ID1 | NM_002165 | AGAACCGCAAGGTGAGCAAGGTGGAGATTCTCCAGCAC GTCATCGACTACATCAGGGACCTTCAGTTGGA | 565 |
| IFITM1 | NM_003641 | CACGCAGAAAACCACACTTCTCAAACCTTCACTCAACACT TCCTTCCCCAAAGCCAGAAGATGCACAAGGAGGAACATG | 566 |
| IGF1 R | NM_000875 | GCATGGTAGCCGAAGATTTCACAGTCAAAATCGGAGATTT TGGTATGACGCGAGATATCTATGAGACAGACTATTACCG GAAA | 567 |
| IGFBP2 | NM_000597 | GTGGACAGCACCATGAACATGTTGGGCGGGGGAGGCAG TGCTGGCCGGAAGCCCCTCAAGTCGGGTATGAAGG | 568 |
| IGFBP3 | NM_000598 | ACGCACCGGGTGTCTGATCCCAAGTTCCACCCCCTCCAT TCAAAGATAATCATCATCAAGAAAGGGCA | 569 |
| IGFBP5 | NM_000599 | TGGACAAGTACGGGATGAAGCTGCCAGGCATGGAGTAC GTTGACGGGGACTTTCAGTGCCACACCTTCG | 570 |
| IL2RA | NM_000417 | TCTGCGTGGTTCCTTTCTCAGCCGCTTCTGACTGCTGATT CTCCCGTTCACGTTGCCTAATAAACATCCTTCAA | 571 |
| IL6 | NM_000600 | CCTGAACCTTCCAAAGATGGCTGAAAAAGATGGATGCTT CCAATCTGGATTCAATGAGGAGACTTGCCTGGT | 572 |
| IL-7 | NM_000880 | GCGGTGATTCGGAAATTCGCGAATTCCTCTGGTCCTCAT | 573 |
| | | CCAGGTGCGCGGGAAGCAGGTGCCCAGGAGAG | |
| IL-8 | NM_000584 | AAGGAACCATCTCACTGTGTGTAAACATGACTTCCAAGCT GGCCGTGGCTCTCTTGGCAGCCTTCCTGAT | 574 |
| ILBRB | NM_001557 | CCGCTCCGTCACTGATGTCTACCTGCTGAACCTAGCCTT GGCCGACCTACTCTTTGCCCTGACCTTGC | 575 |
| ILK | NM_0010147 94 | CTCAGGATTTTCTCGCATCCAAATGTGCTCCCAGTGCTAG GTGCCTGCCAGTCTCCACCTGCTCCT | 576 |
| ILT-2 | NM_006669 | AGCCATCACTCTCAGTGCAGCCAGGTCCTATCGTGGCCC CTGAGGAGACCCTGACTCTGCAGT | 577 |
| INCENP | NM_020238 | GCCAGGATACTGGAGTCCATCACAGTGAGCTCCCTGATG GCTACACCCCAGGACCCCAAGGGTCAAG | 578 |
| IRAK2 | NM_001570 | GGATGGAGTTCGCCTCCTACGTGATCACAGACCTGACCC AGCTGCGGAAGATCAAGTCCATGGAGCG | 579 |
| IRS1 | NM_005544 | CCACAGCTCACCTTCTGTCAGGTGTCCATCCCAGCTCCA GCCAGCTCCCAGAGAGGAAGAGACTGGCACTGAGG | 580 |
| ITGB1 | NM 002211 | TCAGAATTGGATTTGGCTCATTTGTGGAAAAGACTGTGAT GCCTTACATTAGCACAACACCAGCTAAGCTCAGG | 581 |
| K-Alpha-1 | NM_006082 | TGAGGAAGAAGGAGAGGAATACTAATTATCCATTCCTTTT GGCCCTGCAGCATGTCATGCTCCCAGAATTTCAG | 582 |
| KDR | NM_002253 | GAGGACGAAGGCCTCTACACCTGCCAGGCATGCAGTGTT CTTGGCTGTGCAAAAGTGGAGGCATTTTT | 583 |
| Ki-67 | NM_002417 | CGGACTTTGGGTGCGACTTGACGAGCGGTGGTTCGACAA GTGGCCTTGCGGGCCGGATCGTCCCAGTGGAAGAGTTG TAA | 584 |
| KIF11 | NM_004523 | TGGAGGTTGTAAGCCAATGTTGTGAGGCTTCAAGTTCAG ACATCACTGAGAAATCAGATGGACGTAAGGCA | 585 |
| KIF22 | NM_007317 | CTAAGGCACTTGCTGGAAGGGCAGAATGCCAGTGTGCTT GCCTATGGACCCACAGGAGCTGGGAAGA | 586 |
| KIF2C | NM_006845 | AATTCCTGCTCCAAAAGAAAGTCTTCGAAGCCGCTCCACT CGCATGTCCACTGTCTCAGAGCTTCGCATCACG | 587 |
| KIFC1 | NM_002263 | CCACAGGGTTGAAGAACCAGAAGCCAGTTCCTGCTGTTC CTGTCCAGAAGTCTGGCACATCAGGTG | 588 |
| KLK10 | NM_002776 | GCCCAGAGGCTCCATCGTCCATCCTCTTCCTCCCCAGTC GGCTGAACTCTCCCCTTGTCTGCACTGTTCAAACCTCTG | 589 |
| KNS2 | NM_005552 | CAAACAGAGGGTGGCAGAAGTGCTCAATGACCCTGAGAA CATGGAGAAGCGCAGGAGCCGTGAGAGCCTC | 590 |
| KNTC1 | NM 014708 | AGCCGAGGCTTTGTTGAAGAAGCTTCATATCCAGTACCG GCGATCGGGCACAGAAGCTGTGCTCATAGCCCA | 591 |
| KNTC2 | NM_006101 | ATGTGCCAGTGAGCTTGAGTCCTTGGAGAAACACAAGCA CCTGCTAGAAAGTACTGTTAACCAGGGGCTCA | 592 |
| KRT14 | NM_000526 | GGCCTGCTGAGATCAAAGACTACAGTCCCTACTTCAAGA CCATTGAGGACCTGAGGAACAAGATTCTCACAGCCACAG TGGAC | 593 |
| KRT17 | NM_000422 | CGAGGATTGGTTCTTCAGCAAGACAGAGGAACTGAACCG CGAGGTGGCCACCAACAGTGAGCTGGTGCAGAGT | 594 |
| KRT19 | NM_002276 | TGAGCGGCAGAATCAGGAGTACCAGCGGCTCATGGACAT CAAGTCGCGGCTGGAGCAGGAGATTGCCACCTACCGCA | 595 |
| KRT5 | NM_000424 | TCAGTGGAGAAGGAGTTGGACCAGTCAACATCTCTGTTG TCACAAGCAGTGTTTCCTCTGGATATGGCA | 596 |
| L1CAM | NM_000425 | CTTGCTGGCCAATGCCTACATCTACGTTGTCCAGCTGCC AGCCAAGATCCTGACTGCGGACAATCA | 597 |
| LAMC2 | NM_005562 | ACTCAAGCGGAAATTGAAGCAGATAGGTCTTATCAGCAC AGTCTCCGCCTCCTGGATTCAGTGTCTCGGCTTCAGGGA GT | 598 |
| LAPTM4B | NM_018407 | AGCGATGAAGATGGTCGCGCCCTGGACGCGGTTCTACTC CAACAGCTGCTGCTTGTGCTGCCATGTC | 599 |
| LIMK1 | NM_016735 | GCTTCAGGTGTTGTGACTGCAGTGCCTCCCTGTCGCACC AGTACTATGAGAAGGATGGGCAGCTCTT | 600 |
| LIMK2 | NM_005569 | CTTTGGGCCAGGAGGAATCTGTTACTCGAATCCACCCAG GAACTCCCTGGCAGTGGATTGTGGGAG | 601 |
| MAD1L1 | NM_003550 | AGAAGCTGTCCCTGCAAGAGCAGGATGCAGCGATTGTGA AGAACATGAAGTCTGAGCTGGTACGGCT | 602 |
| MAD2L1 | NM_002358 | CCGGGAGCAGGGAATCACCCTGCGCGGAGCGCCGAAA TCGTGGCCGAGTTCTTCTCATTCGGCATCAACAGCAT | 603 |
| MAD2L1BP | NM_014628 | CTGTCATGTGGCAGACCTTCCATCCGAACCACGGCTTGG GAAGACTACATTTGGTTCCAGGCACCAGTGACATTTA | 604 |
| MAD2L2 | NM_006341 | GCCCAGTGGAGAAATTCGTCTTTGAGATCACCCAGCCTC CACTGCTGTCCATCAGCTCAGACTCGC | 605 |
| MAGE2 | NM_005361 | CCTCAGAAATTGCCAGGACTTCTTTCCCGTGATCTTCAGC AAAGCCTCCGAGTACTTGCAGCTGGTCTTTGG | 606 |
| MAGE6 | NM_005363 | AGGACTCCAGCAACCAAGAAGAGGAGGGGCCAAGCACC TTCCCTGACCTGGAGTCTGAGTTCCAAGCAGCACTC | 607 |
| MAP2 | NM_002374 | CGGACCACCAGGTCAGAGCCAATTCGCAGAGCAGGGAA GAGTGGTACCTCAACACCCACTACCCCTG | 608 |
| MAP2K3 | NM_002756 | GCCCTCCAATGTCCTTATCAACAAGGAGGGCCATGTGAA GATGTGTGACTTTGGCATCAGTGGCTAC | 609 |
| MAP4 | NM_002375 | GCCGGTCAGGCACACAAGGGGCCCTTGGAGCGTGGACT GGTTGGTTTTGCCATTTTGTTGTGTGTATGCTGC | 610 |
| MAP6 | NM_033063 | CCCTCAACCGGCAAATCCGCGAGGAGGTGGCGAGTGCA GTGAGCAGCTCCTACAGGAATGAATTCAGGGCATGGACG | 611 |
| MAPK14 | NM_139012 | TGAGTGGAAAAGCCTGACCTATGATGAAGTCATCAGCTTT GTGCCACCACCCCTTGACCAAGAAGAGATGGAGTCC | 612 |
| MAPK8 | NM_002750 | CAACACCCGTACATCAATGTCTGGTATGATCCTTCTGAAG CAGAAGCTCCACCACCAAAGATCCCTGACAAGCAGTTAG ATGA | 613 |
| MAPRE1 | NM_012325 | GACCTTGGAACCTTTGGAACCTGCTGTCAACAGGTCTTA CAGGGCTGCTTGAACCCTCATAGGCCTAGG | 614 |
| MAPT | NM_016835 | CACAAGCTGACCTTCCGCGAGAACGCCAAAGCCAAGACA GACCACGGGGCGGAGATCGTGTACAAGT | 615 |
| Maspin | NM_002639 | CAGATGGCCACTTTGAGAACATTTTAGCTGACAACAGTGT GAACGACCAGACCAAAATCCTTGTGGTTAATGCTGCC | 616 |
| MCL1 | NM_021960 | CTTCGGAAACTGGACATCAAAAACGAAGACGATGTGAAA TCGTTGTCTCGAGTGATGATCCATGTTTTCAGCGAC | 617 |
| MCM2 | NM_004526 | GACTTTTGCCCGCTACCTTTCATTCCGGCGTGACAACAAT GAGCTGTTGCTCTTCATACTGAAGCAGTTAGTGGC | 618 |
| MCM6 | NM_005915 | TGATGGTCCTATGTGTCACATTCATCACAGGTTTCATACC AACACAGGCTTCAGCACTTCCTTTGGTGTGTTTCCTGTCC CA | 619 |
| MCP1 | NM_002982 | CGCTCAGCCAGATGCAATCAATGCCCCAGTCACCTGCTG TTATAACTTCACCAATAGGAAGATCTCAGTGC | 620 |
| MGMT | NM_002412 | GTGAAATGAAACGCACCACACTGGACAGCCCTTTGGGGA AGCTGGAGCTGTCTGGTTGTGAGCAGGGTC | 621 |
| MMP12 | NM_002426 | CCAACGCTTGCCAAATCCTGACAATTCAGAACCAGCTCTC TGTGACCCCAATTTGAGTTTTGATGCTGTCACTACCGT | 622 |
| MMP2 | NM_004530 | CCATGATGGAGAGGCAGACATCATGATCAACTTTGGCCG CTGGGAGCATGGCGATGGATACCCCTTTGACGGTAAGGA CGGACTCC | 623 |
| MMP9 | NM_004994 | GAGAACCAATCTCACCGACAGGCAGCTGGCAGAGGAATA CCTGTACCGCTATGGTTACACTCGGGTG | 624 |
| MRE11A | NM_005590 | GCCATGCTGGCTCAGTCTGAGCTGTGGGCCACATCAGCT AGTGGCTCTTCTCATGCATCAGTTAGGTGGGTCTGGGTG | 625 |
| MRP1 | NM 004996 | TCATGGTGCCCGTCAATGCTGTGATGGCGATGAAGACCA AGACGTATCAGGTGGCCCACATGAAGAGCAAAGACAATC G | 626 |
| MRP2 | NM_000392 | AGGGGATGACTTGGACACATCTGCCATTCGACATGACTG CAATTTTGACAAAGCCATGCAGTTTT | 627 |
| MRP3 | NM_003786 | TCATCCTGGCGATCTACTTCCTCTGGCAGAACCTAGGTC CCTCTGTCCTGGCTGGAGTCGCTTTCATGGTCTTGCTGAT TCCACTCAACGG | 628 |
| MSH3 | NM_002439 | TGATTACCATCATGGCTCAGATTGGCTCCTATGTTCCTGC AGAAGAAGCGACAATTGGGATTGTGGATGGCATTTTCAC AAG | 629 |
| MUC1 | NM_002456 | GGCCAGGATCTGTGGTGGTACAATTGACTCTGGCCTTCC GAGAAGGTACCATCAATGTCCACGACGTGGAG | 630 |
| MX1 | NM_002462 | GAAGGAATGGGAATCAGTCATGAGCTAATCACCCTGGAG ATCAGCTCCCGAGATGTCCCGGATCTGACTCTAATAGAC | 631 |
| MYBL2 | NM_002466 | GCCGAGATCGCCAAGATGTTGCCAGGGAGGACAGACAA TGCTGTGAAGAATCACTGGAACTCTACCATCAAAAG | 632 |
| MYH11 | NM 002474 | CGGTACTTCTCAGGGCTAATATATACGTACTCTGGCCTCT TCTGCGTGGTGGTCAACCCCTATAAACACCTGCCCATCT ACTCGG | 633 |
| NEK2 | NM_002497 | GTGAGGCAGCGCGACTCTGGCGACTGGCCGGCCATGCC TTCCCGGGCTGAGGACTATGAAGTGTTGTACACCATTGG CA | 634 |
| NFKBp50 | NM_003998 | CAGACCAAGGAGATGGACCTCAGCGTGGTGCGGCTCAT GTTTACAGCTTTTCTTCCGGATAGCACTGGCAGCT | 635 |
| NFKBp65 | NM_021975 | CTGCCGGGATGGCTTCTATGAGGCTGAGCTCTGCCCGG ACCGCTGCATCCACAGTTTCCAGAACCTGG | 636 |
| NME6 | NM_005793 | CACTGACACCCGCAACACCACCCATGGTTCGGACTCTGT GGTTTCAGCCAGCAGAGAGATTGCAGCC | 637 |
| NPC2 | NM_006432 | CTGCTTCTTTCCCGAGCTTGGAACTTCGTTATCCGCGATG CGTTTCCTGGCAGCTACATTCCTGCT | 638 |
| NPD009 (ABAT official) | NM_020686 | GGCTGTGGCTGAGGCTGTAGCATCTCTGCTGGAGGTGA GACACTCTGGGAACTGATTTGACCTCGAATGCTCC | 639 |
| NTSR2 | NM_012344 | CGGACCTGAATGTAATGCAAGAATGAACAGAACAAGCAA AATGACCAGCTGCTTAGTCACCTGGCAAAG | 640 |
| NUSAP1 | NM_016359 | CAAAGGAAGAGCAACGGAAGAAACGCGAGCAAGAACGA AAGGAGAAGAAAGCAAAGGTTTTGGGAAT | 641 |
| p21 | NM 000389 | TGGAGACTCTCAGGGTCGAAAACGGCGGCAGACCAGCA TGACAGATTTCTACCACTCCAAACGCC | 642 |
| p27 | NM 004064 | CGGTGGACCACGAAGAGTTAACCCGGACTTGGAGAAG CACTGCAGAGACATGGAAGAGGCGAGCC | 643 |
| PCTK1 | NM_006201 | TCACTACCAGCTGACATCCGGCTGCCTGAGGGCTACCTG GAGAAGCTGACCCTCAATAGCCCCATCT | 644 |
| PDGFRb | NM_002609 | CCAGCTCTCCTTCCAGCTACAGATCAATGTCCCTGTCCG AGTGCTGGAGCTAAGTGAGAGCCACCC | 645 |
| PFDN5 | NM_145897 | GAGAAGCACGCCATGAAACAGGCCGTCATGGAAATGATG AGTCAGAAGATTCAGCAGCTCACAGCC | 646 |
| PGK1 | NM_000291 | AGAGCCAGTTGCTGTAGAACTCAAATCTCTGCTGGGCAA GGATGTTCTGTTCTTGAAGGACTGTGTAGGCCCAG | 647 |
| PHB | NM_002634 | GACATTGTGGTAGGGGAAGGGACTCATTTTCTCATCCCG TGGGTACAGAAACCAATTATCTTTGACTGCCG | 648 |
| PI3KC2A | NM 002645 | ATACCAATCACCGCACAAACCCAGGCTATTTGTTAAGTCC AGTCACAGCGCAAAGAAACATATGCGGAGAAAATGCTAG TGTG | 649 |
| PIM1 | NM_002648 | CTGCTCAAGGACACCGTCTACACGGACTTCGATGGGACC CGAGTGTATAGCCCTCCAGAGTGGATCC | 650 |
| PIM2 | NM_006875 | TGGGGACATTCCCTTTGAGAGGGACCAGGAGATTCTGGA AGCTGAGCTCCACTTCCCAGCCCATGTC | 651 |
| PLAUR | NM_002659 | CCCATGGATGCTCCTCTGAAGAGACTTTCCTCATTGACTG CCGAGGCCCCATGAATCAATGTCTGGTAGCCACCGG | 652 |
| PLD3 | NM_012268 | CCAAGTTCTGGGTGGTGGACCAGACCCACTTCTACCTGG GCAGTGCCAACATGGACTGGCGTTCAC | 653 |
| PLK | NM_005030 | AATGAATACAGTATTCCCAAGCACATCAACCCCGTGGCC GCCTCCCTCATCCAGAAGATGCTTCAGACA | 654 |
| PMS1 | NM_000534 | CTTACGGTTTTCGTGGAGAAGCCTTGGGGTCAATTTGTTG TATAGCTGAGGTTTTAATTACAACAAGAACGGCTGCT | 655 |
| PMS2 | NM_000535 | GATGTGGACTGCCATTCAAACCAGGAAGATACCGGATGT AAATTTCGAGTTTTGCCTCAGCCAACTAATCTCGCA | 656 |
| PP591 | NM_025207 | CCACATACCGTCCAGCCTATCTACTGGAGAACGAAGAAG AGGAGCGGAACTCCCGCACATGACCTC | 657 |
| PPP2CA | NM_002715 | GCAATCATGGAACTTGACGATACTCTAAAATACTCTTTCTT GCAGTTTGACCCAGCACCTCGTAGAGGCGAGCCACAT | 658 |
| PR | NM_000926 | GCATCAGGCTGTCATTATGGTGTCCTTACCTGTGGGAGC TGTAAGGTCTTCTTTAAGAGGGCAATGGAAGGGCAGCAC AACTACT | 659 |
| PRDX1 | NM_002574 | AGGACTGGGACCCATGAACATTCCTTTGGTATCAGACCC GAAGCGCACCATTGCTCAGGATTATGGG | 660 |
| PRDX2 | NM_005809 | GGTGTCCTTCGCCAGATCACTGTTAATGATTTGCCTGTGG GACGCTCCGTGGATGAGGCTCTGCGGCTG | 661 |
| PRKCA | NM_002737 | CAAGCAATGCGTCATCAATGTCCCCAGCCTCTGCGGAAT GGATCACACTGAGAAGAGGGGGCGGATTTAC | 662 |
| PRKCD | NM_006254 | CTGACACTTGCCGCAGAGAATCCCTTTCTCACCCACCTC ATCTGCACCTTCCAGACCAAGGACCACCT | 663 |
| PRKCG | NM_002739 | GGGTTCTAGACGCCCCTCCCAAGCGTTCCTGGCCTTCTG AACTCCATACAGCCTCTACAGCCGTCC | 664 |
| PRKCH | NM_006255 | CTCCACCTATGAGCGTCTGTCTCTGTGGGCTTGGGATGT TAACAGGAGCCAAAAGGAGGGAAAGTGTG | 665 |
| pS2 | NM_003225 | GCCCTCCCAGTGTGCAAATAAGGGCTGCTGTTTCGACGA CACCGTTCGTGGGGTCCCCTGGTGCTTCTATCCTAATAC CATCGACG | 666 |
| PTEN | NM 000314 | TGGCTAAGTGAAGATGACAATCATGTTGCAGCAATTCACT GTAAAGCTGGAAAGGGACGAACTGGTGTAATGATATGTG | 667 |
| | | CA | |
| PTPD1 | NM_007039 | CGCTTGCCTAACTCATACTTTCCCGTTGACACTTGATCCA CGCAGCGTGGCACTGGGACGTAAGTGGCGCAGTCTGAA TGG | 668 |
| PTTG1 | NM_004219 | GGCTACTCTGATCTATGTTGATAAGGAAAATGGAGAACCA GGCACCCGTGTGGTTGCTAAGGATGGGCTGAAGC | 669 |
| RAB27B | NM_004163 | GGGACACTGCGGACAAGAGCGGTTCCGGAGTCTCACC ACTGCATTTTTCAGAGACGCCATGGGC | 670 |
| RAB31 | NM_006868 | CTGAAGGACCCTACGCTCGGTGGCCTGGCACCTCACTTT GAGAAGAGTGAGCACACTGGCTTTGCAT | 671 |
| RAB6C | NM_032144 | GCGACAGCTCCTCTAGTTCCACCATGTCCGCGGCGGA GACTTCGGGAATCCGCTGAGGAAATTCAAGCTGGTGTTC C | 672 |
| RAD1 | NM_002853 | GAGGAGTGGTGACAGTCTGCAAAATCAATACACAGGAAC CTGAGGAGACCCTGGACTTTGATTTCTGCAGC | 673 |
| RAD54L | NM_003579 | AGCTAGCCTCAGTGACACACATGACAGGTTGCACTGCCG ACGTTGTGTCAACAGCCGTCAGATCCGG | 674 |
| RAF1 | NM_002880 | CGTCGTATGCGAGAGTCTGTTTCCAGGATGCCTGTTAGT TCTCAGCACAGATATTCTACACCTCACGCCTTCA | 675 |
| RALBP1 | NM_006788 | GGTGTCAGATATAAATGTGCAAATGCCTTCTTGCTGTCCT GTCGGTCTCAGTACGTTCACTTTATAGCTGCTGGCAATAT CGAA | 676 |
| RAP1GDS1 | NM_021159 | TGTGGATGCTGGATTGATTTCACCACTGGTGCAGCTGCT AAATAGCAAAGACCAGGAAGTGCTGCTT | 677 |
| RASSF1 | NM_007182 | AGTGGGAGACACCTGACCTTTCTCAAGCTGAGATTGAGC AGAAGATCAAGGAGTACAATGCCCAGATCA | 678 |
| RB1 | NM_000321 | CGAAGCCCTTACAAGTTTCCTAGTTCACCCTTACGGATTC CTGGAGGGAACATCTATATTTCACCCCTGAAGAGTCC | 679 |
| RBM17 | NM_032905 | CCCAGTGTACGAGGAACAAGACAGACCGAGATCTCCAAC CGGACCTAGCAACTCCTTCCTCGCTAA | 680 |
| RCC1 | NM_001269 | GGGCTGGGTGAGAATGTGATGGAGAGGAAGAAGCCGGC CCTGGTATCCATTCCGGAGGATGTTGTG | 681 |
| REG1A | NM_002909 | CCTACAAGTCCTGGGGCATTGGAGCCCCAAGCAGTGTTA ATCCTGGCTACTGTGTGAGCCTGACCTCA | 682 |
| RELB | NM_006509 | GCGAGGAGCTCTACTTGCTCTGCGACAAGGTGCAGAAAG AGGACATATCAGTGGTGTTCAGCAGGGC | 683 |
| RhoB | NM_004040 | AAGCATGAACAGGACTTGACCATCTTTCCAACCCCTGGG GAAGACATTTGCAACTGACTTGGGGAGG | 684 |
| rhoC | NM_175744 | CCCGTTCGGTCTGAGGAAGGCCGGGACATGGCGAACCG GATCAGTGCCTTTGGCTACCTTGAGTGCTC | 685 |
| RIZ1 | NM_012231 | CCAGACGAGCGATTAGAAGCGGCAGCTTGTGAGGTGAAT GATTTGGGGGAAGAGGAGGAGGAGGAAGAGGAGGA | 686 |
| ROCK1 | NM 005406 | TGTGCACATAGGAATGAGCTTCAGATGCAGTTGGCCAGC AAAGAGAGTGATATTGAGCAATTGCGTGCTAAAC | 687 |
| RPL37A | NM_000998 | GATCTGGCACTGTGGTTCCTGCATGAAGACAGTGGCTGG CGGTGCCTGGACGTACAATACCACTTCCGCTGTCA | 688 |
| RPLPO | NM_001002 | CCATTCTATCATCAACGGGTACAAACGAGTCCTGGCCTT GTCTGTGGAGACGGATTACACCTTCCCACTTGCTGA | 689 |
| RPN2 | NM_002951 | CTGTCTTCCTGTTGGCCCTGACAATCATAGCCAGCACCT GGGCTCTGACGCCCACTCACTACCTCAC | 690 |
| RPS6KB1 | NM_003161 | GCTCATTATGAAAAACATCCCAAACTTTAAAATGCGAAATT | 691 |
| | | ATTGGTTGGTGTGAAGAAAGCCAGACAACTTCTGTTTCTT | |
| RXRA | NM_002957 | GCTCTGTTGTGTCCTGTTGCCGGCTCTGGCCTTCCTGTG ACTGACTGTGAAGTGGCTTCTCCGTAC | 692 |
| RXRB | NM_021976 | CGAGGAGATGCCTGTGGACAGGATCCTGGAGGCAGAGC TTGCTGTGGAACAGAAGAGTGACCAGGGCGTTG | 693 |
| S100A10 | NM_002966 | ACACCAAAATGCCATCTCAAATGGAACACGCCATGGAAA CCATGATGTTTACATTTCACAAATTCGCTGGGGATAAA | 694 |
| SEC61 A | NM_013336 | CTTCTGAGCCCGTCTCCCGGACAGGTTGAGGAAGCTGCT CCAGAAGCGCCTCGGAAGGGGAGCTCTC | 695 |
| SEMA3F | NM_004186 | CGCGAGCCCCTCATTATACACTGGGCAGCCTCCCCACAG CGCATCGAGGAATGCGTGCTCTCAGGCAAGGATGTCAAC GGCGAGTG | 696 |
| SFN | NM_006142 | GAGAGAGCCAGTCTGATCCAGAAGGCCAAGCTGGCAGA GCAGGCCGAACGCTATGAGGACATGGCAGCCT | 697 |
| SGCB | NM_000232 | CAGTGGAGACCAGTTGGGTAGTGGTGACTGGGTACGCTA CAAGCTCTGCATGTGTGCTGATGGGACGCTCTTCAAGG | 698 |
| SGK | NM_005627 | TCCGCAAGACACCTCCTGGAGGGCCTCCTGCAGAAGGA CAGGACAAAGCGGCTCGGGGCCAAGGATGACTTCA | 699 |
| SGKL | NM_170709 | TGCATTCGTTGGTTTCTCTTATGCACCTCCTTCAGAAGAC TTATTTTTGTGAGCAGTTTGCCATTCAGAAA | 700 |
| SHC1 | NM_003029 | CCAACACCTTCTTGGCTTCTGGACCTGTGTTCTTGCTGA GCACCCTCTCCGGTTTGGGTTGGGATAACAG | 701 |
| SIR2 | NM_012238 | AGCTGGGGTGTCTGTTTCATGTGGAATACCTGACTTCAG GTCAAGGGATGGTATTTATGCTCGCCTTGCTGT | 702 |
| SLC1A3 | NM_004172 | GTGGGGAGCCCATCATCTCGCCAAGCCATCACAGGCTCT GCATACACATGCACTCAGTGTGGACTGG | 703 |
| SLC25A3 | NM_213611 | TCTGCCAGTGCTGAATTCTTTGCTGACATTGCCCTGGCTC CTATGGAAGCTGCTAAGGTTCGAA | 704 |
| SLC35B1 | NM_005827 | CCCAACTCAGGTCCTTGGTAAATCCTGCAAGCCAATCCC AGTCATGCTCCTTGGGGTGACCCTCTTG | 705 |
| SLC7A11 | NM_014331 | AGATGCATACTTGGAAGCACAGTCATATCACACTGGGAG GCAATGCAATGTGGTTACCTGGTCCTAGGTT | 706 |
| SLC7A5 | NM_003486 | GCGCAGAGGCCAGTTAAAGTAGATCACCTCCTCGAACCC ACTCCGGTTCCCCGCAACCCACAGCTCAGCT | 707 |
| SNAI2 | NM_003068 | GGCTGGCCAAACATAAGCAGCTGCACTGCGATGCCCAGT CTAGAAAATCTTTCAGCTGTAAATACTGTGACAAGGA | 708 |
| SNCA | NM_007308 | AGTGACAAATGTTGGAGGAGCAGTGGTGACGGGTGTGA CAGCAGTAGCCCAGAAGACAGTGGAGGG | 709 |
| SNCG | NM_003087 | ACCCACCATGGATGTCTTCAAGAAGGGCTTCTCCATCGC CAAGGAGGGCGTGGTGGGTGCGGTGGAAAAGACCAAGC AGG | 710 |
| SOD1 | NM_000454 | TGAAGAGAGGCATGTTGGAGACTTGGGCAATGTGACTGC TGACAAAGATGGTGTGGCCGATGTGTCTATT | 711 |
| SRC | NM_005417 | TGAGGAGTGGTATTTTGGCAAGATCACCAGACGGGAGTC AGAGCGGTTACTGCTCAATGCAGAGAACCCGAGAG | 712 |
| SRI | NM_003130 | ATACAGCACCAATGGAAAGATCACCTTCGACGACTACATC GCCTGCTGCGTCAAACTGAGGGCTCTTACAGACA | 713 |
| STAT1 | NM_007315 | GGGCTCAGCTTTCAGAAGTGCTGAGTTGGCAGTTTTCTT CTGTCACCAAAAGAGGTCTCAATGTGGACCAGCTGAACA TGT | 714 |
| STAT3 | NM 003150 | TCACATGCCACTTTGGTGTTTCATAATCTCCTGGGAGAGA | 715 |
| | | TTGACCAGCAGTATAGCCGCTTCCTGCAAG | |
| STK10 | NM_005990 | CAAGAGGGACTCGGACTGCAGCAGCCTCTGCACCTCTGA GAGCATGGACTATGGTACCAATCTCTCCACTGACCTG | 716 |
| STK11 | NM_000455 | GGACTCGGAGACGCTGTGCAGGAGGGCCGTCAAGATCC TCAAGAAGAAGAAGTTGCGAAGGATCCC | 717 |
| STK15 | NM_003600 | CATCTTCCAGGAGGACCACTCTCTGTGGCACCCTGGACT ACCTGCCCCCTGAAATGATTGAAGGTCGGA | 718 |
| STMN1 | NM_005563 | AATACCCAACGCACAAATGACCGCACGTTCTCTGCCCCG TTTCTTGCCCCAGTGTGGTTTGCATTGTCTCC | 719 |
| STMY3 | NM_005940 | CCTGGAGGCTGCAACATACCTCAATCCTGTCCCAGGCCG GATCCTCCTGAAGCCCTTTTCGCAGCACTGCTATCCTCCA AAGCCATTGTA | 720 |
| SURV | NM 001168 | TGTTTTGATTCCCGGGCTTACCAGGTGAGAAGTGAGGGA GGAAGAAGGCAGTGTCCCTTTTGCTAGAGCTGACAGCTT TG | 721 |
| TACC3 | NM_006342 | CACCCTTGGACTGGAAAACTCACACCCGGTCTGGACACA GAAAGAGAACCAACAGCTCATCAAGG | 722 |
| TBCA | NM_004607 | GATCCTCGCGTGAGACAGATCAAGATCAAGACCGGCGTG GTGAAGCGGTTGGTCAAAGAAAAAGTG | 723 |
| TBCC | NM_003192 | CTGTTTTCCTGGAGGACTGCAGTGACTGCGTGCTGGCAG TGGCCTGCCAACAGCTCCGCATACACAGT | 724 |
| TBCD | NM_005993 | CAGCCAGGTGTACGAGACATTGCTCACCTACAGTGACGT CGTGGGCGCGGATGTGCTGGACGAGGT | 725 |
| TBCE | NM_003193 | TCCCGAGAGAGGAAAGCATGATGGGAGCCACGAAGGGA CTGTGTATTTTAAATGCAGGCACCCGAC | 726 |
| TBD | NM_016261 | CCTGGTTGAAGCCTGTTAATGCTTTCAACGTGTGGAAAAC CCAGCGGGCCTTTAGCAAATATGAGAAGTCTGCA | 727 |
| TCP1 | NM_030752 | CCAGTGTGTGTAACAGGGTCACAAGAATTCGACAGCCAG ATGCTCCAAGAGGGTGGCCCAAGGCTATA | 728 |
| TFRC | NM_003234 | GCCAACTGCTTTCATTTGTGAGGGATCTGAACCAATACAG AGCAGACATAAAGGAAATGGGCCTGAGT | 729 |
| THBS1 | NM_003246 | CATCCGCAAAGTGACTGAAGAGAACAAAGAGTTGGCCAA TGAGCTGAGGCGGCCTCCCCTATGCTATCACAACGGAGT TCAGTAC | 730 |
| TK1 | NM 003258 | GCCGGGAAGACCGTAATTGTGGCTGCACTGGATGGGAC CTTCCAGAGGAAGCCATTTGGGGCCATCCTGAACCTGGT GCCGCTG | 731 |
| TOP2A | NM_001067 | AATCCAAGGGGGAGAGTGATGACTTCCATATGGACTTTG ACTCAGCTGTGGCTCCTCGGGCAAAATCTGTAC | 732 |
| TOP3B | NM_003935 | GTGATGCCTTCCCTGTGGGCGAGGTGAAGATGCTGGAG AAGCAGACGAACCCACCCGACTACCTGA | 733 |
| TP | NM_001953 | CTATATGCAGCCAGAGATGTGACAGCCACCGTGGACAGC CTGCCACTCATCACAGCCTCCATTCTCAGTAAGAAACTCG TGG | 734 |
| TP53BP1 | NM_005657 | TGCTGTTGCTGAGTCTGTTGCCAGTCCCCAGAAGACCAT GTCTGTGTTGAGCTGTATCTGTGAAGCCAGGCAAG | 735 |
| TPT1 | NM_003295 | GGTGTCGATATTGTCATGAACCATCACCTGCAGGAAACA AGTTTCACAAAAGAAGCCTACAAGAAGTACATCAAAGATT AC | 736 |
| TRAG3 | NM 004909 | GACGCTGGTCTGGTGAAGATGTCCAGGAAACCACGAGC CTCCAGCCCATTGTCCAACAACCACCCA | 737 |
| TRAIL | NM_003810 | CTTCACAGTGCTCCTGCAGTCTCTCTGTGTGGCTGTAACT TACGTGTACTTTACCAACGAGCTGAAGCAGATG | 738 |
| TS | NM_001071 | GCCTCGGTGTGCCTTTCAACATCGCCAGCTACGCCCTGC TCACGTACATGATTGCGCACATCACG | 739 |
| TSPAN4 | NM_003271 | CTGGTCAGCCTTCAGGGACCCTGAGCACCGCCTGGTCTC TTTCCTGTGGCCAGCCCAGAACTGAAG | 740 |
| TTK | NM_003318 | TGCTTGTCAGTTGTCAACACCTTATGGCCAACCTGCCTGT TTCCAGCAGCAACAGCATCAAATACTTGCCACTCCA | 741 |
| TUBA1 | NM_006000 | TGTCACCCCGACTCAACGTGAGACGCACCGCCCGGACT CACCATGCGTGAATGCATCTCAGTCCACGT | 742 |
| TUBA2 | NM_006001 | AGCTCAACATGCGTGAGTGTATCTCTATCCACGTGGGGC AGGCAGGAGTCCAGATCGGCAAT | 743 |
| TUBA3 | NM_006009 | CTCTTACATCGACCGCCTAAGAGTCGCGCTGTAAGAAGC AACAACCTCTCCTCTTCGTCTCCGCCATCAGC | 744 |
| TUBA4 | M_025019 | GAGGAGGGTGAGTTCTCCAAGGCCCATGAGGATATGACT GCCCTGGAGAAGGATTACAAGGAGGTGGGCAT | 745 |
| TUBA6 | NM_032704 | GTCCCTTCGCCTCCTTCACCGCCGCAGACCCCTTCAAGT TCTAGTCATGCGTGAGTGCATCTCCATCCACG | 746 |
| TUBA8 | NM_018943 | CGCCCTACCTATACCAACCTCAACCGCCTCATCAGTCAG ATTGTGTCCTCAATCACTGCTTCTCTCCG | 747 |
| TUBB | NM_001069 | CGAGGACGAGGCTTAAAAACTTCTCAGATCAATCGTGCA TCCTTAGTGAACTTCTGTTGTCCTCAAGCATGGT | 748 |
| TUBB classIII | NM_006086 | CGCCCTCCTGCAGTATTTATGGCCTCGTCCTCCCCCACC TAGGCCACGTGTGAGCTGCTCCTGTCTCTGT | 749 |
| TUBB1 | NM_030773 | ACACTGACTGGCATCCTGCTTTCCAGTGCCTGCCAGCCT CCAGAAGAGCCAGGTGCCTGACTAGTACATGGGGAGCTA CAGAGC | 750 |
| TUBB2 | NM_006088 | GTGGCCTAGAGCCTTCAGTCACTGGGGAAAGCAGGGAA GCAGTGTGAACTCTTTATTCACTCCCAGCCTG | 751 |
| TUBB5 | NM_006087 | ACAGGCCCCATGCATCCTCCCTGCCTCACTCCCCTCAGC CCCTGCCGACCTTAGCTTATCTGGGAGAGAAACA | 752 |
| TUBBM | NM_032525 | CCCTATGGCCCTGAATGGTGCACTGGTTTAATTGTGTTG GTGTCGGCCCCTCACAAATGCAGCCAAGTCATGTAATTA GT | 753 |
| TUBBOK | NM_178014 | AGTGGAATCCTTCCCTTTCCAACTCTACCTCCCTCACTCA GCTCCTTTCCCCTGATCAGAGAAAGGGATCAAGGG | 754 |
| TUBBP | NM_178012 | GGAAGGAAAGAAGCATGGTCTACTTTAGGTGTGCGCTGG GTCTCTGGTGCTCTTCACTGTTGCCTGTCACTTTTT | 755 |
| TUBG1 | NM_001070 | GATGCCGAGGGAAATCATCACCCTACAGTTGGGCCAGTG CGGCAATCAGATTGGGTTCGAGTTCTGG | 756 |
| TWIST1 | NM_000474 | GCGCTGCGGAAGATCATCCCCACGCTGCCCTCGGACAA GCTGAGCAAGATTCAGACCCTCAAGC | 757 |
| TYRO3 | NM_006293 | CAGTGTGGAGGGGATGGAGGAGCCTGACATCCAGTGGG TGAAGGATGGGGCTGTGGTCCAGAACTTG | 758 |
| UFM1 | NM_016617 | AGTTGTCGTGTGTTCTGGATTCATTCCGGCACCACCATGT CGAAGGTTTCCTTTAAGATCACGCTGACG | 759 |
| upa | NM_002658 | GTGGATGTGCCCTGAAGGACAAGCCAGGCGTCTACACG AGAGTCTCACACTTCTTACCCTGGATCCGCAG | 760 |
| VCAM1 | NM_001078 | TGGCTTCAGGAGCTGAATACCCTCCCAGGCACACACAGG TGGGACACAAATAAGGGTTTTGGAACCACTATTTTCTCAT CACGACAGCA | 761 |
| VEGF | NM_003376 | CTGCTGTCTTGGGTGCATTGGAGCCTTGCCTTGCTGCTC TACCTCCACCATGCCAAGTGGTCCCAGGCTGC | 762 |
| VEGFB | NM_003377 | TGACGATGGCCTGGAGTGTGTGCCCACTGGGCAGCACC AAGTCCGGATGCAGATCCTCATGATCCGGTACC | 763 |
| VEGFC | NM_005429 | CCTCAGCAAGACGTTATTTGAAATTACAGTGCCTCTCTCT CAAGGCCCCAAACCAGTAACAATCAGTTTTGCCAATCACA CTT | 764 |
| VHL | NM_000551 | CGGTTGGTGACTTGTCTGCCTCCTGCTTTGGGAAGACTG AGGCATCCGTGAGGCAGGGACAAGTCTT | 765 |
| VIM | NM 003380 | TGCCCTTAAAGGAACCAATGAGTCCCTGGAACGCCAGAT GCGTGAAATGGAAGAGAACTTTGCCGTTGAAGC | 766 |
| V-RAF | NM_001654 | GGTTGTGCTCTACGAGCTTATGACTGGCTCACTGCCTTA CAGCCACATTGGCTGCCGTGACCAGATTATCTTTATGGT GGGCCG | 767 |
| WAVE3 | NM_006646 | CTCTCCAGTGTGGGCACCAGCCGGCCAGAACAGATGCG AGCAGTCCATGACTCTGGGAGCTACACCGC | 768 |
| Wnt-5a | NM_003392 | GTATCAGGACCACATGCAGTACATCGGAGAAGGCGCGAA GACAGGCATCAAAGAATGCCAGTATCAATTCCGACA | 769 |
| XIAP | NM_001167 | GCAGTTGGAAGACACAGGAAAGTATCCCCAAATTGCAGA TTTATCAACGGCTTTTATCTTGAAAATAGTGCCACGCA | 770 |
| XIST | NR_001564 | CAGGTCAGGCAGAGGAAGTCATGTGCATTGCATGAGCTA AACCTATCTGAATGAATTGATTTGGGGCTTGTTAGG | 771 |
| ZW10 | NM_004724 | TGGTCAGATGCTGCTGAAGTATATCCTTAGGCCGCTGGC ATCTTGCCCATCCCTTCATGCTGTGAT | 772 |
| ZWILCH | NM_017975 | GAGGGAGCAGACAGTGGGTACCACGATCTCCGTAACCAT TTGCATGTGACTTAGCAAGGGCTCTGA | 773 |
| ZWINT | NM_007057 | TAGAGGCCATCAAAATTGGCCTCACCAAGGCCCTGACTC AGATGGAGGAAGCCCAGAGGAAACGGA | 774 |

| **Table 1** | | | | | | | | | | | |
|---|---|---|---|---|---|---|---|---|---|---|---|
| | **Gene** | **p-value** | **Estimated Coefficient** | | **Gene** | **p-value** | **Estimated Coefficient** | | **Gene** | **p-value** | **Estimated Coefficient** |
| 1 | SLC1A3 | 0.0002 | -0.7577 | 27 | RAD1 | 0.0115 | -0.6673 | 52 | ACTR2 | 0.0297 | -0.8754 |
| 2 | TBCC | 0.0006 | -1.0289 | 28 | MRE11A | 0.0120 | -0.6253 | 53 | WNT5A | 0.0321 | 0.5036 |
| 3 | EIF4E2 | 0.0009 | -1.2038 | 29 | DDR1 | 0.0122 | -0.5660 | 54 | HSPA1L | 0.0321 | -1.8702 |
| 4 | TUBB | 0.0017 | -0.7332 | 30 | STK10 | 0.0123 | -0.6002 | 55 | APOC1 | 0.0324 | -0.3434 |
| 5 | TSPAN4 | 0.0027 | -0.7211 | 31 | LILRB1 | 0.0125 | -0.4674 | 56 | ZWINT | 0.0326 | -0.3966 |
| 6 | VHL | 0.0034 | -0.7450 | 32 | BBC3 | 0.0128 | -0.4481 | 57 | APEX1 | 0.0330 | -0.7200 |
| 7 | BAX | 0.0039 | -1.0224 | 33 | BUB3 | 0.0144 | -0.5476 | 58 | KALPHA1 | 0.0351 | -0.7627 |
| 8 | CD247 | 0.0044 | -0.4656 | 34 | CDCA8 | 0.0145 | -0.3759 | 59 | ABCC10 | 0.0354 | -0.5667 |
| 9 | CAPZA1 | 0.0044 | -1.1182 | 35 | TOP3B | 0.0164 | -0.7292 | 60 | PHB | 0.0380 | -0.5832 |
| 10 | STMN1 | 0.0052 | -0.4350 | 36 | RPN2 | 0.0166 | -0.8121 | 61 | TUBB2C | 0.0380 | -0.6664 |
| 11 | ABCC1 | 0.0054 | -0.7653 | 37 | ILK | 0.0169 | -0.6920 | 62 | RALBP1 | 0.0382 | -0.5989 |
| 12 | ZW10 | 0.0055 | -0.8228 | 38 | GBP1 | 0.0170 | -0.3496 | 63 | VEGF | 0.0397 | -0.3673 |
| 13 | HSPA1B | 0.0058 | -0.4740 | 39 | TUBB3 | 0.0173 | -0.3037 | 64 | MCL1 | 0.0398 | -0.6137 |
| 14 | MAPRE1 | 0.0060 | -0.7833 | 40 | NTSR2 | 0.0175 | -2.4355 | 65 | HSPA1A | 0.0402 | -0.3451 |
| 15 | PLD3 | 0.0061 | -0.8595 | 41 | BID | 0.0175 | -0.6228 | 66 | BUB1 | 0.0404 | -0.2911 |
| 16 | APRT | 0.0062 | -0.7714 | 42 | BCL2L13 | 0.0189 | -0.7228 | 67 | MAD2L1 | 0.0412 | -0.3336 |
| 17 | BAK1 | 0.0064 | -0.7515 | 43 | TPX2 | 0.0196 | -0.3148 | 68 | CENPF | 0.0418 | -0.2979 |
| 18 | TUBA6 | 0.0067 | -0.7006 | 44 | ABCC5 | 0.0203 | -0.3906 | 69 | IL2RA | 0.0427 | -0.5023 |
| 19 | CST7 | 0.0069 | -0.4243 | 45 | HDAC6 | 0.0226 | -0.7782 | 70 | TUBA3 | 0.0429 | -0.4528 |
| 20 | SHC1 | 0.0080 | -0.6632 | 46 | CD68 | 0.0226 | -0.6531 | 71 | ACTB | 0.0439 | -0.8259 |
| 21 | ZWILCH | 0.0088 | -0.6902 | 47 | NEK2 | 0.0232 | -0.3657 | 72 | KIF22 | 0.0447 | -0.5427 |
| 22 | SRC | 0.0089 | -0.7011 | 48 | DICER1 | 0.0233 | -0.5537 | 73 | CXCR4 | 0.0462 | -0.4239 |
| 23 | GADD45B | 0.0102 | -0.5253 | 49 | RHOA | 0.0268 | -0.7407 | 74 | STAT1 | 0.0472 | -0.3555 |
| 24 | LIMK2 | 0.0106 | -0.7784 | 50 | TYMS | 0.0291 | -0.3577 | 75 | IL7 | 0.0473 | -0.3973 |
| 25 | CENPA | 0.0106 | -0.3588 | 51 | CCT3 | 0.0292 | -0.5989 | 76 | CHFR | 0.0499 | -0.5387 |
| 26 | CHEK2 | 0.0109 | -0.6737 | | | | | | | | |

| **Table 2** | | | | | | | | | | | |
|---|---|---|---|---|---|---|---|---|---|---|---|
| | **Gene** | **p-value** | **Estimated Coefficient** | | **Gene** | **p-value** | **Estimated Coefficient** | | **Gene** | **p-value** | **Estimated Coefficient** |
| 1 | DDR1 | <.0001 | -1.2307 | 34 | ILK | 0.0084 | -1.1481 | 66 | RXRA | 0.0247 | -0.7973 |
| 2 | EIF4E2 | 0.0001 | -1.8076 | 35 | NTSR2 | 0.0090 | -4.0522 | 67 | ABCD1 | 0.0259 | -0.7533 |
| 3 | TBCC | 0.0001 | -1.5303 | 36 | TOP3B | 0.0091 | -1.0744 | 68 | MAPK3 | 0.0269 | -0.7322 |
| 4 | STK10 | 0.0005 | -1.2320 | 37 | PLD3 | 0.0095 | -1.1126 | 69 | CDCA8 | 0.0275 | -0.5210 |
| 5 | ZW10 | 0.0006 | -1.3917 | 38 | DICER1 | 0.0095 | -0.8849 | 70 | DUSP1 | 0.0284 | -0.3398 |
| 6 | BBC3 | 0.0010 | -0.9034 | 39 | VHL | 0.0104 | -0.9357 | 71 | ABCC1 | 0.0287 | -0.8003 |
| 7 | BAX | 0.0011 | -1.4992 | 40 | GCLC | 0.0108 | -0.7822 | 72 | PRKCH | 0.0291 | -0.6680 |
| 8 | BAK1 | 0.0011 | -1.3122 | 41 | RAD1 | 0.0108 | -1.0141 | 73 | PRDX1 | 0.0301 | -0.8823 |
| 9 | TSPAN4 | 0.0013 | -1.1930 | 42 | GATA3 | 0.0112 | -0.4400 | 74 | TUBA3 | 0.0306 | -0.7331 |
| 10 | SLC1A3 | 0.0014 | -0.9828 | 43 | CXCR4 | 0.0120 | -0.7032 | 75 | VEGFB | 0.0317 | -0.7487 |
| 11 | SHC1 | 0.0015 | -1.1395 | 44 | NME6 | 0.0121 | -0.9873 | 76 | LILRB1 | 0.0320 | -0.5617 |
| 12 | CHFR | 0.0016 | -1.3371 | 45 | UFM1 | 0.0125 | -0.9686 | 77 | LAPTM4 B | 0.0321 | -0.4994 |
| 13 | RHOB | 0.0018 | -0.7059 | 46 | BUB3 | 0.0126 | -0.9054 | 78 | HSPA9B | 0.0324 | -0.9660 |
| 14 | TUBA6 | 0.0019 | -1.1071 | 47 | CD14 | 0.0130 | -0.8152 | 79 | ECGF1 | 0.0329 | -0.5807 |
| 15 | BCL2L13 | 0.0023 | -1.3181 | 48 | MRE11A | 0.0130 | -0.8915 | 80 | GDF15 | 0.0332 | -0.3646 |
| 16 | MAPRE1 | 0.0029 | -1.2233 | 49 | CST7 | 0.0131 | -0.5204 | 81 | ACTR2 | 0.0347 | -1.1827 |
| 17 | GADD45B | 0.0034 | -0.9174 | 50 | APOC1 | 0.0134 | -0.5630 | 82 | IL7 | 0.0349 | -0.5623 |
| 18 | HSPA1B | 0.0036 | -0.6406 | 51 | GNS | 0.0136 | -1.0979 | 83 | HDAC6 | 0.0380 | -0.9486 |
| 19 | FAS | 0.0037 | -0.8571 | 52 | ABCC5 | 0.0146 | -0.5595 | 84 | ZWILCH | 0.0384 | -0.7296 |
| 20 | TUBB | 0.0040 | -1.0178 | 53 | AKT2 | 0.0150 | -1.0824 | 85 | CHEK2 | 0.0392 | -0.7502 |
| 21 | HSPA1A | 0.0041 | -0.6648 | 54 | APRT | 0.0150 | -0.9231 | 86 | REG1A | 0.0398 | -3.4734 |
| 22 | MCL1 | 0.0041 | -1.1459 | 55 | PLAU | 0.0157 | -0.6705 | 87 | APC | 0.0411 | -0.8324 |
| 23 | CCT3 | 0.0048 | -1.0709 | 56 | RCC1 | 0.0163 | -0.9073 | 88 | SLC35B1 | 0.0411 | -0.6801 |
| 24 | VEGF | 0.0049 | -0.8411 | 57 | CAPZA1 | 0.0165 | -1.3542 | 89 | NEK2 | 0.0415 | -0.4609 |
| 25 | TUBB2C | 0.0051 | -1.4181 | 58 | RELA | 0.0168 | -0.8534 | 90 | ACTB | 0.0418 | -1.1482 |
| 26 | AKT1 | 0.0053 | -1.1175 | 59 | NFKB1 | 0.0179 | -0.9847 | 91 | BUB1 | 0.0423 | -0.4612 |
| 27 | MAD2L1BP | 0.0055 | -1.0691 | 60 | RASSF1 | 0.0186 | -0.8078 | 92 | PPP2CA | 0.0423 | -0.9474 |
| 28 | RPN2 | 0.0056 | -1.2688 | 61 | BCL2L11 | 0.0209 | -0.9394 | 93 | TNFRSF 10A | 0.0448 | -0.6415 |
| 29 | RHOA | 0.0063 | -1.3773 | 62 | CSNK1D | 0.0211 | -1.2276 | 94 | TBCD | 0.0456 | -0.6196 |
| 30 | MAP2K3 | 0.0063 | -0.9616 | 63 | SRC | 0.0220 | -0.8341 | 95 | ERBB4 | 0.0460 | -0.2830 |
| 31 | BID | 0.0067 | -1.0502 | 64 | LIMK2 | 0.0221 | -1.0830 | 96 | CDC25B | 0.0467 | -0.5660 |
| 32 | APOE | 0.0074 | -0.8130 | 65 | SIRT1 | 0.0229 | -0.7236 | 97 | STMN1 | 0.0472 | -0.4684 |
| 33 | ESR1 | 0.0077 | -0.3456 | | | | | | | | |

| **Table 3** | | | | | | | | | | | |
|---|---|---|---|---|---|---|---|---|---|---|---|
| | **Gene** | **p-value** | **Estimated Coefficient** | | **Gene** | **p-value** | **Estimated Coefficient** | | **Gene** | **p-value** | **Estimated Coefficient** |
| 1 | DDR1 | <.0001 | -1.3498 | 56 | APRT | 0.0027 | -1.2629 | 111 | RBM17 | 0.0171 | -1.3981 |
| 2 | ZW10 | <.0001 | -2.1657 | 57 | MAP2K3 | 0.0031 | -1.1297 | 112 | TP53BP1 | 0.0184 | -0.9442 |
| 3 | RELA | <.0001 | -1.5759 | 58 | MGC52057 | 0.0033 | -1.0906 | 113 | CD247 | 0.0188 | -0.5768 |
| 4 | BAX | <.0001 | -1.8857 | 59 | MAPK3 | 0.0033 | -1.0390 | 114 | ABCA9 | 0.0190 | -0.5489 |
| 5 | RHOB | <.0001 | -1.1694 | 60 | APC | 0.0034 | -1.2719 | 115 | NTSR2 | 0.0192 | -3.9043 |
| 6 | TSPAN4 | <.0001 | -1.7067 | 61 | RAD1 | 0.0036 | -1.2744 | 116 | FOS | 0.0195 | -0.4437 |
| 7 | BBC3 | <.0001 | -1.2017 | 62 | COL6A3 | 0.0039 | -0.8240 | 117 | TNFRSF10A | 0.0196 | -0.7666 |
| 8 | SHC1 | <.0001 | -1.4625 | 63 | RXRB | 0.0039 | -1.2638 | 118 | MSH3 | 0.0200 | -0.9585 |
| 9 | CAPZA1 | <.0001 | -2.4068 | 64 | CCT3 | 0.0040 | -1.3329 | 119 | PTEN | 0.0202 | -1.0307 |
| 10 | STK10 | 0.0001 | -1.4013 | 65 | ABCC3 | 0.0040 | -0.8170 | 120 | GBP2 | 0.0204 | -0.6414 |
| 11 | TBCC | 0.0001 | -1.6385 | 66 | GPX1 | 0.0042 | -1.5547 | 121 | STK11 | 0.0206 | -0.9807 |
| 12 | EIF4E2 | 0.0002 | -1.9122 | 67 | TUBB2C | 0.0042 | -1.6184 | 122 | ERBB4 | 0.0213 | -0.3933 |
| 13 | MCL1 | 0.0003 | -1.6617 | 68 | HSPA1A | 0.0043 | -0.7875 | 123 | TFF1 | 0.0220 | -0.2020 |
| 14 | RASSF1 | 0.0003 | -1.3201 | 69 | AKT1 | 0.0045 | -1.1777 | 124 | ABCC1 | 0.0222 | -0.9438 |
| 15 | VEGF | 0.0003 | -1.0800 | 70 | TUBA6 | 0.0046 | -1.2048 | 125 | IL7 | 0.0223 | -0.6920 |
| 16 | SLC1A3 | 0.0004 | -1.0855 | 71 | TOP3B | 0.0048 | -1.1950 | 126 | CDC25B | 0.0228 | -0.7338 |
| 17 | DICER1 | 0.0004 | -1.4236 | 72 | CSNK1D | 0.0049 | -1.6201 | 127 | TUBD1 | 0.0234 | -0.6092 |
| 18 | ILK | 0.0004 | -1.7221 | 73 | SOD1 | 0.0049 | -1.2383 | 128 | BIRC4 | 0.0236 | -0.9072 |
| 19 | FAS | 0.0005 | -1.1671 | 74 | BUB3 | 0.0050 | -1.0111 | 129 | ACTR3 | 0.0246 | -1.3384 |
| 20 | RAB6C | 0.0005 | -1.6154 | 75 | MAP4 | 0.0052 | -1.5220 | 130 | SLC35B1 | 0.0253 | -0.7793 |
| 21 | ESR1 | 0.0006 | -0.4845 | 76 | NFKB1 | 0.0060 | -1.2355 | 131 | COL1A1 | 0.0256 | -0.4945 |
| 22 | MRE11A | 0.0006 | -1.2537 | 77 | SEC61A1 | 0.0060 | -1.4777 | 132 | FOXA1 | 0.0262 | -0.4554 |
| 23 | APOE | 0.0006 | -1.0602 | 78 | MAD1L1 | 0.0060 | -1.1168 | 133 | DUSP1 | 0.0264 | -0.4205 |
| 24 | BAK1 | 0.0006 | -1.4288 | 79 | PRKCH | 0.0073 | -0.8259 | 134 | CXCR4 | 0.0265 | -0.6550 |
| 25 | UFM1 | 0.0006 | -1.4110 | 80 | RXRA | 0.0074 | -0.9693 | 135 | IL2RA | 0.0268 | -0.9731 |
| 26 | AKT2 | 0.0007 | -1.6213 | 81 | PLAU | 0.0074 | -0.7987 | 136 | GGPS1 | 0.0268 | -0.7915 |
| 27 | SIRT1 | 0.0007 | -1.1651 | 82 | CD63 | 0.0074 | -1.3830 | 137 | KNS2 | 0.0281 | -0.8758 |
| 28 | BCL2L13 | 0.0008 | -1.5059 | 83 | CD14 | 0.0075 | -0.9409 | 138 | RB1 | 0.0289 | -0.9291 |
| 29 | ACTR2 | 0.0008 | -1.9690 | 84 | RHOC | 0.0077 | -1.0341 | 139 | BCL2L1 | 0.0289 | -0.9123 |
| 30 | LIMK2 | 0.0009 | -1.6937 | 85 | STAT1 | 0.0093 | -0.7663 | 140 | XIST | 0.0294 | -0.6529 |
| 31 | HDAC6 | 0.0010 | -1.5715 | 86 | NPC2 | 0.0094 | -1.2302 | 141 | BIRC3 | 0.0294 | -0.4739 |
| 32 | RPN2 | 0.0010 | -1.5839 | 87 | NME6 | 0.0095 | -1.2091 | 142 | BID | 0.0303 | -0.8691 |
| 33 | PLD3 | 0.0010 | -1.5460 | 88 | PDGFRB | 0.0096 | -0.7932 | 143 | BCL2 | 0.0303 | -0.5525 |
| 34 | CHGA | 0.0011 | -0.8275 | 89 | MGMT | 0.0098 | -1.0325 | 144 | STAT3 | 0.0311 | -0.9289 |
| 35 | RHOA | 0.0011 | -1.6934 | 90 | GBP1 | 0.0098 | -0.5896 | 145 | PECAM1 | 0.0319 | -0.6803 |
| 36 | MAPK14 | 0.0014 | -1.6611 | 91 | ERCC1 | 0.0105 | -1.2240 | 146 | DIABLO | 0.0328 | -0.9572 |
| 37 | ECGF1 | 0.0014 | -0.8835 | 92 | RCC1 | 0.0107 | -1.0453 | 147 | CYBA | 0.0333 | -0.6642 |
| 38 | MAPRE1 | 0.0016 | -1.3329 | 93 | FUS | 0.0117 | -1.2869 | 148 | TBCE | 0.0336 | -0.7411 |
| 39 | HSPA1B | 0.0017 | -0.8048 | 94 | TUBA3 | 0.0117 | -0.8905 | 149 | CYP1B1 | 0.0337 | -0.6013 |
| 40 | GATA3 | 0.0017 | -0.6153 | 95 | CHEK2 | 0.0120 | -1.0057 | 150 | APEX1 | 0.0357 | -1.0916 |
| 41 | PPP2CA | 0.0017 | -1.6176 | 96 | APOC1 | 0.0123 | -0.6422 | 151 | TBCD | 0.0383 | -0.5893 |
| 42 | ABCD1 | 0.0018 | -1.1669 | 97 | ABCC10 | 0.0124 | -0.9400 | 152 | HRAS | 0.0390 | -0.8411 |
| 43 | MAD2L1BP | 0.0018 | -1.1725 | 98 | SRC | 0.0128 | -1.1170 | 153 | TNFRSF10B | 0.0394 | -0.7293 |
| 44 | VHL | 0.0022 | -1.1855 | 99 | TUBB | 0.0136 | -0.9398 | 154 | ELP3 | 0.0398 | -0.9560 |
| 45 | GCLC | 0.0023 | -1.1240 | 100 | FLAD1 | 0.0139 | -1.0396 | 155 | PIK3C2A | 0.0408 | -0.9158 |
| 46 | ACTB | 0.0023 | -1.8754 | 101 | MAD2L2 | 0.0141 | -1.0834 | 156 | HSPA5 | 0.0417 | -1.5232 |
| 47 | BCL2L11 | 0.0024 | -1.5415 | 102 | LAPTM4B | 0.0149 | -0.5932 | 157 | VEGFC | 0.0427 | -0.7309 |
| 48 | PRDX1 | 0.0025 | -1.3943 | 103 | REG1A | 0.0150 | -5.1214 | 158 | CRABP1 | 0.0440 | 0.2492 |
| 49 | LILRB1 | 0.0025 | -0.8462 | 104 | PRKCD | 0.0152 | -1.0120 | 159 | MMP11 | 0.0456 | -0.3894 |
| 50 | GNS | 0.0025 | -1.3307 | 105 | CST7 | 0.0157 | -0.5499 | 160 | SGK | 0.0456 | -0.6740 |
| 51 | CHFR | 0.0026 | -1.3292 | 106 | IGFBP2 | 0.0161 | -0.5019 | 161 | CTSD | 0.0463 | -0.7166 |
| 52 | CD68 | 0.0026 | -1.1941 | 107 | FYN | 0.0162 | -0.7670 | 162 | BAD | 0.0479 | -0.6436 |
| 53 | LIMK1 | 0.0026 | -1.5655 | 108 | KDR | 0.0168 | -0.8204 | 163 | PTPN21 | 0.0484 | -0.5636 |
| 54 | GADD45B | 0.0027 | -1.0162 | 109 | STMN1 | 0.0169 | -0.6791 | 164 | HSPA9B | 0.0487 | -0.9657 |
| 55 | VEGFB | 0.0027 | -1.1252 | 110 | ZWILCH | 0.0170 | -0.8897 | 165 | PMS1 | 0.0498 | -0.9283 |

| **Table 4** | | | | | | | | | | | |
|---|---|---|---|---|---|---|---|---|---|---|---|
| | Gene | p-value | Estimated Coefficient | | Gene | p-value | Estimated Coefficient | | Gene | p-value | Estimated Coefficient |
| 1 | CD247 | 0.0101 | -0.6642 | 4 | ACTG2 | 0.0280 | -0.2775 | 7 | CHEK2 | 0.0438 | -0.9595 |
| 2 | TYMS | 0.0225 | -0.5949 | 5 | CCND1 | 0.0355 | 0.4802 | 8 | STMN1 | 0.0441 | -0.5369 |
| 3 | IGF1R | 0.0270 | -0.5243 | 6 | CAPZA1 | 0.0401 | -1.1408 | 9 | ZWILCH | 0.0476 | -0.8264 |

| **Table 5** | | | |
|---|---|---|---|
| **Official Symbol** | **Name** | **Entrez** | **Role** |
| CHUK | Conserved helix-loop-helix ubiquitous kinase | 1147 | Activates |
| BCL3 | B-cell CLL/lymphoma 3 | 602 | Transcriptional co-activator |
| FADD | Fas (TNFRSF6)-associated via death domain | 8772 | Stimulates pathway |
| IKBKB | Inhibitor of kappa light polypeptide gene enhancer in B-cells, kinase beta | 3551 | Activates; triggers degradation of NFKBIA, NFKBIB |
| IKBKG | Inhibitor of kappa light polypeptide gene enhancer in B-cells, kinase gamma | 8517 | Activates; triggers degradation of NFKBIA, NFKBIB |
| IL1A | Interleukin 1, alpha | 3552 | Stimulates pathway |
| IL1R1 | Interleukin 1 receptor, type I | 3554 | Stimulates pathway |
| IRAK1 | Interleukin-1 receptor-associated kinase 1 | 3654 | Stimulates pathway |
| NFKB1 | Nuclear factor of kappa light polypeptide gene enhancer in B-cells 1 (p105) | 4790 | Core subunit |
| NFKB2 | Nuclear factor of kappa light polypeptide gene enhancer in B-cells 2 (p49/p100) | 4791 | Core subunit |
| NFKBIA | Nuclear factor of kappa light polypeptide gene enhancer in B-cells inhibitor, alpha | 4792 | Inhibits |
| NFKBIB | Nuclear factor of kappa light polypeptide gene enhancer in B-cells inhibitor, beta | 4793 | Inhibits |
| NFKBIE | nuclear factor of kappa light polypeptide gene enhancer in B-cells inhibitor, epsilon | 4794 | Inhibits |
| REL | v-rel reticuloendotheliosis viral oncogene homolog (avian) | 5966 | Transcriptional co-activator |
| RELA | V-rel reticuloendotheliosis viral oncogene homolog A, nuclear factor of kappa light polypeptide gene enhancer in B-cells 3, p65 (avian) | 5970 | Transcriptional co-activator |
| RELB | v-rel reticuloendotheliosis viral oncogene homolog B, nuclear factor of kappa light polypeptide gene enhancer in B-cells 3 (avian) | 5971 | Transcriptional co-activator |
| RHOC | ras homolog gene family, member C | 389 | Induce activation of pathway |
| TNFAIP3 | Tumor necrosis factor, alpha-induced protein 3 | 7128 | Activates |
| TNFRSF1A | Tumor necrosis factor receptor superfamily, member 1A | 7132 | Activates |
| TNFRSF18 | TNFRSF1A-associated via death domain | 7133 | Activates |
| TRAF6 | TNF receptor-associated factor 6 | 7189 | Activates CHUK |

## Claims

1. A method of predicting whether a breast cancer patient will exhibit a beneficial response to chemotherapy, comprising
measuring an expression level of BAK1, or its expression product, in a tumour sample obtained from the patient;
using the expression level to determine a likelihood of a beneficial response to a treatment including a taxane, wherein expression of BAK1 is positively correlated with increased likelihood of a beneficial response to a treatment including a taxane; and
generating a report including information based on the likelihood of a beneficial response to chemotherapy including a taxane.

2. A method as claimed in claim 1, wherein the method further comprises using the expression level to determine a likelihood of a beneficial response to a treatment including a cyclophosphamide,
wherein expression of BAK1 is negatively correlated with an increased likelihood of a beneficial response to a treatment including a cyclophosphamide, and
wherein the report includes information based on the likelihood of a beneficial response to chemotherapy including a cyclophosphamide.

3. A method as claimed in claim 1 or 2, wherein the breast cancer is hormone-receptor positive.

4. A method as claimed in claim 1, wherein the chemotherapy includes an anthracycline.

5. A method as claimed in claim 4, wherein the anthracycline is doxorubicin.

6. A method as claimed in claim 1, wherein the taxane is docetaxel.

7. A method as claimed in any preceding claim, wherein said measuring is by quantitative polymerase chain reaction (qPCR).

8. A method as claimed in any preceding claim, wherein said measuring is by detection of an intron-based sequence of an RNA transcript of the gene, wherein the expression of which correlates with the expression of a corresponding exon sequence.

9. A method as claimed in any preceding claim, wherein the tumour sample is a formalin-fixed and paraffin-embedded (FPE) tumour section.

10. A method as claimed in any of claims 1 to 8, wherein the tumour sample is a frozen tumour section.

## Patentansprüche

1. Verfahren zur Vorhersage davon, ob ein Brustkrebspatient positiv auf eine Chemotherapie anspricht, wobei man
ein BAK1-Expressionsniveau oder sein Expressionsprodukt in einer von einem Patienten erhaltenen Tumorprobe misst;
das Expressionsniveau dazu verwendet, eine Wahrscheinlichkeit eines positiven Ansprechens auf eine Behandlung unter Einschluss eines Taxans zu bestimmen, wobei die Expression von BAK1 eine positive Korrelation mit einer erhöhten Wahrscheinlichkeit eines positiven Ansprechens auf eine Behandlung unter Einschluss eines Taxans aufweist; und
einen Bericht erstellt, der auf die Wahrscheinlichkeit eines positiven Ansprechens auf eine Chemotherapie unter Einschluss eines Taxans bezogene Informationen enthält.

2. Verfahren nach Anspruch 1, wobei das Verfahren ferner die Verwendung des Expressionsniveaus zur Bestimmung einer Wahrscheinlichkeit eines positiven Ansprechens auf eine Behandlung unter Einschluss eines Cyclophosphamids umfasst,
wobei die Expression von BAK1 eine negative Korrelation mit einer erhöhten Wahrscheinlichkeit eines positiven Ansprechens auf eine Behandlung unter Einschluss eines Cyclophosphamids aufweist; und
wobei der Bericht auf die Wahrscheinlichkeit eines positiven Ansprechens auf eine Chemotherapie unter Einschluss eines Cyclophosphamids bezogene Informationen enthält.

3. Verfahren nach Anspruch 1 oder 2, wobei es sich um eine hormonrezeptor-positive Brustkrebserkrankung handelt.

4. Verfahren nach Anspruch 1, wobei es sich um eine Chemotherapie unter Einschluss eines Anthracyclins handelt.

5. Verfahren nach Anspruch 4, wobei es sich bei dem Anthracyclin um Doxorubicin handelt.

6. Verfahren nach Anspruch 1, wobei es sich bei dem Taxan um Docetaxel handelt.

7. Verfahren nach einem vorhergehenden Anspruch, wobei die Messung mittels quantitativer Polymerasekettenreaktion (qPCR) erfolgt.

8. Verfahren nach einem vorhergehenden Anspruch, wobei die Messung mittels Nachweis einer intronbasierten Sequenz eines RNA-Transkripts des Gens erfolgt, wobei ihre Expression mit der Expression einer entsprechenden Exonsequenz korreliert.

9. Verfahren nach einem vorhergehenden Anspruch, wobei es sich bei der Tumorprobe um einen mit Formalin fixierten und in Paraffin eingebetteten (FPE-) Tumorschnitt handelt.

10. Verfahren nach einem der Ansprüche 1 bis 8, wobei es sich bei der Tumorprobe um einen Tumorgefrierschnitt handelt.

## Revendications

1. Procédé consistant à prédire si un patient atteint de cancer du sein présente une réponse bénéfique à une chimiothérapie, comprenant la mesure d'un niveau d'expression de BAK1, ou son produit d'expression, dans un échantillon de tumeur obtenu à partir du patient ;
l'utilisation du niveau d'expression pour déterminer une probabilité d'une réponse bénéfique à un traitement comprenant un taxane, dans lequel l'expression de BAK1 est positivement corrélée avec une probabilité augmentée d'une réponse bénéfique à un traitement comprenant un taxane ; et
la génération d'un rapport comprenant des informations basées sur la probabilité d'une réponse bénéfique à une chimiothérapie comprenant un taxane.

2. Procédé selon la revendication 1, le procédé comprenant en outre l'utilisation du niveau d'expression pour déterminer une probabilité d'une réponse bénéfique à un traitement comprenant un cyclophosphamide,
dans lequel l'expression de BAK1 est négativement corrélée à une probabilité augmentée d'une réponse bénéfique à un traitement comprenant un cyclophosphamide, et
dans lequel le rapport comprend des informations basées sur la probabilité d'une réponse bénéfique à une chimiothérapie comprenant un cyclophosphamide.

3. Procédé selon la revendication 1 ou 2, dans lequel le cancer du sein est à récepteur hormonal positif.

4. Procédé selon la revendication 1, dans lequel la chimiothérapie comprend une anthracycline.

5. Procédé selon la revendication 4, dans lequel l'anthracycline est la doxorubicine.

6. Procédé selon la revendication 1, dans lequel le taxane est le docétaxel.

7. Procédé selon l'une quelconque des revendications précédentes, dans lequel ladite mesure est effectuée par réaction de polymérase en chaîne quantitative (qPCR).

8. Procédé selon l'une quelconque des revendications précédentes, dans lequel ladite mesure est effectuée par détection d'une séquence à base d'introduction d'un produit de transcription d'ARN du gène, dont l'expression est corrélée avec l'expression d'une séquence d'exon correspondante.

9. Procédé selon l'une quelconque des revendications précédentes, dans lequel l'échantillon de tumeur est une coupe de tumeur fixée au formol et incluse dans la paraffine (FPE).

10. Procédé selon l'une quelconque des revendications 1 à 8, dans lequel l'échantillon de tumeur est une coupe de tumeur congelée.
